# EUROPEAN PATENT SPECIFICATION

(11) **EP 2 545 073 B1**
(45) Date of publication and mention of the grant of the patent: **30.09.2015**
(21) Application number: 11730113.5
(22) Date of filing: 11.03.2011
(51) Int. Cl.: C07K 14/705, A61K 47/48, A61K 38/00

(54) **CTLA4 PROTEINS AND THEIR USES**
CTLA4-PROTEINE UND IHRE VERWENDUNGEN
PROTÉINES CTLA4 ET LEURS UTILISATIONS

(30) Priority: 12.03.2010 US 313516 P
(43) Date of publication of application: 16.01.2013
(73) Proprietor: AbbVie Biotherapeutics Inc., Redwood City, CA 94063 (US)
(72) Inventor: AKAMATSU, Yoshiko, Palo Alto California 94306 (US); DUBRIDGE, Robert, B., Belmont California 94002 (US); JUAN, Veronica, Redwood City California 94062 (US)
(74) Representative: Roques, Sarah Elizabeth
(86) International application number: PCT/US2011/028221
(87) International publication number: WO 2011/113019

(56) References cited:
- WO-A2-01/92337
- WO-A2-02/02638
- WO-A2-2005/108432
- WO-A2-2008/047150
- WO-A2-2009/058564
- WO-A2-2011/103584
- LARSEN C P ET AL: "Rational development of LEA29Y (belatacept), a high-affinity variant of CTLA4-Ig with potent immunosuppressive properties", AMERICAN JOURNAL OF TRANSPLANTATION, BLACKWELL MUNKSGAARD, DK, vol. 5, no. 3, 1 March 2005 (2005-03-01), pages 443-453, XP002393471, ISSN: 1600-6135, DOI: 10.1111/J.1600-6143.2005.00749.X

### SEQUENCE LISTING

<110> AKAMATSU, YOSHIKO
   DUBRIDGE, ROBERT B.
   POWERS, DAVID B.
   JUAN, VERONICA
<120> CTLA4 PROTEINS AND THEIR USES
<130> 381493-237US (106186)
<140>
   <141>
<150> 61/313,516
   <151> 2010-03-12
<160> 761
<170> PatentIn version 3.5
<210> 1
   <211> 124
   <212> PRT
   <213> Homo sapiens
<400> 1
<210> 2
   <211> 8
   <212> PRT
   <213> Homo sapiens
<400> 2
   Pro Gly Lys Ala Thr Glu Val Arg
   1               5
<210> 3
   <211> 13
   <212> PRT
   <213> Homo sapiens
<400> 3
   Thr Tyr Met Met Gly Asn Glu Leu Thr Phe Leu Asp Asp
   1               5                   10
<210> 4
   <211> 12
   <212> PRT
   <213> Homo sapiens
<400> 4
   Lys Val Glu Leu Met Tyr Pro Pro Pro Tyr Tyr Leu
   1               5                   10
<210> 5
   <211> 186
   <212> PRT
   <213> Homo sapiens
<400> 5
<210> 6
   <211> 357
   <212> PRT
   <213> Artificial Sequence
<220>
   <223> Description of Artificial Sequence: Synthetic polypeptide
<400> 6
<210> 7
   <211> 233
   <212> PRT
   <213> Homo sapiens
<400> 7
   Val Glu Pro Lys Ser Cys Asp Lys Thr His Thr Cys Pro Pro Cys Pro
   1               5                   10                  15
   Ala Pro Glu Leu Leu Gly Gly Pro Ser Val Phe Leu Phe Pro Pro Lys
               20                  25                  30
   Pro Lys Asp Thr Leu Met Ile Ser Arg Thr Pro Glu Val Thr Cys Val
           35                  40                  45
   Val Val Asp Val Ser His Glu Asp Pro Glu Val Lys Phe Asn Trp Tyr
       50                  55                  60
   Val Asp Gly Val Glu Val His Asn Ala Lys Thr Lys Pro Arg Glu Glu
   65                  70                  75                  80
   Gln Tyr Asn Ser Thr Tyr Arg Val Val Ser Val Leu Thr Val Leu His
                   85                  90                  95
   Gln Asp Trp Leu Asn Gly Lys Glu Tyr Lys Cys Lys Val Ser Asn Lys
               100                 105                 110
   Ala Leu Pro Ala Pro Ile Glu Lys Thr Ile Ser Lys Ala Lys Gly Gln
           115                 120                 125
   Pro Arg Glu Pro Gln Val Tyr Thr Leu Pro Pro Ser Arg Asp Glu Leu
       130                 135                 140
   Thr Lys Asn Gln Val Ser Leu Thr Cys Leu Val Lys Gly Phe Tyr Pro
   145                 150                 155                 160
   Ser Asp Ile Ala Val Glu Trp Glu Ser Asn Gly Gln Pro Glu Asn Asn
                   165                 170                 175
   Tyr Lys Thr Thr Pro Pro Val Leu Asp Ser Asp Gly Ser Phe Phe Leu
               180                 185                 190
   Tyr Ser Lys Leu Thr Val Asp Lys Ser Arg Trp Gln Gln Gly Asn Val
           195                 200                 205
   Phe Ser Cys Ser Val Met His Glu Ala Leu His Asn His Tyr Thr Gln
       210                 215                 220
   Lys Ser Leu Ser Leu Ser Pro Gly Lys
   225                 230
<210> 8
   <211> 8
   <212> PRT
   <213> Artificial Sequence
<220>
   <223> Description of Artificial Sequence: Synthetic peptide
<400> 8
   Pro Gly His Ala Thr Glu Val Arg
   1               5
<210> 9
   <211> 8
   <212> PRT
   <213> Artificial Sequence
<220>
   <223> Description of Artificial Sequence: Synthetic peptide
<400> 9
   Pro Gly Thr Ala Thr Glu Val Arg
   1               5
<210> 10
   <211> 8
   <212> PRT
   <213> Artificial Sequence
<220>
   <223> Description of Artificial Sequence: Synthetic peptide
<400> 10
   Pro Gly Lys His Thr Glu Val Arg
   1               5
<210> 11
   <211> 13
   <212> PRT
   <213> Artificial Sequence
<220>
   <223> Description of Artificial Sequence: Synthetic peptide
<400> 11
   Tyr Tyr Met Met Gly Asn Glu Leu Thr Phe Leu Asp Asp
   1               5                   10
<210> 12
   <211> 13
   <212> PRT
   <213> Artificial Sequence
<220>
   <223> Description of Artificial Sequence: Synthetic peptide
<400> 12
   Thr Tyr Phe Met Gly Asn Glu Leu Thr Phe Leu Asp Asp
   1 5 10
<210> 13
   <211> 13
   <212> PRT
   <213> Artificial Sequence
<220>
   <223> Description of Artificial Sequence: Synthetic peptide
<400> 13
   Thr Tyr Tyr Met Gly Asn Glu Leu Thr Phe Leu Asp Asp
   1 5 10
<210> 14
   <211> 13
   <212> PRT
   <213> Artificial Sequence
<220>
   <223> Description of Artificial Sequence: Synthetic peptide
<400> 14
   Thr Tyr Met Met Gly Asn Glu Gly Thr Phe Leu Asp Asp
   1 5 10
<210> 15
   <211> 13
   <212> PRT
   <213> Artificial Sequence
<220>
   <223> Description of Artificial Sequence: Synthetic peptide
<400> 15
   Thr Tyr Met Met Gly Asn Glu Leu Thr Phe His Asp Asp
   1 5 10
<210> 16
   <211> 13
   <212> PRT
   <213> Artificial Sequence
<220>
   <223> Description of Artificial Sequence: Synthetic peptide
<400> 16
   Thr Tyr Met Met Gly Asn Glu Leu Thr Phe Tyr Asp Asp
   1 5 10
<210> 17
   <211> 12
   <212> PRT
   <213> Artificial Sequence
<220>
   <223> Description of Artificial Sequence: Synthetic peptide
<400> 17
   Met Val Glu Leu Met Tyr Pro Pro Pro Tyr Tyr Leu
   1 5 10
<210> 18
   <211> 12
   <212> PRT
   <213> Artificial Sequence
<220>
   <223> Description of Artificial Sequence: Synthetic peptide
<400> 18
   Gln Val Glu Leu Met Tyr Pro Pro Pro Tyr Tyr Leu
   1 5 10
<210> 19
   <211> 13
   <212> PRT
   <213> Artificial Sequence
<220>
   <223> Description of Artificial Sequence: Synthetic peptide
<400> 19
   Thr Tyr Met Met Gly Asn Glu Ala Thr Phe Leu Asp Asp
   1 5 10
<210> 20
   <211> 13
   <212> PRT
   <213> Artificial Sequence
<220>
   <223> Description of Artificial Sequence: Synthetic peptide
<400> 20
   Thr Tyr Met Met Gly Asn Glu Asn Thr Phe Leu Asp Asp
   1 5 10
<210> 21
   <211> 13
   <212> PRT
   <213> Artificial Sequence
<220>
   <223> Description of Artificial Sequence: Synthetic peptide
<400> 21
   Thr Tyr Met Met Gly Asn Glu Gln Thr Phe Leu Asp Asp
   1 5 10
<210> 22
   <211> 13
   <212> PRT
   <213> Artificial Sequence
<220>
   <223> Description of Artificial Sequence: Synthetic peptide
<400> 22
   Thr Tyr Met Met Gly Asn Glu Leu Thr Phe Ala Asp Asp
   1 5 10
<210> 23
   <211> 12
   <212> PRT
   <213> Artificial Sequence
<220>
   <223> Description of Artificial Sequence: Synthetic peptide
<400> 23
   Val Val Glu Leu Met Tyr Pro Pro Pro Tyr Tyr Leu
   1 5 10
<210> 24
   <211> 12
   <212> PRT
   <213> Artificial Sequence
<220>
   <223> Description of Artificial Sequence: Synthetic peptide
<400> 24
   Lys Val Glu Leu Met Tyr Pro Pro Pro Tyr Tyr His
   1 5 10
<210> 25
   <211> 12
   <212> PRT
   <213> Artificial Sequence
<220>
   <223> Description of Artificial Sequence: Synthetic peptide
<400> 25
   Lys Val Glu Leu Met Tyr Pro Pro Pro Tyr Tyr Tyr
   1 5 10
<210> 26
   <211> 8
   <212> PRT
   <213> Artificial Sequence
<220>
   <223> Description of Artificial Sequence: Synthetic peptide
<400> 26
   Pro Gly Lys Ala Glu Glu Val Arg
   1 5
<210> 27
   <211> 13
   <212> PRT
   <213> Artificial Sequence
<220>
   <223> Description of Artificial Sequence: Synthetic peptide
<400> 27
   Thr Tyr Met Arg Gly Asn Glu Leu Thr Phe Leu Asp Asp
   1 5 10
<210> 28
   <211> 13
   <212> PRT
   <213> Artificial Sequence
<220>
   <223> Description of Artificial Sequence: Synthetic peptide
<400> 28
   Thr Tyr Met Met Pro Asn Glu Leu Thr Phe Leu Asp Asp
   1 5 10
<210> 29
   <211> 13
   <212> PRT
   <213> Artificial Sequence
<220>
   <223> Description of Artificial Sequence: Synthetic peptide
<400> 29
   Thr Tyr Met Met Gly Asn Pro Leu Thr Phe Leu Asp Asp
   1 5 10
<210> 30
   <211> 13
   <212> PRT
   <213> Artificial Sequence
<220>
   <223> Description of Artificial Sequence: Synthetic peptide
<400> 30
   Thr Tyr Met Met Gly Asn Glu Glu Thr Phe Leu Asp Asp
   1 5 10
<210> 31
   <211> 13
   <212> PRT
   <213> Artificial Sequence
<220>
   <223> Description of Artificial Sequence: Synthetic peptide
<400> 31
   Thr Tyr Met Met Gly Asn Glu Pro Thr Phe Leu Asp Asp
   1 5 10
<210> 32
   <211> 13
   <212> PRT
   <213> Artificial Sequence
<220>
   <223> Description of Artificial Sequence: Synthetic peptide
<400> 32
   Thr Tyr Met Met Gly Asn Glu Tyr Thr Phe Leu Asp Asp
   1 5 10
<210> 33
   <211> 13
   <212> PRT
   <213> Artificial Sequence
<220>
   <223> Description of Artificial Sequence: Synthetic peptide
<400> 33
   Thr Tyr Met Met Gly Asn Glu Leu Thr Phe Phe Asp Asp
   1 5 10
<210> 34
   <211> 12
   <212> PRT
   <213> Artificial Sequence
<220>
   <223> Description of Artificial Sequence: Synthetic peptide
<400> 34
   Lys Val Glu Leu Met Tyr Pro Pro Pro Tyr Tyr Ile
   1               5                   10
<210> 35
   <211> 12
   <212> PRT
   <213> Artificial Sequence
<220>
   <223> Description of Artificial Sequence: Synthetic peptide
<400> 35
   Ile Val Glu Leu Met Tyr Pro Pro Pro Tyr Tyr Leu
   1               5                   10
<210> 36
   <211> 13
   <212> PRT
   <213> Artificial Sequence
<220>
   <223> Description of Artificial Sequence: Synthetic peptide
<400> 36
   Thr Tyr Met Met Gly Asn Glu His Thr Phe Leu Asp Asp
   1               5                   10
<210> 37
   <211> 13
   <212> PRT
   <213> Artificial Sequence
<220>
   <223> Description of Artificial Sequence: Synthetic peptide
<400> 37
   Thr Tyr Met Met Gly Asn Glu Lys Thr Phe Leu Asp Asp
   1               5                   10
<210> 38
   <211> 13
   <212> PRT
   <213> Artificial Sequence
<220>
   <223> Description of Artificial Sequence: Synthetic peptide
<400> 38
   Thr Tyr Met Met Gly Asn Glu Leu Thr Phe Leu His Asp
   1 5 10
<210> 39
   <211> 13
   <212> PRT
   <213> Artificial Sequence
<220>
   <223> Description of Artificial Sequence: Synthetic peptide
<400> 39
   Thr Tyr Met Trp Gly Asn Glu Leu Thr Phe Leu Asp Asp
   1 5 10
<210> 40
   <211> 13
   <212> PRT
   <213> Artificial Sequence
<220>
   <223> Description of Artificial Sequence: Synthetic peptide
<400> 40
   Thr Tyr Met Tyr Gly Asn Glu Leu Thr Phe Leu Asp Asp
   1 5 10
<210> 41
   <211> 8
   <212> PRT
   <213> Artificial Sequence
<220>
   <223> Description of Artificial Sequence: Synthetic peptide
<400> 41
   Pro Gly Lys Ala Thr Glu Val Asp
   1 5
<210> 42
   <211> 13
   <212> PRT
   <213> Artificial Sequence
<220>
   <223> Description of Artificial Sequence: Synthetic peptide
<400> 42
   Thr Tyr Met Met Gly Asn Glu Leu Thr Phe Trp Asp Asp
   1 5 10
<210> 43
   <211> 8
   <212> PRT
   <213> Artificial Sequence
<220>
   <223> Description of Artificial Sequence: Synthetic peptide
<400> 43
   Pro Gly Lys Lys Thr Glu Val Arg
   1 5
<210> 44
   <211> 13
   <212> PRT
   <213> Artificial Sequence
<220>
   <223> Description of Artificial Sequence: Synthetic peptide
<400> 44
   Asn Tyr Met Met Gly Asn Glu Leu Thr Phe Leu Asp Asp
   1 5 10
<210> 45
   <211> 13
   <212> PRT
   <213> Artificial Sequence
<220>
   <223> Description of Artificial Sequence: Synthetic peptide
<400> 45
   Thr Tyr Trp Met Gly Asn Glu Leu Thr Phe Leu Asp Asp
   1 5 10
<210> 46
   <211> 12
   <212> PRT
   <213> Artificial Sequence
<220>
   <223> Description of Artificial Sequence: Synthetic peptide
<400> 46
   Lys Val Glu Lys Met Tyr Pro Pro Pro Tyr Tyr Leu
   1 5 10
<210> 47
   <211> 12
   <212> PRT
   <213> Artificial Sequence
<220>
   <223> Description of Artificial Sequence: Synthetic peptide
<400> 47
   Lys Val Glu Arg Met Tyr Pro Pro Pro Tyr Tyr Leu
   1 5 10
<210> 48
   <211> 8
   <212> PRT
   <213> Artificial Sequence
<220>
   <223> Description of Artificial Sequence: Synthetic peptide
<400> 48
   Pro Ala Lys Ala Thr Glu Val Arg
   1 5
<210> 49
   <211> 13
   <212> PRT
   <213> Artificial Sequence
<220>
   <223> Description of Artificial Sequence: Synthetic peptide
<400> 49
   Thr Phe Met Met Gly Asn Glu Leu Thr Phe Leu Asp Asp
   1 5 10
<210> 50
   <211> 13
   <212> PRT
   <213> Artificial Sequence
<220>
   <223> Description of Artificial Sequence: Synthetic peptide
<400> 50
   Thr Tyr Met Met Gly Thr Glu Leu Thr Phe Leu Asp Asp
   1 5 10
<210> 51
   <211> 12
   <212> PRT
   <213> Artificial Sequence
<220>
   <223> Description of Artificial Sequence: Synthetic peptide
<400> 51
   Lys Val Glu Leu Met Tyr Pro Pro Pro Tyr Tyr Met
   1 5 10
<210> 52
   <211> 8
   <212> PRT
   <213> Artificial Sequence
<220>
   <223> Description of Artificial Sequence: Synthetic peptide
<400> 52
   Ile Gly Lys Ala Thr Glu Val Arg
   1 5
<210> 53
   <211> 8
   <212> PRT
   <213> Artificial Sequence
<220>
   <223> Description of Artificial Sequence: Synthetic peptide
<400> 53
   Leu Gly Lys Ala Thr Glu Val Arg
   1 5
<210> 54
   <211> 8
   <212> PRT
   <213> Artificial Sequence
<220>
   <223> Description of Artificial Sequence: Synthetic peptide
<400> 54
   Met Gly Lys Ala Thr Glu Val Arg
   1 5
<210> 55
   <211> 8
   <212> PRT
   <213> Artificial Sequence
<220>
   <223> Description of Artificial Sequence: Synthetic peptide
<400> 55
   Gln Gly Lys Ala Thr Glu Val Arg
   1 5
<210> 56
   <211> 8
   <212> PRT
   <213> Artificial Sequence
<220>
   <223> Description of Artificial Sequence: Synthetic peptide
<400> 56
   Thr Gly Lys Ala Thr Glu Val Arg
   1 5
<210> 57
   <211> 8
   <212> PRT
   <213> Artificial Sequence
<220>
   <223> Description of Artificial Sequence: Synthetic peptide
<400> 57
   Val Gly Lys Ala Thr Glu Val Arg
   1 5
<210> 58
   <211> 8
   <212> PRT
   <213> Artificial Sequence
<220>
   <223> Description of Artificial Sequence: Synthetic peptide
<400> 58
   Pro Gly Arg Ala Thr Glu Val Arg
   1 5
<210> 59
   <211> 8
   <212> PRT
   <213> Artificial Sequence
<220>
   <223> Description of Artificial Sequence: Synthetic peptide
<400> 59
   Pro Gly Lys Pro Thr Glu Val Arg
   1 5
<210> 60
   <211> 8
   <212> PRT
   <213> Artificial Sequence
<220>
   <223> Description of Artificial Sequence: Synthetic peptide
<400> 60
   Pro Gly Lys Ser Thr Glu Val Arg
   1 5
<210> 61
   <211> 8
   <212> PRT
   <213> Artificial Sequence
<220>
   <223> Description of Artificial Sequence: Synthetic peptide
<400> 61
   Pro Gly Lys Ala His Glu Val Arg
   1 5
<210> 62
   <211> 8
   <212> PRT
   <213> Artificial Sequence
<220>
   <223> Description of Artificial Sequence: Synthetic peptide
<400> 62
   Pro Gly Lys Ala Met Glu Val Arg
   1 5
<210> 63
   <211> 8
   <212> PRT
   <213> Artificial Sequence
<220>
   <223> Description of Artificial Sequence: Synthetic peptide
<400> 63
   Pro Gly Lys Ala Ser Glu Val Arg
   1 5
<210> 64
   <211> 8
   <212> PRT
   <213> Artificial Sequence
<220>
   <223> Description of Artificial Sequence: Synthetic peptide
<400> 64
   Pro Gly Lys Ala Thr Glu Glu Arg
   1 5
<210> 65
   <211> 8
   <212> PRT
   <213> Artificial Sequence
<220>
   <223> Description of Artificial Sequence: Synthetic peptide
<400> 65
   Pro Gly Lys Ala Thr Glu Val Pro
   1 5
<210> 66
   <211> 13
   <212> PRT
   <213> Artificial Sequence
<220>
   <223> Description of Artificial Sequence: Synthetic peptide
<400> 66
   Ala Tyr Met Met Gly Asn Glu Leu Thr Phe Leu Asp Asp
   1 5 10
<210> 67
   <211> 13
   <212> PRT
   <213> Artificial Sequence
<220>
   <223> Description of Artificial Sequence: Synthetic peptide
<400> 67
   Thr His Met Met Gly Asn Glu Leu Thr Phe Leu Asp Asp
   1 5 10
<210> 68
   <211> 13
   <212> PRT
   <213> Artificial Sequence
<220>
   <223> Description of Artificial Sequence: Synthetic peptide
<400> 68
   Thr Met Met Met Gly Asn Glu Leu Thr Phe Leu Asp Asp
   1 5 10
<210> 69
   <211> 13
   <212> PRT
   <213> Artificial Sequence
<220>
   <223> Description of Artificial Sequence: Synthetic peptide
<400> 69
   Thr Trp Met Met Gly Asn Glu Leu Thr Phe Leu Asp Asp
   1 5 10
<210> 70
   <211> 13
   <212> PRT
   <213> Artificial Sequence
<220>
   <223> Description of Artificial Sequence: Synthetic peptide
<400> 70
   Thr Tyr Asp Met Gly Asn Glu Leu Thr Phe Leu Asp Asp
   1 5 10
<210> 71
   <211> 13
   <212> PRT
   <213> Artificial Sequence
<220>
   <223> Description of Artificial Sequence: Synthetic peptide
<400> 71
   Thr Tyr Met Asp Gly Asn Glu Leu Thr Phe Leu Asp Asp
   1 5 10
<210> 72
   <211> 13
   <212> PRT
   <213> Artificial Sequence
<220>
   <223> Description of Artificial Sequence: Synthetic peptide
<400> 72
   Thr Tyr Met Gly Gly Asn Glu Leu Thr Phe Leu Asp Asp
   1 5 10
<210> 73
   <211> 13
   <212> PRT
   <213> Artificial Sequence
<220>
   <223> Description of Artificial Sequence: Synthetic peptide
<400> 73
   Thr Tyr Met His Gly Asn Glu Leu Thr Phe Leu Asp Asp
   1 5 10
<210> 74
   <211> 13
   <212> PRT
   <213> Artificial Sequence
<220>
   <223> Description of Artificial Sequence: Synthetic peptide
<400> 74
   Thr Tyr Met Leu Gly Asn Glu Leu Thr Phe Leu Asp Asp
   1 5 10
<210> 75
   <211> 13
   <212> PRT
   <213> Artificial Sequence
<220>
   <223> Description of Artificial Sequence: Synthetic peptide
<400> 75
   Thr Tyr Met Met Asp Asn Glu Leu Thr Phe Leu Asp Asp
   1 5 10
<210> 76
   <211> 13
   <212> PRT
   <213> Artificial Sequence
<220>
   <223> Description of Artificial Sequence: Synthetic peptide
<400> 76
   Thr Tyr Met Met His Asn Glu Leu Thr Phe Leu Asp Asp
   1 5 10
<210> 77
   <211> 13
   <212> PRT
   <213> Artificial Sequence
<220>
   <223> Description of Artificial Sequence: Synthetic peptide
<400> 77
   Thr Tyr Met Met Thr Asn Glu Leu Thr Phe Leu Asp Asp
   1 5 10
<210> 78
   <211> 13
   <212> PRT
   <213> Artificial Sequence
<220>
   <223> Description of Artificial Sequence: Synthetic peptide
<400> 78
   Thr Tyr Met Met Gly Ala Glu Leu Thr Phe Leu Asp Asp
   1               5                   10
<210> 79
   <211> 13
   <212> PRT
   <213> Artificial Sequence
<220>
   <223> Description of Artificial Sequence: Synthetic peptide
<400> 79
   Thr Tyr Met Met Gly Phe Glu Leu Thr Phe Leu Asp Asp
   1               5                   10
<210> 80
   <211> 13
   <212> PRT
   <213> Artificial Sequence
<220>
   <223> Description of Artificial Sequence: Synthetic peptide
<400> 80
   Thr Tyr Met Met Gly Gly Glu Leu Thr Phe Leu Asp Asp
   1               5                   10
<210> 81
   <211> 13
   <212> PRT
   <213> Artificial Sequence
<220>
   <223> Description of Artificial Sequence: Synthetic peptide
<400> 81
   Thr Tyr Met Met Gly His Glu Leu Thr Phe Leu Asp Asp
   1               5                   10
<210> 82
   <211> 13
   <212> PRT
   <213> Artificial Sequence
<220>
   <223> Description of Artificial Sequence: Synthetic peptide
<400> 82
   Thr Tyr Met Met Gly Ile Glu Leu Thr Phe Leu Asp Asp
   1 5 10
<210> 83
   <211> 13
   <212> PRT
   <213> Artificial Sequence
<220>
   <223> Description of Artificial Sequence: Synthetic peptide
<400> 83
   Thr Tyr Met Met Gly Lys Glu Leu Thr Phe Leu Asp Asp
   1 5 10
<210> 84
   <211> 13
   <212> PRT
   <213> Artificial Sequence
<220>
   <223> Description of Artificial Sequence: Synthetic peptide
<400> 84
   Thr Tyr Met Met Gly Leu Glu Leu Thr Phe Leu Asp Asp
   1 5 10
<210> 85
   <211> 13
   <212> PRT
   <213> Artificial Sequence
<220>
   <223> Description of Artificial Sequence: Synthetic peptide
<400> 85
   Thr Tyr Met Met Gly Met Glu Leu Thr Phe Leu Asp Asp
   1 5 10
<210> 86
   <211> 13
   <212> PRT
   <213> Artificial Sequence
<220>
   <223> Description of Artificial Sequence: Synthetic peptide
<400> 86
   Thr Tyr Met Met Gly Gln Glu Leu Thr Phe Leu Asp Asp
   1 5 10
<210> 87
   <211> 13
   <212> PRT
   <213> Artificial Sequence
<220>
   <223> Description of Artificial Sequence: Synthetic peptide
<400> 87
   Thr Tyr Met Met Gly Arg Glu Leu Thr Phe Leu Asp Asp
   1 5 10
<210> 88
   <211> 13
   <212> PRT
   <213> Artificial Sequence
<220>
   <223> Description of Artificial Sequence: Synthetic peptide
<400> 88
   Thr Tyr Met Met Gly Ser Glu Leu Thr Phe Leu Asp Asp
   1 5 10
<210> 89
   <211> 13
   <212> PRT
   <213> Artificial Sequence
<220>
   <223> Description of Artificial Sequence: Synthetic peptide
<400> 89
   Thr Tyr Met Met Gly Val Glu Leu Thr Phe Leu Asp Asp
   1 5 10
<210> 90
   <211> 13
   <212> PRT
   <213> Artificial Sequence
<220>
   <223> Description of Artificial Sequence: Synthetic peptide
<400> 90
   Thr Tyr Met Met Gly Tyr Glu Leu Thr Phe Leu Asp Asp
   1 5 10
<210> 91
   <211> 13
   <212> PRT
   <213> Artificial Sequence
<220>
   <223> Description of Artificial Sequence: Synthetic peptide
<400> 91
   Thr Tyr Met Met Gly Asn Ala Leu Thr Phe Leu Asp Asp
   1 5 10
<210> 92
   <211> 13
   <212> PRT
   <213> Artificial Sequence
<220>
   <223> Description of Artificial Sequence: Synthetic peptide
<400> 92
   Thr Tyr Met Met Gly Asn Asp Leu Thr Phe Leu Asp Asp
   1 5 10
<210> 93
   <211> 13
   <212> PRT
   <213> Artificial Sequence
<220>
   <223> Description of Artificial Sequence: Synthetic peptide
<400> 93
   Thr Tyr Met Met Gly Asn His Leu Thr Phe Leu Asp Asp
   1 5 10
<210> 94
   <211> 13
   <212> PRT
   <213> Artificial Sequence
<220>
   <223> Description of Artificial Sequence: Synthetic peptide
<400> 94
   Thr Tyr Met Met Gly Asn Lys Leu Thr Phe Leu Asp Asp
   1 5 10
<210> 95
   <211> 13
   <212> PRT
   <213> Artificial Sequence
<220>
   <223> Description of Artificial Sequence: Synthetic peptide
<400> 95
   Thr Tyr Met Met Gly Asn Met Leu Thr Phe Leu Asp Asp
   1 5 10
<210> 96
   <211> 13
   <212> PRT
   <213> Artificial Sequence
<220>
   <223> Description of Artificial Sequence: Synthetic peptide
<400> 96
   Thr Tyr Met Met Gly Asn Asn Leu Thr Phe Leu Asp Asp
   1 5 10
<210> 97
   <211> 13
   <212> PRT
   <213> Artificial Sequence
<220>
   <223> Description of Artificial Sequence: Synthetic peptide
<400> 97
   Thr Tyr Met Met Gly Asn Gln Leu Thr Phe Leu Asp Asp
   1 5 10
<210> 98
   <211> 13
   <212> PRT
   <213> Artificial Sequence
<220>
   <223> Description of Artificial Sequence: Synthetic peptide
<400> 98
   Thr Tyr Met Met Gly Asn Arg Leu Thr Phe Leu Asp Asp
   1 5 10
<210> 99
   <211> 13
   <212> PRT
   <213> Artificial Sequence
<220>
   <223> Description of Artificial Sequence: Synthetic peptide
<400> 99
   Thr Tyr Met Met Gly Asn Ser Leu Thr Phe Leu Asp Asp
   1 5 10
<210> 100
   <211> 13
   <212> PRT
   <213> Artificial Sequence
<220>
   <223> Description of Artificial Sequence: Synthetic peptide
<400> 100
   Thr Tyr Met Met Gly Asn Thr Leu Thr Phe Leu Asp Asp
   1 5 10
<210> 101
   <211> 13
   <212> PRT
   <213> Artificial Sequence
<220>
   <223> Description of Artificial Sequence: Synthetic peptide
<400> 101
   Thr Tyr Met Met Gly Asn Val Leu Thr Phe Leu Asp Asp
   1 5 10
<210> 102
   <211> 13
   <212> PRT
   <213> Artificial Sequence
<220>
   <223> Description of Artificial Sequence: Synthetic peptide
<400> 102
   Thr Tyr Met Met Gly Asn Glu Ile Thr Phe Leu Asp Asp
   1 5 10
<210> 103
   <211> 13
   <212> PRT
   <213> Artificial Sequence
<220>
   <223> Description of Artificial Sequence: Synthetic peptide
<400> 103
   Thr Tyr Met Met Gly Asn Glu Arg Thr Phe Leu Asp Asp
   1 5 10
<210> 104
   <211> 13
   <212> PRT
   <213> Artificial Sequence
<220>
   <223> Description of Artificial Sequence: Synthetic peptide
<400> 104
   Thr Tyr Met Met Gly Asn Glu Thr Thr Phe Leu Asp Asp
   1               5                   10
<210> 105
   <211> 13
   <212> PRT
   <213> Artificial Sequence
<220>
   <223> Description of Artificial Sequence: Synthetic peptide
<400> 105
   Thr Tyr Met Met Gly Asn Glu Leu Ile Phe Leu Asp Asp
   1               5                   10
<210> 106
   <211> 13
   <212> PRT
   <213> Artificial Sequence
<220>
   <223> Description of Artificial Sequence: Synthetic peptide
<400> 106
   Thr Tyr Met Met Gly Asn Glu Leu Met Phe Leu Asp Asp
   1               5                   10
<210> 107
   <211> 13
   <212> PRT
   <213> Artificial Sequence
<220>
   <223> Description of Artificial Sequence: Synthetic peptide
<400> 107
   Thr Tyr Met Met Gly Asn Glu Leu Asn Phe Leu Asp Asp
   1               5                   10
<210> 108
   <211> 13
   <212> PRT
   <213> Artificial Sequence
<220>
   <223> Description of Artificial Sequence: Synthetic peptide
<400> 108
   Thr Tyr Met Met Gly Asn Glu Leu Gln Phe Leu Asp Asp
   1               5                   10
<210> 109
   <211> 13
   <212> PRT
   <213> Artificial Sequence
<220>
   <223> Description of Artificial Sequence: Synthetic peptide
<400> 109
   Thr Tyr Met Met Gly Asn Glu Leu Arg Phe Leu Asp Asp
   1 5 10
<210> 110
   <211> 13
   <212> PRT
   <213> Artificial Sequence
<220>
   <223> Description of Artificial Sequence: Synthetic peptide
<400> 110
   Thr Tyr Met Met Gly Asn Glu Leu Ser Phe Leu Asp Asp
   1 5 10
<210> 111
   <211> 13
   <212> PRT
   <213> Artificial Sequence
<220>
   <223> Description of Artificial Sequence: Synthetic peptide
<400> 111
   Thr Tyr Met Met Gly Asn Glu Leu Val Phe Leu Asp Asp
   1 5 10
<210> 112
   <211> 13
   <212> PRT
   <213> Artificial Sequence
<220>
   <223> Description of Artificial Sequence: Synthetic peptide
<400> 112
   Thr Tyr Met Met Gly Asn Glu Leu Thr Phe Glu Asp Asp
   1 5 10
<210> 113
   <211> 13
   <212> PRT
   <213> Artificial Sequence
<220>
   <223> Description of Artificial Sequence: Synthetic peptide
<400> 113
   Thr Tyr Met Met Gly Asn Glu Leu Thr Phe Ile Asp Asp
   1 5 10
<210> 114
   <211> 13
   <212> PRT
   <213> Artificial Sequence
<220>
   <223> Description of Artificial Sequence: Synthetic peptide
<400> 114
   Thr Tyr Met Met Gly Asn Glu Leu Thr Phe Met Asp Asp
   1 5 10
<210> 115
   <211> 13
   <212> PRT
   <213> Artificial Sequence
<220>
   <223> Description of Artificial Sequence: Synthetic peptide
<400> 115
   Thr Tyr Met Met Gly Asn Glu Leu Thr Phe Thr Asp Asp
   1 5 10
<210> 116
   <211> 13
   <212> PRT
   <213> Artificial Sequence
<220>
   <223> Description of Artificial Sequence: Synthetic peptide
<400> 116
   Thr Tyr Met Met Gly Asn Glu Leu Thr Phe Val Asp Asp
   1 5 10
<210> 117
   <211> 13
   <212> PRT
   <213> Artificial Sequence
<220>
   <223> Description of Artificial Sequence: Synthetic peptide
<400> 117
   Thr Tyr Met Met Gly Asn Glu Leu Thr Phe Leu Arg Asp
   1 5 10
<210> 118
   <211> 13
   <212> PRT
   <213> Artificial Sequence
<220>
   <223> Description of Artificial Sequence: Synthetic peptide
<400> 118
   Thr Tyr Met Met Gly Asn Glu Leu Thr Phe Leu Asp Glu
   1 5 10
<210> 119
   <211> 13
   <212> PRT
   <213> Artificial Sequence
<220>
   <223> Description of Artificial Sequence: Synthetic peptide
<400> 119
   Thr Tyr Met Met Gly Asn Glu Leu Thr Phe Leu Asp Gly
   1 5 10
<210> 120
   <211> 13
   <212> PRT
   <213> Artificial Sequence
<220>
   <223> Description of Artificial Sequence: Synthetic peptide
<400> 120
   Thr Tyr Met Met Gly Asn Glu Leu Thr Phe Leu Asp His
   1 5 10
<210> 121
   <211> 13
   <212> PRT
   <213> Artificial Sequence
<220>
   <223> Description of Artificial Sequence: Synthetic peptide
<400> 121
   Thr Tyr Met Met Gly Asn Glu Leu Thr Phe Leu Asp Thr
   1               5                   10
<210> 122
   <211> 12
   <212> PRT
   <213> Artificial Sequence
<220>
   <223> Description of Artificial Sequence: Synthetic peptide
<400> 122
   Gly Val Glu Leu Met Tyr Pro Pro Pro Tyr Tyr Leu
   1               5                   10
<210> 123
   <211> 12
   <212> PRT
   <213> Artificial Sequence
<220>
   <223> Description of Artificial Sequence: Synthetic peptide
<400> 123
   Lys Gly Glu Leu Met Tyr Pro Pro Pro Tyr Tyr Leu
   1               5                   10
<210> 124
   <211> 12
   <212> PRT
   <213> Artificial Sequence
<220>
   <223> Description of Artificial Sequence: Synthetic peptide
<400> 124
   Lys Ile Glu Leu Met Tyr Pro Pro Pro Tyr Tyr Leu
   1               5                   10
<210> 125
   <211> 12
   <212> PRT
   <213> Artificial Sequence
<220>
   <223> Description of Artificial Sequence: Synthetic peptide
<400> 125
   Lys Val Trp Leu Met Tyr Pro Pro Pro Tyr Tyr Leu
   1               5                   10
<210> 126
   <211> 12
   <212> PRT
   <213> Artificial Sequence
<220>
   <223> Description of Artificial Sequence: Synthetic peptide
<400> 126
   Lys Val Glu Leu Met Tyr His Pro Pro Tyr Tyr Leu
   1 5 10
<210> 127
   <211> 12
   <212> PRT
   <213> Artificial Sequence
<220>
   <223> Description of Artificial Sequence: Synthetic peptide
<400> 127
   Lys Val Glu Leu Met Tyr Thr Pro Pro Tyr Tyr Leu
   1 5 10
<210> 128
   <211> 12
   <212> PRT
   <213> Artificial Sequence
<220>
   <223> Description of Artificial Sequence: Synthetic peptide
<400> 128
   Lys Val Glu Leu Met Tyr Leu Pro Pro Tyr Tyr Leu
   1 5 10
<210> 129
   <211> 12
   <212> PRT
   <213> Artificial Sequence
<220>
   <223> Description of Artificial Sequence: Synthetic peptide
<400> 129
   Lys Val Glu Leu Met Tyr Pro Pro Pro Thr Tyr Leu
   1 5 10
<210> 130
   <211> 8
   <212> PRT
   <213> Artificial Sequence
<220>
   <223> Description of Artificial Sequence: Synthetic peptide
<400> 130
   Pro Gly Tyr Ala Thr Glu Val Arg
   1 5
<210> 131
   <211> 8
   <212> PRT
   <213> Artificial Sequence
<220>
   <223> Description of Artificial Sequence: Synthetic peptide
<400> 131
   Asn Gly Lys Ala Thr Glu Val Arg
   1 5
<210> 132
   <211> 8
   <212> PRT
   <213> Artificial Sequence
<220>
   <223> Description of Artificial Sequence: Synthetic peptide
<400> 132
   Pro Ile Lys Ala Thr Glu Val Arg
   1 5
<210> 133
   <211> 8
   <212> PRT
   <213> Artificial Sequence
<220>
   <223> Description of Artificial Sequence: Synthetic peptide
<400> 133
   Pro Gln Lys Ala Thr Glu Val Arg
   1 5
<210> 134
   <211> 8
   <212> PRT
   <213> Artificial Sequence
<220>
   <223> Description of Artificial Sequence: Synthetic peptide
<400> 134
   Pro Trp Lys Ala Thr Glu Val Arg
   1 5
<210> 135
   <211> 8
   <212> PRT
   <213> Artificial Sequence
<220>
   <223> Description of Artificial Sequence: Synthetic peptide
<400> 135
   Pro Gly Glu Ala Thr Glu Val Arg
   1 5
<210> 136
   <211> 8
   <212> PRT
   <213> Artificial Sequence
<220>
   <223> Description of Artificial Sequence: Synthetic peptide
<400> 136
   Pro Gly Ile Ala Thr Glu Val Arg
   1 5
<210> 137
   <211> 8
   <212> PRT
   <213> Artificial Sequence
<220>
   <223> Description of Artificial Sequence: Synthetic peptide
<400> 137
   Pro Gly Met Ala Thr Glu Val Arg
   1 5
<210> 138
   <211> 8
   <212> PRT
   <213> Artificial Sequence
<220>
   <223> Description of Artificial Sequence: Synthetic peptide
<400> 138
   Pro Gly Pro Ala Thr Glu Val Arg
   1 5
<210> 139
   <211> 8
   <212> PRT
   <213> Artificial Sequence
<220>
   <223> Description of Artificial Sequence: Synthetic peptide
<400> 139
   Pro Gly Gln Ala Thr Glu Val Arg
   1 5
<210> 140
   <211> 8
   <212> PRT
   <213> Artificial Sequence
<220>
   <223> Description of Artificial Sequence: Synthetic peptide
<400> 140
   Pro Gly Val Ala Thr Glu Val Arg
   1 5
<210> 141
   <211> 8
   <212> PRT
   <213> Artificial Sequence
<220>
   <223> Description of Artificial Sequence: Synthetic peptide
<400> 141
   Pro Gly Lys Ala Ile Glu Val Arg
   1 5
<210> 142
   <211> 8
   <212> PRT
   <213> Artificial Sequence
<220>
   <223> Description of Artificial Sequence: Synthetic peptide
<400> 142
   Pro Gly Lys Ala Leu Glu Val Arg
   1 5
<210> 143
   <211> 13
   <212> PRT
   <213> Artificial Sequence
<220>
   <223> Description of Artificial Sequence: Synthetic peptide
<400> 143
   Thr Tyr Glu Met Gly Asn Glu Leu Thr Phe Leu Asp Asp
   1 5 10
<210> 144
   <211> 13
   <212> PRT
   <213> Artificial Sequence
<220>
   <223> Description of Artificial Sequence: Synthetic peptide
<400> 144
   Thr Tyr Leu Met Gly Asn Glu Leu Thr Phe Leu Asp Asp
   1 5 10
<210> 145
   <211> 13
   <212> PRT
   <213> Artificial Sequence
<220>
   <223> Description of Artificial Sequence: Synthetic peptide
<400> 145
   Thr Tyr Asn Met Gly Asn Glu Leu Thr Phe Leu Asp Asp
   1 5 10
<210> 146
   <211> 13
   <212> PRT
   <213> Artificial Sequence
<220>
   <223> Description of Artificial Sequence: Synthetic peptide
<400> 146
   Thr Tyr Met Ala Gly Asn Glu Leu Thr Phe Leu Asp Asp
   1 5 10
<210> 147
   <211> 13
   <212> PRT
   <213> Artificial Sequence
<220>
   <223> Description of Artificial Sequence: Synthetic peptide
<400> 147
   Thr Tyr Met Gln Gly Asn Glu Leu Thr Phe Leu Asp Asp
   1 5 10
<210> 148
   <211> 13
   <212> PRT
   <213> Artificial Sequence
<220>
   <223> Description of Artificial Sequence: Synthetic peptide
<400> 148
   Thr Tyr Met Ser Gly Asn Glu Leu Thr Phe Leu Asp Asp
   1 5 10
<210> 149
   <211> 13
   <212> PRT
   <213> Artificial Sequence
<220>
   <223> Description of Artificial Sequence: Synthetic peptide
<400> 149
   Thr Tyr Met Met Gly Asn Glu Asp Thr Phe Leu Asp Asp
   1 5 10
<210> 150
   <211> 13
   <212> PRT
   <213> Artificial Sequence
<220>
   <223> Description of Artificial Sequence: Synthetic peptide
<400> 150
   Thr Tyr Met Met Gly Asn Glu Val Thr Phe Leu Asp Asp
   1 5 10
<210> 151
   <211> 13
   <212> PRT
   <213> Artificial Sequence
<220>
   <223> Description of Artificial Sequence: Synthetic peptide
<400> 151
   Thr Tyr Met Met Gly Asn Glu Leu His Phe Leu Asp Asp
   1 5 10
<210> 152
   <211> 13
   <212> PRT
   <213> Artificial Sequence
<220>
   <223> Description of Artificial Sequence: Synthetic peptide
<400> 152
   Thr Tyr Met Met Gly Asn Glu Leu Lys Phe Leu Asp Asp
   1 5 10
<210> 153
   <211> 13
   <212> PRT
   <213> Artificial Sequence
<220>
   <223> Description of Artificial Sequence: Synthetic peptide
<400> 153
   Thr Tyr Met Met Gly Asn Glu Leu Leu Phe Leu Asp Asp
   1 5 10
<210> 154
   <211> 13
   <212> PRT
   <213> Artificial Sequence
<220>
   <223> Description of Artificial Sequence: Synthetic peptide
<400> 154
   Thr Tyr Met Met Gly Asn Glu Leu Pro Phe Leu Asp Asp
   1 5 10
<210> 155
   <211> 13
   <212> PRT
   <213> Artificial Sequence
<220>
   <223> Description of Artificial Sequence: Synthetic peptide
<400> 155
   Thr Tyr Met Met Gly Asn Glu Leu Thr Phe Asp Asp Asp
   1 5 10
<210> 156
   <211> 13
   <212> PRT
   <213> Artificial Sequence
<220>
   <223> Description of Artificial Sequence: Synthetic peptide
<400> 156
   Thr Tyr Met Met Gly Asn Glu Leu Thr Phe Gly Asp Asp
   1 5 10
<210> 157
   <211> 13
   <212> PRT
   <213> Artificial Sequence
<220>
   <223> Description of Artificial Sequence: Synthetic peptide
<400> 157
   Thr Tyr Met Met Gly Asn Glu Leu Thr Phe Gln Asp Asp
   1 5 10
<210> 158
   <211> 13
   <212> PRT
   <213> Artificial Sequence
<220>
   <223> Description of Artificial Sequence: Synthetic peptide
<400> 158
   Thr Tyr Met Met Gly Asn Glu Leu Thr Phe Arg Asp Asp
   1 5 10
<210> 159
   <211> 13
   <212> PRT
   <213> Artificial Sequence
<220>
   <223> Description of Artificial Sequence: Synthetic peptide
<400> 159
   Thr Tyr Met Met Gly Asn Glu Leu Thr Phe Leu Ala Asp
   1 5 10
<210> 160
   <211> 13
   <212> PRT
   <213> Artificial Sequence
<220>
   <223> Description of Artificial Sequence: Synthetic peptide
<400> 160
   Thr Tyr Met Met Gly Asn Glu Leu Thr Phe Leu Glu Asp
   1 5 10
<210> 161
   <211> 13
   <212> PRT
   <213> Artificial Sequence
<220>
   <223> Description of Artificial Sequence: Synthetic peptide
<400> 161
   Thr Tyr Met Met Gly Asn Glu Leu Thr Phe Leu Ser Asp
   1               5                   10
<210> 162
   <211> 13
   <212> PRT
   <213> Artificial Sequence
<220>
   <223> Description of Artificial Sequence: Synthetic peptide
<400> 162
   Thr Tyr Met Met Gly Asn Glu Leu Thr Phe Leu Asp Gln
   1               5                   10
<210> 163
   <211> 13
   <212> PRT
   <213> Artificial Sequence
<220>
   <223> Description of Artificial Sequence: Synthetic peptide
<400> 163
   Thr Tyr Met Met Gly Asn Glu Leu Thr Phe Leu Asp Ser
   1               5                   10
<210> 164
   <211> 12
   <212> PRT
   <213> Artificial Sequence
<220>
   <223> Description of Artificial Sequence: Synthetic peptide
<400> 164
   Arg Val Glu Leu Met Tyr Pro Pro Pro Tyr Tyr Leu
   1               5                   10
<210> 165
   <211> 8
   <212> PRT
   <213> Artificial Sequence
<220>
   <223> Description of Artificial Sequence: Synthetic peptide
<400> 165
   Arg Gly Lys Ala Thr Glu Val Arg
   1 5
<210> 166
   <211> 8
   <212> PRT
   <213> Artificial Sequence
<220>
   <223> Description of Artificial Sequence: Synthetic peptide
<400> 166
   Pro Asp Lys Ala Thr Glu Val Arg
   1 5
<210> 167
   <211> 8
   <212> PRT
   <213> Artificial Sequence
<220>
   <223> Description of Artificial Sequence: Synthetic peptide
<400> 167
   Pro Pro Lys Ala Thr Glu Val Arg
   1 5
<210> 168
   <211> 8
   <212> PRT
   <213> Artificial Sequence
<220>
   <223> Description of Artificial Sequence: Synthetic peptide
<400> 168
   Pro Gly Lys Asn Thr Glu Val Arg
   1 5
<210> 169
   <211> 8
   <212> PRT
   <213> Artificial Sequence
<220>
   <223> Description of Artificial Sequence: Synthetic peptide
<400> 169
   Pro Gly Lys Ala Lys Glu Val Arg
   1 5
<210> 170
   <211> 8
   <212> PRT
   <213> Artificial Sequence
<220>
   <223> Description of Artificial Sequence: Synthetic peptide
<400> 170
   Pro Gly Lys Ala Thr Asp Val Arg
   1               5
<210> 171
   <211> 8
   <212> PRT
   <213> Artificial Sequence
<220>
   <223> Description of Artificial Sequence: Synthetic peptide
<400> 171
   Pro Gly Lys Ala Thr Gly Val Arg
   1               5
<210> 172
   <211> 8
   <212> PRT
   <213> Artificial Sequence
<220>
   <223> Description of Artificial Sequence: Synthetic peptide
<400> 172
   Pro Gly Lys Ala Thr Pro Val Arg
   1               5
<210> 173
   <211> 8
   <212> PRT
   <213> Artificial Sequence
<220>
   <223> Description of Artificial Sequence: Synthetic peptide
<400> 173
   Pro Gly Lys Ala Thr Arg Val Arg
   1               5
<210> 174
   <211> 8
   <212> PRT
   <213> Artificial Sequence
<220>
   <223> Description of Artificial Sequence: Synthetic peptide
<400> 174
   Pro Gly Lys Ala Thr Thr Val Arg
   1 5
<210> 175
   <211> 8
   <212> PRT
   <213> Artificial Sequence
<220>
   <223> Description of Artificial Sequence: Synthetic peptide
<400> 175
   Pro Gly Lys Ala Thr Glu Gly Arg
   1 5
<210> 176
   <211> 8
   <212> PRT
   <213> Artificial Sequence
<220>
   <223> Description of Artificial Sequence: Synthetic peptide
<400> 176
   Pro Gly Lys Ala Thr Glu His Arg
   1 5
<210> 177
   <211> 8
   <212> PRT
   <213> Artificial Sequence
<220>
   <223> Description of Artificial Sequence: Synthetic peptide
<400> 177
   Pro Gly Lys Ala Thr Glu Thr Arg
   1 5
<210> 178
   <211> 8
   <212> PRT
   <213> Artificial Sequence
<220>
   <223> Description of Artificial Sequence: Synthetic peptide
<400> 178
   Pro Gly Lys Ala Thr Glu Val Glu
   1 5
<210> 179
   <211> 8
   <212> PRT
   <213> Artificial Sequence
<220>
   <223> Description of Artificial Sequence: Synthetic peptide
<400> 179
   Pro Gly Lys Ala Thr Glu Val Gly
   1 5
<210> 180
   <211> 8
   <212> PRT
   <213> Artificial Sequence
<220>
   <223> Description of Artificial Sequence: Synthetic peptide
<400> 180
   Pro Gly Lys Ala Thr Glu Val Ile
   1 5
<210> 181
   <211> 8
   <212> PRT
   <213> Artificial Sequence
<220>
   <223> Description of Artificial Sequence: Synthetic peptide
<400> 181
   Pro Gly Lys Ala Thr Glu Val Met
   1 5
<210> 182
   <211> 8
   <212> PRT
   <213> Artificial Sequence
<220>
   <223> Description of Artificial Sequence: Synthetic peptide
<400> 182
   Pro Gly Lys Ala Thr Glu Val Asn
   1 5
<210> 183
   <211> 8
   <212> PRT
   <213> Artificial Sequence
<220>
   <223> Description of Artificial Sequence: Synthetic peptide
<400> 183
   Pro Gly Lys Ala Thr Glu Val Gln
   1 5
<210> 184
   <211> 8
   <212> PRT
   <213> Artificial Sequence
<220>
   <223> Description of Artificial Sequence: Synthetic peptide
<400> 184
   Pro Gly Lys Ala Thr Glu Val Trp
   1 5
<210> 185
   <211> 8
   <212> PRT
   <213> Artificial Sequence
<220>
   <223> Description of Artificial Sequence: Synthetic peptide
<400> 185
   Pro Gly Lys Ala Thr Glu Val Tyr
   1 5
<210> 186
   <211> 13
   <212> PRT
   <213> Artificial Sequence
<220>
   <223> Description of Artificial Sequence: Synthetic peptide
<400> 186
   Glu Tyr Met Met Gly Asn Glu Leu Thr Phe Leu Asp Asp
   1 5 10
<210> 187
   <211> 13
   <212> PRT
   <213> Artificial Sequence
<220>
   <223> Description of Artificial Sequence: Synthetic peptide
<400> 187
   Gly Tyr Met Met Gly Asn Glu Leu Thr Phe Leu Asp Asp
   1               5                   10
<210> 188
   <211> 13
   <212> PRT
   <213> Artificial Sequence
<220>
   <223> Description of Artificial Sequence: Synthetic peptide
<400> 188
   Ile Tyr Met Met Gly Asn Glu Leu Thr Phe Leu Asp Asp
   1               5                   10
<210> 189
   <211> 13
   <212> PRT
   <213> Artificial Sequence
<220>
   <223> Description of Artificial Sequence: Synthetic peptide
<400> 189
   Val Tyr Met Met Gly Asn Glu Leu Thr Phe Leu Asp Asp
   1               5                   10
<210> 190
   <211> 13
   <212> PRT
   <213> Artificial Sequence
<220>
   <223> Description of Artificial Sequence: Synthetic peptide
<400> 190
   Thr Ala Met Met Gly Asn Glu Leu Thr Phe Leu Asp Asp
   1               5                   10
<210> 191
   <211> 13
   <212> PRT
   <213> Artificial Sequence
<220>
   <223> Description of Artificial Sequence: Synthetic peptide
<400> 191
   Thr Asp Met Met Gly Asn Glu Leu Thr Phe Leu Asp Asp
   1 5 10
<210> 192
   <211> 13
   <212> PRT
   <213> Artificial Sequence
<220>
   <223> Description of Artificial Sequence: Synthetic peptide
<400> 192
   Thr Ile Met Met Gly Asn Glu Leu Thr Phe Leu Asp Asp
   1 5 10
<210> 193
   <211> 13
   <212> PRT
   <213> Artificial Sequence
<220>
   <223> Description of Artificial Sequence: Synthetic peptide
<400> 193
   Thr Lys Met Met Gly Asn Glu Leu Thr Phe Leu Asp Asp
   1 5 10
<210> 194
   <211> 13
   <212> PRT
   <213> Artificial Sequence
<220>
   <223> Description of Artificial Sequence: Synthetic peptide
<400> 194
   Thr Leu Met Met Gly Asn Glu Leu Thr Phe Leu Asp Asp
   1 5 10
<210> 195
   <211> 13
   <212> PRT
   <213> Artificial Sequence
<220>
   <223> Description of Artificial Sequence: Synthetic peptide
<400> 195
   Thr Gln Met Met Gly Asn Glu Leu Thr Phe Leu Asp Asp
   1 5 10
<210> 196
   <211> 13
   <212> PRT
   <213> Artificial Sequence
<220>
   <223> Description of Artificial Sequence: Synthetic peptide
<400> 196
   Thr Arg Met Met Gly Asn Glu Leu Thr Phe Leu Asp Asp
   1 5 10
<210> 197
   <211> 13
   <212> PRT
   <213> Artificial Sequence
<220>
   <223> Description of Artificial Sequence: Synthetic peptide
<400> 197
   Thr Ser Met Met Gly Asn Glu Leu Thr Phe Leu Asp Asp
   1 5 10
<210> 198
   <211> 13
   <212> PRT
   <213> Artificial Sequence
<220>
   <223> Description of Artificial Sequence: Synthetic peptide
<400> 198
   Thr Thr Met Met Gly Asn Glu Leu Thr Phe Leu Asp Asp
   1 5 10
<210> 199
   <211> 13
   <212> PRT
   <213> Artificial Sequence
<220>
   <223> Description of Artificial Sequence: Synthetic peptide
<400> 199
   Thr Tyr His Met Gly Asn Glu Leu Thr Phe Leu Asp Asp
   1 5 10
<210> 200
   <211> 13
   <212> PRT
   <213> Artificial Sequence
<220>
   <223> Description of Artificial Sequence: Synthetic peptide
<400> 200
   Thr Tyr Pro Met Gly Asn Glu Leu Thr Phe Leu Asp Asp
   1               5                   10
<210> 201
   <211> 13
   <212> PRT
   <213> Artificial Sequence
<220>
   <223> Description of Artificial Sequence: Synthetic peptide
<400> 201
   Thr Tyr Ser Met Gly Asn Glu Leu Thr Phe Leu Asp Asp
   1               5                   10
<210> 202
   <211> 13
   <212> PRT
   <213> Artificial Sequence
<220>
   <223> Description of Artificial Sequence: Synthetic peptide
<400> 202
   Thr Tyr Met Met Ala Asn Glu Leu Thr Phe Leu Asp Asp
   1               5                   10
<210> 203
   <211> 13
   <212> PRT
   <213> Artificial Sequence
<220>
   <223> Description of Artificial Sequence: Synthetic peptide
<400> 203
   Thr Tyr Met Met Ile Asn Glu Leu Thr Phe Leu Asp Asp
   1               5                   10
<210> 204
   <211> 13
   <212> PRT
   <213> Artificial Sequence
<220>
   <223> Description of Artificial Sequence: Synthetic peptide
<400> 204
   Thr Tyr Met Met Leu Asn Glu Leu Thr Phe Leu Asp Asp
   1 5 10
<210> 205
   <211> 13
   <212> PRT
   <213> Artificial Sequence
<220>
   <223> Description of Artificial Sequence: Synthetic peptide
<400> 205
   Thr Tyr Met Met Asn Asn Glu Leu Thr Phe Leu Asp Asp
   1 5 10
<210> 206
   <211> 13
   <212> PRT
   <213> Artificial Sequence
<220>
   <223> Description of Artificial Sequence: Synthetic peptide
<400> 206
   Thr Tyr Met Met Gln Asn Glu Leu Thr Phe Leu Asp Asp
   1 5 10
<210> 207
   <211> 13
   <212> PRT
   <213> Artificial Sequence
<220>
   <223> Description of Artificial Sequence: Synthetic peptide
<400> 207
   Thr Tyr Met Met Arg Asn Glu Leu Thr Phe Leu Asp Asp
   1 5 10
<210> 208
   <211> 13
   <212> PRT
   <213> Artificial Sequence
<220>
   <223> Description of Artificial Sequence: Synthetic peptide
<400> 208
   Thr Tyr Met Met Tyr Asn Glu Leu Thr Phe Leu Asp Asp
   1 5 10
<210> 209
   <211> 13
   <212> PRT
   <213> Artificial Sequence
<220>
   <223> Description of Artificial Sequence: Synthetic peptide
<400> 209
   Thr Tyr Met Met Gly Asn Gly Leu Thr Phe Leu Asp Asp
   1 5 10
<210> 210
   <211> 13
   <212> PRT
   <213> Artificial Sequence
<220>
   <223> Description of Artificial Sequence: Synthetic peptide
<400> 210
   Thr Tyr Met Met Gly Asn Ile Leu Thr Phe Leu Asp Asp
   1 5 10
<210> 211
   <211> 13
   <212> PRT
   <213> Artificial Sequence
<220>
   <223> Description of Artificial Sequence: Synthetic peptide
<400> 211
   Thr Tyr Met Met Gly Asn Trp Leu Thr Phe Leu Asp Asp
   1 5 10
<210> 212
   <211> 13
   <212> PRT
   <213> Artificial Sequence
<220>
   <223> Description of Artificial Sequence: Synthetic peptide
<400> 212
   Thr Tyr Met Met Gly Asn Tyr Leu Thr Phe Leu Asp Asp
   1 5 10
<210> 213
   <211> 13
   <212> PRT
   <213> Artificial Sequence
<220>
   <223> Description of Artificial Sequence: Synthetic peptide
<400> 213
   Thr Tyr Met Met Gly Asn Glu Leu Asp Phe Leu Asp Asp
   1 5 10
<210> 214
   <211> 13
   <212> PRT
   <213> Artificial Sequence
<220>
   <223> Description of Artificial Sequence: Synthetic peptide
<400> 214
   Thr Tyr Met Met Gly Asn Glu Leu Gly Phe Leu Asp Asp
   1 5 10
<210> 215
   <211> 13
   <212> PRT
   <213> Artificial Sequence
<220>
   <223> Description of Artificial Sequence: Synthetic peptide
<400> 215
   Thr Tyr Met Met Gly Asn Glu Leu Tyr Phe Leu Asp Asp
   1 5 10
<210> 216
   <211> 13
   <212> PRT
   <213> Artificial Sequence
<220>
   <223> Description of Artificial Sequence: Synthetic peptide
<400> 216
   Thr Tyr Met Met Gly Asn Glu Leu Thr Asp Leu Asp Asp
   1 5 10
<210> 217
   <211> 13
   <212> PRT
   <213> Artificial Sequence
<220>
   <223> Description of Artificial Sequence: Synthetic peptide
<400> 217
   Thr Tyr Met Met Gly Asn Glu Leu Thr Gly Leu Asp Asp
   1 5 10
<210> 218
   <211> 13
   <212> PRT
   <213> Artificial Sequence
<220>
   <223> Description of Artificial Sequence: Synthetic peptide
<400> 218
   Thr Tyr Met Met Gly Asn Glu Leu Thr His Leu Asp Asp
   1 5 10
<210> 219
   <211> 13
   <212> PRT
   <213> Artificial Sequence
<220>
   <223> Description of Artificial Sequence: Synthetic peptide
<400> 219
   Thr Tyr Met Met Gly Asn Glu Leu Thr Leu Leu Asp Asp
   1 5 10
<210> 220
   <211> 13
   <212> PRT
   <213> Artificial Sequence
<220>
   <223> Description of Artificial Sequence: Synthetic peptide
<400> 220
   Thr Tyr Met Met Gly Asn Glu Leu Thr Phe Leu Gly Asp
   1 5 10
<210> 221
   <211> 13
   <212> PRT
   <213> Artificial Sequence
<220>
   <223> Description of Artificial Sequence: Synthetic peptide
<400> 221
   Thr Tyr Met Met Gly Asn Glu Leu Thr Phe Leu Ile Asp
   1 5 10
<210> 222
   <211> 13
   <212> PRT
   <213> Artificial Sequence
<220>
   <223> Description of Artificial Sequence: Synthetic peptide
<400> 222
   Thr Tyr Met Met Gly Asn Glu Leu Thr Phe Leu Trp Asp
   1               5                   10
<210> 223
   <211> 13
   <212> PRT
   <213> Artificial Sequence
<220>
   <223> Description of Artificial Sequence: Synthetic peptide
<400> 223
   Thr Tyr Met Met Gly Asn Glu Leu Thr Phe Leu Asp Ala
   1               5                   10
<210> 224
   <211> 13
   <212> PRT
   <213> Artificial Sequence
<220>
   <223> Description of Artificial Sequence: Synthetic peptide
<400> 224
   Thr Tyr Met Met Gly Asn Glu Leu Thr Phe Leu Asp Tyr
   1               5                   10
<210> 225
   <211> 12
   <212> PRT
   <213> Artificial Sequence
<220>
   <223> Description of Artificial Sequence: Synthetic peptide
<400> 225
   Asp Val Glu Leu Met Tyr Pro Pro Pro Tyr Tyr Leu
   1               5                   10
<210> 226
   <211> 12
   <212> PRT
   <213> Artificial Sequence
<220>
   <223> Description of Artificial Sequence: Synthetic peptide
<400> 226
   His Val Glu Leu Met Tyr Pro Pro Pro Tyr Tyr Leu
   1 5 10
<210> 227
   <211> 12
   <212> PRT
   <213> Artificial Sequence
<220>
   <223> Description of Artificial Sequence: Synthetic peptide
<400> 227
   Asn Val Glu Leu Met Tyr Pro Pro Pro Tyr Tyr Leu
   1 5 10
<210> 228
   <211> 12
   <212> PRT
   <213> Artificial Sequence
<220>
   <223> Description of Artificial Sequence: Synthetic peptide
<400> 228
   Pro Val Glu Leu Met Tyr Pro Pro Pro Tyr Tyr Leu
   1 5 10
<210> 229
   <211> 12
   <212> PRT
   <213> Artificial Sequence
<220>
   <223> Description of Artificial Sequence: Synthetic peptide
<400> 229
   Trp Val Glu Leu Met Tyr Pro Pro Pro Tyr Tyr Leu
   1 5 10
<210> 230
   <211> 12
   <212> PRT
   <213> Artificial Sequence
<220>
   <223> Description of Artificial Sequence: Synthetic peptide
<400> 230
   Lys Ala Glu Leu Met Tyr Pro Pro Pro Tyr Tyr Leu
   1 5 10
<210> 231
   <211> 12
   <212> PRT
   <213> Artificial Sequence
<220>
   <223> Description of Artificial Sequence: Synthetic peptide
<400> 231
   Lys Asp Glu Leu Met Tyr Pro Pro Pro Tyr Tyr Leu
   1 5 10
<210> 232
   <211> 12
   <212> PRT
   <213> Artificial Sequence
<220>
   <223> Description of Artificial Sequence: Synthetic peptide
<400> 232
   Lys His Glu Leu Met Tyr Pro Pro Pro Tyr Tyr Leu
   1 5 10
<210> 233
   <211> 12
   <212> PRT
   <213> Artificial Sequence
<220>
   <223> Description of Artificial Sequence: Synthetic peptide
<400> 233
   Lys Asn Glu Leu Met Tyr Pro Pro Pro Tyr Tyr Leu
   1 5 10
<210> 234
   <211> 12
   <212> PRT
   <213> Artificial Sequence
<220>
   <223> Description of Artificial Sequence: Synthetic peptide
<400> 234
   Lys Pro Glu Leu Met Tyr Pro Pro Pro Tyr Tyr Leu
   1 5 10
<210> 235
   <211> 12
   <212> PRT
   <213> Artificial Sequence
<220>
   <223> Description of Artificial Sequence: Synthetic peptide
<400> 235
   Lys Arg Glu Leu Met Tyr Pro Pro Pro Tyr Tyr Leu
   1 5 10
<210> 236
   <211> 12
   <212> PRT
   <213> Artificial Sequence
<220>
   <223> Description of Artificial Sequence: Synthetic peptide
<400> 236
   Lys Thr Glu Leu Met Tyr Pro Pro Pro Tyr Tyr Leu
   1 5 10
<210> 237
   <211> 12
   <212> PRT
   <213> Artificial Sequence
<220>
   <223> Description of Artificial Sequence: Synthetic peptide
<400> 237
   Lys Trp Glu Leu Met Tyr Pro Pro Pro Tyr Tyr Leu
   1 5 10
<210> 238
   <211> 12
   <212> PRT
   <213> Artificial Sequence
<220>
   <223> Description of Artificial Sequence: Synthetic peptide
<400> 238
   Lys Val Gly Leu Met Tyr Pro Pro Pro Tyr Tyr Leu
   1 5 10
<210> 239
   <211> 12
   <212> PRT
   <213> Artificial Sequence
<220>
   <223> Description of Artificial Sequence: Synthetic peptide
<400> 239
   Lys Val Ile Leu Met Tyr Pro Pro Pro Tyr Tyr Leu
   1 5 10
<210> 240
   <211> 12
   <212> PRT
   <213> Artificial Sequence
<220>
   <223> Description of Artificial Sequence: Synthetic peptide
<400> 240
   Lys Val Lys Leu Met Tyr Pro Pro Pro Tyr Tyr Leu
   1               5                   10
<210> 241
   <211> 12
   <212> PRT
   <213> Artificial Sequence
<220>
   <223> Description of Artificial Sequence: Synthetic peptide
<400> 241
   Lys Val Pro Leu Met Tyr Pro Pro Pro Tyr Tyr Leu
   1               5                   10
<210> 242
   <211> 12
   <212> PRT
   <213> Artificial Sequence
<220>
   <223> Description of Artificial Sequence: Synthetic peptide
<400> 242
   Lys Val Gln Leu Met Tyr Pro Pro Pro Tyr Tyr Leu
   1               5                   10
<210> 243
   <211> 12
   <212> PRT
   <213> Artificial Sequence
<220>
   <223> Description of Artificial Sequence: Synthetic peptide
<400> 243
   Lys Val Arg Leu Met Tyr Pro Pro Pro Tyr Tyr Leu
   1               5                   10
<210> 244
   <211> 12
   <212> PRT
   <213> Artificial Sequence
<220>
   <223> Description of Artificial Sequence: Synthetic peptide
<400> 244
   Lys Val Ser Leu Met Tyr Pro Pro Pro Tyr Tyr Leu
   1 5 10
<210> 245
   <211> 12
   <212> PRT
   <213> Artificial Sequence
<220>
   <223> Description of Artificial Sequence: Synthetic peptide
<400> 245
   Lys Val Thr Leu Met Tyr Pro Pro Pro Tyr Tyr Leu
   1 5 10
<210> 246
   <211> 12
   <212> PRT
   <213> Artificial Sequence
<220>
   <223> Description of Artificial Sequence: Synthetic peptide
<400> 246
   Lys Val Val Leu Met Tyr Pro Pro Pro Tyr Tyr Leu
   1 5 10
<210> 247
   <211> 12
   <212> PRT
   <213> Artificial Sequence
<220>
   <223> Description of Artificial Sequence: Synthetic peptide
<400> 247
   Lys Val Tyr Leu Met Tyr Pro Pro Pro Tyr Tyr Leu
   1 5 10
<210> 248
   <211> 12
   <212> PRT
   <213> Artificial Sequence
<220>
   <223> Description of Artificial Sequence: Synthetic peptide
<400> 248
   Lys Val Glu Ala Met Tyr Pro Pro Pro Tyr Tyr Leu
   1 5 10
<210> 249
   <211> 12
   <212> PRT
   <213> Artificial Sequence
<220>
   <223> Description of Artificial Sequence: Synthetic peptide
<400> 249
   Lys Val Glu Gly Met Tyr Pro Pro Pro Tyr Tyr Leu
   1 5 10
<210> 250
   <211> 12
   <212> PRT
   <213> Artificial Sequence
<220>
   <223> Description of Artificial Sequence: Synthetic peptide
<400> 250
   Lys Val Glu His Met Tyr Pro Pro Pro Tyr Tyr Leu
   1 5 10
<210> 251
   <211> 12
   <212> PRT
   <213> Artificial Sequence
<220>
   <223> Description of Artificial Sequence: Synthetic peptide
<400> 251
   Lys Val Glu Met Met Tyr Pro Pro Pro Tyr Tyr Leu
   1 5 10
<210> 252
   <211> 12
   <212> PRT
   <213> Artificial Sequence
<220>
   <223> Description of Artificial Sequence: Synthetic peptide
<400> 252
   Lys Val Glu Pro Met Tyr Pro Pro Pro Tyr Tyr Leu
   1 5 10
<210> 253
   <211> 12
   <212> PRT
   <213> Artificial Sequence
<220>
   <223> Description of Artificial Sequence: Synthetic peptide
<400> 253
   Lys Val Glu Gln Met Tyr Pro Pro Pro Tyr Tyr Leu
   1 5 10
<210> 254
   <211> 12
   <212> PRT
   <213> Artificial Sequence
<220>
   <223> Description of Artificial Sequence: Synthetic peptide
<400> 254
   Lys Val Glu Leu Asp Tyr Pro Pro Pro Tyr Tyr Leu
   1 5 10
<210> 255
   <211> 12
   <212> PRT
   <213> Artificial Sequence
<220>
   <223> Description of Artificial Sequence: Synthetic peptide
<400> 255
   Lys Val Glu Leu Gly Tyr Pro Pro Pro Tyr Tyr Leu
   1 5 10
<210> 256
   <211> 12
   <212> PRT
   <213> Artificial Sequence
<220>
   <223> Description of Artificial Sequence: Synthetic peptide
<400> 256
   Lys Val Glu Leu Ile Tyr Pro Pro Pro Tyr Tyr Leu
   1 5 10
<210> 257
   <211> 12
   <212> PRT
   <213> Artificial Sequence
<220>
   <223> Description of Artificial Sequence: Synthetic peptide
<400> 257
   Lys Val Glu Leu Asn Tyr Pro Pro Pro Tyr Tyr Leu
   1 5 10
<210> 258
   <211> 12
   <212> PRT
   <213> Artificial Sequence
<220>
   <223> Description of Artificial Sequence: Synthetic peptide
<400> 258
   Lys Val Glu Leu Ser Tyr Pro Pro Pro Tyr Tyr Leu
   1 5 10
<210> 259
   <211> 12
   <212> PRT
   <213> Artificial Sequence
<220>
   <223> Description of Artificial Sequence: Synthetic peptide
<400> 259
   Lys Val Glu Leu Val Tyr Pro Pro Pro Tyr Tyr Leu
   1 5 10
<210> 260
   <211> 12
   <212> PRT
   <213> Artificial Sequence
<220>
   <223> Description of Artificial Sequence: Synthetic peptide
<400> 260
   Lys Val Glu Leu Met Tyr Ile Pro Pro Tyr Tyr Leu
   1 5 10
<210> 261
   <211> 12
   <212> PRT
   <213> Artificial Sequence
<220>
   <223> Description of Artificial Sequence: Synthetic peptide
<400> 261
   Lys Val Glu Leu Met Tyr Met Pro Pro Tyr Tyr Leu
   1 5 10
<210> 262
   <211> 12
   <212> PRT
   <213> Artificial Sequence
<220>
   <223> Description of Artificial Sequence: Synthetic peptide
<400> 262
   Lys Val Glu Leu Met Tyr Trp Pro Pro Tyr Tyr Leu
   1 5 10
<210> 263
   <211> 12
   <212> PRT
   <213> Artificial Sequence
<220>
   <223> Description of Artificial Sequence: Synthetic peptide
<400> 263
   Lys Val Glu Leu Met Tyr Tyr Pro Pro Tyr Tyr Leu
   1 5 10
<210> 264
   <211> 12
   <212> PRT
   <213> Artificial Sequence
<220>
   <223> Description of Artificial Sequence: Synthetic peptide
<400> 264
   Lys Val Glu Leu Met Tyr Pro His Pro Tyr Tyr Leu
   1 5 10
<210> 265
   <211> 12
   <212> PRT
   <213> Artificial Sequence
<220>
   <223> Description of Artificial Sequence: Synthetic peptide
<400> 265
   Lys Val Glu Leu Met Tyr Pro Leu Pro Tyr Tyr Leu
   1 5 10
<210> 266
   <211> 12
   <212> PRT
   <213> Artificial Sequence
<220>
   <223> Description of Artificial Sequence: Synthetic peptide
<400> 266
   Lys Val Glu Leu Met Tyr Pro Thr Pro Tyr Tyr Leu
   1 5 10
<210> 267
   <211> 12
   <212> PRT
   <213> Artificial Sequence
<220>
   <223> Description of Artificial Sequence: Synthetic peptide
<400> 267
   Lys Val Glu Leu Met Tyr Pro Val Pro Tyr Tyr Leu
   1 5 10
<210> 268
   <211> 12
   <212> PRT
   <213> Artificial Sequence
<220>
   <223> Description of Artificial Sequence: Synthetic peptide
<400> 268
   Lys Val Glu Leu Met Tyr Pro Trp Pro Tyr Tyr Leu
   1 5 10
<210> 269
   <211> 12
   <212> PRT
   <213> Artificial Sequence
<220>
   <223> Description of Artificial Sequence: Synthetic peptide
<400> 269
   Lys Val Glu Leu Met Tyr Pro Pro Leu Tyr Tyr Leu
   1 5 10
<210> 270
   <211> 12
   <212> PRT
   <213> Artificial Sequence
<220>
   <223> Description of Artificial Sequence: Synthetic peptide
<400> 270
   Lys Val Glu Leu Met Tyr Pro Pro Pro His Tyr Leu
   1 5 10
<210> 271
   <211> 12
   <212> PRT
   <213> Artificial Sequence
<220>
   <223> Description of Artificial Sequence: Synthetic peptide
<400> 271
   Lys Val Glu Leu Met Tyr Pro Pro Pro Tyr His Leu
   1 5 10
<210> 272
   <211> 12
   <212> PRT
   <213> Artificial Sequence
<220>
   <223> Description of Artificial Sequence: Synthetic peptide
<400> 272
   Lys Val Glu Leu Met Tyr Pro Pro Pro Tyr Leu Leu
   1 5 10
<210> 273
   <211> 12
   <212> PRT
   <213> Artificial Sequence
<220>
   <223> Description of Artificial Sequence: Synthetic peptide
<400> 273
   Lys Val Glu Leu Met Tyr Pro Pro Pro Tyr Pro Leu
   1 5 10
<210> 274
   <211> 12
   <212> PRT
   <213> Artificial Sequence
<220>
   <223> Description of Artificial Sequence: Synthetic peptide
<400> 274
   Lys Val Glu Leu Met Tyr Pro Pro Pro Tyr Thr Leu
   1 5 10
<210> 275
   <211> 12
   <212> PRT
   <213> Artificial Sequence
<220>
   <223> Description of Artificial Sequence: Synthetic peptide
<400> 275
   Lys Val Glu Leu Met Tyr Pro Pro Pro Tyr Tyr Pro
   1               5                   10
<210> 276
   <211> 12
   <212> PRT
   <213> Artificial Sequence
<220>
   <223> Description of Artificial Sequence: Synthetic peptide
<400> 276
   Lys Val Glu Leu Met Tyr Pro Pro Pro Tyr Tyr Glu
   1               5                   10
<210> 277
   <211> 8
   <212> PRT
   <213> Artificial Sequence
<220>
   <223> Description of Artificial Sequence: Synthetic peptide
<400> 277
   Pro His Lys Ala Thr Glu Val Arg
   1               5
<210> 278
   <211> 8
   <212> PRT
   <213> Artificial Sequence
<220>
   <223> Description of Artificial Sequence: Synthetic peptide
<400> 278
   Pro Thr Lys Ala Thr Glu Val Arg
   1               5
<210> 279
   <211> 8
   <212> PRT
   <213> Artificial Sequence
<220>
   <223> Description of Artificial Sequence: Synthetic peptide
<400> 279
   Pro Gly Gly Ala Thr Glu Val Arg
   1 5
<210> 280
   <211> 8
   <212> PRT
   <213> Artificial Sequence
<220>
   <223> Description of Artificial Sequence: Synthetic peptide
<400> 280
   Pro Gly Asn Ala Thr Glu Val Arg
   1 5
<210> 281
   <211> 8
   <212> PRT
   <213> Artificial Sequence
<220>
   <223> Description of Artificial Sequence: Synthetic peptide
<400> 281
   Pro Gly Ser Ala Thr Glu Val Arg
   1 5
<210> 282
   <211> 13
   <212> PRT
   <213> Artificial Sequence
<220>
   <223> Description of Artificial Sequence: Synthetic peptide
<400> 282
   Thr Tyr Met Lys Gly Asn Glu Leu Thr Phe Leu Asp Asp
   1 5 10
<210> 283
   <211> 13
   <212> PRT
   <213> Artificial Sequence
<220>
   <223> Description of Artificial Sequence: Synthetic peptide
<400> 283
   Thr Tyr Met Met Gly Asn Glu Leu Thr Phe Pro Asp Asp
   1 5 10
<210> 284
   <211> 8
   <212> PRT
   <213> Artificial Sequence
<220>
   <223> Description of Artificial Sequence: Synthetic peptide
<400> 284
   Pro Ser Lys Ala Thr Glu Val Arg
   1               5
<210> 285
   <211> 8
   <212> PRT
   <213> Artificial Sequence
<220>
   <223> Description of Artificial Sequence: Synthetic peptide
<400> 285
   Pro Gly Lys Thr Thr Glu Val Arg
   1               5
<210> 286
   <211> 8
   <212> PRT
   <213> Artificial Sequence
<220>
   <223> Description of Artificial Sequence: Synthetic peptide
<400> 286
   Pro Gly Lys Trp Thr Glu Val Arg
   1               5
<210> 287
   <211> 8
   <212> PRT
   <213> Artificial Sequence
<220>
   <223> Description of Artificial Sequence: Synthetic peptide
<400> 287
   Pro Gly Lys Tyr Thr Glu Val Arg
   1               5
<210> 288
   <211> 13
   <212> PRT
   <213> Artificial Sequence
<220>
   <223> Description of Artificial Sequence: Synthetic peptide
<400> 288
   Ser Tyr Met Met Gly Asn Glu Leu Thr Phe Leu Asp Asp
   1 5 10
<210> 289
   <211> 8
   <212> PRT
   <213> Artificial Sequence
<220>
   <223> Description of Artificial Sequence: Synthetic peptide
<400> 289
   Pro Gly Lys Ala Gly Glu Val Arg
   1 5
<210> 290
   <211> 8
   <212> PRT
   <213> Artificial Sequence
<220>
   <223> Description of Artificial Sequence: Synthetic peptide
<400> 290
   His Gly Lys Ala Thr Glu Val Arg
   1 5
<210> 291
   <211> 8
   <212> PRT
   <213> Artificial Sequence
<220>
   <223> Description of Artificial Sequence: Synthetic peptide
<400> 291
   Lys Gly Lys Ala Thr Glu Val Arg
   1 5
<210> 292
   <211> 8
   <212> PRT
   <213> Artificial Sequence
<220>
   <223> Description of Artificial Sequence: Synthetic peptide
<400> 292
   Ser Gly Lys Ala Thr Glu Val Arg
   1 5
<210> 293
   <211> 8
   <212> PRT
   <213> Artificial Sequence
<220>
   <223> Description of Artificial Sequence: Synthetic peptide
<400> 293
   Pro Gly Lys Gly Thr Glu Val Arg
   1 5
<210> 294
   <211> 8
   <212> PRT
   <213> Artificial Sequence
<220>
   <223> Description of Artificial Sequence: Synthetic peptide
<400> 294
   Pro Gly Lys Val Thr Glu Val Arg
   1 5
<210> 295
   <211> 8
   <212> PRT
   <213> Artificial Sequence
<220>
   <223> Description of Artificial Sequence: Synthetic peptide
<400> 295
   Pro Gly Lys Ala Ala Glu Val Arg
   1 5
<210> 296
   <211> 8
   <212> PRT
   <213> Artificial Sequence
<220>
   <223> Description of Artificial Sequence: Synthetic peptide
<400> 296
   Pro Gly Lys Ala Asp Glu Val Arg
   1 5
<210> 297
   <211> 8
   <212> PRT
   <213> Artificial Sequence
<220>
   <223> Description of Artificial Sequence: Synthetic peptide
<400> 297
   Pro Gly Lys Ala Asn Glu Val Arg
   1 5
<210> 298
   <211> 8
   <212> PRT
   <213> Artificial Sequence
<220>
   <223> Description of Artificial Sequence: Synthetic peptide
<400> 298
   Pro Gly Lys Ala Gln Glu Val Arg
   1 5
<210> 299
   <211> 8
   <212> PRT
   <213> Artificial Sequence
<220>
   <223> Description of Artificial Sequence: Synthetic peptide
<400> 299
   Pro Gly Lys Ala Tyr Glu Val Arg
   1 5
<210> 300
   <211> 13
   <212> PRT
   <213> Artificial Sequence
<220>
   <223> Description of Artificial Sequence: Synthetic peptide
<400> 300
   Thr Tyr Val Met Gly Asn Glu Leu Thr Phe Leu Asp Asp
   1 5 10
<210> 301
   <211> 13
   <212> PRT
   <213> Artificial Sequence
<220>
   <223> Description of Artificial Sequence: Synthetic peptide
<400> 301
   Thr Tyr Ile Met Gly Asn Glu Leu Thr Phe Leu Asp Asp
   1 5 10
<210> 302
   <211> 13
   <212> PRT
   <213> Artificial Sequence
<220>
   <223> Description of Artificial Sequence: Synthetic peptide
<400> 302
   Thr Tyr Met Val Gly Asn Glu Leu Thr Phe Leu Asp Asp
   1 5 10
<210> 303
   <211> 13
   <212> PRT
   <213> Artificial Sequence
<220>
   <223> Description of Artificial Sequence: Synthetic peptide
<400> 303
   Thr Tyr Met Met Glu Asn Glu Leu Thr Phe Leu Asp Asp
   1 5 10
<210> 304
   <211> 13
   <212> PRT
   <213> Artificial Sequence
<220>
   <223> Description of Artificial Sequence: Synthetic peptide
<400> 304
   Thr Tyr Met Met Gly Asp Glu Leu Thr Phe Leu Asp Asp
   1 5 10
<210> 305
   <211> 13
   <212> PRT
   <213> Artificial Sequence
<220>
   <223> Description of Artificial Sequence: Synthetic peptide
<400> 305
   Thr Tyr Met Met Gly Asn Glu Ser Thr Phe Leu Asp Asp
   1 5 10
<210> 306
   <211> 13
   <212> PRT
   <213> Artificial Sequence
<220>
   <223> Description of Artificial Sequence: Synthetic peptide
<400> 306
   Thr Tyr Met Met Gly Asn Glu Leu Ala Phe Leu Asp Asp
   1               5                   10
<210> 307
   <211> 13
   <212> PRT
   <213> Artificial Sequence
<220>
   <223> Description of Artificial Sequence: Synthetic peptide
<400> 307
   Thr Tyr Met Met Gly Asn Glu Leu Thr Phe Leu Asn Asp
   1               5                   10
<210> 308
   <211> 12
   <212> PRT
   <213> Artificial Sequence
<220>
   <223> Description of Artificial Sequence: Synthetic peptide
<400> 308
   Glu Val Glu Leu Met Tyr Pro Pro Pro Tyr Tyr Leu
   1               5                   10
<210> 309
   <211> 12
   <212> PRT
   <213> Artificial Sequence
<220>
   <223> Description of Artificial Sequence: Synthetic peptide
<400> 309
   Lys Val Glu Leu Met Ile Pro Pro Pro Tyr Tyr Leu
   1               5                   10
<210> 310
   <211> 12
   <212> PRT
   <213> Artificial Sequence
<220>
   <223> Description of Artificial Sequence: Synthetic peptide
<400> 310
   Lys Val Glu Leu Met Tyr Pro Pro Pro Tyr Tyr Gln
   1 5 10
<210> 311
   <211> 12
   <212> PRT
   <213> Artificial Sequence
<220>
   <223> Description of Artificial Sequence: Synthetic peptide
<400> 311
   Lys Val Glu Leu Met Tyr Pro Pro Pro Tyr Tyr Trp
   1 5 10
<210> 312
   <211> 8
   <212> PRT
   <213> Artificial Sequence
<220>
   <223> Description of Artificial Sequence: Synthetic peptide
<400> 312
   Ala Gly Lys Ala Thr Glu Val Arg
   1 5
<210> 313
   <211> 8
   <212> PRT
   <213> Artificial Sequence
<220>
   <223> Description of Artificial Sequence: Synthetic peptide
<400> 313
   Pro Val Lys Ala Thr Glu Val Arg
   1 5
<210> 314
   <211> 8
   <212> PRT
   <213> Artificial Sequence
<220>
   <223> Description of Artificial Sequence: Synthetic peptide
<400> 314
   Pro Gly Ala Ala Thr Glu Val Arg
   1 5
<210> 315
   <211> 8
   <212> PRT
   <213> Artificial Sequence
<220>
   <223> Description of Artificial Sequence: Synthetic peptide
<400> 315
   Pro Gly Asp Ala Thr Glu Val Arg
   1 5
<210> 316
   <211> 8
   <212> PRT
   <213> Artificial Sequence
<220>
   <223> Description of Artificial Sequence: Synthetic peptide
<400> 316
   Pro Gly Lys Ala Thr Glu Ile Arg
   1 5
<210> 317
   <211> 8
   <212> PRT
   <213> Artificial Sequence
<220>
   <223> Description of Artificial Sequence: Synthetic peptide
<400> 317
   Pro Arg Lys Ala Thr Glu Val Arg
   1 5
<210> 318
   <211> 8
   <212> PRT
   <213> Artificial Sequence
<220>
   <223> Description of Artificial Sequence: Synthetic peptide
<400> 318
   Pro Gly Lys Gln Thr Glu Val Arg
   1 5
<210> 319
   <211> 8
   <212> PRT
   <213> Artificial Sequence
<220>
   <223> Description of Artificial Sequence: Synthetic peptide
<400> 319
   Pro Gly Lys Ala Arg Glu Val Arg
   1 5
<210> 320
   <211> 13
   <212> PRT
   <213> Artificial Sequence
<220>
   <223> Description of Artificial Sequence: Synthetic peptide
<400> 320
   Thr Tyr Met Met Gly Asn Glu Leu Thr Phe Leu Asp Lys
   1 5 10
<210> 321
   <211> 12
   <212> PRT
   <213> Artificial Sequence
<220>
   <223> Description of Artificial Sequence: Synthetic peptide
<400> 321
   Ser Val Glu Leu Met Tyr Pro Pro Pro Tyr Tyr Leu
   1 5 10
<210> 322
   <211> 12
   <212> PRT
   <213> Artificial Sequence
<220>
   <223> Description of Artificial Sequence: Synthetic peptide
<400> 322
   Lys Val Glu Leu Met Pro Pro Pro Pro Tyr Tyr Leu
   1 5 10
<210> 323
   <211> 12
   <212> PRT
   <213> Artificial Sequence
<220>
   <223> Description of Artificial Sequence: Synthetic peptide
<400> 323
   Lys Val Glu Leu Met Tyr Pro Ala Pro Tyr Tyr Leu
   1               5                   10
<210> 324
   <211> 12
   <212> PRT
   <213> Artificial Sequence
<220>
   <223> Description of Artificial Sequence: Synthetic peptide
<400> 324
   Lys Val Glu Leu Met Tyr Pro Pro Pro Tyr Tyr Asp
   1               5                   10
<210> 325
   <211> 12
   <212> PRT
   <213> Artificial Sequence
<220>
   <223> Description of Artificial Sequence: Synthetic peptide
<400> 325
   Lys Val Glu Leu Met Tyr Pro Pro Pro Tyr Tyr Ser
   1               5                   10
<210> 326
   <211> 12
   <212> PRT
   <213> Artificial Sequence
<220>
   <223> Description of Artificial Sequence: Synthetic peptide
<400> 326
   Lys Val Glu Leu Met Tyr Pro Pro Pro Tyr Tyr Val
   1               5                   10
<210> 327
   <211> 8
   <212> PRT
   <213> Artificial Sequence
<220>
   <223> Description of Artificial Sequence: Synthetic peptide
<400> 327
   Pro Gly Lys Ala Thr Ser Val Arg
   1 5
<210> 328
   <211> 8
   <212> PRT
   <213> Artificial Sequence
<220>
   <223> Description of Artificial Sequence: Synthetic peptide
<400> 328
   Pro Gly Lys Ala Thr Glu Val Ser
   1 5
<210> 329
   <211> 8
   <212> PRT
   <213> Artificial Sequence
<220>
   <223> Description of Artificial Sequence: Synthetic peptide
<400> 329
   Pro Gly Ala Ala Thr Ala Val Ala
   1 5
<210> 330
   <211> 8
   <212> PRT
   <213> Artificial Sequence
<220>
   <223> Description of Artificial Sequence: Synthetic peptide
<400> 330
   Pro Gly Lys Tyr Thr Glu Val Arg
   1 5
<210> 331
   <211> 13
   <212> PRT
   <213> Artificial Sequence
<220>
   <223> Description of Artificial Sequence: Synthetic peptide
<400> 331
   Ser Tyr Met Met Gly Asn Glu Leu Thr Phe Leu Asp Asp
   1 5 10
<210> 332
   <211> 13
   <212> PRT
   <213> Artificial Sequence
<220>
   <223> Description of Artificial Sequence: Synthetic peptide
<400> 332
   Thr Tyr Ile Met Gly Asn Glu Leu Thr Phe Leu Asp Asp
   1 5 10
<210> 333
   <211> 12
   <212> PRT
   <213> Artificial Sequence
<220>
   <223> Description of Artificial Sequence: Synthetic peptide
<400> 333
   Ser Val Glu Leu Met Tyr Pro Pro Pro Tyr Tyr Leu
   1 5 10
<210> 334
   <211> 12
   <212> PRT
   <213> Artificial Sequence
<220>
   <223> Description of Artificial Sequence: Synthetic peptide
<400> 334
   Lys Val Glu Leu Met Pro Pro Pro Pro Tyr Tyr Leu
   1 5 10
<210> 335
   <211> 12
   <212> PRT
   <213> Artificial Sequence
<220>
   <223> Description of Artificial Sequence: Synthetic peptide
<400> 335
   Lys Val Glu Leu Met Tyr Pro Pro Pro Pro Tyr Leu
   1 5 10
<210> 336
   <211> 12
   <212> PRT
   <213> Artificial Sequence
<220>
   <223> Description of Artificial Sequence: Synthetic peptide
<400> 336
   Ala Val Ala Leu Met Tyr Pro Pro Pro Tyr Tyr Leu
   1 5 10
<210> 337
   <211> 12
   <212> PRT
   <213> Artificial Sequence
<220>
   <223> Description of Artificial Sequence: Synthetic peptide
<400> 337
   Lys Val Glu Leu Ala Tyr Pro Pro Pro Tyr Tyr Leu
   1 5 10
<210> 338
   <211> 12
   <212> PRT
   <213> Artificial Sequence
<220>
   <223> Description of Artificial Sequence: Synthetic peptide
<400> 338
   Lys Val Glu Leu Met Ala Pro Pro Pro Tyr Tyr Leu
   1 5 10
<210> 339
   <211> 12
   <212> PRT
   <213> Artificial Sequence
<220>
   <223> Description of Artificial Sequence: Synthetic peptide
<400> 339
   Lys Val Glu Leu Met Tyr Ala Pro Pro Tyr Tyr Leu
   1 5 10
<210> 340
   <211> 12
   <212> PRT
   <213> Artificial Sequence
<220>
   <223> Description of Artificial Sequence: Synthetic peptide
<400> 340
   Lys Val Glu Leu Met Tyr Pro Ala Pro Tyr Tyr Leu
   1 5 10
<210> 341
   <211> 12
   <212> PRT
   <213> Artificial Sequence
<220>
   <223> Description of Artificial Sequence: Synthetic peptide
<400> 341
   Lys Val Glu Leu Met Tyr Pro Pro Ala Tyr Tyr Leu
   1 5 10
<210> 342
   <211> 12
   <212> PRT
   <213> Artificial Sequence
<220>
   <223> Description of Artificial Sequence: Synthetic peptide
<400> 342
   Lys Val Glu Leu Met Tyr Pro Pro Pro Ala Tyr Leu
   1 5 10
<210> 343
   <211> 12
   <212> PRT
   <213> Artificial Sequence
<220>
   <223> Description of Artificial Sequence: Synthetic peptide
<400> 343
   Lys Val Glu Leu Ala Ala Ala Ala Ala Ala Tyr Leu
   1 5 10
<210> 344
   <211> 12
   <212> PRT
   <213> Artificial Sequence
<220>
   <223> Description of Artificial Sequence: Synthetic peptide
<400> 344
   Lys Val Glu Leu Met Tyr Pro Pro Pro Tyr Tyr Glu
   1 5 10
<210> 345
   <211> 12
   <212> PRT
   <213> Artificial Sequence
<220>
   <223> Description of Artificial Sequence: Synthetic peptide
<400> 345
   Lys Val Glu Leu Met Phe Pro Pro Pro Tyr Tyr Leu
   1 5 10
<210> 346
   <211> 8
   <212> PRT
   <213> Artificial Sequence
<220>
   <223> Description of Artificial Sequence: Synthetic peptide
<400> 346
   Pro Gly Lys Ala Gly Glu Val Arg
   1 5
<210> 347
   <211> 8
   <212> PRT
   <213> Artificial Sequence
<220>
   <223> Description of Artificial Sequence: Synthetic peptide
<400> 347
   Pro Gly Lys Ala Asn Glu Val Arg
   1 5
<210> 348
   <211> 8
   <212> PRT
   <213> Artificial Sequence
<220>
   <223> Description of Artificial Sequence: Synthetic peptide
<400> 348
   Pro Gly Lys Tyr Thr Glu Val Arg
   1 5
<210> 349
   <211> 8
   <212> PRT
   <213> Artificial Sequence
<220>
   <223> Description of Artificial Sequence: Synthetic peptide
<400> 349
   Pro Gly Lys Leu Thr Glu Val Arg
   1 5
<210> 350
   <211> 8
   <212> PRT
   <213> Artificial Sequence
<220>
   <223> Description of Artificial Sequence: Synthetic peptide
<400> 350
   Pro Gly Lys Trp Thr Glu Val Arg
   1 5
<210> 351
   <211> 8
   <212> PRT
   <213> Artificial Sequence
<220>
   <223> Description of Artificial Sequence: Synthetic peptide
<400> 351
   Pro Gly Lys Thr Thr Glu Val Arg
   1 5
<210> 352
   <211> 8
   <212> PRT
   <213> Artificial Sequence
<220>
   <223> Description of Artificial Sequence: Synthetic peptide
<400> 352
   Lys Arg Gly Val Gln Glu Val Arg
   1 5
<210> 353
   <211> 8
   <212> PRT
   <213> Artificial Sequence
<220>
   <223> Description of Artificial Sequence: Synthetic peptide
<400> 353
   Tyr Thr Gly Tyr Gly Glu Val Arg
   1 5
<210> 354
   <211> 8
   <212> PRT
   <213> Artificial Sequence
<220>
   <223> Description of Artificial Sequence: Synthetic peptide
<400> 354
   Tyr Arg Asn Gln Gln Glu Val Arg
   1 5
<210> 355
   <211> 12
   <212> PRT
   <213> Artificial Sequence
<220>
   <223> Description of Artificial Sequence: Synthetic peptide
<400> 355
   Lys Val Glu Leu Met Tyr Pro Pro Pro Tyr Tyr Glu
   1 5 10
<210> 356
   <211> 12
   <212> PRT
   <213> Artificial Sequence
<220>
   <223> Description of Artificial Sequence: Synthetic peptide
<400> 356
   Lys Val Glu Leu Met Tyr Pro Pro Pro Tyr Tyr Ser
   1 5 10
<210> 357
   <211> 12
   <212> PRT
   <213> Artificial Sequence
<220>
   <223> Description of Artificial Sequence: Synthetic peptide
<400> 357
   Lys Val Glu Leu Met Tyr Pro Pro Pro Tyr Tyr Thr
   1 5 10
<210> 358
   <211> 12
   <212> PRT
   <213> Artificial Sequence
<220>
   <223> Description of Artificial Sequence: Synthetic peptide
<400> 358
   Lys Val Glu Leu Met Tyr Pro Pro Pro Tyr Tyr Ala
   1 5 10
<210> 359
   <211> 12
   <212> PRT
   <213> Artificial Sequence
<220>
   <223> Description of Artificial Sequence: Synthetic peptide
<400> 359
   Lys Val Glu Leu Met Tyr Pro Pro Pro Tyr Tyr Trp
   1 5 10
<210> 360
   <211> 12
   <212> PRT
   <213> Artificial Sequence
<220>
   <223> Description of Artificial Sequence: Synthetic peptide
<400> 360
   Lys Val Glu Leu Met Tyr Pro Pro Pro Tyr Tyr Gln
   1 5 10
<210> 361
   <211> 12
   <212> PRT
   <213> Artificial Sequence
<220>
   <223> Description of Artificial Sequence: Synthetic peptide
<400> 361
   Lys Val Glu Leu Met Tyr Pro Pro Pro Tyr Tyr Lys
   1 5 10
<210> 362
   <211> 12
   <212> PRT
   <213> Artificial Sequence
<220>
   <223> Description of Artificial Sequence: Synthetic peptide
<400> 362
   Lys Val Glu Leu Met Tyr Pro Pro Pro Tyr Tyr Arg
   1 5 10
<210> 363
   <211> 12
   <212> PRT
   <213> Artificial Sequence
<220>
   <223> Description of Artificial Sequence: Synthetic peptide
<400> 363
   Lys Val Glu Leu Met Tyr Pro Pro Pro Tyr Tyr Gly
   1 5 10
<210> 364
   <211> 12
   <212> PRT
   <213> Artificial Sequence
<220>
   <223> Description of Artificial Sequence: Synthetic peptide
<400> 364
   Lys Val Glu Leu Met Tyr Pro Pro Pro Tyr Tyr Asp
   1 5 10
<210> 365
   <211> 12
   <212> PRT
   <213> Artificial Sequence
<220>
   <223> Description of Artificial Sequence: Synthetic peptide
<400> 365
   Lys Val Glu Leu Met Tyr Pro Pro Pro Tyr Tyr Glu
   1 5 10
<210> 366
   <211> 8
   <212> PRT
   <213> Artificial Sequence
<220>
   <223> Description of Artificial Sequence: Synthetic peptide
<400> 366
   Pro Tyr Asp Gly Gln Glu Val Arg
   1 5
<210> 367
   <211> 8
   <212> PRT
   <213> Artificial Sequence
<220>
   <223> Description of Artificial Sequence: Synthetic peptide
<400> 367
   His Val Ser Arg Arg Glu Val Arg
   1 5
<210> 368
   <211> 12
   <212> PRT
   <213> Artificial Sequence
<220>
   <223> Description of Artificial Sequence: Synthetic peptide
<400> 368
   Lys Val Glu Leu Ala Tyr Pro Pro Pro Tyr Tyr Leu
   1               5                   10
<210> 369
   <211> 12
   <212> PRT
   <213> Artificial Sequence
<220>
   <223> Description of Artificial Sequence: Synthetic peptide
<400> 369
   Lys Val Glu Leu Met Ala Pro Pro Pro Tyr Tyr Leu
   1               5                   10
<210> 370
   <211> 12
   <212> PRT
   <213> Artificial Sequence
<220>
   <223> Description of Artificial Sequence: Synthetic peptide
<400> 370
   Lys Val Glu Leu Met Tyr Ala Pro Pro Tyr Tyr Leu
   1               5                   10
<210> 371
   <211> 12
   <212> PRT
   <213> Artificial Sequence
<220>
   <223> Description of Artificial Sequence: Synthetic peptide
<400> 371
   Lys Val Glu Leu Met Tyr Pro Ala Pro Tyr Tyr Leu
   1               5                   10
<210> 372
   <211> 12
   <212> PRT
   <213> Artificial Sequence
<220>
   <223> Description of Artificial Sequence: Synthetic peptide
<400> 372
   Lys Val Glu Leu Met Tyr Pro Pro Ala Tyr Tyr Leu
   1 5 10
<210> 373
   <211> 12
   <212> PRT
   <213> Artificial Sequence
<220>
   <223> Description of Artificial Sequence: Synthetic peptide
<400> 373
   Lys Val Glu Leu Met Tyr Pro Pro Pro Ala Tyr Leu
   1 5 10
<210> 374
   <211> 12
   <212> PRT
   <213> Artificial Sequence
<220>
   <223> Description of Artificial Sequence: Synthetic peptide
<400> 374
   Lys Val Glu Leu Met Asp Pro Pro Pro Tyr Tyr Leu
   1 5 10
<210> 375
   <211> 12
   <212> PRT
   <213> Artificial Sequence
<220>
   <223> Description of Artificial Sequence: Synthetic peptide
<400> 375
   Lys Val Glu Leu Met Glu Pro Pro Pro Tyr Tyr Leu
   1 5 10
<210> 376
   <211> 12
   <212> PRT
   <213> Artificial Sequence
<220>
   <223> Description of Artificial Sequence: Synthetic peptide
<400> 376
   Lys Val Glu Leu Met Phe Pro Pro Pro Tyr Tyr Leu
   1 5 10
<210> 377
   <211> 12
   <212> PRT
   <213> Artificial Sequence
<220>
   <223> Description of Artificial Sequence: Synthetic peptide
<400> 377
   Lys Val Glu Leu Met Gly Pro Pro Pro Tyr Tyr Leu
   1 5 10
<210> 378
   <211> 12
   <212> PRT
   <213> Artificial Sequence
<220>
   <223> Description of Artificial Sequence: Synthetic peptide
<400> 378
   Lys Val Glu Leu Met His Pro Pro Pro Tyr Tyr Leu
   1 5 10
<210> 379
   <211> 12
   <212> PRT
   <213> Artificial Sequence
<220>
   <223> Description of Artificial Sequence: Synthetic peptide
<400> 379
   Lys Val Glu Leu Met Ile Pro Pro Pro Tyr Tyr Leu
   1 5 10
<210> 380
   <211> 12
   <212> PRT
   <213> Artificial Sequence
<220>
   <223> Description of Artificial Sequence: Synthetic peptide
<400> 380
   Lys Val Glu Leu Met Lys Pro Pro Pro Tyr Tyr Leu
   1 5 10
<210> 381
   <211> 12
   <212> PRT
   <213> Artificial Sequence
<220>
   <223> Description of Artificial Sequence: Synthetic peptide
<400> 381
   Lys Val Glu Leu Met Leu Pro Pro Pro Tyr Tyr Leu
   1 5 10
<210> 382
   <211> 12
   <212> PRT
   <213> Artificial Sequence
<220>
   <223> Description of Artificial Sequence: Synthetic peptide
<400> 382
   Lys Val Glu Leu Met Met Pro Pro Pro Tyr Tyr Leu
   1 5 10
<210> 383
   <211> 12
   <212> PRT
   <213> Artificial Sequence
<220>
   <223> Description of Artificial Sequence: Synthetic peptide
<400> 383
   Lys Val Glu Leu Met Asn Pro Pro Pro Tyr Tyr Leu
   1 5 10
<210> 384
   <211> 12
   <212> PRT
   <213> Artificial Sequence
<220>
   <223> Description of Artificial Sequence: Synthetic peptide
<400> 384
   Lys Val Glu Leu Met Pro Pro Pro Pro Tyr Tyr Leu
   1 5 10
<210> 385
   <211> 8
   <212> PRT
   <213> Artificial Sequence
<220>
   <223> Description of Artificial Sequence: Synthetic peptide
<400> 385
   Pro Gly Lys Tyr Thr Glu Val Arg
   1 5
<210> 386
   <211> 8
   <212> PRT
   <213> Artificial Sequence
<220>
   <223> Description of Artificial Sequence: Synthetic peptide
<400> 386
   Ala Gly Lys Ala Ala Glu Val Arg
   1 5
<210> 387
   <211> 8
   <212> PRT
   <213> Artificial Sequence
<220>
   <223> Description of Artificial Sequence: Synthetic peptide
<400> 387
   Ser Gly Lys Ala Asp Glu Val Arg
   1 5
<210> 388
   <211> 8
   <212> PRT
   <213> Artificial Sequence
<220>
   <223> Description of Artificial Sequence: Synthetic peptide
<400> 388
   Ser Gly Asn Ala Ala Lys Phe Arg
   1 5
<210> 389
   <211> 8
   <212> PRT
   <213> Artificial Sequence
<220>
   <223> Description of Artificial Sequence: Synthetic peptide
<400> 389
   Pro Gly Lys Ala Tyr Glu Val Arg
   1 5
<210> 390
   <211> 8
   <212> PRT
   <213> Artificial Sequence
<220>
   <223> Description of Artificial Sequence: Synthetic peptide
<400> 390
   Ser Gly Asn Ala Ala Glu Val Arg
   1 5
<210> 391
   <211> 8
   <212> PRT
   <213> Artificial Sequence
<220>
   <223> Description of Artificial Sequence: Synthetic peptide
<400> 391
   Pro Ser Lys Ala Thr Glu Val Arg
   1 5
<210> 392
   <211> 13
   <212> PRT
   <213> Artificial Sequence
<220>
   <223> Description of Artificial Sequence: Synthetic peptide
<400> 392
   Thr Tyr Met Thr Gly Asn Glu Leu Thr Phe Leu Asp Asp
   1 5 10
<210> 393
   <211> 13
   <212> PRT
   <213> Artificial Sequence
<220>
   <223> Description of Artificial Sequence: Synthetic peptide
<400> 393
   Thr Tyr Thr Val Glu Asp Glu Leu Thr Phe Leu Asp Asp
   1 5 10
<210> 394
   <211> 13
   <212> PRT
   <213> Artificial Sequence
<220>
   <223> Description of Artificial Sequence: Synthetic peptide
<400> 394
   Thr Tyr Met Val Glu Asp Glu Leu Thr Phe Leu Asp Asp
   1 5 10
<210> 395
   <211> 13
   <212> PRT
   <213> Artificial Sequence
<220>
   <223> Description of Artificial Sequence: Synthetic peptide
<400> 395
   Thr Tyr Thr Val Glu Asn Glu Leu Thr Ala Leu Asn Asp
   1 5 10
<210> 396
   <211> 13
   <212> PRT
   <213> Artificial Sequence
<220>
   <223> Description of Artificial Sequence: Synthetic peptide
<400> 396
   Thr Tyr Met Thr Gly Asn Glu Leu Thr Phe Leu Asp Asp
   1 5 10
<210> 397
   <211> 13
   <212> PRT
   <213> Artificial Sequence
<220>
   <223> Description of Artificial Sequence: Synthetic peptide
<400> 397
   Thr Tyr Thr Val Glu Asp Glu Leu Ala Phe Leu Asp Asp
   1 5 10
<210> 398
   <211> 13
   <212> PRT
   <213> Artificial Sequence
<220>
   <223> Description of Artificial Sequence: Synthetic peptide
<400> 398
   Thr Tyr Thr Val Glu Asn Glu Leu Ala Phe Leu Asp Asp
   1 5 10
<210> 399
   <211> 13
   <212> PRT
   <213> Artificial Sequence
<220>
   <223> Description of Artificial Sequence: Synthetic peptide
<400> 399
   Thr Tyr Val Met Gly Asn Glu Ser Thr Phe Leu Asp Asp
   1 5 10
<210> 400
   <211> 13
   <212> PRT
   <213> Artificial Sequence
<220>
   <223> Description of Artificial Sequence: Synthetic peptide
<400> 400
   Thr Tyr Met Met Gly Asn Glu Ser Thr Phe Leu Asp Asp
   1 5 10
<210> 401
   <211> 12
   <212> PRT
   <213> Artificial Sequence
<220>
   <223> Description of Artificial Sequence: Synthetic peptide
<400> 401
   Lys Val Glu Leu Met Gln Pro Pro Pro Tyr Tyr Leu
   1 5 10
<210> 402
   <211> 12
   <212> PRT
   <213> Artificial Sequence
<220>
   <223> Description of Artificial Sequence: Synthetic peptide
<400> 402
   Lys Val Glu Leu Met Arg Pro Pro Pro Tyr Tyr Leu
   1 5 10
<210> 403
   <211> 12
   <212> PRT
   <213> Artificial Sequence
<220>
   <223> Description of Artificial Sequence: Synthetic peptide
<400> 403
   Lys Val Glu Leu Met Ser Pro Pro Pro Tyr Tyr Leu
   1               5                   10
<210> 404
   <211> 12
   <212> PRT
   <213> Artificial Sequence
<220>
   <223> Description of Artificial Sequence: Synthetic peptide
<400> 404
   Lys Val Glu Leu Met Thr Pro Pro Pro Tyr Tyr Leu
   1               5                   10
<210> 405
   <211> 12
   <212> PRT
   <213> Artificial Sequence
<220>
   <223> Description of Artificial Sequence: Synthetic peptide
<400> 405
   Lys Val Glu Leu Met Val Pro Pro Pro Tyr Tyr Leu
   1               5                   10
<210> 406
   <211> 12
   <212> PRT
   <213> Artificial Sequence
<220>
   <223> Description of Artificial Sequence: Synthetic peptide
<400> 406
   Lys Val Glu Leu Met Trp Pro Pro Pro Tyr Tyr Leu
   1               5                   10
<210> 407
   <211> 12
   <212> PRT
   <213> Artificial Sequence
<220>
   <223> Description of Artificial Sequence: Synthetic peptide
<400> 407
   Lys Val Glu Leu Leu Tyr Pro Pro Pro Tyr Tyr Val
   1 5 10
<210> 408
   <211> 12
   <212> PRT
   <213> Artificial Sequence
<220>
   <223> Description of Artificial Sequence: Synthetic peptide
<400> 408
   Lys Val Glu Leu Met Tyr Pro Pro Pro Tyr Tyr Val
   1 5 10
<210> 409
   <211> 12
   <212> PRT
   <213> Artificial Sequence
<220>
   <223> Description of Artificial Sequence: Synthetic peptide
<400> 409
   Lys Val Glu Leu Met Tyr Pro Pro Pro Tyr Tyr Ala
   1 5 10
<210> 410
   <211> 12
   <212> PRT
   <213> Artificial Sequence
<220>
   <223> Description of Artificial Sequence: Synthetic peptide
<400> 410
   Glu Val Glu Leu Met Tyr Pro Pro Pro Tyr Tyr Leu
   1 5 10
<210> 411
   <211> 8
   <212> PRT
   <213> Artificial Sequence
<220>
   <223> Description of Artificial Sequence: Synthetic peptide
<400> 411
   Pro Gly Asn Ala Thr Glu Val Arg
   1 5
<210> 412
   <211> 8
   <212> PRT
   <213> Artificial Sequence
<220>
   <223> Description of Artificial Sequence: Synthetic peptide
<400> 412
   Pro Gly Lys Ala Asn Glu Val Arg
   1               5
<210> 413
   <211> 8
   <212> PRT
   <213> Artificial Sequence
<220>
   <223> Description of Artificial Sequence: Synthetic peptide
<400> 413
   Pro Gly Lys Ala Asn Glu Ile Arg
   1               5
<210> 414
   <211> 13
   <212> PRT
   <213> Artificial Sequence
<220>
   <223> Description of Artificial Sequence: Synthetic peptide
<400> 414
   Thr Tyr Met Met Gly Asn Glu Ser Thr Phe Pro Asp Asp
   1               5                   10
<210> 415
   <211> 13
   <212> PRT
   <213> Artificial Sequence
<220>
   <223> Description of Artificial Sequence: Synthetic peptide
<400> 415
   Thr Tyr Met Met Gly Asn Glu Ser Thr Phe Leu Asp Asp
   1               5                   10
<210> 416
   <211> 13
   <212> PRT
   <213> Artificial Sequence
<220>
   <223> Description of Artificial Sequence: Synthetic peptide
<400> 416
   Thr Tyr Met Met Gly Asp Glu Leu Thr Phe Leu Asp Asp
   1 5 10
<210> 417
   <211> 13
   <212> PRT
   <213> Artificial Sequence
<220>
   <223> Description of Artificial Sequence: Synthetic peptide
<400> 417
   Thr Tyr Met Met Glu Asn Glu Leu Thr Phe Leu Asp Asp
   1 5 10
<210> 418
   <211> 13
   <212> PRT
   <213> Artificial Sequence
<220>
   <223> Description of Artificial Sequence: Synthetic peptide
<400> 418
   Thr Tyr Met Lys Glu Asn Glu Leu Thr Phe Leu Asp Asp
   1 5 10
<210> 419
   <211> 13
   <212> PRT
   <213> Artificial Sequence
<220>
   <223> Description of Artificial Sequence: Synthetic peptide
<400> 419
   Thr Tyr Met Lys Glu Asp Glu Leu Thr Phe Leu Asp Asp
   1 5 10
<210> 420
   <211> 13
   <212> PRT
   <213> Artificial Sequence
<220>
   <223> Description of Artificial Sequence: Synthetic peptide
<400> 420
   Thr Tyr Met Val Glu Asp Glu Leu Thr Phe Leu Asp Asp
   1 5 10
<210> 421
   <211> 13
   <212> PRT
   <213> Artificial Sequence
<220>
   <223> Description of Artificial Sequence: Synthetic peptide
<400> 421
   Thr Tyr Met Lys Gly Asp Glu Leu Thr Phe Leu Asp Asp
   1               5                   10
<210> 422
   <211> 13
   <212> PRT
   <213> Artificial Sequence
<220>
   <223> Description of Artificial Sequence: Synthetic peptide
<400> 422
   Thr Tyr Met Lys Gly Asn Glu Leu Thr Phe Leu Asp Asp
   1               5                   10
<210> 423
   <211> 12
   <212> PRT
   <213> Artificial Sequence
<220>
   <223> Description of Artificial Sequence: Synthetic peptide
<400> 423
   Glu Val Glu Leu Met Tyr Pro Pro Pro Tyr Tyr Leu
   1               5                   10
<210> 424
   <211> 12
   <212> PRT
   <213> Artificial Sequence
<220>
   <223> Description of Artificial Sequence: Synthetic peptide
<400> 424
   Lys Val Glu Leu Met Tyr Pro Pro Pro Tyr Tyr Glu
   1               5                   10
<210> 425
   <211> 8
   <212> PRT
   <213> Artificial Sequence
<220>
   <223> Description of Artificial Sequence: Synthetic peptide
<400> 425
   Pro Gly Lys Ala Asn Glu Ile Arg
   1               5
<210> 426
   <211> 8
   <212> PRT
   <213> Artificial Sequence
<220>
   <223> Description of Artificial Sequence: Synthetic peptide
<400> 426
   Pro Gly Lys Ala Thr Glu Ile Arg
   1               5
<210> 427
   <211> 8
   <212> PRT
   <213> Artificial Sequence
<220>
   <223> Description of Artificial Sequence: Synthetic peptide
<400> 427
   Pro His Lys Ala Thr Glu Ile Arg
   1               5
<210> 428
   <211> 8
   <212> PRT
   <213> Artificial Sequence
<220>
   <223> Description of Artificial Sequence: Synthetic peptide
<400> 428
   Pro Gly Lys Ala Asp Glu Ile Arg
   1               5
<210> 429
   <211> 8
   <212> PRT
   <213> Artificial Sequence
<220>
   <223> Description of Artificial Sequence: Synthetic peptide
<400> 429
   Pro His Lys Ala Asp Glu Ile Arg
   1 5
<210> 430
   <211> 8
   <212> PRT
   <213> Artificial Sequence
<220>
   <223> Description of Artificial Sequence: Synthetic peptide
<400> 430
   Pro His Lys Ala Asn Glu Ile Arg
   1 5
<210> 431
   <211> 8
   <212> PRT
   <213> Artificial Sequence
<220>
   <223> Description of Artificial Sequence: Synthetic peptide
<400> 431
   Pro His Lys Ala Asn Glu Val Arg
   1 5
<210> 432
   <211> 8
   <212> PRT
   <213> Artificial Sequence
<220>
   <223> Description of Artificial Sequence: Synthetic peptide
<400> 432
   Pro His Asn Ala Ala Glu Ile Arg
   1 5
<210> 433
   <211> 8
   <212> PRT
   <213> Artificial Sequence
<220>
   <223> Description of Artificial Sequence: Synthetic peptide
<400> 433
   Pro His Asn Ala Asp Glu Ile Arg
   1 5
<210> 434
   <211> 8
   <212> PRT
   <213> Artificial Sequence
<220>
   <223> Description of Artificial Sequence: Synthetic peptide
<400> 434
   Pro Gly Lys Ile Thr Glu Val Arg
   1 5
<210> 435
   <211> 8
   <212> PRT
   <213> Artificial Sequence
<220>
   <223> Description of Artificial Sequence: Synthetic peptide
<400> 435
   Pro Gly Lys Ala Thr Glu Val Phe
   1 5
<210> 436
   <211> 8
   <212> PRT
   <213> Artificial Sequence
<220>
   <223> Description of Artificial Sequence: Synthetic peptide
<400> 436
   Pro Gly Lys Tyr Thr Glu Val Arg
   1 5
<210> 437
   <211> 13
   <212> PRT
   <213> Artificial Sequence
<220>
   <223> Description of Artificial Sequence: Synthetic peptide
<400> 437
   Thr Tyr Met Lys Gly Asp Glu Leu Thr Phe Leu Asp Asp
   1 5 10
<210> 438
   <211> 13
   <212> PRT
   <213> Artificial Sequence
<220>
   <223> Description of Artificial Sequence: Synthetic peptide
<400> 438
   Thr Tyr Met Met Gly Asp Glu Leu Thr Phe Leu Asp Asp
   1 5 10
<210> 439
   <211> 13
   <212> PRT
   <213> Artificial Sequence
<220>
   <223> Description of Artificial Sequence: Synthetic peptide
<400> 439
   Thr Tyr Met Met Glu Asp Glu Leu Thr Phe Leu Asp Asp
   1 5 10
<210> 440
   <211> 13
   <212> PRT
   <213> Artificial Sequence
<220>
   <223> Description of Artificial Sequence: Synthetic peptide
<400> 440
   Thr Tyr Met Glu Gly Asp Glu Leu Thr Phe Leu Asp Asp
   1 5 10
<210> 441
   <211> 13
   <212> PRT
   <213> Artificial Sequence
<220>
   <223> Description of Artificial Sequence: Synthetic peptide
<400> 441
   Thr Tyr Met Val Glu Asp Glu Leu Thr Phe Leu Asp Asp
   1 5 10
<210> 442
   <211> 13
   <212> PRT
   <213> Artificial Sequence
<220>
   <223> Description of Artificial Sequence: Synthetic peptide
<400> 442
   Thr Tyr Met Lys Glu Asp Glu Leu Thr Phe Leu Asp Asp
   1 5 10
<210> 443
   <211> 13
   <212> PRT
   <213> Artificial Sequence
<220>
   <223> Description of Artificial Sequence: Synthetic peptide
<400> 443
   Thr Tyr Met Glu Glu Asp Glu Leu Thr Phe Leu Asp Asp
   1 5 10
<210> 444
   <211> 13
   <212> PRT
   <213> Artificial Sequence
<220>
   <223> Description of Artificial Sequence: Synthetic peptide
<400> 444
   Thr Tyr Met Val Glu Asp Glu Leu Thr Phe Leu Asp Lys
   1 5 10
<210> 445
   <211> 13
   <212> PRT
   <213> Artificial Sequence
<220>
   <223> Description of Artificial Sequence: Synthetic peptide
<400> 445
   Thr Tyr Met Met Gly Asn Glu Leu Thr Phe Lys Asp Asp
   1 5 10
<210> 446
   <211> 13
   <212> PRT
   <213> Artificial Sequence
<220>
   <223> Description of Artificial Sequence: Synthetic peptide
<400> 446
   Cys Tyr Met Met Gly Asn Glu Leu Thr Phe Leu Asp Asp
   1 5 10
<210> 447
   <211> 13
   <212> PRT
   <213> Artificial Sequence
<220>
   <223> Description of Artificial Sequence: Synthetic peptide
<400> 447
   Lys Tyr Met Met Gly Asn Glu Leu Thr Phe Leu Asp Asp
   1 5 10
<210> 448
   <211> 13
   <212> PRT
   <213> Artificial Sequence
<220>
   <223> Description of Artificial Sequence: Synthetic peptide
<400> 448
   Thr Tyr Met Met Gly Asn Glu Leu Thr Phe Tyr Asp Asp
   1 5 10
<210> 449
   <211> 12
   <212> PRT
   <213> Artificial Sequence
<220>
   <223> Description of Artificial Sequence: Synthetic peptide
<400> 449
   Lys Val Glu Leu Met Tyr Pro Pro Pro Tyr Tyr Glu
   1 5 10
<210> 450
   <211> 12
   <212> PRT
   <213> Artificial Sequence
<220>
   <223> Description of Artificial Sequence: Synthetic peptide
<400> 450
   Lys Val Phe Leu Met Tyr Pro Pro Pro Tyr Tyr Leu
   1 5 10
<210> 451
   <211> 12
   <212> PRT
   <213> Artificial Sequence
<220>
   <223> Description of Artificial Sequence: Synthetic peptide
<400> 451
   Lys Val Met Leu Met Tyr Pro Pro Pro Tyr Tyr Leu
   1 5 10
<210> 452
   <211> 124
   <212> PRT
   <213> Artificial Sequence
<220>
   <223> Description of Artificial Sequence: Synthetic polypeptide
<400> 452
   Met His Val Ala Gln Pro Ala Val Val Leu Ala Ser Ser Arg Gly Ile
   1 5 10 15
   Ala Ser Phe Val Cys Glu Tyr Ala Ser Pro Gly His Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 453
   <211> 124
   <212> PRT
   <213> Artificial Sequence
<220>
   <223> Description of Artificial Sequence: Synthetic polypeptide
<400> 453
   Met His Val Ala Gln Pro Ala Val Val Leu Ala Ser Ser Arg Gly Ile
   1 5 10 15
   Ala Ser Phe Val Cys Glu Tyr Ala Ser Pro Gly Thr Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 454
   <211> 124
   <212> PRT
   <213> Artificial Sequence
<220>
   <223> Description of Artificial Sequence: Synthetic polypeptide
<400> 454
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys His Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 455
   <211> 124
   <212> PRT
   <213> Artificial Sequence
<220>
   <223> Description of Artificial Sequence: Synthetic polypeptide
<400> 455
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Tyr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 456
   <211> 124
   <212> PRT
   <213> Artificial Sequence
<220>
   <223> Description of Artificial Sequence: Synthetic polypeptide
<400> 456
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Phe Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 457
   <211> 124
   <212> PRT
   <213> Artificial Sequence
<220>
   <223> Description of Artificial Sequence: Synthetic polypeptide
<400> 457
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Tyr Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 458
   <211> 124
   <212> PRT
   <213> Artificial Sequence
<220>
   <223> Description of Artificial Sequence: Synthetic polypeptide
<400> 458
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Gly Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 459
   <211> 124
   <212> PRT
   <213> Artificial Sequence
<220>
   <223> Description of Artificial Sequence: Synthetic polypeptide
<400> 459
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe His Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 460
   <211> 124
   <212> PRT
   <213> Artificial Sequence
<220>
   <223> Description of Artificial Sequence: Synthetic polypeptide
<400> 460
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Tyr Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 461
   <211> 124
   <212> PRT
   <213> Artificial Sequence
<220>
   <223> Description of Artificial Sequence: Synthetic polypeptide
<400> 461
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Met Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 462
   <211> 124
   <212> PRT
   <213> Artificial Sequence
<220>
   <223> Description of Artificial Sequence: Synthetic polypeptide
<400> 462
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Gln Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 463
   <211> 124
   <212> PRT
   <213> Artificial Sequence
<220>
   <223> Description of Artificial Sequence: Synthetic polypeptide
<400> 463
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Ala Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 464
   <211> 124
   <212> PRT
   <213> Artificial Sequence
<220>
   <223> Description of Artificial Sequence: Synthetic polypeptide
<400> 464
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Asn Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 465
   <211> 124
   <212> PRT
   <213> Artificial Sequence
<220>
   <223> Description of Artificial Sequence: Synthetic polypeptide
<400> 465
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Gln Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 466
   <211> 124
   <212> PRT
   <213> Artificial Sequence
<220>
   <223> Description of Artificial Sequence: Synthetic polypeptide
<400> 466
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Ala Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
                   100                 105                 110
   Tyr Val Ile Asp Pro Glu Pro Cys Pro Asp Ser Asp
           115                 120
<210> 467
   <211> 124
   <212> PRT
   <213> Artificial Sequence
<220>
   <223> Description of Artificial Sequence: Synthetic polypeptide
<400> 467
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Val Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 468
   <211> 124
   <212> PRT
   <213> Artificial Sequence
<220>
   <223> Description of Artificial Sequence: Synthetic polypeptide
<400> 468
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr His Gly Ile Gly Asn Gly Thr Gln Ile
   100 105 110
   Tyr Val Ile Asp Pro Glu Pro Cys Pro Asp Ser Asp
   115 120
<210> 469
   <211> 124
   <212> PRT
   <213> Artificial Sequence
<220>
   <223> Description of Artificial Sequence: Synthetic polypeptide
<400> 469
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Tyr Gly Ile Gly Asn Gly Thr Gln Ile
               100                 105                 110
   Tyr Val Ile Asp Pro Glu Pro Cys Pro Asp Ser Asp
       115                 120
<210> 470
   <211> 124
   <212> PRT
   <213> Artificial Sequence
<220>
   <223> Description of Artificial Sequence: Synthetic polypeptide
<400> 470
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Glu Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 471
   <211> 124
   <212> PRT
   <213> Artificial Sequence
<220>
   <223> Description of Artificial Sequence: Synthetic polypeptide
<400> 471
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Arg Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 472
   <211> 124
   <212> PRT
   <213> Artificial Sequence
<220>
   <223> Description of Artificial Sequence: Synthetic polypeptide
<400> 472
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Pro Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<211> 124
   <212> PRT
   <213> Artificial Sequence
<220>
   <223> Description of Artificial Sequence: Synthetic polypeptide
<400> 473
<210> 474
   <211> 124
   <212> PRT
   <213> Artificial Sequence
<220>
   <223> Description of Artificial Sequence: Synthetic polypeptide
<400> 474
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Glu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 475
   <211> 124
   <212> PRT
   <213> Artificial Sequence
<220>
   <223> Description of Artificial Sequence: Synthetic polypeptide
<400> 475
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Pro Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 476
   <211> 124
   <212> PRT
   <213> Artificial Sequence
<220>
   <223> Description of Artificial Sequence: Synthetic polypeptide
<400> 476
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Tyr Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 477
   <211> 124
   <212> PRT
   <213> Artificial Sequence
<220>
   <223> Description of Artificial Sequence: Synthetic polypeptide
<400> 477
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Phe Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 478
   <211> 124
   <212> PRT
   <213> Artificial Sequence
<220>
   <223> Description of Artificial Sequence: Synthetic polypeptide
<400> 478
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Ile Gly Ile Gly Asn Gly Thr Gln Ile
               100                 105                 110
   Tyr Val Ile Asp Pro Glu Pro Cys Pro Asp Ser Asp
           115                 120
<210> 479
   <211> 124
   <212> PRT
   <213> Artificial Sequence
<220>
   <223> Description of Artificial Sequence: Synthetic polypeptide
<400> 479
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Ile Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 480
   <211> 124
   <212> PRT
   <213> Artificial Sequence
<220>
   <223> Description of Artificial Sequence: Synthetic polypeptide
<400> 480
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu His Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 481
   <211> 124
   <212> PRT
   <213> Artificial Sequence
<220>
   <223> Description of Artificial Sequence: Synthetic polypeptide
<400> 481
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Lys Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 482
   <211> 124
   <212> PRT
   <213> Artificial Sequence
<220>
   <223> Description of Artificial Sequence: Synthetic polypeptide
<400> 482
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu His Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 483
   <211> 124
   <212> PRT
   <213> Artificial Sequence
<220>
   <223> Description of Artificial Sequence: Synthetic polypeptide
<400> 483
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Trp Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 484
   <211> 124
   <212> PRT
   <213> Artificial Sequence
<220>
   <223> Description of Artificial Sequence: Synthetic polypeptide
<400> 484
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Tyr Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 485
   <211> 124
   <212> PRT
   <213> Artificial Sequence
<220>
   <223> Description of Artificial Sequence: Synthetic polypeptide
<400> 485
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Asp Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 486
   <211> 124
   <212> PRT
   <213> Artificial Sequence
<220>
   <223> Description of Artificial Sequence: Synthetic polypeptide
<400> 486
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Trp Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 487
   <211> 124
   <212> PRT
   <213> Artificial Sequence
<220>
   <223> Description of Artificial Sequence: Synthetic polypeptide
<400> 487
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Lys Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 488
   <211> 124
   <212> PRT
   <213> Artificial Sequence
<220>
   <223> Description of Artificial Sequence: Synthetic polypeptide
<400> 488
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Asn Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 489
   <211> 124
   <212> PRT
   <213> Artificial Sequence
<220>
   <223> Description of Artificial Sequence: Synthetic polypeptide
<400> 489
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Trp Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 490
   <211> 124
   <212> PRT
   <213> Artificial Sequence
<220>
   <223> Description of Artificial Sequence: Synthetic polypeptide
<400> 490
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Lys
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 491
   <211> 124
   <212> PRT
   <213> Artificial Sequence
<220>
   <223> Description of Artificial Sequence: Synthetic polypeptide
<400> 491
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Arg
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 492
   <211> 124
   <212> PRT
   <213> Artificial Sequence
<220>
   <223> Description of Artificial Sequence: Synthetic polypeptide
<400> 492
   Met His Val Ala Gln Pro Ala Val Val Leu Ala Ser Ser Arg Gly Ile
   1 5 10 15
   Ala Ser Phe Val Cys Glu Tyr Ala Ser Pro Ala Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 493
   <211> 124
   <212> PRT
   <213> Artificial Sequence
<220>
   <223> Description of Artificial Sequence: Synthetic polypeptide
<400> 493
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Phe Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 494
   <211> 124
   <212> PRT
   <213> Artificial Sequence
<220>
   <223> Description of Artificial Sequence: Synthetic polypeptide
<400> 494
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Thr Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 495
   <211> 124
   <212> PRT
   <213> Artificial Sequence
<220>
   <223> Description of Artificial Sequence: Synthetic polypeptide
<400> 495
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Met Gly Ile Gly Asn Gly Thr Gln Ile
   100 105 110
   Tyr Val Ile Asp Pro Glu Pro Cys Pro Asp Ser Asp
   115 120
<210> 496
   <211> 124
   <212> PRT
   <213> Artificial Sequence
<220>
   <223> Description of Artificial Sequence: Synthetic polypeptide
<400> 496
   Met His Val Ala Gln Pro Ala Val Val Leu Ala Ser Ser Arg Gly Ile
   1               5                   10                  15
   Ala Ser Phe Val Cys Glu Tyr Ala Ser Ile Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 497
   <211> 124
   <212> PRT
   <213> Artificial Sequence
<220>
   <223> Description of Artificial Sequence: Synthetic polypeptide
<400> 497
   Met His Val Ala Gln Pro Ala Val Val Leu Ala Ser Ser Arg Gly Ile
   1               5                   10                  15
   Ala Ser Phe Val Cys Glu Tyr Ala Ser Leu Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 498
   <211> 124
   <212> PRT
   <213> Artificial Sequence
<220>
   <223> Description of Artificial Sequence: Synthetic polypeptide
<400> 498
   Met His Val Ala Gln Pro Ala Val Val Leu Ala Ser Ser Arg Gly Ile
   1 5 10 15
   Ala Ser Phe Val Cys Glu Tyr Ala Ser Met Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 499
   <211> 124
   <212> PRT
   <213> Artificial Sequence
<220>
   <223> Description of Artificial Sequence: Synthetic polypeptide
<400> 499
   Met His Val Ala Gln Pro Ala Val Val Leu Ala Ser Ser Arg Gly Ile
   1 5 10 15
   Ala Ser Phe Val Cys Glu Tyr Ala Ser Gln Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 500
   <211> 124
   <212> PRT
   <213> Artificial Sequence
<220>
   <223> Description of Artificial Sequence: Synthetic polypeptide
<400> 500
   Met His Val Ala Gln Pro Ala Val Val Leu Ala Ser Ser Arg Gly Ile
   1               5                   10                  15
   Ala Ser Phe Val Cys Glu Tyr Ala Ser Thr Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 501
   <211> 124
   <212> PRT
   <213> Artificial Sequence
<220>
   <223> Description of Artificial Sequence: Synthetic polypeptide
<400> 501
   Met His Val Ala Gln Pro Ala Val Val Leu Ala Ser Ser Arg Gly Ile
   1               5                   10                  15
   Ala Ser Phe Val Cys Glu Tyr Ala Ser Val Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 502
   <211> 124
   <212> PRT
   <213> Artificial Sequence
<220>
   <223> Description of Artificial Sequence: Synthetic polypeptide
<400> 502
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Arg Ala Thr Glu Val
               20                  25                  30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 503
   <211> 124
   <212> PRT
   <213> Artificial Sequence
<220>
   <223> Description of Artificial Sequence: Synthetic polypeptide
<400> 503
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Pro Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 504
   <211> 124
   <212> PRT
   <213> Artificial Sequence
<220>
   <223> Description of Artificial Sequence: Synthetic polypeptide
<400> 504
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ser Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 505
   <211> 124
   <212> PRT
   <213> Artificial Sequence
<220>
   <223> Description of Artificial Sequence: Synthetic polypeptide
<400> 505
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala His Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 506
   <211> 124
   <212> PRT
   <213> Artificial Sequence
<220>
   <223> Description of Artificial Sequence: Synthetic polypeptide
<400> 506
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Met Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 507
   <211> 124
   <212> PRT
   <213> Artificial Sequence
<220>
   <223> Description of Artificial Sequence: Synthetic polypeptide
<400> 507
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Ser Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 508
   <211> 124
   <212> PRT
   <213> Artificial Sequence
<220>
   <223> Description of Artificial Sequence: Synthetic polypeptide
<400> 508
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Glu
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 509
   <211> 124
   <212> PRT
   <213> Artificial Sequence
<220>
   <223> Description of Artificial Sequence: Synthetic polypeptide
<400> 509
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Pro Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 510
   <211> 124
   <212> PRT
   <213> Artificial Sequence
<220>
   <223> Description of Artificial Sequence: Synthetic polypeptide
<400> 510
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Ala Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 511
   <211> 124
   <212> PRT
   <213> Artificial Sequence
<220>
   <223> Description of Artificial Sequence: Synthetic polypeptide
<400> 511
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr His Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 512
   <211> 124
   <212> PRT
   <213> Artificial Sequence
<220>
   <223> Description of Artificial Sequence: Synthetic polypeptide
<400> 512
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Met Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 513
   <211> 124
   <212> PRT
   <213> Artificial Sequence
<220>
   <223> Description of Artificial Sequence: Synthetic polypeptide
<400> 513
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Trp Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 514
   <211> 124
   <212> PRT
   <213> Artificial Sequence
<220>
   <223> Description of Artificial Sequence: Synthetic polypeptide
<400> 514
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Asp Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 515
   <211> 124
   <212> PRT
   <213> Artificial Sequence
<220>
   <223> Description of Artificial Sequence: Synthetic polypeptide
<400> 515
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Asp Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 516
   <211> 124
   <212> PRT
   <213> Artificial Sequence
<220>
   <223> Description of Artificial Sequence: Synthetic polypeptide
<400> 516
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Gly Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 517
   <211> 124
   <212> PRT
   <213> Artificial Sequence
<220>
   <223> Description of Artificial Sequence: Synthetic polypeptide
<400> 517
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met His Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 518
   <211> 124
   <212> PRT
   <213> Artificial Sequence
<220>
   <223> Description of Artificial Sequence: Synthetic polypeptide
<400> 518
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Leu Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 519
   <211> 124
   <212> PRT
   <213> Artificial Sequence
<220>
   <223> Description of Artificial Sequence: Synthetic polypeptide
<400> 519
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Asp Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 520
   <211> 124
   <212> PRT
   <213> Artificial Sequence
<220>
   <223> Description of Artificial Sequence: Synthetic polypeptide
<400> 520
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met His Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 521
   <211> 124
   <212> PRT
   <213> Artificial Sequence
<220>
   <223> Description of Artificial Sequence: Synthetic polypeptide
<400> 521
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Thr Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 522
   <211> 124
   <212> PRT
   <213> Artificial Sequence
<220>
   <223> Description of Artificial Sequence: Synthetic polypeptide
<400> 522
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Ala Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 523
   <211> 124
   <212> PRT
   <213> Artificial Sequence
<220>
   <223> Description of Artificial Sequence: Synthetic polypeptide
<400> 523
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Phe Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 524
   <211> 124
   <212> PRT
   <213> Artificial Sequence
<220>
   <223> Description of Artificial Sequence: Synthetic polypeptide
<400> 524
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Gly Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 525
   <211> 124
   <212> PRT
   <213> Artificial Sequence
<220>
   <223> Description of Artificial Sequence: Synthetic polypeptide
<400> 525
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly His Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 526
   <211> 124
   <212> PRT
   <213> Artificial Sequence
<220>
   <223> Description of Artificial Sequence: Synthetic polypeptide
<400> 526
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Ile Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 527
   <211> 124
   <212> PRT
   <213> Artificial Sequence
<220>
   <223> Description of Artificial Sequence: Synthetic polypeptide
<400> 527
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Lys Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 528
   <211> 124
   <212> PRT
   <213> Artificial Sequence
<220>
   <223> Description of Artificial Sequence: Synthetic polypeptide
<400> 528
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Leu Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 529
   <211> 124
   <212> PRT
   <213> Artificial Sequence
<220>
   <223> Description of Artificial Sequence: Synthetic polypeptide
<400> 529
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Met Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 530
   <211> 124
   <212> PRT
   <213> Artificial Sequence
<220>
   <223> Description of Artificial Sequence: Synthetic polypeptide
<400> 530
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Gln Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 531
   <211> 124
   <212> PRT
   <213> Artificial Sequence
<220>
   <223> Description of Artificial Sequence: Synthetic polypeptide
<400> 531
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Arg Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
                   100                 105                 110
   Tyr Val Ile Asp Pro Glu Pro Cys Pro Asp Ser Asp
           115                 120
<210> 532
   <211> 124
   <212> PRT
   <213> Artificial Sequence
<220>
   <223> Description of Artificial Sequence: Synthetic polypeptide
<400> 532
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Ser Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 533
   <211> 124
   <212> PRT
   <213> Artificial Sequence
<220>
   <223> Description of Artificial Sequence: Synthetic polypeptide
<400> 533
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Val Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 534
   <211> 124
   <212> PRT
   <213> Artificial Sequence
<220>
   <223> Description of Artificial Sequence: Synthetic polypeptide
<400> 534
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Tyr Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 535
   <211> 124
   <212> PRT
   <213> Artificial Sequence
<220>
   <223> Description of Artificial Sequence: Synthetic polypeptide
<400> 535
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Ala Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 536
   <211> 124
   <212> PRT
   <213> Artificial Sequence
<220>
   <223> Description of Artificial Sequence: Synthetic polypeptide
<400> 536
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Asp Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 537
   <211> 124
   <212> PRT
   <213> Artificial Sequence
<220>
   <223> Description of Artificial Sequence: Synthetic polypeptide
<400> 537
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn His Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 538
   <211> 124
   <212> PRT
   <213> Artificial Sequence
<220>
   <223> Description of Artificial Sequence: Synthetic polypeptide
<400> 538
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Lys Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 539
   <211> 124
   <212> PRT
   <213> Artificial Sequence
<220>
   <223> Description of Artificial Sequence: Synthetic polypeptide
<400> 539
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Met Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 540
   <211> 124
   <212> PRT
   <213> Artificial Sequence
<220>
   <223> Description of Artificial Sequence: Synthetic polypeptide
<400> 540
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Asn Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 541
   <211> 124
   <212> PRT
   <213> Artificial Sequence
<220>
   <223> Description of Artificial Sequence: Synthetic polypeptide
<400> 541
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Gln Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 542
   <211> 124
   <212> PRT
   <213> Artificial Sequence
<220>
   <223> Description of Artificial Sequence: Synthetic polypeptide
<400> 542
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Arg Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 543
   <211> 124
   <212> PRT
   <213> Artificial Sequence
<220>
   <223> Description of Artificial Sequence: Synthetic polypeptide
<400> 543
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Ser Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 544
   <211> 124
   <212> PRT
   <213> Artificial Sequence
<220>
   <223> Description of Artificial Sequence: Synthetic polypeptide
<400> 544
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Thr Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 545
   <211> 124
   <212> PRT
   <213> Artificial Sequence
<220>
   <223> Description of Artificial Sequence: Synthetic polypeptide
<400> 545
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Val Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 546
   <211> 124
   <212> PRT
   <213> Artificial Sequence
<220>
   <223> Description of Artificial Sequence: Synthetic polypeptide
<400> 546
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Ile Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 547
   <211> 124
   <212> PRT
   <213> Artificial Sequence
<220>
   <223> Description of Artificial Sequence: Synthetic polypeptide
<400> 547
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Arg Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 548
   <211> 124
   <212> PRT
   <213> Artificial Sequence
<220>
   <223> Description of Artificial Sequence: Synthetic polypeptide
<400> 548
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Thr Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 549
   <211> 124
   <212> PRT
   <213> Artificial Sequence
<220>
   <223> Description of Artificial Sequence: Synthetic polypeptide
<400> 549
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Ile Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 550
   <211> 124
   <212> PRT
   <213> Artificial Sequence
<220>
   <223> Description of Artificial Sequence: Synthetic polypeptide
<400> 550
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Met Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 551
   <211> 124
   <212> PRT
   <213> Artificial Sequence
<220>
   <223> Description of Artificial Sequence: Synthetic polypeptide
<400> 551
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Asn Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 552
   <211> 124
   <212> PRT
   <213> Artificial Sequence
<220>
   <223> Description of Artificial Sequence: Synthetic polypeptide
<400> 552
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Gln Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 553
   <211> 124
   <212> PRT
   <213> Artificial Sequence
<220>
   <223> Description of Artificial Sequence: Synthetic polypeptide
<400> 553
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Arg Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 554
   <211> 124
   <212> PRT
   <213> Artificial Sequence
<220>
   <223> Description of Artificial Sequence: Synthetic polypeptide
<400> 554
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Ser Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 555
   <211> 124
   <212> PRT
   <213> Artificial Sequence
<220>
   <223> Description of Artificial Sequence: Synthetic polypeptide
<400> 555
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Val Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 556
   <211> 124
   <212> PRT
   <213> Artificial Sequence
<220>
   <223> Description of Artificial Sequence: Synthetic polypeptide
<400> 556
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Glu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 557
   <211> 124
   <212> PRT
   <213> Artificial Sequence
<220>
   <223> Description of Artificial Sequence: Synthetic polypeptide
<400> 557
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Ile Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 558
   <211> 124
   <212> PRT
   <213> Artificial Sequence
<220>
   <223> Description of Artificial Sequence: Synthetic polypeptide
<400> 558
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Met Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 559
   <211> 124
   <212> PRT
   <213> Artificial Sequence
<220>
   <223> Description of Artificial Sequence: Synthetic polypeptide
<400> 559
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Thr Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 560
   <211> 124
   <212> PRT
   <213> Artificial Sequence
<220>
   <223> Description of Artificial Sequence: Synthetic polypeptide
<400> 560
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Val Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 561
   <211> 124
   <212> PRT
   <213> Artificial Sequence
<220>
   <223> Description of Artificial Sequence: Synthetic polypeptide
<400> 561
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Arg Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 562
   <211> 124
   <212> PRT
   <213> Artificial Sequence
<220>
   <223> Description of Artificial Sequence: Synthetic polypeptide
<400> 562
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Glu Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 563
   <211> 124
   <212> PRT
   <213> Artificial Sequence
<220>
   <223> Description of Artificial Sequence: Synthetic polypeptide
<400> 563
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Gly Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 564
   <211> 124
   <212> PRT
   <213> Artificial Sequence
<220>
   <223> Description of Artificial Sequence: Synthetic polypeptide
<400> 564
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp His Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 565
   <211> 124
   <212> PRT
   <213> Artificial Sequence
<220>
   <223> Description of Artificial Sequence: Synthetic polypeptide
<400> 565
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Thr Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 566
   <211> 124
   <212> PRT
   <213> Artificial Sequence
<220>
   <223> Description of Artificial Sequence: Synthetic polypeptide
<400> 566
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Gly Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 567
   <211> 124
   <212> PRT
   <213> Artificial Sequence
<220>
   <223> Description of Artificial Sequence: Synthetic polypeptide
<400> 567
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Gly Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 568
   <211> 124
   <212> PRT
   <213> Artificial Sequence
<220>
   <223> Description of Artificial Sequence: Synthetic polypeptide
<400> 568
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Ile Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 569
   <211> 124
   <212> PRT
   <213> Artificial Sequence
<220>
   <223> Description of Artificial Sequence: Synthetic polypeptide
<400> 569
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Trp Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 570
   <211> 124
   <212> PRT
   <213> Artificial Sequence
<220>
   <223> Description of Artificial Sequence: Synthetic polypeptide
<400> 570
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr His Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 571
   <211> 124
   <212> PRT
   <213> Artificial Sequence
<220>
   <223> Description of Artificial Sequence: Synthetic polypeptide
<400> 571
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Thr Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 572
   <211> 124
   <212> PRT
   <213> Artificial Sequence
<220>
   <223> Description of Artificial Sequence: Synthetic polypeptide
<400> 572
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Leu Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 573
   <211> 124
   <212> PRT
   <213> Artificial Sequence
<220>
   <223> Description of Artificial Sequence: Synthetic polypeptide
<400> 573
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Thr Tyr Leu Gly Ile Gly Asn Gly Thr Gln Ile
   100 105 110
   Tyr Val Ile Asp Pro Glu Pro Cys Pro Asp Ser Asp
   115 120
<210> 574
   <211> 124
   <212> PRT
   <213> Artificial Sequence
<220>
   <223> Description of Artificial Sequence: Synthetic polypeptide
<400> 574
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Tyr Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 575
   <211> 124
   <212> PRT
   <213> Artificial Sequence
<220>
   <223> Description of Artificial Sequence: Synthetic polypeptide
<400> 575
   Met His Val Ala Gln Pro Ala Val Val Leu Ala Ser Ser Arg Gly Ile
   1 5 10 15
   Ala Ser Phe Val Cys Glu Tyr Ala Ser Asn Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 576
   <211> 124
   <212> PRT
   <213> Artificial Sequence
<220>
   <223> Description of Artificial Sequence: Synthetic polypeptide
<400> 576
   Met His Val Ala Gln Pro Ala Val Val Leu Ala Ser Ser Arg Gly Ile
   1 5 10 15
   Ala Ser Phe Val Cys Glu Tyr Ala Ser Pro Ile Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 577
   <211> 124
   <212> PRT
   <213> Artificial Sequence
<220>
   <223> Description of Artificial Sequence: Synthetic polypeptide
<400> 577
   Met His Val Ala Gln Pro Ala Val Val Leu Ala Ser Ser Arg Gly Ile
   1 5 10 15
   Ala Ser Phe Val Cys Glu Tyr Ala Ser Pro Gln Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 578
   <211> 124
   <212> PRT
   <213> Artificial Sequence
<220>
   <223> Description of Artificial Sequence: Synthetic polypeptide
<400> 578
   Met His Val Ala Gln Pro Ala Val Val Leu Ala Ser Ser Arg Gly Ile
   1               5                   10                  15
   Ala Ser Phe Val Cys Glu Tyr Ala Ser Pro Trp Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 579
   <211> 124
   <212> PRT
   <213> Artificial Sequence
<220>
   <223> Description of Artificial Sequence: Synthetic polypeptide
<400> 579
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Glu Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 580
   <211> 124
   <212> PRT
   <213> Artificial Sequence
<220>
   <223> Description of Artificial Sequence: Synthetic polypeptide
<400> 580
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Ile Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 581
   <211> 124
   <212> PRT
   <213> Artificial Sequence
<220>
   <223> Description of Artificial Sequence: Synthetic polypeptide
<400> 581
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Met Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 582
   <211> 124
   <212> PRT
   <213> Artificial Sequence
<220>
   <223> Description of Artificial Sequence: Synthetic polypeptide
<400> 582
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Pro Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 583
   <211> 124
   <212> PRT
   <213> Artificial Sequence
<220>
   <223> Description of Artificial Sequence: Synthetic polypeptide
<400> 583
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Gln Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 584
   <211> 124
   <212> PRT
   <213> Artificial Sequence
<220>
   <223> Description of Artificial Sequence: Synthetic polypeptide
<400> 584
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Val Ala Thr Glu Val
               20                  25                  30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 585
   <211> 124
   <212> PRT
   <213> Artificial Sequence
<220>
   <223> Description of Artificial Sequence: Synthetic polypeptide
<400> 585
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Ile Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 586
   <211> 124
   <212> PRT
   <213> Artificial Sequence
<220>
   <223> Description of Artificial Sequence: Synthetic polypeptide
<400> 586
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Leu Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 587
   <211> 124
   <212> PRT
   <213> Artificial Sequence
<220>
   <223> Description of Artificial Sequence: Synthetic polypeptide
<400> 587
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Glu Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 588
   <211> 124
   <212> PRT
   <213> Artificial Sequence
<220>
   <223> Description of Artificial Sequence: Synthetic polypeptide
<400> 588
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Leu Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 589
   <211> 124
   <212> PRT
   <213> Artificial Sequence
<220>
   <223> Description of Artificial Sequence: Synthetic polypeptide
<400> 589
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Asn Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 590
   <211> 124
   <212> PRT
   <213> Artificial Sequence
<220>
   <223> Description of Artificial Sequence: Synthetic polypeptide
<400> 590
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Ala Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 591
   <211> 124
   <212> PRT
   <213> Artificial Sequence
<220>
   <223> Description of Artificial Sequence: Synthetic polypeptide
<400> 591
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Gln Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 592
   <211> 124
   <212> PRT
   <213> Artificial Sequence
<220>
   <223> Description of Artificial Sequence: Synthetic polypeptide
<400> 592
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Ser Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 593
   <211> 124
   <212> PRT
   <213> Artificial Sequence
<220>
   <223> Description of Artificial Sequence: Synthetic polypeptide
<400> 593
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Asp Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 594
   <211> 124
   <212> PRT
   <213> Artificial Sequence
<220>
   <223> Description of Artificial Sequence: Synthetic polypeptide
<400> 594
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Val Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 595
   <211> 124
   <212> PRT
   <213> Artificial Sequence
<220>
   <223> Description of Artificial Sequence: Synthetic polypeptide
<400> 595
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu His Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 596
   <211> 124
   <212> PRT
   <213> Artificial Sequence
<220>
   <223> Description of Artificial Sequence: Synthetic polypeptide
<400> 596
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Lys Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
                   100                 105                 110
   Tyr Val Ile Asp Pro Glu Pro Cys Pro Asp Ser Asp
           115                 120
<210> 597
   <211> 124
   <212> PRT
   <213> Artificial Sequence
<220>
   <223> Description of Artificial Sequence: Synthetic polypeptide
<400> 597
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Leu Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 598
   <211> 124
   <212> PRT
   <213> Artificial Sequence
<220>
   <223> Description of Artificial Sequence: Synthetic polypeptide
<400> 598
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
   35 40 45
   Ala Ala Thr Tyr Met Met Gly Asn Glu Leu Pro Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 599
   <211> 124
   <212> PRT
   <213> Artificial Sequence
<220>
   <223> Description of Artificial Sequence: Synthetic polypeptide
<400> 599
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Asp Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 600
   <211> 124
   <212> PRT
   <213> Artificial Sequence
<220>
   <223> Description of Artificial Sequence: Synthetic polypeptide
<400> 600
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Gly Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 601
   <211> 124
   <212> PRT
   <213> Artificial Sequence
<220>
   <223> Description of Artificial Sequence: Synthetic polypeptide
<400> 601
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Gln Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 602
   <211> 124
   <212> PRT
   <213> Artificial Sequence
<220>
   <223> Description of Artificial Sequence: Synthetic polypeptide
<400> 602
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Arg Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 603
   <211> 124
   <212> PRT
   <213> Artificial Sequence
<220>
   <223> Description of Artificial Sequence: Synthetic polypeptide
<400> 603
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Ala Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 604
   <211> 124
   <212> PRT
   <213> Artificial Sequence
<220>
   <223> Description of Artificial Sequence: Synthetic polypeptide
<400> 604
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Glu Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 605
   <211> 124
   <212> PRT
   <213> Artificial Sequence
<220>
   <223> Description of Artificial Sequence: Synthetic polypeptide
<400> 605
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Ser Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 606
   <211> 124
   <212> PRT
   <213> Artificial Sequence
<220>
   <223> Description of Artificial Sequence: Synthetic polypeptide
<400> 606
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Gln Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 607
   <211> 124
   <212> PRT
   <213> Artificial Sequence
<220>
   <223> Description of Artificial Sequence: Synthetic polypeptide
<400> 607
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Ser Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 608
   <211> 124
   <212> PRT
   <213> Artificial Sequence
<220>
   <223> Description of Artificial Sequence: Synthetic polypeptide
<400> 608
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Arg Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 609
   <211> 124
   <212> PRT
   <213> Artificial Sequence
<220>
   <223> Description of Artificial Sequence: Synthetic polypeptide
<400> 609
   Met His Val Ala Gln Pro Ala Val Val Leu Ala Ser Ser Arg Gly Ile
   1 5 10 15
   Ala Ser Phe Val Cys Glu Tyr Ala Ser Arg Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 610
   <211> 124
   <212> PRT
   <213> Artificial Sequence
<220>
   <223> Description of Artificial Sequence: Synthetic polypeptide
<400> 610
   Met His Val Ala Gln Pro Ala Val Val Leu Ala Ser Ser Arg Gly Ile
   1 5 10 15
   Ala Ser Phe Val Cys Glu Tyr Ala Ser Pro Asp Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 611
   <211> 124
   <212> PRT
   <213> Artificial Sequence
<220>
   <223> Description of Artificial Sequence: Synthetic polypeptide
<400> 611
   Met His Val Ala Gln Pro Ala Val Val Leu Ala Ser Ser Arg Gly Ile
   1               5                   10                  15
   Ala Ser Phe Val Cys Glu Tyr Ala Ser Pro Pro Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 612
   <211> 124
   <212> PRT
   <213> Artificial Sequence
<220>
   <223> Description of Artificial Sequence: Synthetic polypeptide
<400> 612
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Asn Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 613
   <211> 124
   <212> PRT
   <213> Artificial Sequence
<220>
   <223> Description of Artificial Sequence: Synthetic polypeptide
<400> 613
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Lys Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 614
   <211> 124
   <212> PRT
   <213> Artificial Sequence
<220>
   <223> Description of Artificial Sequence: Synthetic polypeptide
<400> 614
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Asp Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 615
   <211> 124
   <212> PRT
   <213> Artificial Sequence
<220>
   <223> Description of Artificial Sequence: Synthetic polypeptide
<400> 615
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Gly Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 616
   <211> 124
   <212> PRT
   <213> Artificial Sequence
<220>
   <223> Description of Artificial Sequence: Synthetic polypeptide
<400> 616
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Pro Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 617
   <211> 124
   <212> PRT
   <213> Artificial Sequence
<220>
   <223> Description of Artificial Sequence: Synthetic polypeptide
<400> 617
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Arg Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 618
   <211> 124
   <212> PRT
   <213> Artificial Sequence
<220>
   <223> Description of Artificial Sequence: Synthetic polypeptide
<400> 618
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Thr Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 619
   <211> 124
   <212> PRT
   <213> Artificial Sequence
<220>
   <223> Description of Artificial Sequence: Synthetic polypeptide
<400> 619
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Gly
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 620
   <211> 124
   <212> PRT
   <213> Artificial Sequence
<220>
   <223> Description of Artificial Sequence: Synthetic polypeptide
<400> 620
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu His
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 621
   <211> 124
   <212> PRT
   <213> Artificial Sequence
<220>
   <223> Description of Artificial Sequence: Synthetic polypeptide
<400> 621
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Thr
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 622
   <211> 124
   <212> PRT
   <213> Artificial Sequence
<220>
   <223> Description of Artificial Sequence: Synthetic polypeptide
<400> 622
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Glu Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 623
   <211> 124
   <212> PRT
   <213> Artificial Sequence
<220>
   <223> Description of Artificial Sequence: Synthetic polypeptide
<400> 623
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Gly Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 624
   <211> 124
   <212> PRT
   <213> Artificial Sequence
<220>
   <223> Description of Artificial Sequence: Synthetic polypeptide
<400> 624
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Ile Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 625
   <211> 124
   <212> PRT
   <213> Artificial Sequence
<220>
   <223> Description of Artificial Sequence: Synthetic polypeptide
<400> 625
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Met Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 626
   <211> 124
   <212> PRT
   <213> Artificial Sequence
<220>
   <223> Description of Artificial Sequence: Synthetic polypeptide
<400> 626
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Asn Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 627
   <211> 124
   <212> PRT
   <213> Artificial Sequence
<220>
   <223> Description of Artificial Sequence: Synthetic polypeptide
<400> 627
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Gln Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 628
   <211> 124
   <212> PRT
   <213> Artificial Sequence
<220>
   <223> Description of Artificial Sequence: Synthetic polypeptide
<400> 628
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Trp Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 629
   <211> 124
   <212> PRT
   <213> Artificial Sequence
<220>
   <223> Description of Artificial Sequence: Synthetic polypeptide
<400> 629
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Tyr Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 630
   <211> 124
   <212> PRT
   <213> Artificial Sequence
<220>
   <223> Description of Artificial Sequence: Synthetic polypeptide
<400> 630
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Glu Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 631
   <211> 124
   <212> PRT
   <213> Artificial Sequence
<220>
   <223> Description of Artificial Sequence: Synthetic polypeptide
<400> 631
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Gly Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 632
   <211> 124
   <212> PRT
   <213> Artificial Sequence
<220>
   <223> Description of Artificial Sequence: Synthetic polypeptide
<400> 632
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Ile Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 633
   <211> 124
   <212> PRT
   <213> Artificial Sequence
<220>
   <223> Description of Artificial Sequence: Synthetic polypeptide
<400> 633
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Val Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 634
   <211> 124
   <212> PRT
   <213> Artificial Sequence
<220>
   <223> Description of Artificial Sequence: Synthetic polypeptide
<400> 634
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Ala Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 635
   <211> 124
   <212> PRT
   <213> Artificial Sequence
<220>
   <223> Description of Artificial Sequence: Synthetic polypeptide
<400> 635
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Asp Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 636
   <211> 124
   <212> PRT
   <213> Artificial Sequence
<220>
   <223> Description of Artificial Sequence: Synthetic polypeptide
<400> 636
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Ile Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 637
   <211> 124
   <212> PRT
   <213> Artificial Sequence
<220>
   <223> Description of Artificial Sequence: Synthetic polypeptide
<400> 637
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Lys Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 638
   <211> 124
   <212> PRT
   <213> Artificial Sequence
<220>
   <223> Description of Artificial Sequence: Synthetic polypeptide
<400> 638
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Leu Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 639
   <211> 124
   <212> PRT
   <213> Artificial Sequence
<220>
   <223> Description of Artificial Sequence: Synthetic polypeptide
<400> 639
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Gln Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 640
   <211> 124
   <212> PRT
   <213> Artificial Sequence
<220>
   <223> Description of Artificial Sequence: Synthetic polypeptide
<400> 640
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Arg Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 641
   <211> 124
   <212> PRT
   <213> Artificial Sequence
<220>
   <223> Description of Artificial Sequence: Synthetic polypeptide
<400> 641
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Ser Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 642
   <211> 124
   <212> PRT
   <213> Artificial Sequence
<220>
   <223> Description of Artificial Sequence: Synthetic polypeptide
<400> 642
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Thr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 643
   <211> 124
   <212> PRT
   <213> Artificial Sequence
<220>
   <223> Description of Artificial Sequence: Synthetic polypeptide
<400> 643
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr His Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 644
   <211> 124
   <212> PRT
   <213> Artificial Sequence
<220>
   <223> Description of Artificial Sequence: Synthetic polypeptide
<400> 644
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Pro Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 645
   <211> 124
   <212> PRT
   <213> Artificial Sequence
<220>
   <223> Description of Artificial Sequence: Synthetic polypeptide
<400> 645
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Ser Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 646
   <211> 124
   <212> PRT
   <213> Artificial Sequence
<220>
   <223> Description of Artificial Sequence: Synthetic polypeptide
<400> 646
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Ala Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 647
   <211> 124
   <212> PRT
   <213> Artificial Sequence
<220>
   <223> Description of Artificial Sequence: Synthetic polypeptide
<400> 647
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Ile Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 648
   <211> 124
   <212> PRT
   <213> Artificial Sequence
<220>
   <223> Description of Artificial Sequence: Synthetic polypeptide
<400> 648
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Leu Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 649
   <211> 124
   <212> PRT
   <213> Artificial Sequence
<220>
   <223> Description of Artificial Sequence: Synthetic polypeptide
<400> 649
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Asn Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 650
   <211> 124
   <212> PRT
   <213> Artificial Sequence
<220>
   <223> Description of Artificial Sequence: Synthetic polypeptide
<400> 650
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gln Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 651
   <211> 124
   <212> PRT
   <213> Artificial Sequence
<220>
   <223> Description of Artificial Sequence: Synthetic polypeptide
<400> 651
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Arg Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 652
   <211> 124
   <212> PRT
   <213> Artificial Sequence
<220>
   <223> Description of Artificial Sequence: Synthetic polypeptide
<400> 652
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Tyr Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 653
   <211> 124
   <212> PRT
   <213> Artificial Sequence
<220>
   <223> Description of Artificial Sequence: Synthetic polypeptide
<400> 653
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Gly Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 654
   <211> 124
   <212> PRT
   <213> Artificial Sequence
<220>
   <223> Description of Artificial Sequence: Synthetic polypeptide
<400> 654
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Ile Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 655
   <211> 124
   <212> PRT
   <213> Artificial Sequence
<220>
   <223> Description of Artificial Sequence: Synthetic polypeptide
<400> 655
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Trp Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 656
   <211> 124
   <212> PRT
   <213> Artificial Sequence
<220>
   <223> Description of Artificial Sequence: Synthetic polypeptide
<400> 656
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Tyr Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 657
   <211> 124
   <212> PRT
   <213> Artificial Sequence
<220>
   <223> Description of Artificial Sequence: Synthetic polypeptide
<400> 657
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Asp Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 658
   <211> 124
   <212> PRT
   <213> Artificial Sequence
<220>
   <223> Description of Artificial Sequence: Synthetic polypeptide
<400> 658
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Gly Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 659
   <211> 124
   <212> PRT
   <213> Artificial Sequence
<220>
   <223> Description of Artificial Sequence: Synthetic polypeptide
<400> 659
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Tyr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 660
   <211> 124
   <212> PRT
   <213> Artificial Sequence
<220>
   <223> Description of Artificial Sequence: Synthetic polypeptide
<400> 660
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Asp Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 661
   <211> 124
   <212> PRT
   <213> Artificial Sequence
<220>
   <223> Description of Artificial Sequence: Synthetic polypeptide
<400> 661
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Gly Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 662
   <211> 124
   <212> PRT
   <213> Artificial Sequence
<220>
   <223> Description of Artificial Sequence: Synthetic polypeptide
<400> 662
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr His Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 663
   <211> 124
   <212> PRT
   <213> Artificial Sequence
<220>
   <223> Description of Artificial Sequence: Synthetic polypeptide
<400> 663
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
   35 40 45
   Ala Ala Thr Tyr Met Met Gly Asn Glu Leu Thr Leu Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 664
   <211> 124
   <212> PRT
   <213> Artificial Sequence
<220>
   <223> Description of Artificial Sequence: Synthetic polypeptide
<400> 664
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Gly Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
       115                 120
<210> 665
   <211> 124
   <212> PRT
   <213> Artificial Sequence
<220>
   <223> Description of Artificial Sequence: Synthetic polypeptide
<400> 665
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Ile Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 666
   <211> 124
   <212> PRT
   <213> Artificial Sequence
<220>
   <223> Description of Artificial Sequence: Synthetic polypeptide
<400> 666
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Trp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 667
   <211> 124
   <212> PRT
   <213> Artificial Sequence
<220>
   <223> Description of Artificial Sequence: Synthetic polypeptide
<400> 667
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Ala Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 668
   <211> 124
   <212> PRT
   <213> Artificial Sequence
<220>
   <223> Description of Artificial Sequence: Synthetic polypeptide
<400> 668
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Tyr Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 669
   <211> 124
   <212> PRT
   <213> Artificial Sequence
<220>
   <223> Description of Artificial Sequence: Synthetic polypeptide
<400> 669
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Asp Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 670
   <211> 124
   <212> PRT
   <213> Artificial Sequence
<220>
   <223> Description of Artificial Sequence: Synthetic polypeptide
<400> 670
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys His Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 671
   <211> 124
   <212> PRT
   <213> Artificial Sequence
<220>
   <223> Description of Artificial Sequence: Synthetic polypeptide
<400> 671
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Asn Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 672
   <211> 124
   <212> PRT
   <213> Artificial Sequence
<220>
   <223> Description of Artificial Sequence: Synthetic polypeptide
<400> 672
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Pro Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 673
   <211> 124
   <212> PRT
   <213> Artificial Sequence
<220>
   <223> Description of Artificial Sequence: Synthetic polypeptide
<400> 673
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Trp Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 674
   <211> 124
   <212> PRT
   <213> Artificial Sequence
<220>
   <223> Description of Artificial Sequence: Synthetic polypeptide
<400> 674
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Ala Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 675
   <211> 124
   <212> PRT
   <213> Artificial Sequence
<220>
   <223> Description of Artificial Sequence: Synthetic polypeptide
<400> 675
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Asp Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 676
   <211> 124
   <212> PRT
   <213> Artificial Sequence
<220>
   <223> Description of Artificial Sequence: Synthetic polypeptide
<400> 676
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys His Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 677
   <211> 124
   <212> PRT
   <213> Artificial Sequence
<220>
   <223> Description of Artificial Sequence: Synthetic polypeptide
<400> 677
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Asn Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 678
   <211> 124
   <212> PRT
   <213> Artificial Sequence
<220>
   <223> Description of Artificial Sequence: Synthetic polypeptide
<400> 678
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Pro Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 679
   <211> 124
   <212> PRT
   <213> Artificial Sequence
<220>
   <223> Description of Artificial Sequence: Synthetic polypeptide
<400> 679
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Arg Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 680
   <211> 124
   <212> PRT
   <213> Artificial Sequence
<220>
   <223> Description of Artificial Sequence: Synthetic polypeptide
<400> 680
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Thr Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 681
   <211> 124
   <212> PRT
   <213> Artificial Sequence
<220>
   <223> Description of Artificial Sequence: Synthetic polypeptide
<400> 681
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Trp Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 682
   <211> 124
   <212> PRT
   <213> Artificial Sequence
<220>
   <223> Description of Artificial Sequence: Synthetic polypeptide
<400> 682
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Gly Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 683
   <211> 124
   <212> PRT
   <213> Artificial Sequence
<220>
   <223> Description of Artificial Sequence: Synthetic polypeptide
<400> 683
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Ile Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 684
   <211> 124
   <212> PRT
   <213> Artificial Sequence
<220>
   <223> Description of Artificial Sequence: Synthetic polypeptide
<400> 684
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Lys Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 685
   <211> 124
   <212> PRT
   <213> Artificial Sequence
<220>
   <223> Description of Artificial Sequence: Synthetic polypeptide
<400> 685
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Pro Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 686
   <211> 124
   <212> PRT
   <213> Artificial Sequence
<220>
   <223> Description of Artificial Sequence: Synthetic polypeptide
<400> 686
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Gln Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 687
   <211> 124
   <212> PRT
   <213> Artificial Sequence
<220>
   <223> Description of Artificial Sequence: Synthetic polypeptide
<400> 687
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Arg Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 688
   <211> 124
   <212> PRT
   <213> Artificial Sequence
<220>
   <223> Description of Artificial Sequence: Synthetic polypeptide
<400> 688
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Ser Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 689
   <211> 124
   <212> PRT
   <213> Artificial Sequence
<220>
   <223> Description of Artificial Sequence: Synthetic polypeptide
<400> 689
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Thr Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 690
   <211> 124
   <212> PRT
   <213> Artificial Sequence
<220>
   <223> Description of Artificial Sequence: Synthetic polypeptide
<400> 690
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Val Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 691
   <211> 124
   <212> PRT
   <213> Artificial Sequence
<220>
   <223> Description of Artificial Sequence: Synthetic polypeptide
<400> 691
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Tyr Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 692
   <211> 124
   <212> PRT
   <213> Artificial Sequence
<220>
   <223> Description of Artificial Sequence: Synthetic polypeptide
<400> 692
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Ala
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 693
   <211> 124
   <212> PRT
   <213> Artificial Sequence
<220>
   <223> Description of Artificial Sequence: Synthetic polypeptide
<400> 693
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Gly
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 694
   <211> 124
   <212> PRT
   <213> Artificial Sequence
<220>
   <223> Description of Artificial Sequence: Synthetic polypeptide
<400> 694
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu His
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 695
   <211> 124
   <212> PRT
   <213> Artificial Sequence
<220>
   <223> Description of Artificial Sequence: Synthetic polypeptide
<400> 695
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Met
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 696
   <211> 124
   <212> PRT
   <213> Artificial Sequence
<220>
   <223> Description of Artificial Sequence: Synthetic polypeptide
<400> 696
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Pro
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 697
   <211> 124
   <212> PRT
   <213> Artificial Sequence
<220>
   <223> Description of Artificial Sequence: Synthetic polypeptide
<400> 697
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Gln
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 698
   <211> 124
   <212> PRT
   <213> Artificial Sequence
<220>
   <223> Description of Artificial Sequence: Synthetic polypeptide
<400> 698
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Asp Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 699
   <211> 124
   <212> PRT
   <213> Artificial Sequence
<220>
   <223> Description of Artificial Sequence: Synthetic polypeptide
<400> 699
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Gly Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 700
   <211> 124
   <212> PRT
   <213> Artificial Sequence
<220>
   <223> Description of Artificial Sequence: Synthetic polypeptide
<400> 700
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Ile Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 701
   <211> 124
   <212> PRT
   <213> Artificial Sequence
<220>
   <223> Description of Artificial Sequence: Synthetic polypeptide
<400> 701
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Asn Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 702
   <211> 124
   <212> PRT
   <213> Artificial Sequence
<220>
   <223> Description of Artificial Sequence: Synthetic polypeptide
<400> 702
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Ser Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 703
   <211> 124
   <212> PRT
   <213> Artificial Sequence
<220>
   <223> Description of Artificial Sequence: Synthetic polypeptide
<400> 703
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Val Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 704
   <211> 124
   <212> PRT
   <213> Artificial Sequence
<220>
   <223> Description of Artificial Sequence: Synthetic polypeptide
<400> 704
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Ile Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 705
   <211> 124
   <212> PRT
   <213> Artificial Sequence
<220>
   <223> Description of Artificial Sequence: Synthetic polypeptide
<400> 705
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Met Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 706
   <211> 124
   <212> PRT
   <213> Artificial Sequence
<220>
   <223> Description of Artificial Sequence: Synthetic polypeptide
<400> 706
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Trp Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 707
   <211> 124
   <212> PRT
   <213> Artificial Sequence
<220>
   <223> Description of Artificial Sequence: Synthetic polypeptide
<400> 707
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Tyr Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 708
   <211> 124
   <212> PRT
   <213> Artificial Sequence
<220>
   <223> Description of Artificial Sequence: Synthetic polypeptide
<400> 708
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro His Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 709
   <211> 124
   <212> PRT
   <213> Artificial Sequence
<220>
   <223> Description of Artificial Sequence: Synthetic polypeptide
<400> 709
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Leu Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 710
   <211> 124
   <212> PRT
   <213> Artificial Sequence
<220>
   <223> Description of Artificial Sequence: Synthetic polypeptide
<400> 710
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Thr Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 711
   <211> 124
   <212> PRT
   <213> Artificial Sequence
<220>
   <223> Description of Artificial Sequence: Synthetic polypeptide
<400> 711
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Val Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 712
   <211> 124
   <212> PRT
   <213> Artificial Sequence
<220>
   <223> Description of Artificial Sequence: Synthetic polypeptide
<400> 712
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Trp Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 713
   <211> 124
   <212> PRT
   <213> Artificial Sequence
<220>
   <223> Description of Artificial Sequence: Synthetic polypeptide
<400> 713
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Leu Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 714
   <211> 124
   <212> PRT
   <213> Artificial Sequence
<220>
   <223> Description of Artificial Sequence: Synthetic polypeptide
<400> 714
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro His Tyr Leu Gly Ile Gly Asn Gly Thr Gln Ile
   100 105 110
   Tyr Val Ile Asp Pro Glu Pro Cys Pro Asp Ser Asp
   115 120
<210> 715
   <211> 124
   <212> PRT
   <213> Artificial Sequence
<220>
   <223> Description of Artificial Sequence: Synthetic polypeptide
<400> 715
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr His Leu Gly Ile Gly Asn Gly Thr Gln Ile
               100                 105                 110
   Tyr Val Ile Asp Pro Glu Pro Cys Pro Asp Ser Asp
           115                 120
<210> 716
   <211> 124
   <212> PRT
   <213> Artificial Sequence
<220>
   <223> Description of Artificial Sequence: Synthetic polypeptide
<400> 716
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Leu Leu Gly Ile Gly Asn Gly Thr Gln Ile
   100 105 110
   Tyr Val Ile Asp Pro Glu Pro Cys Pro Asp Ser Asp
   115 120
<210> 717
   <211> 124
   <212> PRT
   <213> Artificial Sequence
<220>
   <223> Description of Artificial Sequence: Synthetic polypeptide
<400> 717
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Pro Leu Gly Ile Gly Asn Gly Thr Gln Ile
   100 105 110
   Tyr Val Ile Asp Pro Glu Pro Cys Pro Asp Ser Asp
   115 120
<210> 718
   <211> 124
   <212> PRT
   <213> Artificial Sequence
<220>
   <223> Description of Artificial Sequence: Synthetic polypeptide
<400> 718
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Thr Leu Gly Ile Gly Asn Gly Thr Gln Ile
   100 105 110
   Tyr Val Ile Asp Pro Glu Pro Cys Pro Asp Ser Asp
   115 120
<210> 719
   <211> 124
   <212> PRT
   <213> Artificial Sequence
<220>
   <223> Description of Artificial Sequence: Synthetic polypeptide
<400> 719
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Pro Gly Ile Gly Asn Gly Thr Gln Ile
               100                 105                 110
   Tyr Val Ile Asp Pro Glu Pro Cys Pro Asp Ser Asp
           115                 120
<210> 720
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 720
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Ser Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 721
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 721
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Pro Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 722
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 722
   Met His Val Ala Gln Pro Ala Val Val Leu Ala Ser Ser Arg Gly Ile
   1 5 10 15
   Ala Ser Phe Val Cys Glu Tyr Ala Ser Pro His Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 723
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 723
   Met His Val Ala Gln Pro Ala Val Val Leu Ala Ser Ser Arg Gly Ile
   1               5                   10                  15
   Ala Ser Phe Val Cys Glu Tyr Ala Ser Pro Thr Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 724
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 724
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Asn Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 725
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 725
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Lys Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 726
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 726
   Met His Val Ala Gln Pro Ala Val Val Leu Ala Ser Ser Arg Gly Ile
   1 5 10 15
   Ala Ser Phe Val Cys Glu Tyr Ala Ser Pro Ser Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 727
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 727
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Thr Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 728
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 728
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Trp Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 729
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 729
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Gly Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 730
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 730
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Gly Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 731
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 731
   Met His Val Ala Gln Pro Ala Val Val Leu Ala Ser Ser Arg Gly Ile
   1               5                   10                  15
   Ala Ser Phe Val Cys Glu Tyr Ala Ser His Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 732
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 732
   Met His Val Ala Gln Pro Ala Val Val Leu Ala Ser Ser Arg Gly Ile
   1 5 10 15
   Ala Ser Phe Val Cys Glu Tyr Ala Ser Lys Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 733
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 733
   Met His Val Ala Gln Pro Ala Val Val Leu Ala Ser Ser Arg Gly Ile
   1               5                   10                  15
   Ala Ser Phe Val Cys Glu Tyr Ala Ser Ser Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 734
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 734
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Val Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 735
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 735
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Ala Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 736
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 736
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Asp Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 737
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 737
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Asn Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 738
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 738
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Gln Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 739
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 739
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Tyr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 740
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 740
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Val Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 741
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 741
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Val Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 742
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 742
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Glu Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 743
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 743
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asp Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 744
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 744
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Ser Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 745
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 745
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Ala Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 746
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 746
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asn Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
                   100                 105                 110
   Tyr Val Ile Asp Pro Glu Pro Cys Pro Asp Ser Asp
           115                 120
<210> 747
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 747
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Glu Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 748
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 748
   Met His Val Ala Gln Pro Ala Val Val Leu Ala Ser Ser Arg Gly Ile
   1 5 10 15
   Ala Ser Phe Val Cys Glu Tyr Ala Ser Pro Gly Asp Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 749
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 749
   Met His Val Ala Gln Pro Ala Val Val Leu Ala Ser Ser Arg Gly Ile
   1 5 10 15
   Ala Ser Phe Val Cys Glu Tyr Ala Ser Ala Gly Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 750
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 750
   Met His Val Ala Gln Pro Ala Val Val Leu Ala Ser Ser Arg Gly Ile
   1 5 10 15
   Ala Ser Phe Val Cys Glu Tyr Ala Ser Pro Val Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 751
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 751
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Ala Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 752
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 752
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Ile
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 753
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 753
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Gly Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 754
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 754
   Met His Val Ala Gln Pro Ala Val Val Leu Ala Ser Ser Arg Gly Ile
   1 5 10 15
   Ala Ser Phe Val Cys Glu Tyr Ala Ser Pro Arg Lys Ala Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 755
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 755
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Gln Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 756
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 756
   Met His Val Ala Gln Pro Ala Val Val Leu Ala Ser Ser Arg Gly Ile
   1 5 10 15
   Ala Ser Phe Val Cys Glu Tyr Ala Ser Pro Gly Lys Ala Arg Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 757
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 757
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Lys Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys Pro Asp Ser Asp
           115                 120
<210> 758
   <211> 124
   <212> PRT
   <213> Artificial Sequence
<220>
   <223> Description of Artificial Sequence: Synthetic polypeptide
<220>
   <223> see specification as filed for detailed description of substitutions and preferred embodiments
<400> 758
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Val Gly Ile Gly Asn Gly Thr Gln Ile
               100                 105                 110
   Tyr Val Ile Asp Pro Glu Pro Cys Pro Asp Ser Asp
           115                 120
<210> 759
   <211> 124
   <212> PRT
   <213> Artificial Sequence
<220>
   <223> Description of Artificial Sequence: Synthetic polypeptide
<400> 759
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Tyr Thr Glu Val
   20 25 30
   Arg Val Thr Val Leu Arg Gln Ala Asp Ser Gln Val Thr Glu Val Cys
   35 40 45
   Ala Ala Thr Tyr Met Met Gly Asn Glu Leu Thr Phe Leu Asp Asp Ser
   50 55 60
   Ile Cys Thr Gly Thr Ser Ser Gly Asn Gln Val Asn Leu Thr Ile Gln
   65 70 75 80
   Gly Leu Arg Ala Met Asp Thr Gly Leu Tyr Ile Cys Lys Val Glu Leu
   85 90 95
   Met Tyr Pro Pro Pro Tyr Tyr Leu Gly Ile Gly Asn Gly Thr Gln Ile
   100 105 110
   Tyr Val Ile Asp Pro Glu Pro Cys Pro Asp Ser Asp
   115 120
<210> 760
   <211> 124
   <212> PRT
   <213> Artificial Sequence
<220>
   <223> Description of Artificial Sequence: Synthetic polypeptide
<400> 760
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Glu Gly Ile Gly Asn Gly Thr Gln Ile
               100                 105                 110
   Tyr Val Ile Asp Pro Glu Pro Cys Pro Asp Ser Asp
           115                 120
<210> 761
   <211> 124
   <212> PRT
   <213> Artificial Sequence
<220>
   <223> Description of Artificial Sequence: Synthetic polypeptide
<400> 761
   Met His Val Ala Gln Pro Ala Val Val Leu Ala Ser Ser Arg Gly Ile
   1               5                   10                  15
   Ala Ser Phe Val Cys Glu Tyr Ala Ser Pro Gly Lys Tyr Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Glu Gly Ile Gly Asn Gly Thr Gln Ile
               100                 105                 110
   Tyr Val Ile Asp Pro Glu Pro Cys Pro Asp Ser Asp
           115                 120

## Description

### 1. CROSS REFERENCE TO RELATED APPLICATIONS

### 1.1 SEQUENCE LISTING

The instant application contains a Sequence Listing which has been submitted in ASCII format via EFS-Web.

### 2. FIELD OF THE INVENTION

The present invention relates to CTLA4 proteins, pharmaceutical compositions comprising CTLA4 proteins, and therapeutic uses of such proteins.

### 3. BACKGROUND

T-cell lymphocytes play a critical role in cell-mediated immunity by providing for an adaptive response to specific pathogens. T-cell activation depends on activation of at least two signaling pathways, one that is antigen specific and the other that is antigen nonspecific. A T-helper cell antigenic response requires activation of a first signaling pathway by the binding of the T-cell receptor to an antigen bound to MHC (major histocompatibility complex) on the surface of an antigen presenting cell (APC). The antigenic response also requires activation of a second signaling pathway, which produces a co-stimulatory signal, by the binding of CD28 protein on the surface of the T-cell to CD80 (B7-1) and CD86 (B7-2) on the surface of the APC. Because the antigen-specific signal is not sufficient to generate a full antigenic response, T-cell receptor/antigen-MHC binding without co-stimulation may lead to clonal inactivation or anergy. (*See, e.g.,* Jenkins et al., 1993, Curr. Opin. Immunol. 5:361). The co-stimulatory pathway plays a role not only in T-cell activation and differentiation, but also in tissue migration and peripheral tolerance induction. (*See* Salomon et al., 2001, Ann. Rev. Immunol. 19:225.)

Activated T-cells also express on their cell surface cytotoxic T-lymphocyte-associated antigen 4 (CTLA4), a homologue of CD28 that binds CD80 and CD86 with higher affinity. CTLA4 expression results in competitive binding to CD80 and CD86, blocking the CD80/86-CD28 interaction and terminating T-cell activation. Because these signaling pathways determine the magnitude of a T-cell response to antigen, as well as downstream responses to antigen, agents that modulate one or more costimulatory signals, e.g., by blocking one or more of the interactions between CD80/CD86 and CD28 and/or CTLA4, may be effective in treating disorders that result from disregulated cell-mediated immune responses. (*See, e.g.,* Mueller, et al., 1989, Annu. Rev. Immunol. 7:445).

Suitable agents for the disruption of the CD80/86-CD28 interaction include antibodies and fusion proteins. For example, in an experimental autoimmune encephalomyelitis (EAE) model, T-cells were blocked using anti-CD80 and anti-CD86 antibodies. (*See* Racke, et al., 1995, J. Clin. Invest. 96:2195). In another example, anti-CD80 and anti-CD86 antibodies were used to block type 2 *in vivo* immune responses (*i.e.,* immune responses mediated by IgG antibodies and directed against cell-surface or matrix antigens) and the production of IL-4. (Greenwald, et al., 1997, J. Immunol. 158:4088) CD28-Ig fusion proteins have been demonstrated to interact with CD80 and/or CD86 on dendritic cells and to activate them, which results in an adjuvant activity of soluble CD28 that produces enhanced T-cell mediated immune responses *in vivo*. (Orabona et al., 2004, Nature Immunol. 5(11):1134). Alternatively, CTLA4-Ig fusion proteins have been used to disrupt CD80/86-CD28 interactions. (*See, e.g.,* McIntyre, et al., 2005, Drugs of the Future, 30:873).

Recent studies of T-cell activation and the costimulatory pathway indicate that CD80 and CD86 can play different roles in immune responses, and especially in autoimmunity. Thus, blocking the binding of only one of CD80 or CD86 to CD28 has been shown to produce disparate effects *in vivo,* depending on the disease being studied and the timing of administration (*See* Salomon, 2001, Annu. Rev. Immunol. 19:225; Kuchroo, et al., 1995, Cell 80:707; Racke, et al., 1995, J. Clin. Invest. 96:2195; Lenschow et al., 1995, J. Exp. Med. 181:1145; and Greenwald, et al., J. Immunol., 1997, 158:4088).

Published PCT applications WO 2008/047150 and WO 2009/058564 disclose protein variants and mutants respectively of CTLA4, for the treatment of disorders, whereby attentuation of the T cell response would be beneficial.

Antibodies that bind to CTLA4 and disrupt CTLA4 inhibition of CD28-mediated T-cell responses are currently in development for the treatment of various cancers. Ipilimumab, a human anti-CTLA4 IgG₁ antibody, is in Phase III trials for melanoma and in Phase II trials for breast cancer and prostate cancer. Tremelimumab, a human anti-CTLA4 IgG₂ antibody, is in Phase II trials for prostate cancer, bladder cancer, and colorectal cancer.

Fusion proteins that bind to CD80 and/or CD86 and disrupt the interaction with CTLA4 and CD28 are good candidates for the development of immunomodulatory agents. Fusion proteins allow for the engineering of proteins with, for example, improved solubility, stability, deliverability, and/or activity. One such immunomodulatory agent is the soluble fusion protein abatacept (Orencia®), developed for the treatment of rheumatoid arthritis (RA) and juvenile idiopathic arthritis, autoimmune disorders which attack the joints, causing painful swelling that can eventually result in bone erosion and joint deformity.

Abatacept is a CTLA4-Ig fusion protein consisting of the extracellular domain of human CTLA4 and a modified Fc portion of human immunoglobulin G₁ (IgG₁) that includes hinge, CH2, and CH3 domains. The CTLA4 domain provides binding activity and the Fc portion is believed to provide stability, solubility and deliverability. Abatacept inhibits the interaction of CD28 on T-cells with CD80 and/or CD86 proteins on APC cells, thereby resulting in inhibition of T-cell activation. (*See, e.g.,* Schwartz, 1992, Cell 71:1065). Accordingly, administration of abatacept results in a decrease in T-cell proliferation, activation and signaling, and attenuates the autoimmune response in rheumatoid arthritis. A second-generation soluble recombinant CTLA4-Ig fusion protein known as belatacept differs from abatacept in two amino acids and has a greater binding affinity for CD80 and CD86. Belatacept is in ongoing Phase III trials for use in kidney and other organ transplantation. (*See* Linsley et al., 2009, Immunol. Reviews 229:307-321). A third fusion protein, MAXY-4, a protein derived from abatacept but having increased binding to CTLA4 targets, is currently in preclinical development by Perseid Therapeutics, LLC and Astellas Pharma, Inc. for treatment of autoimmune diseases and transplant rejection.

Therapy with abatacept can be cost prohibitive. Higher affinity variants of abatacept could reduce the required effective therapeutic dose and therefore reduce the cost of therapy. Moreover, because less of the agent is required to achieve a beneficial result, the risk of developing an immunogenic response to the agent itself may also be decreased. Other side-effects may also be reduced. There is a need to provide improved fusion proteins with differential binding to the CD80 and CD86 proteins, for example, by generating variants with higher relative affinity for one isoform relative to the other. Such improved variants may provide targeted immune modulation for specific conditions or pathologies. Adverse effects or exacerbation of immune responses observed in some cases of CTLA4-Ig and anti-CTLA4 antibody therapies may also be reduced.

Citation or identification of any reference in Section 2 or in any other section of this application shall not be construed as an admission that such reference is available as prior art to the present disclosure.

### 4. SUMMARY

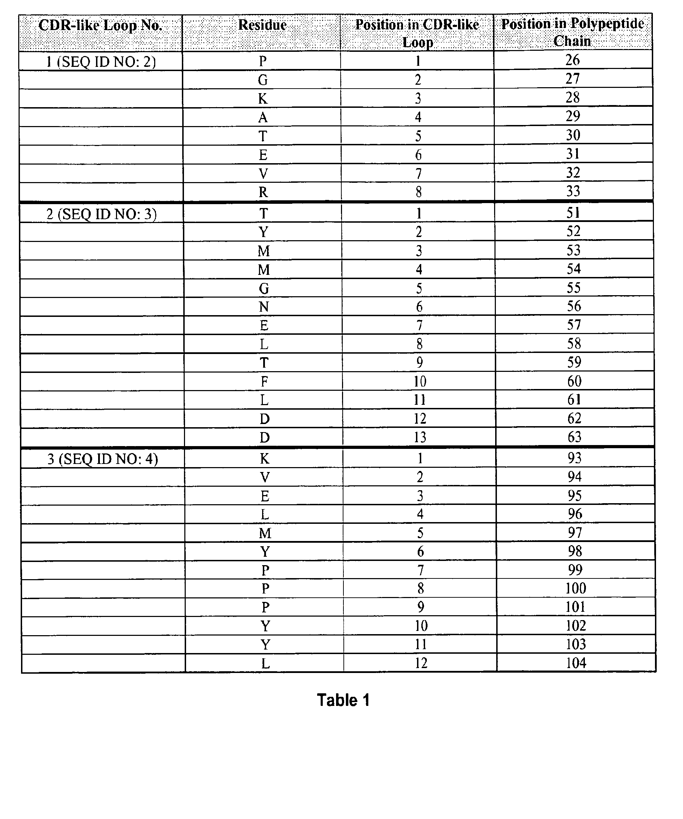

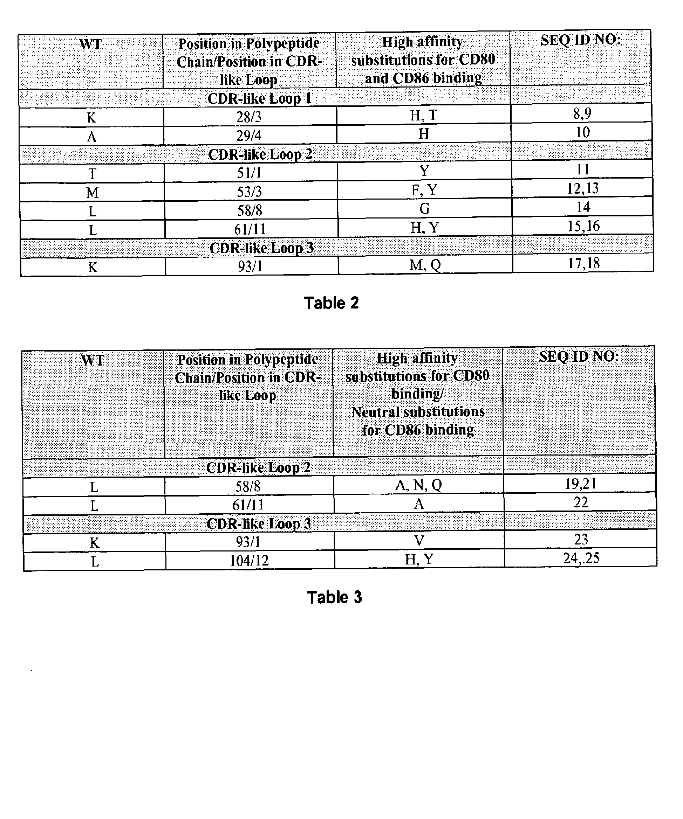

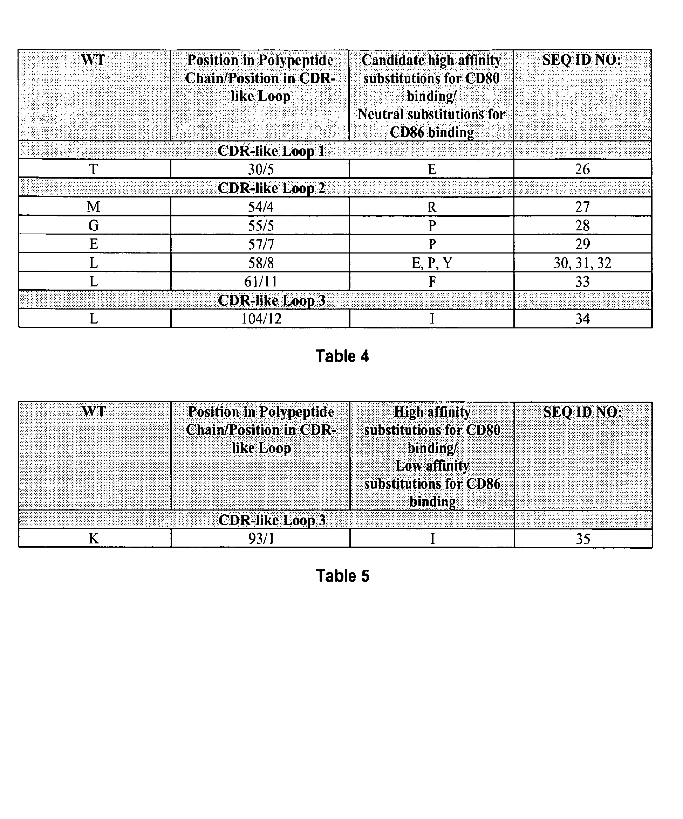

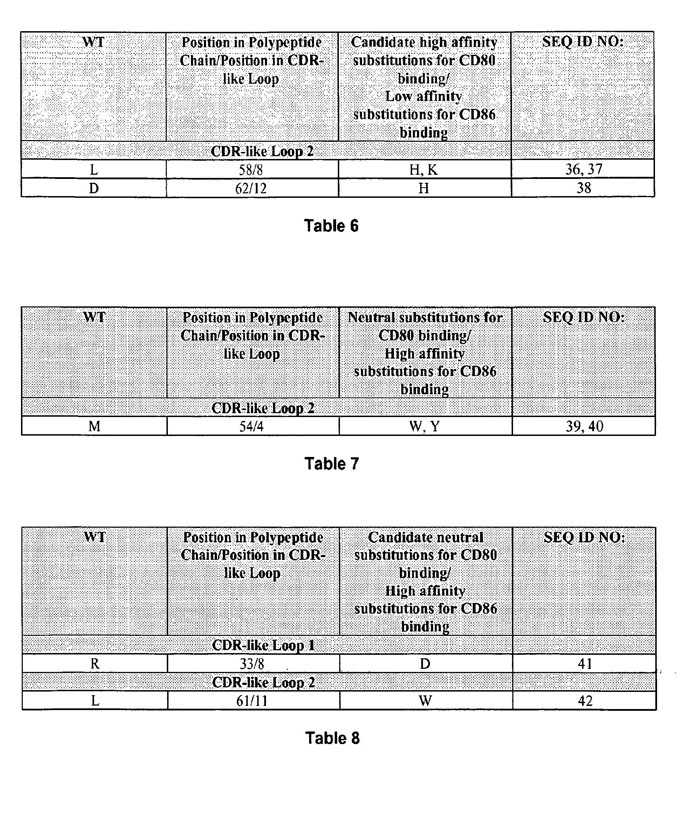

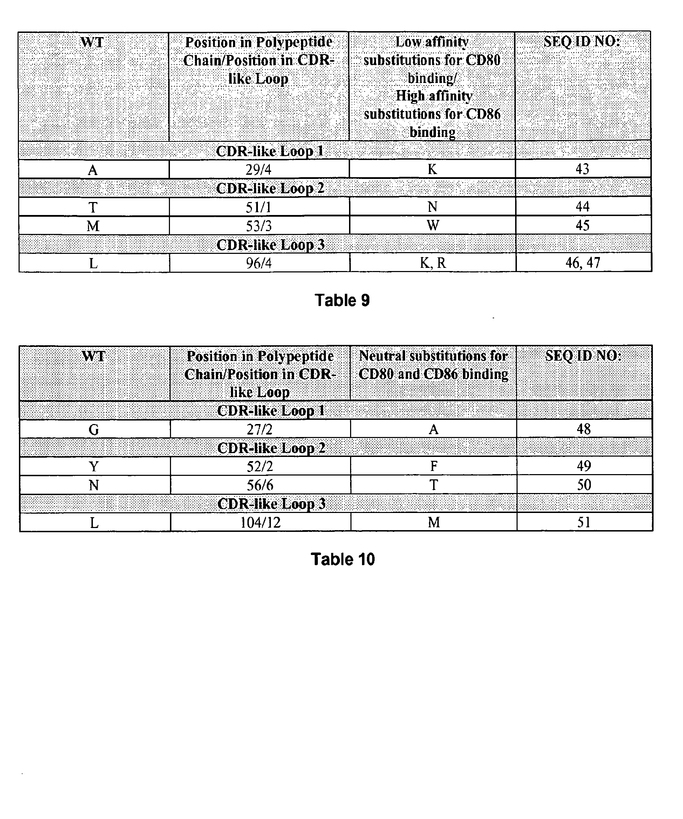

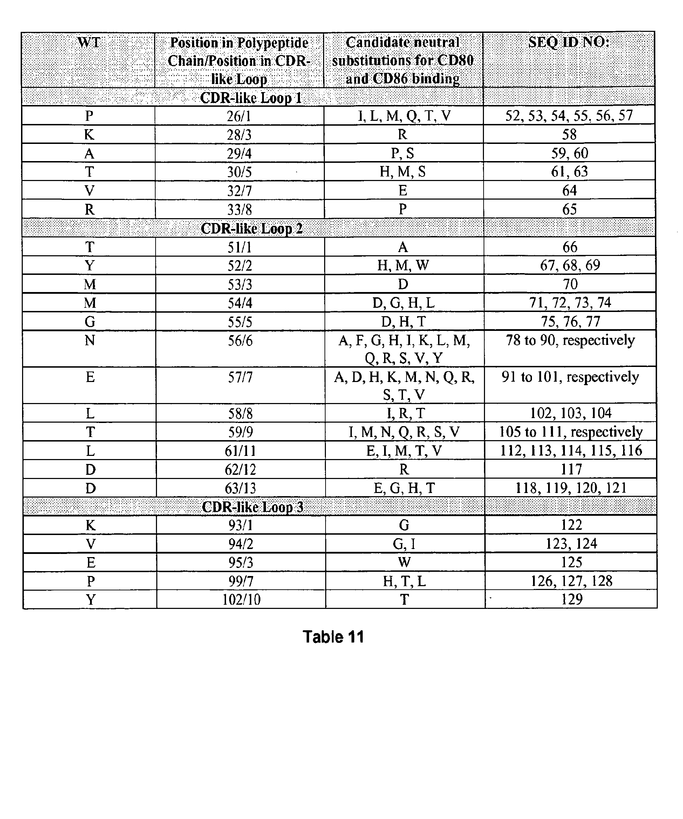

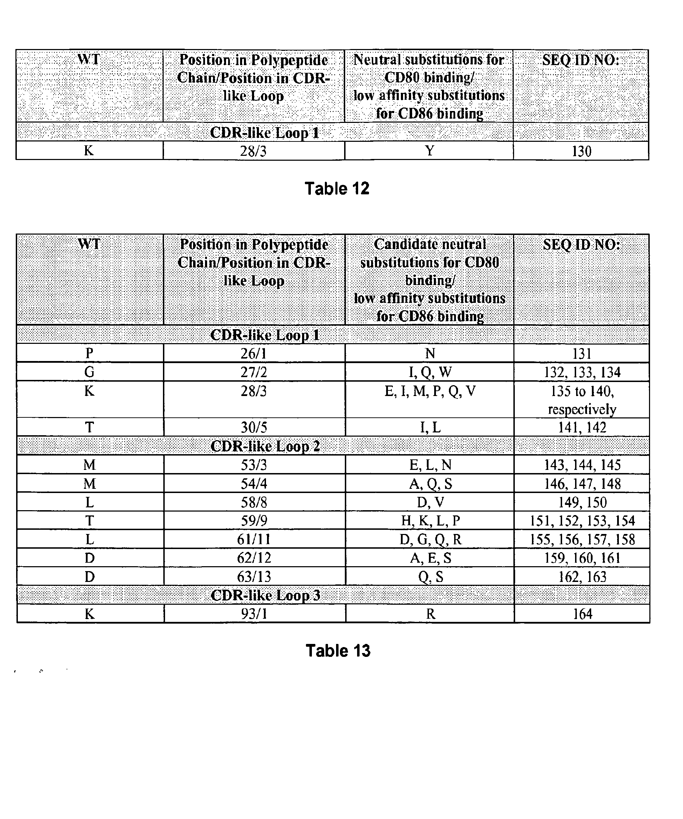

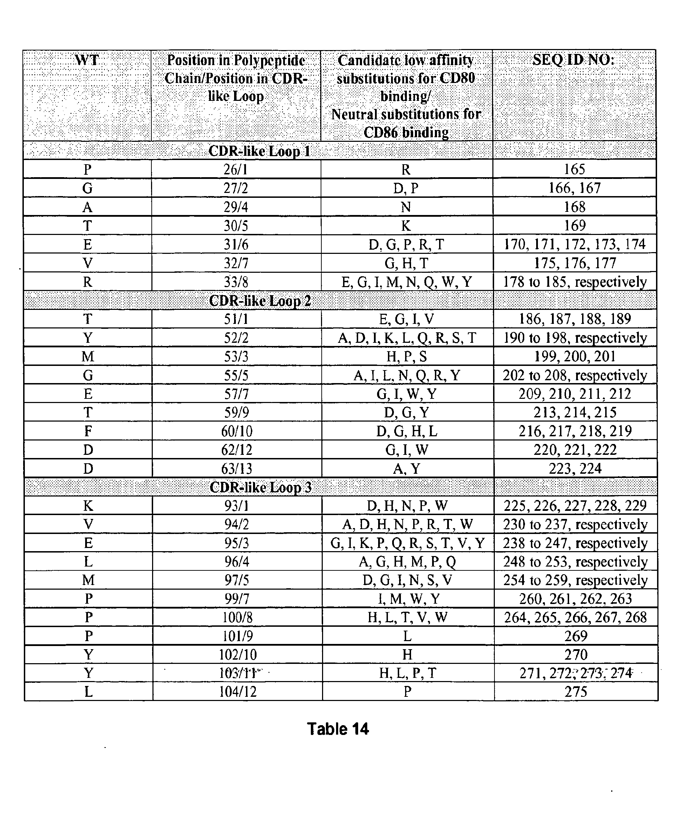

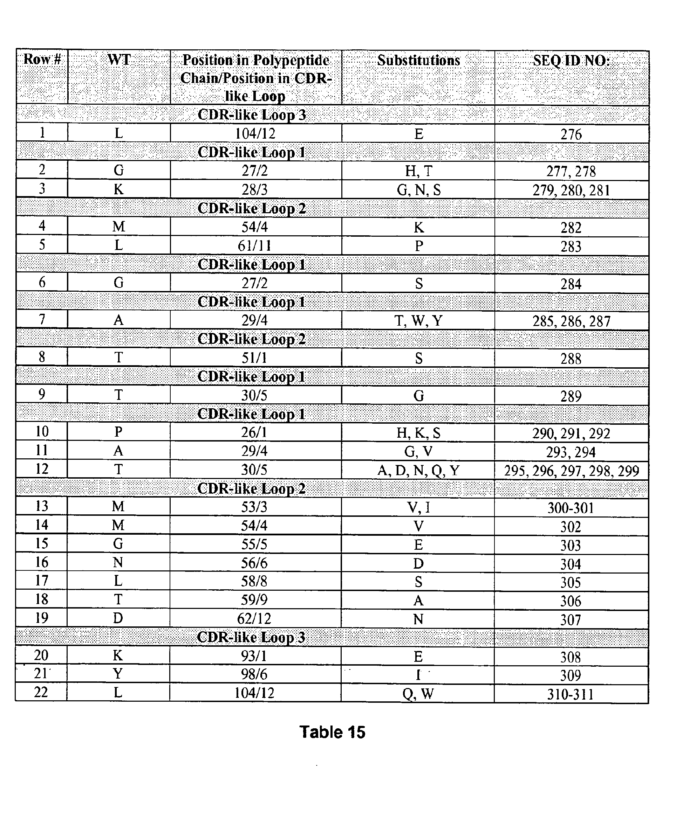

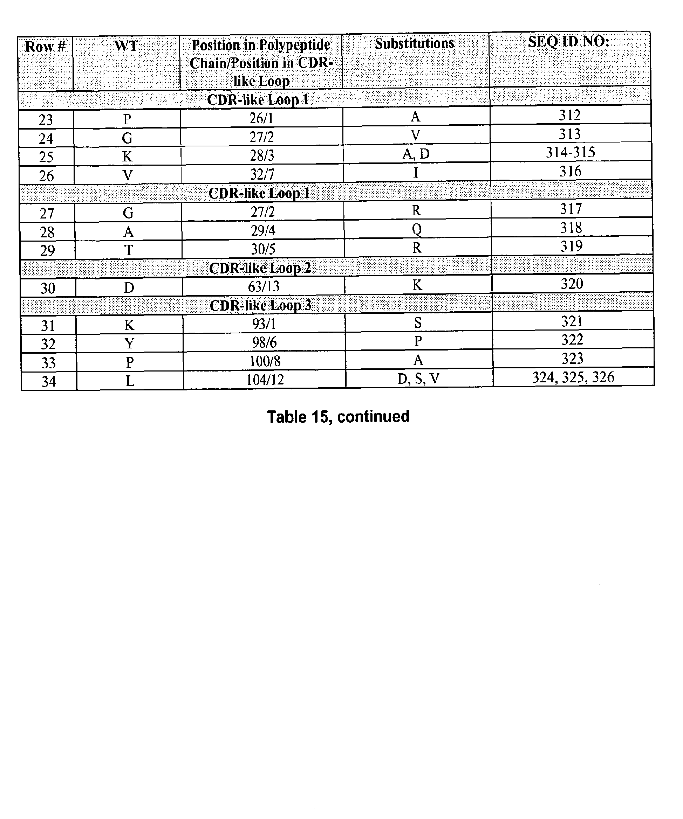

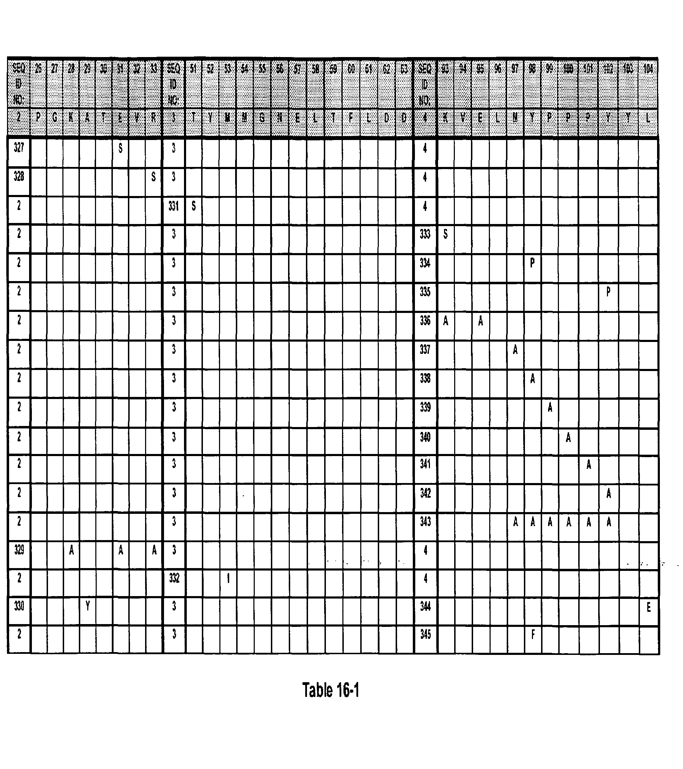

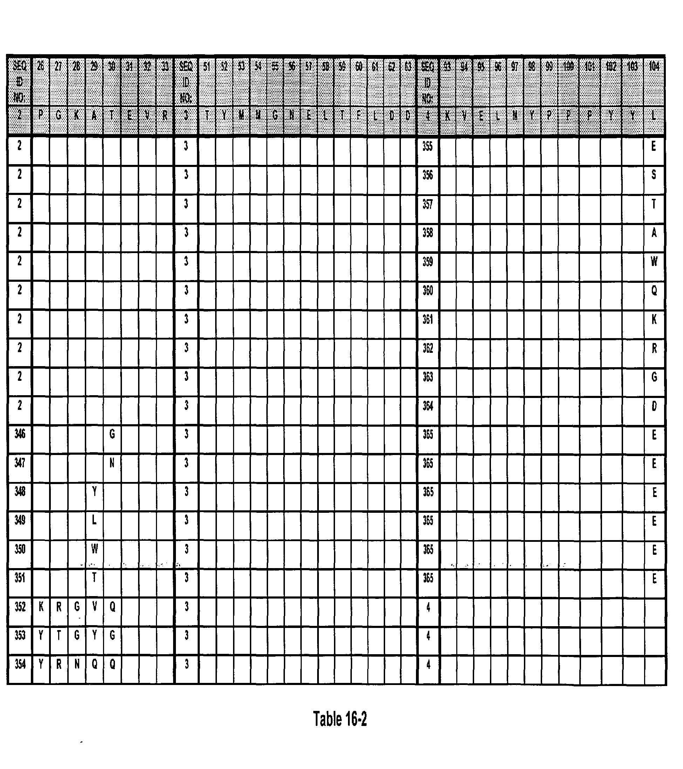

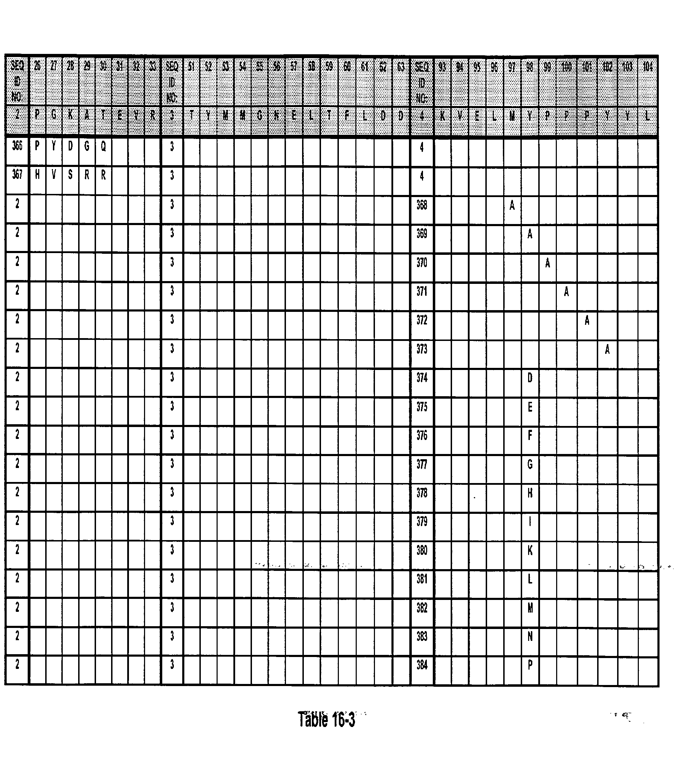

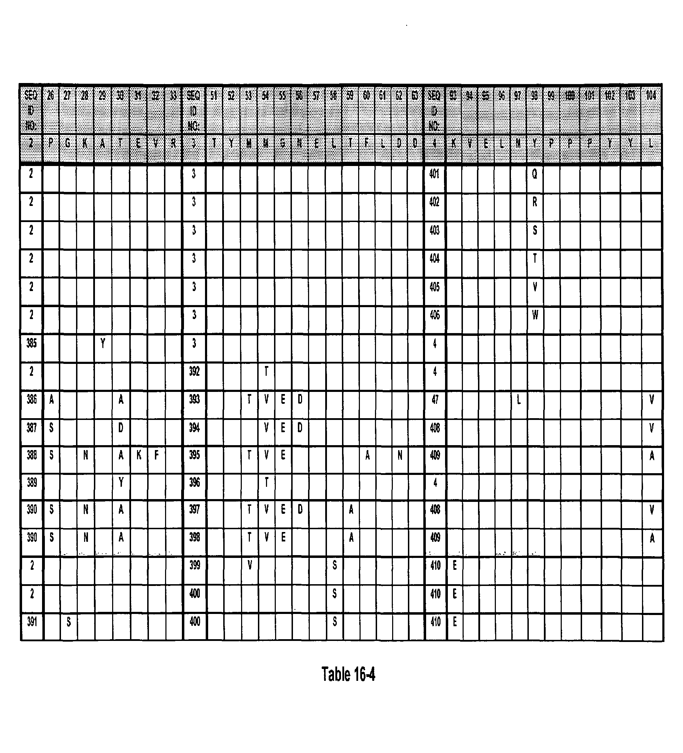

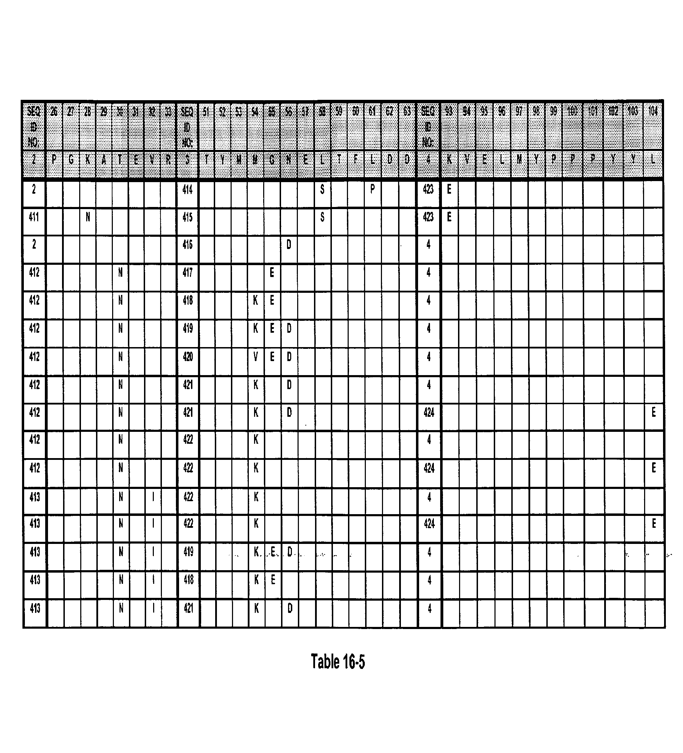

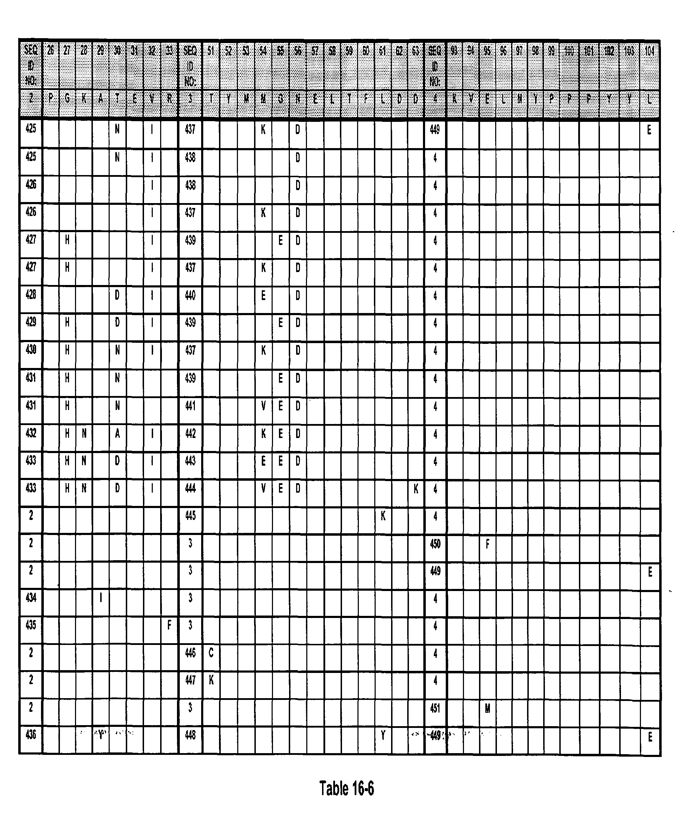

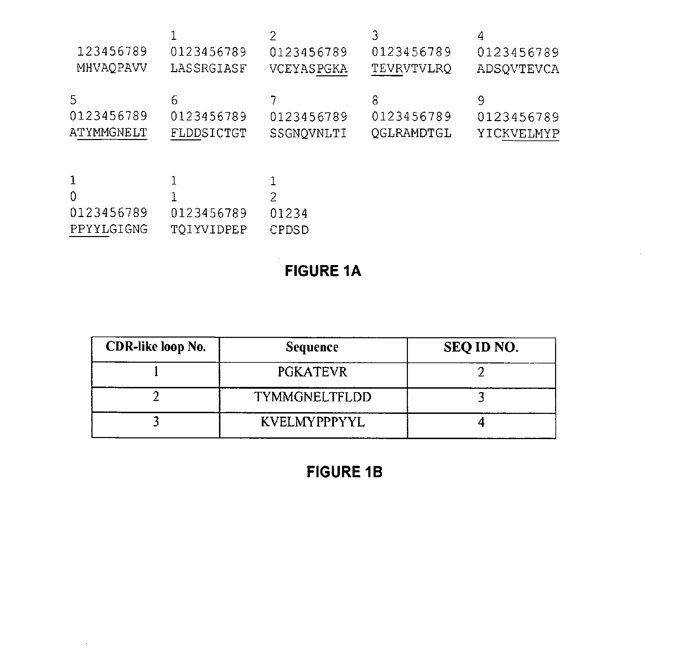

The present disclosure relates to proteins comprising a CTLA4 binding domain that are capable of binding CD80 and/or CD86 and that are related in sequence to SEQ ID NO: 1 (Figure 1A). In certain aspects, the CTLA4 protein of the disclosure further comprises a fusion partner, such as an immunoglobulin domain, which is a non-CTLA4 protein.

CTLA4 has three CDR-like loops, referred to herein (in amino- to carboxy-terminal order) as CDR-like loop 1, CDR-like loop 2 and CDR-like loop 3. The CDR-like loops of CTLA4 is shown in Figure 1B.

CTLA4 was subjected to comprehensive mutagenesis in its CDR-like loops to produce a population of variants with single point mutations. The variant population was then screened to identify point mutants that resulted in similar or increased binding affinity to CD80 or CD86 based on the variants' behavior in a population.

Variants were identified whose behavior in the population indicated a similar or higher binding affinity to CD80 or to CD86 relative to wild type CTLA4. Some of the variants were isolated from the population and their biological properties, such as binding to CD80 and CD86 (described in Example 1), inhibition of IL-2 secretion by Jurkat cells (described in Example 1) and inhibition of T-cell proliferation (described in Example 2), were further characterized. The amino acid substitutions in these variants are listed in Tables 2, 3, 5, 7, 9, 10 and 12. Other variants were not isolated from the population and their binding characteristics are based on their behavior in the population. The amino acid substitutions in these variants, which are referred to as "candidate" mutations, are listed in Tables 4, 6, 8, 11, 13, 14.

A variant CTLA4 polypeptide that has greater affinity to CD80 and/or CD86 than the CTLA4 binding domain of amino acids 21-112 of SEQ ID NO:1, said variant CTLA4 polypeptide comprising an amino acid sequence having at least 90%, at least 95%, at least 97%, at least 98% or at least 99% sequence identity to said CTLA4 binding domain and the amino acid substitution K28H as compared to SEQ ID NO:1.

Accordingly, the present disclosure provides CTLA4 proteins comprising CDR-like loops that have at least one amino acid substitution as compared to the CDR-like loops of SEQ ID NO: 1. Exemplary substitutions in the CDR-like loops are found in Tables 2, 3, 4, 5, 6, 7, 8, 9, 10, 11, 12, 13, and 14. Preferred amino acid substitutions that can be incorporated into the CTLA4 proteins of the disclosure (singly or in combination) include, but are not limited to, the amino acid substitutions K28H and A29H in CDR-like loop 1.

In certain aspects, the present disclosure provides CTLA4 proteins that have at least one amino acid substitution in the CDR-like loops as compared to SEQ ID NO: 1 and that bind with high affinity to CD80 and CD86 as compared to a wild-type CTLA4 binding domain. Exemplary substitutions in the CDR-like loops that result in high affinity binding to CD80 and CD86 are found in Table 2 and in row 1 of Table 15.

In other aspects, the present disclosure provides CTLA4 proteins that have at least one amino acid substitution in the CDR-like loops as compared to the CDR-like loops of SEQ ID NO: 1 that bind with high affinity to CD80 and with neutral affinity (*i.e.,* comparable to abatacept) to CD86 as compared to a wild-type CTLA4 binding domain. Exemplary substitutions in the CDR-like loops that result in high affinity binding to CD80 and with neutral affinity binding to CD86 are found in Tables 3, 4 and in rows 2-5 of Table 15.

In yet other embodiments, the present disclosure provides CTLA4 proteins that have at least one amino acid substitution in the CDR-like loops as compared to the CDR-like loops of SEQ ID NO: 1 that bind with high affinity to CD80 and with low affinity to CD86 as compared to a wild-type CTLA4 binding domain. Exemplary substitutions that result in high affinity binding to CD80 and low affinity binding to CD86 are found in Tables 5, 6 and in row 6 of Table 15.

In some embodiments, the present disclosure provides CTLA4 proteins that have at least one amino acid substitution in the CDR-like loops as compared to the CDR-like loops of SEQ ID NO: 1 that bind with neutral affinity to CD80 and with high affinity to CD86 as compared to a wild-type CTLA4 binding domain. Exemplary substitutions that result in neutral affinity binding to CD80 and high affinity binding to CD86 are found in Tables 7, 8 and rows 7-8 of Table 15.

In various embodiments, the present disclosure provides CTLA4 proteins that have at least one amino acid substitution in the CDR-like loops as compared to the CDR-like loops of SEQ ID NO: 1 and that bind with low affinity to CD80 and with high affinity to CD86 as compared to wild-type CTLA4 binding domain. Exemplary substitutions that result in low affinity binding to CD80 and high affinity binding to CD86 are found in Table 9 and in row 9 of Table 15.

Described herein are CTLA4 proteins that have at least one amino acid substitution in the CDR-like loops as compared to the CDR-like loops of SEQ ID NO: 1 that bind with neutral affinity to CD80 and CD86 as compared to a wild-type CTLA4 binding domain. Exemplary substitutions that result in neutral affinity binding to CD80 and CD86 are found in Tables 10, 11 and rows 10-22 of Table 15.

Described herein is CTLA4 proteins that have at least one amino acid substitution in the CDR-like loops as compared to the CDR-like loops of SEQ ID NO: 1 that bind with neutral affinity to CD80 and with low affinity to CD86 as compared to a wild-type CTLA4 binding domain. Exemplary substitutions that bind with neutral affinity to CD80 and with low affinity to CD86 are found in Tables 12, 13 and rows 23-26 of Table 15.

Described herein is CTLA4 proteins that have at least one amino acid substitution in the CDR-like loops as compared to the CDR-like loops of SEQ ID NO: 1 that bind with low affinity to CD80 and with neutral affinity to CD86 as compared to a wild-type CTLA4 binding domain. Exemplary substitutions that bind with low affinity to CD80 and with neutral affinity to CD86 are found in Table 14 and in rows 27-34 of Table 15.

Described herein is a variant CTLA4 polypeptide comprising an amino acid sequence having at least 90%, at least 95%, at least 97%, at least 98% or at least 99% sequence identity to amino acids 21 to 112 of SEQ ID NO: 1, wherein the variant polypeptide has at least one amino acid substitution as compared to SEQ ID NO: 1 selected from K28H, K28T, A29H, T51Y, M53F, M53Y, L58G, L61H, L61Y, K93M and K93Q.

Described herein is a variant CTLA4 polypeptide having at least 90%, at least 95%, at least 97%, at least 98% or at least 99% sequence identity to amino acids 21 to 112 of SEQ ID NO: 1, wherein the variant polypeptide has at least one amino acid substitution as compared to SEQ ID NO: 1 selected from L58A, L58N, L58Q, L61A, K93V, L104H and L104Y.

Also described herein is a variant CTLA4 polypeptide having at least 90%, at least 95%, at least 97%, at least 98% or at least 99% sequence identity to amino acids 21 to 112 of SEQ ID NO: 1, wherein the variant polypeptide has at least one amino acid substitution as compared to SEQ ID NO: 1 selected from T30E, M54R, G55P, E57P, L58E, L58P, L58Y, L61F and L104I.

In various embodiments, the present disclosure provides a variant CTLA4 polypeptide having at least 90%, at least 95%, at least 97%, at least 98% or at least 99% sequence identity to amino acids 21 to 112 of SEQ ID NO: 1, wherein the variant polypeptide includes the amino acid substitution K93I as compared to SEQ ID NO: 1.

In other embodiments, the present disclosure provides a variant CTLA4 polypeptide having at least 90%, at least 95%, at least 97%, at least 98% or at least 99% sequence identity to amino acids 21 to 112 of SEQ ID NO: 1, wherein the variant polypeptide has at least one amino acid substitution as compared to SEQ ID NO: 1 selected from L58H, L58K and D62H.

In some embodiments, the present disclosure provides a variant CTLA4 polypeptide having at least 90%, at least 95%, at least 97%, at least 98% or at least 99% sequence identity to amino acids 21 to 112 of SEQ ID NO: 1, wherein the variant polypeptide includes at least one amino acid substitution selected from M54W and M54Y as compared to SEQ ID NO: 1.

In other embodiments, the present disclosure provides a variant CTLA4 polypeptide having at least 90%, at least 95%, at least 97%, at least 98% or at least 99% sequence identity to amino acids 21 to 112 of SEQ ID NO: 1, wherein the variant polypeptide has at least one amino acid substitution as compared to SEQ ID NO: 1 selected from R33D and L61W.

In still other embodiments, the present disclosure provides a variant CTLA4 polypeptide having at least 90%, at least 95%, at least 97%, at least 98% or at least 99% sequence identity to amino acids 21 to 112 of SEQ ID NO: 1, wherein the variant polypeptide has at least one amino acid substitution as compared to SEQ ID NO: 1 selected from A29K, T51N, M53W, L96K and L96R.

In various embodiments, the present disclosure provies a variant CTLA4 polypeptide comprising an amino acid sequence having at least 90%, at least 95%, at least 97%, at least 98% or at least 99% sequence identity to amino acids 21 to 112 of SEQ ID NO: 1, wherein the variant polypeptide has at least one amino acid substitution as compared to SEQ ID NO: 1 selected from G27A, Y52F, N56T and L104M.

Described herein is a variant CTLA4 polypeptide having at least 90%, at least 95%, at least 97%, at least 98% or at least 99% sequence identity to amino acids 21 to 112 of SEQ ID NO: 1, wherein the variant polypeptide has at least one amino acid substitution as compared to SEQ ID NO: 1 selected from P26I, P26L, P26M, P26Q, P26T, P26V, K28R, A29P, A29S, T30H, T30M, T30S, V32E, R33P, T51A, Y52H, Y52M, Y52W, M53D, M54D, M54G, M54H, M54L, G55D, G55H, G55T, N56A, N56F, N56G, N56H, N56I, N56K, N56L, N56M, N56Q, N56R, N56S, N56V, N56Y, E57A, E57D, E57H, E57K, E57M, E57N, E57Q, E57R, E57S, E57T, E57V, L58I, L58R, L58T, T59I, T59M, T59N, T59Q, T59R, T59S, T59V, L61E, L61I, L61M, L61T, L61V, D62R, D63E, D63G, D63H, D63T, K93G, V94G, V94I, E95W, P99H, P99T, P99L and Y102T.

Described herein is a variant CTLA4 polypeptide having at least 90%, at least 95%, at least 97%, at least 98% or at least 99% sequence identity to amino acids 21 to 112 of SEQ ID NO: 1, wherein the variant polypeptide includes the amino acid substitution K28Y as compared to SEQ ID NO: 1.

Also described herein is a variant CTLA4 polypeptide having at least 90%, at least 95%, at least 97%, at least 98% or at least 99% sequence identity to amino acids 21 to 112 of SEQ ID NO: 1, wherein the variant polypeptide has at least one amino acid substitution as compared to SEQ ID NO: 1 selected from P26N, G27I, G27Q, G27W, K28E, K28I, K28M, K28P, K28Q, K28V, T30I, T30L, M53E, M53L, M53N, M54A, M54Q, M54S, L58D, L58V, T59H, T59K, T59L, T59P, L61D, L61G, L61Q, L61R, D62A, D62E, D62S, D63Q, D63S and K93R.

Also described herein is a variant CTLA4 polypeptide having at least 90%, at least 95%, at least 97%, at least 98% or at least 99% sequence identity to amino acids 21 to 112 of SEQ ID NO: 1, wherein the variant polypeptide has at least one amino acid substitution as compared to SEQ ID NO: 1 selected from P26R, G27D, G27P, A29N, T30K, E31D, E31G, E31P, E31R, E31T, V32G, V32H, V32T, R33E, R33G, R33I, R33M, R33N, R33Q, R33W, R33Y, T51E, T51G, T51I, T51V, Y52A, Y52D, Y52I, Y52K, Y52L, Y52Q, Y52R, Y52S, Y52T, M53H, M53P, M53S, G55A, G55I, G55L, G55N, G55Q, G55R, G55Y, E57G, E57I, E57W, E57Y, T59D, T59G, T59Y, F60D, F60G, F60H, F60L, D62G, D62I, D62W, D63A, D63Y, K93D, K93H, K93N, K93P, K93W, V94A, V94D, V94H, V94N, V94P, V94R, V94T, V94W, E95G, E95I, E95K, E95P, E95Q, E95R, E95S, E95T, E95V, E95Y, L96A, L96G, L96H, L96M, L96P, L96Q, M97D, M97G, M97I, M97N, M97S, M97V, P99I, P99M, P99W, P99Y, P100H, P100L, P100T, P100V, P100W, P101L, Y102H, Y103H, Y103L, Y103P, Y103T, and L104P.

The variant CTLA4 proteins of the disclosure can comprise one or more additional mutations or combinations of mutations selected from one or more of Tables 2, 3, 4, 5, 6, 7, 8, 9, 10, 11, 12, 13, 14, 15, and 16-1 to 16-6.

In certain aspects, the mutations in the CDR-like loops of the variant CTLA4 polypeptides of the disclosure as compared to abatacept (SEQ ID NO: 6) do not comprise (a) A29K; (b) M53E; (c) L61Y; (d) L104M; (e) R33P; (f) T59I; (g) L61F. In other aspects, the mutations in the CDR-like loops of the variant CTLA4 polypeptides of the disclosure as compared to abatacept (SEQ ID NO: 6) do not consist of (a) A29K; (b) M53E; (c) L61Y; (d) L104M; (e) R33P; (f) T59I; (g) L61F; (h) a combination of two or more of A29K, M53E, L61Y, L104M, R33P, T59I and L61F; or (i) a combination of three or more of A29K, M53E, L61Y, L104M, R33P, T59I and L61F. By way of example and not limitation, in specific embodiments, the mutations in the CDR-like loops of the variant CTLA4 polypeptides of the disclosure as compared to abatacept (SEQ ID NO: 6) do not comprise or consist of (1) A29K and, optionally, one, two, or more substitutions independently selected from M53E, L61Y, L104M, R33P, T59I and L61F; (2) M53E and, optionally, one, two, or more substitutions independently selected from A29K, L61Y, L104M, R33P, T59I and L61F; (3) L61Y and, optionally, one, two or more substitutions independently selected from A29K, M53E, L104M, R33P, T59I and L61F; (4) L104M and, optionally, one, two, or more substitutions independently selected from A29K, M53E, L61Y, R33P, T59I and L61F; (5) R33P and, optionally, one, two, or more substitutions independently selected from A29K, M53E, L61Y, L104M, T59I and L61F; (6) T59I and, optionally, one, two, or more substitutions independently selected from A29K, M53E, L61Y, L104M, R33P, and L61F; or (7) L61F and, optionally, one, two, or more substitutions independently selected from A29K, M53E, L61Y, L104M, R33P, and T59I.

In certain aspects, the variant CTLA4 polypeptide is a fusion protein. In certain embodiments, the fusion partner is a immunoglobulin constant domain, such as an IgG₁.

The CTLA4 proteins of the disclosure have sequences having at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity or at least 99% sequence identity to the amino acid sequence of a wild-type CTLA4 binding domain (amino acids 21 to 112 of SEQ ID NO: 1).

In various aspects, the CTLA4 proteins of the disclosure have up to 2, up to 3, up to 4, up to 5, up to 6, up to 7, up to 8, up to 9, or up to 10 amino acid substitutions in the CDR-like loops as compared to the CDR-like loops of SEQ ID NO: 1.

Nucleic acids comprise nucleotide sequences encoding the CTLA4 proteins of the disclosure are provided herein, as are vectors comprising nucleic acids. Additionally, prokaryotic and eukaryotic host cells transformed with a vector comprising a nucleotide sequence encoding a CTLA4 protein are described herein, as well as eukaryotic (such as mammalian) host cells engineered to express the nucleotide sequences. Methods of producing CTLA4 proteins by culturing host cells are also described.

The CTLA4 proteins of the disclosure are useful in the treatment of cancers, autoimmune diseases and the rejection of cell, organ or tissue graft transplants. In certain aspects, the CTLA4 proteins are useful in treating an autoimmune disease selected from rheumatoid arthritis and juvenile idiopathic arthritis.

It should be noted that the indefinite articles "a" and "an" and the definite article "the" are used in the present application, as is common in patent applications, to mean one or more unless the context clearly dictates otherwise. Further, the term "or" is used in the present application, as is common in patent applications, to mean the disjunctive "or" or the conjunctive "and."

Any discussion of documents, acts, materials, devices, articles or the like that has been included in this specification is solely for the purpose of providing a context for the present disclosure. It is not to be taken as an admission that any or all of these matters form part of the prior art base or were common general knowledge in the field relevant to the present disclosure as it existed anywhere before the priority date of this application.

The features and advantages of the disclosure will become further apparent from the following detailed description of embodiments thereof.

### 5. BRIEF DESCRIPTION OF THE TABLES AND FIGURES

**Table 1** shows the numbering of the amino acids in the CDR-like loops of wild-type CTLA4-Ig.
**Table 2** shows mutations in the wild-type CTLA4 CDR-like loops that binding studies indicate increase affinity for CD80 and CD86.
**Table 3** shows mutations in the wild-type CTLA4 CDR-like loops that binding studies indicate increase affinity for CD80, and do not substantially impact CD86 binding.
**Table 4** shows candidate mutations that can be incorporated in the wild-type CTLA4 CDR-like loops for increasing affinity toward CD80 while not substantially impacting CD86 binding.
**Table 5** shows mutations in the wild-type CTLA4 CDR-like loops that binding studies indicate increase affinity for CD80 and decrease affinity for CD86.
**Table 6** shows candidate mutations that can be incorporated in the wild-type CTLA4 CDR-like loops for increasing affinity toward CD80 and decreasing affinity toward CD86.
**Table 7** shows mutations in the wild-type CTLA4 CDR-like loops that binding studies indicate increase affinity for CD86 while not substantially impacting CD80 binding.
**Table 8** shows candidate mutations that can be incorporated in the wild-type CTLA4 CDR-like loops for increasing affinity toward CD86 while not substantially impacting CD80 binding.
**Table 9** shows mutations in the wild-type CTLA4 CDR-like loops that binding studies indicate decrease affinity for CD80 and increase affinity for CD86.
**Table 10** shows mutations in the wild-type CTLA4 CDR-like loops that binding studies indicate do not substantially impact binding to CD80 or CD86.
**Table 11** shows candidate mutations that can be incorporated in the wild-type CTLA4 CDR-like loops and that do not substantially impact binding to CD80 or CD86.
**Table 12** shows mutations in the wild-type CTLA4 CDR-like loops that binding studies indicate decrease affinity for CD86 while not substantially impacting binding to CD80.
**Table 13** shows candidate mutations that can be incorporated in the wild-type CTLA4 CDR-like loops for decreasing affinity for CD86 while not substantially impacting CD80 binding.
**Table 14** shows candidate mutations that can be incorporated in the wild-type CTLA4 CDR-like loops for decreasing affinity for CD80 while not substantially impacting CD86 binding.
**Table 15** shows known mutations in the wild-type CTLA4 CDR-like loops that can be incorporated into the CTLA4 proteins described herein. Row 1 shows known mutations that can be incorporated in the wild-type CTLA4 CDR-like loops for increasing affinity for CD80 and CD86. Rows 2-5 show known mutations that can be incorporated in the wild-type CTLA4 CDR-like loops for increasing affinity for CD80 while not substantially impacting CD86 binding. Row 6 shows known mutations that can be incorporated in the wild-type CTLA4 CDR-like loops for increasing affinity for CD80 while decreasing affinity for CD86. Rows 7-8 show known mutations that can be incorporated in the wild-type CTLA4 CDR-like loops for increasing affinity for CD86 while not substantially impacting CD80 binding. Row 9 shows known mutations that can be incorporated in the wild-type CTLA4 CDR-like loops for increasing affinity for CD86 while decreasing affinity for CD80. Rows 10-22 show known mutations that can be incorporated in the wild-type CTLA4 CDR-like loops which do not substantially impact CD80 or CD86 binding. Rows 23-26 show known mutations that can be incorporated in the wild-type CTLA4 CDR-like loops for decreasing affinity for CD86 while not substantially impacting affinity for CD80. Rows 27-34 show known mutations that can be incorporated in the wild-type CTLA4 CDR-like loops for decreasing affinity for CD80 while not substantially impacting affinity for CD86.
**Tables 16-1** to **16-6** show known mutations in the wild-type CTLA4 CDR-like loops that can be incorporated into the CTLA4 proteins described herein.

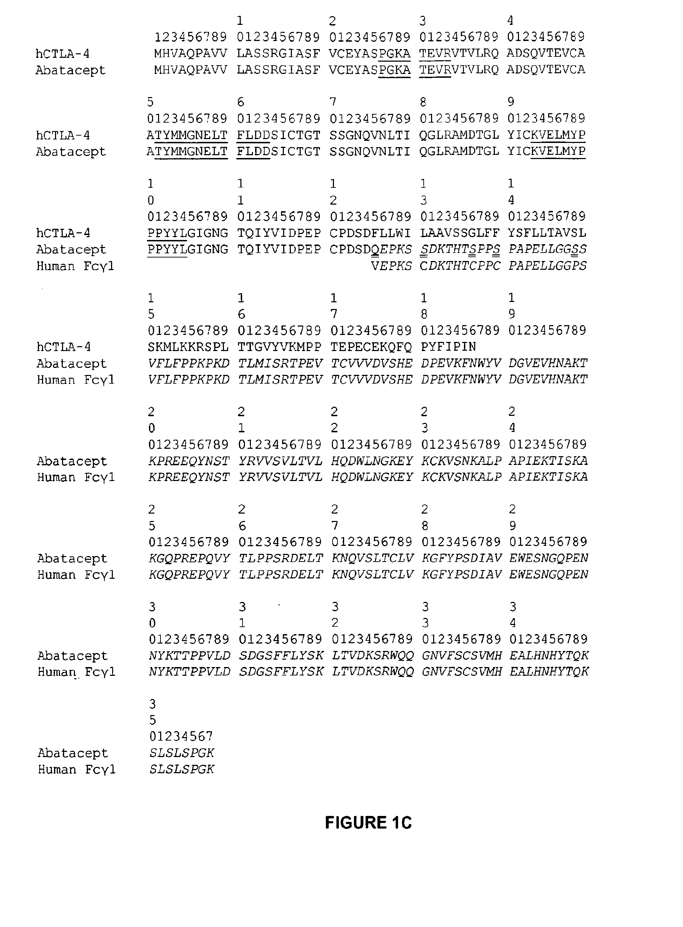

**Figures 1A-1C**. **Figure 1A** shows the amino acid sequence of the human CTLA4 extracellular domain (SEQ ID NO: 1). Amino acids in the CDR-like loops (underlined) were identified by visual inspection of reported CTLA4 co-crystal structures. (Schwartz *et al.,* 2001, Nature 410:604; Stamper *et al.*, 2001, Nature 410:608). **Figure 1B** shows the sequences of the CDR-like loops of wild-type CTLA4 and the corresponding sequence identifiers. **Figure 1C** shows an alignment of (i) full-length human CTLA4 extracellular domain and containing a transmembrane domain (Genbank Accession No. NM_005214; SEQ ID NO: 5), (ii) abatacept as set forth in US Patent No. 5,434,131 (SEQ ID NO: 6); and (iii) human Fcγ1 (SEQ ID NO: 7). The extracellular domain of wild-type CTLA4-Ig comprises amino acids 1-124 of hCTLA4, which corresponds to the CTLA4 extracellular domain. CTLA4 dimerizes through the cysteine residue at position 120. Abatacept has a glutamine residue at position 125, which is at the junction of the hCTLA4 extracellular domain and the human Fcγ1. The CH₁ region of hFcγ1 contains a valine at the equivalent position. The human Fcγ1 region with the IgG₁m(a) allotype starts at position 126 and is italicized. Cysteine residues at positions 130, 136 and 139 that are present in the Fcγ1 and are involved in disulfide bridges have been mutated to serine in abatacept. Position 148 in the CH2 region of Fcγ1 is mutated from proline to serine in abatacept. (Davis *et al.*, 2007, J. Rheumatol. 34(11):2204).

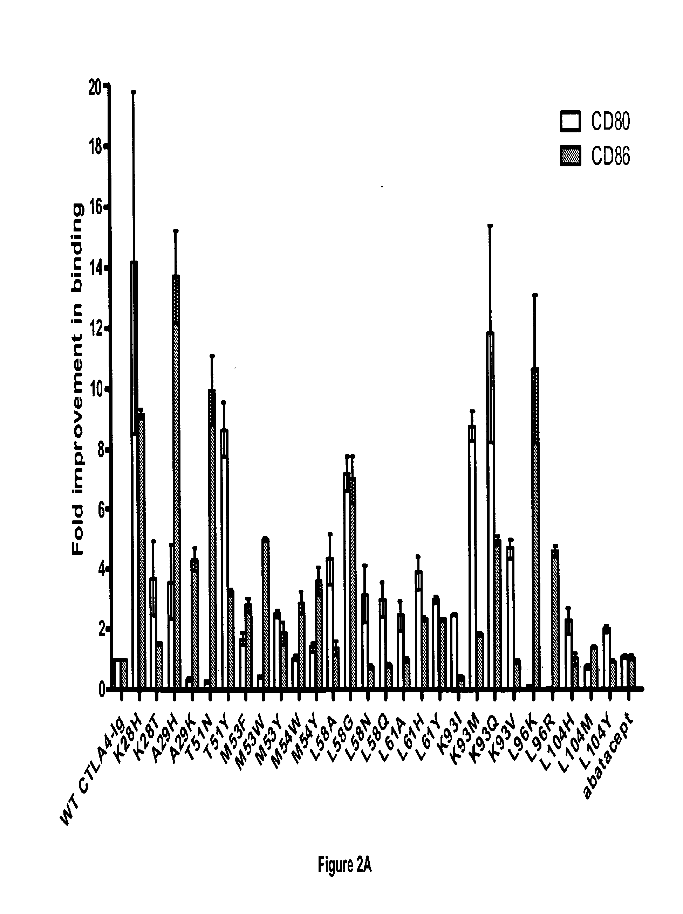

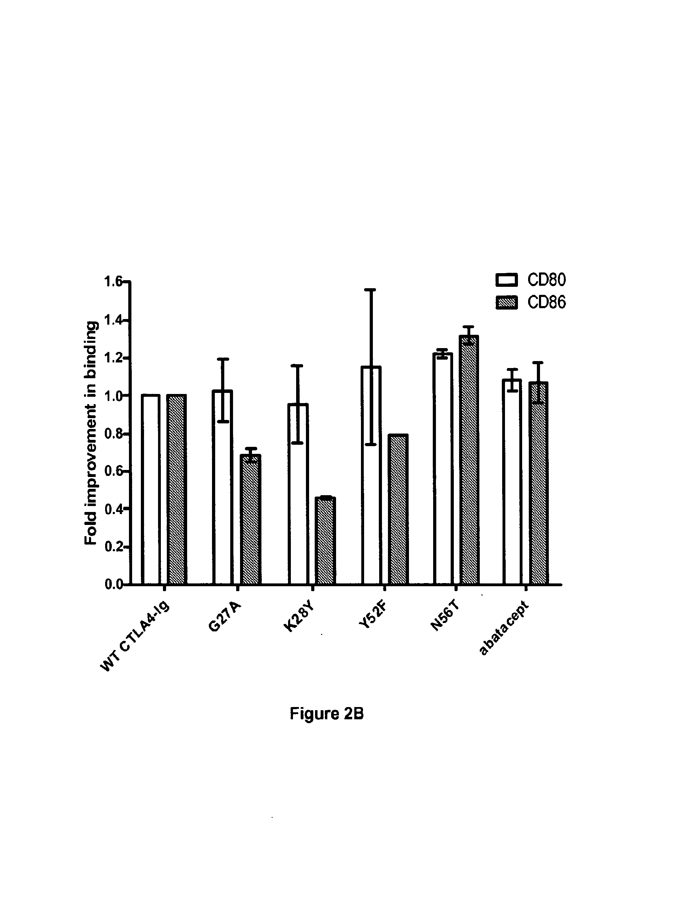

**Figures 2A-2B** show the binding of novel CTLA4-Ig variants to CD80 and CD86 as measured by competition AlphaLISA®. Relative binding activities of variants that showed improved binding to at least one of CD80 and CD86 are shown in **Figure 2A**. Relative binding activities of variants that showed equivalent binding to at least one of CD80 and CD86 are shown in **Figure 2B**. Open and solid bars indicate the binding data with CD80 and CD86, respectively. Data are normalized with an IC₅₀ value obtained from wild-type CTLA4-Ig, and are shown as the average fold improvement from two independent sets of experiments. Binding activity of abatacept that contains the mutation P148S mutation in the CH2 domain was confirmed to have negligible impact on ligand binding. Each data point of the dilution curve was duplicated in the assay plate. The average IC₅₀ value of wild-type CTLA4-Ig was determined to be 0.89 nM ± 0.26 and 4.26 nM ± 0.76 for CD80 and CD86, respectively. Error bars represent the range of values obtained from duplicate experiments.

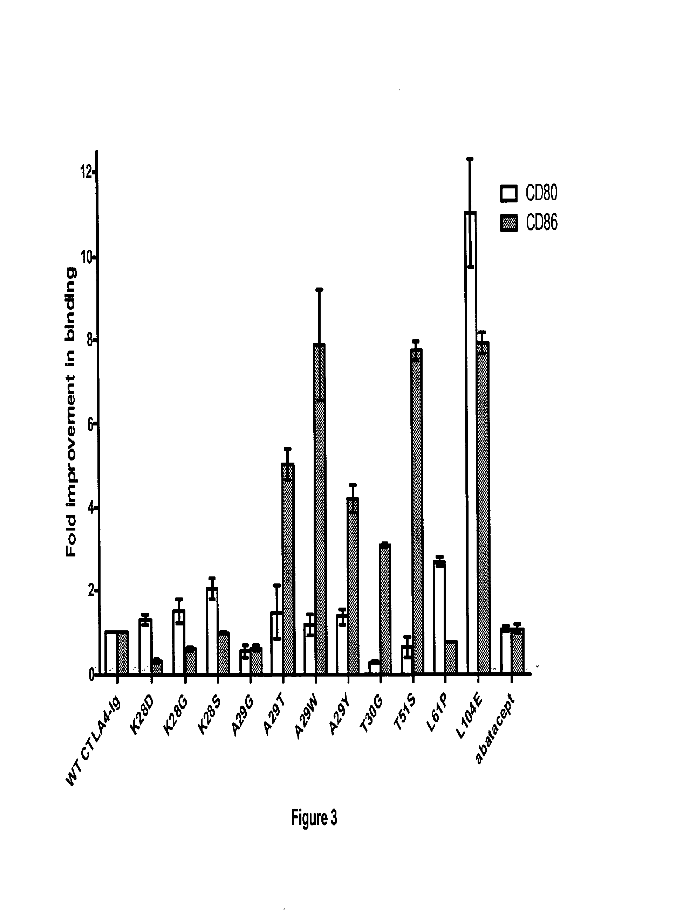

**Figure 3** shows the binding activity of known CTLA4-Ig variants to CD80 and CD86 as measured by competition AlphaLISA®.

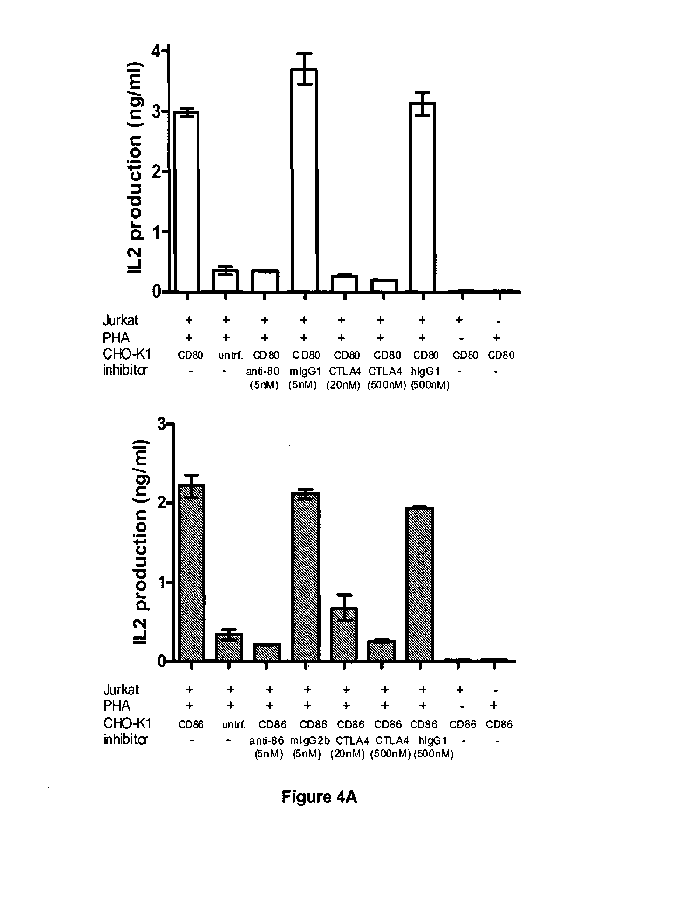

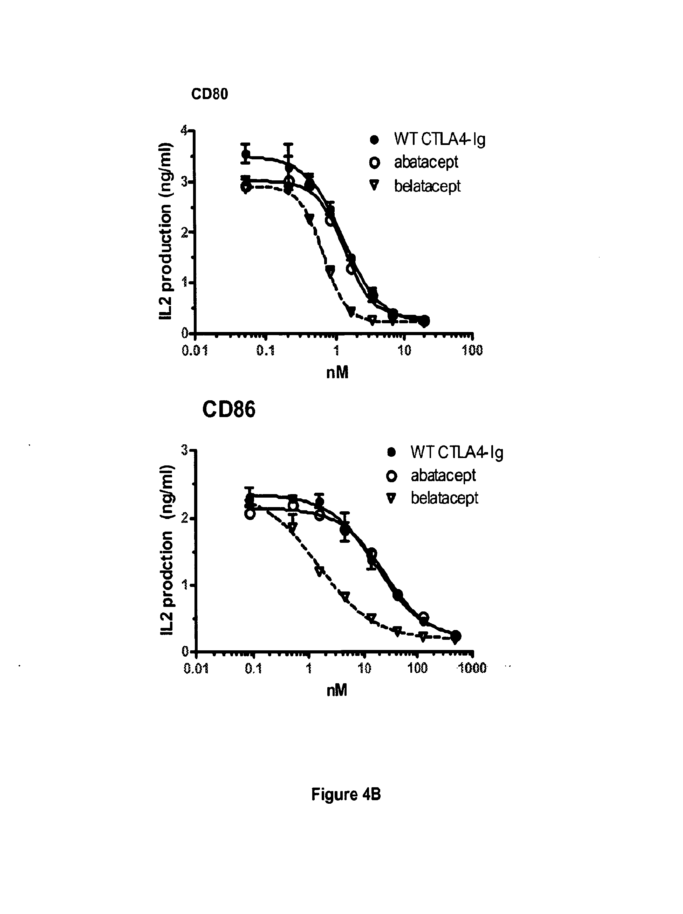

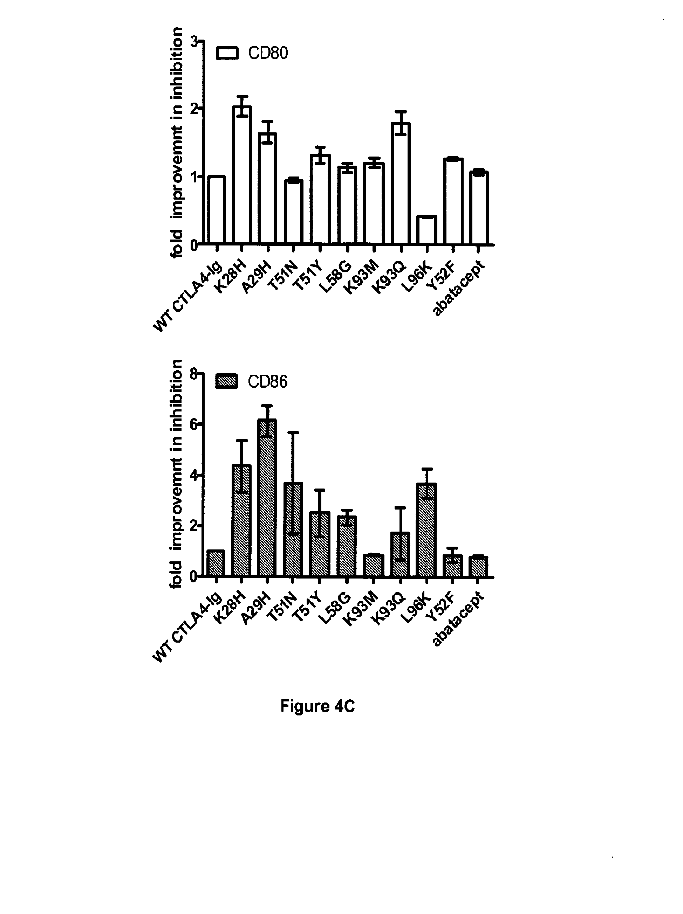

**Figures 4A-4C** show the ability of wild-type and CTLA4-Ig variants to inhibit IL-2 production of Jurkat T-cells upon stimulation. **Figure 4A** shows IL-2 production from Jurkat T-cells upon stimulation with CHO-K1 cells expressing CD80 or CD86 in the presence of wild-type CTLA4-Ig or control antibody. **Figure 4B** shows full inhibition curves with titrating amounts of commercial abatacept or wild-type CTLA4-Ig or belatacept produced in-house. Relative potencies of selected CTLA-Ig variants of the disclosure in the inhibition of IL-2 production normalized with an IC₅₀ value obtained from wild-type CTLA4-Ig are shown in **Figure 4C**. Error bars represent the range of values obtained from duplicate experiments.

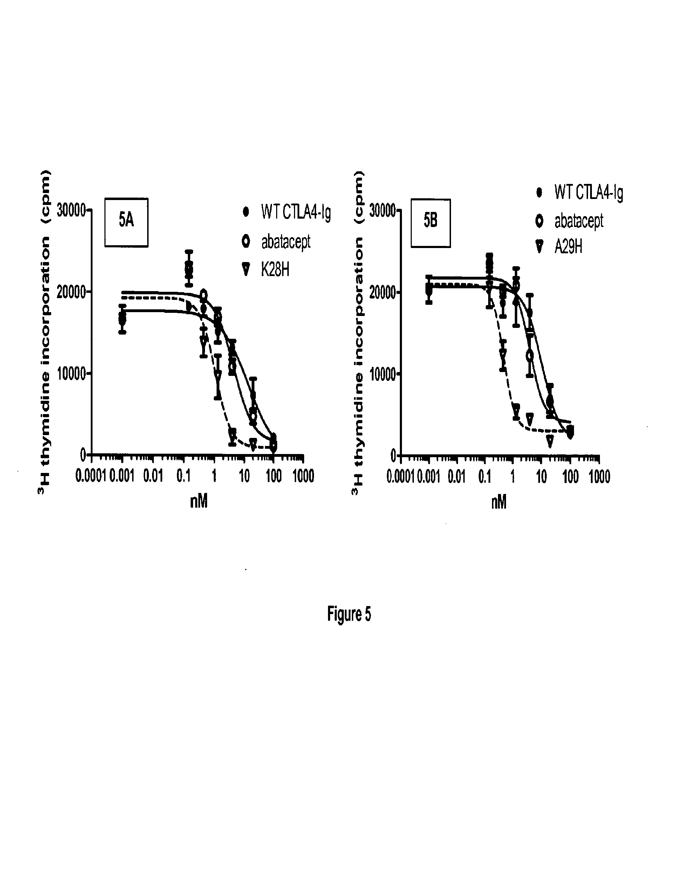

**Figures 5A-5E** show the ability of wild-type and CTLA4-Ig variants to inhibit proliferation of primary allo-stimulated T-cells. Symbols represent means of triplicate determinations. Data are representative of at least three independent experiments.

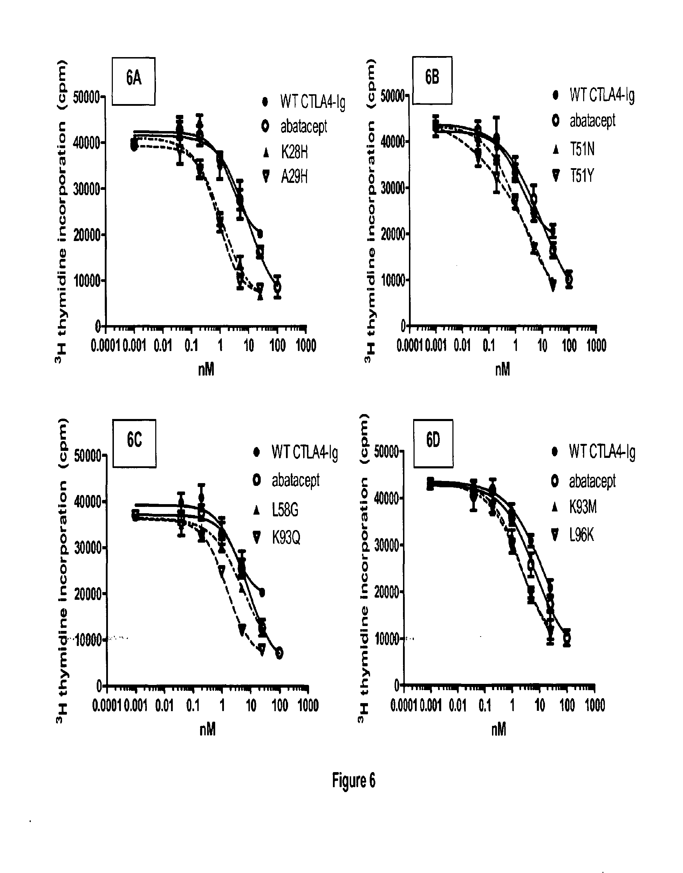

**Figures 6A-6D** show the ability of wild-type and CTLA4-Ig variants to inhibit proliferation of secondary allo-stimulated T-cells. Symbols represent means of triplicate determinations. Data are representative of at least three independent experiments.

### 6. DETAILED DESCRIPTION

The present disclosure provides novel polypeptides comprising variant CD80- and/or CD86-binding fragments of CTLA4 ("CTLA4 binding domain") that inhibit or suppress immune responses. The polypeptides of the disclosure comprise at least a CTLA4 binding domain that has one or more amino acid substitutions that maintain or alter the binding properties of CTLA4 to CD80 and/or CD86. Optionally, the variant CTLA4 binding domains are fused to a different (non-CTLA4) amino acid sequence, such as that of an immunoglobulin constant region. These novel polypeptides are collectively referred to herein as "CTLA4 proteins" or "CTLA4 polypeptides".

### 6.1. CTLA4 Extracellular Domain

The variant polypeptides of the disclosure comprise an amino acid sequence that is related in sequence to the CTLA4 extracellular domain (SEQ ID NO: 1). The CTLA4 extracellular domain is a single chain polypeptide that adopts an immunoglobulin variable fold with three CDR-like loops. The variant polypeptides of the disclosure contain at least one amino acid substitution in at least one CDR-like loop as compared to CTLA4 that maintain or alter the protein's binding properties to CD80 and/or CD86. In certain aspects, the variant CTLA4 polypeptides of the disclosure bind to CD80 and/or CD86 and block their interactions with their respective ligands, e.g., CTLA4 or CD28, thereby inhibiting one or more immune cell mediated responses. The variant CTLA4 polypeptides of the disclosure can also have amino acid mutations (e.g., deletions, insertions, additions or substitutions) in the non-CDR-like loop portions of the CTLA4 extracellular domain. Suitable mutations in the non-CDR-like regions of the CTLA4 extracellular domain include, but are not limited to, those disclosed, for example in U.S. Patent nos. 5,977,318; 6,107,056; 6,750,334; 7,034,121; 7,229,962; 7,432,344; 7,504,554; 7,385,045; 7,105,166; 6,090,914; 5,770,197; and U.S. Patent publication no. 2009/0258031.

The variant CTLA4 polypeptides described herein may have an amino acid sequence that is at least 75% identical to the amino acid sequence of a CTLA4 binding domain (*e.g.,* amino acids 21-112 of SEQ ID NO: 1). The variant CTLA4 polypeptides of the disclosure may have an amino acid sequence that is at least 75% identical to the amino acid sequence of the CTLA4 extracellular domain (*e.g.,* amino acids 1-124 of SEQ ID NO: 1). The variant CTLA4 polypeptides described herein may have an amino acid sequence that is at least 80% identical, at least 85% identical, at least 90% identical, at least 91% identical, at least 92% identical, at least 93% identical, at least 94% identical, at least 95% identical, at least 96% identical, at least 97% identical, at least 98% identical or at least 99% identical to the amino acid sequence of a CTLA4 binding domain or to the amino acid sequence of a CTLA4 extracellular domain.

The variant CTLA4 polypeptides described herein may comprise at least one mutation compared to a wild-type CTLA4 extracellular domain. The variant CTLA4 polypeptides of the disclosure comprise at least one mutation compared to a wild-type CTLA4 binding domain. In other instances, the variant CTLA4 polypeptides described herein comprise up to 2, up to 3, up to 4, up to 5, up to 6, up to 7, up to 8, up to 9, up to 10, up to 11, up to 12, up to 13, up to 14, up to 15, up to 16, up to 17, up to 18, up to 19, or up to 20 amino acid substitutions as compared to a wild-type CTLA4 extracellular domain or as compared to a wild-type CTLA4 binding domain.

The CTLA4 extracellular domain described herein includes amino acid residues 1-124 of the human CTLA4 protein (SEQ ID NO: 1). In various embodiments, the CTLA4 polypeptide of the disclosure is a CD80- and/or CD86-binding fragment of the CTLA4 extracellular domain comprising CDR-like sequences that are related to the CDR-like sequences of CTLA4. In certain instances described herein, the binding fragment comprises a portion of the CTLA4 extracellular domain starting anywhere between amino acids 1 to 12 of SEQ ID NO: 1 and ending anywhere between amino acids 112 to 124 of SEQ ID NO: 1. In certain instances described herein, the binding fragment is from amino acid 12 to amino acid 112 of SEQ ID NO: 1, from amino acid 10 to amino acid 112 of SEQ ID NO: 1, from amino acid 8 to amino acid 112 of SEQ ID NO: 1, from amino acid 6 to amino acid 112 of SEQ ID NO: 1, from amino acid 4 to amino acid 112 of SEQ ID NO: 1, from amino acid 1 to amino acid 112, amino acid 113, amino acid 114, amino acid 115, amino acid 116, amino acid 117, amino acid 118, amino acid 119, amino acid 120, amino acid 121, amino acid 122 or amino acid 123 of SEQ ID NO: 1. In yet other instances described herein, the binding portion is from amino acid 12 to amino acid 114 of SEQ ID NO: 1, from amino acid 12 to amino acid 116 of SEQ ID NO: 1, from amino acid 12 to amino acid 118 of SEQ ID NO: 1, from amino acid 12 to amino acid 120 of SEQ ID NO: 1, from amino acid 12 to amino acid 122 of SEQ ID NO: 1, or from amino acid 12, amino acid 11, amino acid 10, amino acid 9, amino acid 8, amino acid 7, amino acid 6, amino acid 5, amino acid 4, amino acid 3 or amino acid 2 to amino acid 124 of SEQ ID NO: 1.

In certain embodiments, the CDR-like loops of CTLA4 are grafted onto a non-CTLA4 protein scaffold. The non-CTLA4 protein scaffold can be any protein scaffold known in the art that allows the CDR-like loops to maintain a three-dimensional structure that binds to CD80 and/or CD86. In certain embodiments, the non-CTLA4 protein scaffold is a variable domain of a camelid heavy chain antibody (VHH) or a variable domain of the shark new antigen receptor (VNAR) ("nanobodies"). In other embodiments, the scaffold can be from a protein other than an immunoglobulin. Such scaffolds for use in the CTLA4 variant proteins of the disclosure include, but are not limited to, fibronectin III (AdNectin), β-sandwich proteins (iMAB), lipocalins (Anticalin), EETI-II/AGRP (Microbody), BPTI/LACI-D1/ITI-D2 (Kunitz domain), thioredoxin (Peptide aptamer), protein A (Affibody), ankyrin repeat (DARPin), γB-crystallin/ubiquitin (Affilin), CTLD₃ (Tetranectin) and (LDLR-A module)₃ (Avimer).

In some embodiments, the variant CTLA4 polypeptide of the disclosure is a monomer. In other embodiments, the CTLA4 polypeptide is a dimer. In certain embodiments, the CTLA4 dimer is a covalent dimer, *e.g.,* a disulfide-linked dimer. In other embodiments, the CTLA4 dimer is a non-covalent dimer. In still other embodiments, the CTLA4 polypeptide is a multimer, *e.g.,* a tetramer.

The variant CTLA4 polypeptides of the present disclosure have a binding affinity for a CTLA4 ligand. In certain embodiments the CTLA4 polypeptides have a binding affinity for the extracellular domain of human CD80, or a portion thereof. In various embodiments, the CTLA4 polypeptides have a binding affinity for the extracellular domain of human CD86, or a portion thereof.

The variant CTLA4 polypeptides of the disclosure have a binding affinity for CD80 and/or CD86 that is increased compared to the binding affinity of the wild-type CTLA4 extracellular domain, *e.g.,* in the context of abatacept. As used herein, a variant with "increased affinity" for CD80 and/or CD86 (a "high affinity" variant) refers to a polypeptide of the disclosure having a binding affinity that is at least 1.5x, at least 1.75x, at least 2x, at least 2.5x, at least 3x, at least 3.5x, at least 4x, at least 4.5x, at least 5x, at least 5.5x, at least 6x, at least 6.5x, at least 7x, at least 7.5x, at least 8x, at least 8.5x, at least 9x, at least 9.5x, at least 10x, or at least 20x or greater than the binding affinity of the CTLA4 extracellular domain for CD80 and/or CD86. The variant CTLA4 polypeptides described herein may have a binding affinity for CD80 and/or CD86 that is about equal to the binding affinity of the wild-type CTLA4 extracellular domain, e.g., in the context of abatacept for CD80 and/or CD86 (a "neutral affinity" variant). As used herein, a "neutral affinity" variant has a binding affinity for CD80 and/or CD86 that is between about 0.5x and about 1.5x of the binding affinity of the CTLA4 extracellular domain for the ligand, such as about 0.6x, about 0.7x, about 0.8x, about 0.9x, about 1x, about 1.1x, about 1.2x, about 1.3x, about 1.4x or about 1.5x of the binding affinity of the CTLA4 extracellular domain. A variant polypeptide described herein may have a decreased binding affinity for CD80 and/or CD86 as compared to the CTLA4 extracellular domain ("low affinity" variant). As used herein, a "low affinity" variant of the disclosure has a binding affinity that is less than 0.5x, less than 0.4x, less than 0.3x, less than 0.2x, less than 0.1x, less than 0.05x, less than 0.01x or less of the binding affinity of the CTLA4 extracellular domain.

In particular embodiments a variant CTLA4 protein of the disclosure is a high affinity variant with respect to both CD80 and CD86 binding. In some embodiments, the variant polypeptide is a high affinity variant with respect to CD80 and a neutral affinity variant with respect to CD86. In other embodiments, the variant CTLA4 polypeptide is a high affinity variant with respect to CD86 binding and neutral affinity variant with respect to CD80 binding. In still other embodiments, the variant CTLA4 protein of the disclosure is a high affinity variant with respect to CD80 binding and a low affinity variant with respect to CD86 binding. In yet other embodiments, the variant protein is a high affinity variant with respect to CD86 binding and a low affinity variant with respect to CD80 binding. The variant polypeptide described herein may have a neutral affinity variant with respect to both CD80 binding and CD86 binding. The variant CTLA4 polypeptide described herein may have a low affinity variant with respect to CD86 binding and a neutral affinity variant with respect to CD80 binding. The variant CTLA4 polypeptide described herein may have a neutral affinity variant with respect to CD86 binding and a low affinity variant with respect to CD80 binding.

In certain embodiments, the CTLA4 polypeptides of the disclosure comprise a variant CTLA4 binding domain in the context of full length CTLA4 (Genbank Accession No. AAL07473.1

### 6.2. CTLA4 Fusion Partners

In certain aspects, the CTLA4 polypeptide of the disclosure can be a fusion protein in which the variant CTLA4 binding domain is operably linked to the amino acid sequence of another polypeptide, referred to herein as a "fusion partner." As used herein, the term "fusion partner" refers to a non-CTLA4 polypeptide that is covalently attached to the CTLA4 polypeptide variant, either directly or through a linker sequence. In various embodiments, the C-terminus of the CTLA4 binding domain is linked to the N-terminus of the fusion partner. In some embodiments, the fusion partner can comprise a transmembrane domain and/or a cytoplasmic domain. In these embodiments, the CTLA4 fusion protein is expressed on the surface of a cell. In particular embodiments, the fusion partner comprises a polypeptide that facilitates secretion, expression and/or solubility of the CTLA4 polypeptide.

In some embodiments, the CTLA4 fusion proteins of the disclosure are monomeric. In other embodiments, the CTLA4 fusion proteins of the disclosure are dimeric, either due to covalent linkage (*e.g.,* by disulfide bonds) between the CTLA4 portions of two monomers and/or between the fusion partner polypeptides or due to non-covalent association of the CTLA4 and/or fusion partner portions of the monomers.

In some embodiments, the fusion partner is a protein or protein domain that dimerizes, such as lambda repressor, upstream binding factor, and leucine zipper domains (*e.g.,* fos/jun). In particular embodiments, the fusion partner is an immunoglobulin (Ig) Fc polypeptide, *i.e.,* the Fc domain of an Ig polypeptide, or a fragment thereof. In certain embodiments, the Fc polypeptide or fragment can be derived from various species, including, *e.g.,* human, mouse, primate, *etc.*, and can be derived from a different species than the CTLA4 binding domain to which it is linked. Thus, for example, in some embodiments, the CTLA4 binding domain is derived from a human protein and the fusion partner is derived from a mouse protein. In particular embodiments, the CTLA4 polypeptide and the fusion partner are both derived from human proteins.

In certain embodiments, the Ig Fc polypeptide comprises a first constant region (CH₁), a hinge region, a second constant region (CH₂) and a third constant region (CH₃) in the order (from the N-terminus to the C-terminus) CH₁-hinge-CH₂-CH₃. In some embodiments, the fusion partner optionally comprises a fourth constant region (CH₄). In some embodiments, the fusion partner is an immunoglobulin Fc fragment comprising the hinge region, CH₂ domain and CH₃ domain of the Ig polypeptide. In still other embodiments, the Ig Fc fragment comprises a single constant domain, such as the hinge region and a CH₁, CH₂ or CH₃ domain. In certain aspects, the Ig Fc does not have a hinge, *e.g.,* an IgM. In some embodiments, the Ig Fc polypeptide comprises a wild-type Ig Fc polypeptide. Exemplary Fc polypeptides include, but are not limited to, IgG₁, IgG₂, IgG₃, IgG₄, IgA, IgE, IgD and IgM. In particular embodiments, the Fc polypeptide is an IgG.

Alternatively, the immunoglobulin Fc fusion partner is a modified Ig polypeptide. In certain embodiments, the Fc polypeptide has one or more amino acid substitutions compared to wild-type. For example, in certain embodiments, the Fc polypeptide is a mutant IgG in which one or more cysteine residues have been substituted with another amino acid (*e.g.,* a serine residue) to eliminate one or more disulfide bonds formed between two Ig chains. In other embodiments, the Fc polypeptide is an IgG polypeptide in which one or more amino acid residues is substituted with another amino acid (*e.g.,* proline) to reduce effector function (reduced Fc receptor binding). *See* U.S. Patent Publication no. 2009/0258031.

In some embodiments, the Fc fusion partner or a fragment thereof can be modified to alter at least one constant region-mediated biological effector function relative to the corresponding wild type sequence. For example, in some embodiments, the Fc polypeptide can be modified to reduce at least one constant region-mediated biological effector function relative to an unmodified Fc region, *e.g.,* reduced binding to the Fc receptor (FcγR). FcγR binding can be reduced by mutating the immunoglobulin constant region segment at particular regions necessary for FcγR interactions (see *e.g.,* Canfield and Morrison, 1991, J. Exp. Med. 173:1483-1491; and Lund et al., 1991, J. Immunol. 147:2657-2662). Reduction in FcγR binding ability of the Fc region can also reduce other effector functions which rely on FcγR interactions, such as opsonization, phagocytosis and antibody-dependent cellular cytotoxicity ("ADCC").

In other embodiments, the Fc polypeptide can be modified to acquire or improve at least one constant region-mediated biological effector function relative to an unmodified Fc region, *e.g.,* to enhance FcγR interactions (see, *e.g.,* US 2006/0134709). For example, the Fc region can be modified to bind FcγRIIA, FcγRIIB and/or FcγRIIIA with greater affinity than the corresponding wild type constant region.

Thus, Fc fusion proteins of the disclosure can have alterations in biological activity that result in increased or decreased opsonization, phagocytosis, or ADCC as compared to a fusion protein having the corresponding wild-type Fc region. Such alterations are known in the art. For example, the Fc polypeptides can be modified as antibodies are modified to reduce ADCC activity, *e.g.,* as described in U.S. Patent Nos. 5,834,597 and 7,083,784; and US Patent Publication Nos. 2006/0198840, and 2007/0122403. An exemplary ADCC lowering variant corresponds to "mutant 3" shown in Figure 4 of U.S. Patent No. 5,834,597, in which residue 236 is deleted and residues 234, 235 and 237 (using EU numbering) are substituted with alanines.

In some embodiments, the Fc polypeptides for use in CTLA4 proteins of the disclosure have low levels of or lack fucose. Antibodies lacking fucose in the Fc region have been correlated with enhanced ADCC activity, especially at low doses of antibody. See Shields et al., 2002, J. Biol. Chem. 277:26733-26740; Shinkawa et al., 2003, J. Biol. Chem. 278:3466-73. Methods of preparing fucose-less proteins include growth in rat myeloma YB2/0 cells (ATCC CRL 1662). YB2/0 cells express low levels of FUT8 mRNA, which encodes α-1,6-fucosyltransferase, an enzyme necessary for fucosylation of polypeptides.

In yet another aspect, the proteins of the disclosure or fragments thereof can include Fc regions that have been modified to increase or reduce their binding affinities to the fetal Fc receptor, FcRn, for example by mutating the immunoglobulin constant region segment at particular regions involved in FcRn interactions (see *e.g.,* WO 2005/123780). In particular embodiments, the Ig Fc fusion partner of the IgG class is mutated such that at least one of amino acid residues 250, 314, and 428 of the heavy chain constant region is substituted alone, or in any combinations thereof, such as at positions 250 and 428, or at positions 250 and 314, or at positions 314 and 428, or at positions 250, 314, and 428, with positions 250 and 428 a specific combination. For position 250, the substituting amino acid residue can be any amino acid residue other than threonine, including, but not limited to, alanine, cysteine, aspartic acid, glutamic acid, phenylalanine, glycine, histidine, isoleucine, lysine, leucine, methionine, asparagine, proline, glutamine, arginine, serine, valine, tryptophan, or tyrosine. For position 314, the substituting amino acid residue can be any amino acid residue other than leucine, including, but not limited to, alanine, cysteine, aspartic acid, glutamic acid, phenylalanine, glycine, histidine, isoleucine, lysine, methionine, asparagine, proline, glutamine, arginine, serine, threonine, valine, tryptophan, or tyrosine. For position 428, the substituting amino acid residues can be any amino acid residue other than methionine, including, but not limited to, alanine, cysteine, aspartic acid, glutamic acid, phenylalanine, glycine, histidine, isoleucine, lysine, leucine, asparagine, proline, glutamine, arginine, serine, threonine, valine, tryptophan, or tyrosine. Specific combinations of suitable amino acid substitutions are identified in Table 1 of U.S. Patent No. 7,217,797. Such mutations increase binding of the Fc polypeptide to FcRn, which protects the protein from degradation and increases its half-life.

In certain embodiments, the Fc polypeptides for use in CTLA4 fusion proteins of the disclosure have an amino acid sequence that is at least 75% identical to the amino acid sequence of human Fcγ1 (SEQ ID NO: 7). In various embodiments, the Fc polypeptides have an amino acid sequence that is at least 80% identical, at least 85% identical, at least 90% identical, at least 91% identical, at least 92% identical, at least 93% identical, at least 94% identical, at least 95% identical, at least 96% identical, at least 97% identical, at least 98% identical or at least 99% identical to the amino acid sequence of human Fcγ1 (SEQ ID NO: 7).

The Fc polypeptides for use in CTLA4 fusion proteins described herein comprise at least one mutation compared to human Fcγ1 (SEQ ID NO: 7). The variant CTLA4 polypeptides described herein comprise up to 2, up to 3, up to 4, up to 5, up to 6, up to 7, up to 8, up to 9, up to 10, up to 11, up to 12, up to 13, up to 14, up to 15, up to 16, up to 17, up to 18, up to 19, or up to 20 or more amino acid substitutions as compared to wild-type human Fcγ1.

The variant CTLA4 proteins described herein are fusion proteins comprising a CTLA4 binding region comprising about 124 amino acids covalently linked (either directly or indirectly) at the C-terminus to an IgG Fc fusion partner comprising about 231 amino acids (the hinge, CH₂ and CH₃ regions of the Fc). In certain embodiments, the CTLA4 extracellular domain and the Ig Fc domain are linked via a linker, wherein the linker comprises from about 1 to about 10 amino acids. In particular embodiments, the linker comprises 1 amino acid.

The variant fusion proteins of the disclosure comprise at least one amino acid substitution as compared to wild-type abatacept (SEQ ID NO: 5). The amino acid substitution is in the CTLA4 extracellular domain, *e.g.,* in a CDR-like loop of CTLA4. The variant fusion proteins described herein may comprise at least one amino acid substitution in the CTLA4 extracellular domain and at least one amino acid substitution in the Fc domain as compared to wild-type abatacept (SEQ ID NO: 5).

The CTLA4 fusion proteins described herein may have an amino acid sequence that is at least 75% identical to the amino acid sequence of wild-type abatacept (SEQ ID NO: 6). The fusion proteins may have an amino acid sequence that is at least 80% identical, at least 85% identical, at least 90% identical, at least 91% identical, at least 92% identical, at least 93% identical, at least 94% identical, at least 95% identical, at least 96% identical, at least 97% identical, at least 98% identical or at least 99% identical to the amino acid sequence of wild-type abatacept (SEQ ID NO: 6).

The CTLA4 fusion proteins of the disclosure comprise at least one mutation compared to wild-type abatacept (SEQ ID NO: 6). In other embodiments, the variant CTLA4 polypeptides of the disclosure comprise up to 2, up to 3, up to 4, up to 5, up to 6, up to 7, up to 8, up to 9, up to 10, up to 11, up to 12, up to 13, up to 14, up to 15, up to 16, up to 17, up to 18, up to 19, or up to 20 or more amino acid substitutions as compared to wild-type abatacept.

In addition to the modifications described above, a CTLA4 protein of the disclosure can be modified in the CTLA4 portion and/or the fusion partner portion to include a signal sequence for secretion from the host cell or a peptide purification sequence, *e.g,,* an epitope tag, a FLAG tag, a polyhistidine sequence, or a GST fusion. In certain embodiments, the CTLA4 proteins of the disclosure comprise one or more modified amino acids, *e.g.,* a glycosylated amino acid, a PEGylated amino acid, a farnesylated amino acid, an acetylated amino acid, a biotinylated amino acid, an amino acid conjugated to a lipid moiety, and/or an amino acid conjugated to an organic derivatizing agent. In certain embodiments, the presence of modified amino acids is advantageous in, for example, (a) increasing polypeptide serum half-life and/or functional in vivo half-life, (b) reducing polypeptide antigenicity or immunogenicity, (c) increasing polypeptide storage stability, (d) increasing bioavailability, (e) decreasing effector function, and/or (f) decreasing or inhibiting undesired self-association (*e.g.,* aggregate formation) between two or more CTLA4 proteins of the disclosure. Amino acid(s) can be modified, for example, co-translationally or post-translationally during recombinant production (*e.g.,* N-linked glycosylation at N-X-S/T motifs during expression in mammalian cells) or modified by synthetic means.

In another aspect, a CTLA4 protein of the disclosure can be modified by the addition of protecting groups to the side chains of one or more the amino acids in the CTLA4 portion and/or in the fusion partner portion. Such protecting groups can facilitate transport of the polypeptides of the disclosure through membrane(s) or through certain tissue(s), for example, by reducing the hydrophilicity and increasing the lipophilicity of the polypeptides. Examples of suitable protecting groups include ester protecting groups, amine protecting groups, acyl protecting groups, and carboxylic acid protecting groups, which are known in the art (see, *e.g.,* U.S. Patent No. 6,121,236). Other modifications of the CTLA4 proteins of the disclosure, for example, by addition of polyethylene glycol or a detectable label, are discussed in Sections 5.6 and 5.7 below.

### 6.3. Increased Stability of CTLA4 Polypeptides

In certain aspects, the present disclosure provides CTLA4 polypeptides having increased protein stability as compared to wild-type CTLA4 Ig. The present disclosure provides CTLA4 polypeptides having single or multiple amino acid substitutions in their CDR-like loop regions as compared to the CDR-like loops of wild type CTLA4 extracellular domain and/or in the regions of the CTLA4 extracellular domain that are outside the CDR-like loops and/or in the fusion partner polypeptide, if present, wherein at least one substitution results in increased stability of the CTLA4 polypeptide as compared to wild-type CTLA4 Ig. In certain embodiments, the increased stability is upon storage of the CTLA4 polypeptide. In other embodiments, the increased stability is due to resistance to degradation by proteases and the like. In various embodiments, the increased stability is due to increased conformational stability. In some embodiments, the increased stability is due to decreased aggregation.

In certain embodiments, the increased stability does not affect the binding affinity of the variant CTLA4 polypeptides of the disclosure to CD80 and/or CD86. In certain embodiments, the stability of a CTLA4 protein of the disclosure can be at least 0.1-fold, at least 0.2-fold, at least 0.3-fold, at least 0.4-fold, at least 0.5-fold, at least 0.6-fold, at least 0.7-fold, at least 0.8-fold, at least 0.9-fold, at least 1-fold, at least 2-fold, at least 3-fold, at least 4-fold, at least 5-fold, at least 6-fold, at least 7-fold, at least 8-fold, at least 9-fold, or at least 10-fold of the stability of wild-type CTLA4 Ig. In certain embodiments, the stability of a CTLA4 protein of the disclosure is increased relative to the stability of wild-type CTLA4 Ig in a range between any of the foregoing values, *e.g.,* 0.1-fold to 2-fold, 5-fold to 10-fold, 0.9-fold to 6-fold, or the like. In certain aspects, the stability of a CTLA4 polypeptide of the disclosure in a sample is greater than the stability of wild-type CTLA4 Ig in a sample where the samples are substantially identical (*e.g.,* are both in pharmaceutical compositions containing the same ingredients) and/or are processed or handled in substantially the same way (*e.g.,* are stored under the same conditions of temperature and humidity for the same period of time).

In certain aspects, the increased stability of a CTLA4 polypeptide of the disclosure is an increase in shelf-life. An increased shelf life can result from a decrease in amino acid oxidation (for example because an amino acid residue susceptible to oxidation, such as methionine, cysteine or tryptophan, in a CDR-like loop or in a region outside the CDR-like loops of the CTLA4 extracellular domain has been substituted relative to wild-type CTLA4-Ig). An increased shelf life can also result from a decrease in amino acid cyclization (for example because an amino acid residue susceptible to cyclization, such as asparagines or glutamic acid) in a CDR-like loop or in a region outside the CDR-like loops of the CTLA4 extracellular domain has been substituted relative to wild-type CTLA4 Ig. An increased shelf life can also result from the elimination of an amino acid motif that is recognized by proteases that may be in protein purifications at residual levels. An increased shelf life can also result from the elimination of an amino acid motif that causes aggregation of the protein. An increased shelf life can also result from an increase in conformational stability. The increase in shelf life or conformational stability, or decrease in oxidation, cyclization, proteolysis or aggregation levels relative to wild-type CTLA4 Ig can be at least 0.1-fold, at least 0.2-fold, at least 0.3-fold, at least 0.4-fold, at least 0.5-fold, at least 0.6-fold, at least 0.7-fold, at least 0.8-fold, at least 0.9-fold, at least 1-fold, at least 2-fold, at least 3-fold, at least 4-fold, at least 5-fold, at least 6-fold, at least 7-fold, at least 8-fold, at least 9-fold, or at least 10-fold. In certain embodiments, increase in shelf life or conformational stability, or decrease in oxidation, cyclization, proteolysis or aggregation levels relative to wild-type CTLA4 Ig is in a range between any of the foregoing values, *e.g.,* 0.1-fold to 2-fold, 5-fold to 10-fold, 0.9-fold to 6-fold, or the like.

Described herein are methods for screening CTLA4 polypeptides for increased stability relative to wild-type CTLA4 Ig. The methods may comprise the steps of: (a) determining the amount of full-length CTLA4 polypeptide in a first sample; and (b) comparing the amount of full-length CTLA4 polypeptide in the first sample with an amount of full-length wild-type CTLA4 Ig in a second sample. Methods of assaying for protein stability are known in the art.

### 6.4. Reduced Immunogenicity of CTLA4 Polypeptides

In certain aspects, the present disclosure provides CTLA4 polypeptides having reduced immunogenicity as compared to belatacept or MAXY-4 (Maxygen). The present disclosure provides CTLA4 polypeptides having single or multiple amino acid substitutions in their CDR-like loop regions as compared to the CDR-like loops of belatacept or MAXY-4 extracellular domain and/or in the regions of the CTLA4 extracellular domain that are outside the CDR-like loops and/or in the fusion partner polypeptide, if present, wherein at least one substitution results in reduced immunogenicity of the CTLA4 polypeptide as compared to belatacept or MAXY-4. In certain embodiments, the reduced immunogenicity results from one or more amino acid substitutions that result in eliminating or mitigating one or more T cell epitopes.

In certain aspects, the CTLA4 proteins of the disclosure having reduced immunogenicity have comparable or altered biological activity as compared to belatacept or MAXY-4, *e.g.,* affinity towards CD80 and/or CD86.

In certain embodiments, the immunogenicity of a CTLA4 polypeptide of the disclosure is reduced relative to belatacept or MAXY-4. Such polypeptides generally have variant sequences relative to the CTLA4 extracellular domain and/or the fusion partner, if present. In certain embodiments, the CTLA4 polypeptides will generally have 1, 2 or 3 amino acid substitutions in one or both of the CTLA4 extracellular domain sequence and the fusion partner sequence, if present. In other embodiments, the CTLA4 polypeptides can have 4, 5, 6, 7, 8, 9, or 10 amino acid substitutions in one or both of the CTLA4 extracellular domain sequence and the fusion partner sequence, if present. In still other embodiments, the the CTLA4 polypeptides can have 11, 12, 13, 14, 15, 16, 17, 18, 19, or 20 amino acid substitutions in one or both of the CTLA4 extracellular domain sequence and the fusion partner sequence, if present.

As used in the present disclosure, a variant with "reduced immunogenicity" refers to a CTLA4 polypeptide comprising a variant sequence as compared to belatacept or MAXY-4 that elicits a reduced proliferative response in peripheral blood mononuclear cells as compared to belatacept or MAXY-4. An exemplary proliferation assay that can be used to evaluate the proliferative response is set forth in Section 5.6 below. The reduced proliferative response can be reflected in terms of the percentage of responders, the stimulation index, or both.

In other embodiments, a peptide having a variant sequence as compared to the corresponding peptide in belatacept or MAXY-4 results in at least 25% fewer responders, in at least 30% fewer responders, in at least 35% fewer responders, in at least 40% fewer responders, in at least 45% fewer responders, in at least 50% fewer responders, in at least 60% fewer responders, in at least 65% fewer responders, in at least 70% fewer responders, in at least 75% fewer responders, in at least 80% fewer responders, in at least 85% fewer responders, in at least 90% fewer responders, in at least 95% fewer responders, in at least 100% fewer responders, or a reduction in responders in a range between any of the foregoing values, *e.g.,* 25%-75% fewer responders, 50%-90% fewer responders, 60%-100% fewer responders, 70%-90% fewer responders, or the like. In certain aspects, the ability of variant peptides of belatacept or MAXY-4 to elicit a T-cell response as compared respectively to the corresponding wild-type peptides of belatacept or MAXY-4 is determined by the assay set forth in Section 5.6, below.

In other embodiments, a peptide having a variant sequence as compared to the corresponding peptide in belatacept or MAXY-4 results in a stimulation index that is at least 5% less, at least 10% less, at least 15% less, at least 20% less, at least 25% less, at least 30% less, at least 35% less, or at least 40% less than the stimulation index elicited by the corresponding peptide in wild-type CTLA4 Ig, or results in a stimulation reduced by a range between any of the foregoing values as compared to wild-type CTLA4 Ig, *e.g.,* 5%-20% less, 10%-30% less, 30%-40% less, or the like. In certain embodiments, the stimulation index is determined by the assay set forth in Section 5.6, below.

### 6.5. Nucleic Acids and Expression Systems

Described herein are nucleic acid molecules encoding the CTLA4 polypeptides of the disclosure and host cells engineered to recombinantly express the polypeptides.

A CTLA4 protein of the disclosure can be prepared by recombinant expression of a nucleic acid encoding a CTLA4 binding portion or of a nucleic acid encoding a CTLA4 fusion protein in a host cell. To express a CTLA4 protein recombinantly, a host cell is transfected with one or more recombinant expression vectors carrying nucleic acids encoding the CTLA4 proteins such that the proteins are expressed in the host cell and, optionally, secreted into the medium in which the host cells are cultured, from which medium the proteins can be recovered. Standard recombinant DNA methodologies are used to obtain nucleic acids encoding CTLA4 proteins, incorporate these nucleic acids into recombinant expression vectors and introduce the vectors into host cells, such as those described in Molecular Cloning; A Laboratory Manual, Second Edition (Sambrook, Fritsch and Maniatis (eds), Cold Spring Harbor, N. Y., 1989), Current Protocols in Molecular Biology (Ausubel, F.M. et al., eds., Greene Publishing Associates, 1989) and in U.S. Patent No. 4,816,397.

The nucleic acids encoding the variant CTLA4 polypeptides of the disclosure can be synthesized in part or completely. Alternatively, nucleic acids encoding the CTLA4 binding portion are first obtained. These DNAs can be obtained from PCR of total cellular RNA of, for example, H38 (HTLV-II-associated leukemia) cell line. *See* U.S. Patent no. 5,434,131. In addition, if the CTLA4 protein is a fusion protein, nucleic acids encoding the fusion partner, *e.g.,* an immunoglobulin constant region, can be obtained by amplification and modification of germline DNA or cDNA encoding the desired fusion protein, for example using the polymerase chain reaction (PCR). A nucleic acid encoding wild-type abatacept can be synthesized and used as a template for mutagenesis to generate a variant as described herein using routine mutagenesis techniques; alternatively, a nucleic acid encoding the variant can be directly synthesized.

Once nucleic acids encoding the variant CTLA4 binding domains are obtained, the nucleic acid can be further manipulated by standard recombinant DNA techniques, for example to operatively link the CTLA4 binding domain-encoding DNA to another nucleic acid encoding another protein, *e.g.,* a fusion partner, such as an antibody constant region and/or a flexible linker. The term "operatively linked," as used in this context, is intended to mean that the two nucleic acids are joined such that the amino acid sequences encoded by the two nucleic acids remain in-frame.

The isolated DNA encoding the CTLA4 binding domain can be converted to a nucleic acid encoding a fusion protein by operatively linking the CTLA4 binding domain-encoding DNA to another DNA molecule encoding one or more heavy chain constant regions (CH₁, CH₂, CH₃ and, optionally, CH₄). The sequences of human heavy chain constant region nucleic acids are known in the art (see *e.g.,* Kabat, E.A., et al., 1991, Sequences of Proteins of Immunological Interest, Fifth Edition, U.S. Department of Health and Human Services, NIH Publication No. 91-3242) and nucleic acids encompassing these regions can be obtained by standard PCR amplification. The heavy chain constant region can be an IgG₁, IgG₂, IgG₃, IgG₄, IgA, IgE, IgM or IgD constant region, but in certain embodiments is an IgG₁ constant region.

To express the CTLA4 proteins of the disclosure, DNAs encoding partial or full-length proteins, obtained as described above, are inserted into expression vectors such that the nucleic acids are operatively linked to transcriptional and translational control sequences. In this context, the term "operatively linked" is intended to mean that a nucleic acid encoding a CTLA4 protein is ligated into a vector such that transcriptional and translational control sequences within the vector serve their intended function of regulating the transcription and translation of the CTLA4 protein-encoding nucleic acid. The expression vector and expression control sequences are chosen to be compatible with the expression host cell used.

The nucleic acids encoding CTLA4 proteins of the disclosure are inserted into the expression vector by standard methods (*e.g.,* ligation of complementary restriction sites on the CTLA4 protein gene fragment and vector, or blunt end ligation if no restriction sites are present). If the CTLA4 protein is a fusion protein, prior to insertion of the CTLA4 binding domain-encoding sequences, the expression vector can already carry fusion partner sequences, *e.g.,* antibody constant region sequences. For example, one approach to converting a protein of the disclosure comprising only a CTLA4 binding domain into a CTLA4 fusion protein is to insert the nucleic acid encoding the CTLA4 binding domain into expression vectors already encoding immunoglobulin Fc such that the CTLA4-encoding sequence is operatively linked to the Fc-encoding sequence within the vector. Additionally or alternatively, the recombinant expression vector can encode a signal peptide that facilitates secretion of the CTLA4 protein from a host cell. The nucleic acid encoding a CTLA4 protein can be cloned into the vector such that the signal peptide is linked in-frame to the amino terminus of the CTLA4 protein-encoding nucleic acid. The signal peptide can be a CTLA4 signal peptide or a heterologous signal peptide, *e.g.,* an immunoglobulin signal peptide.

In addition to nucleic acids encoding CTLA4 proteins, the recombinant expression vectors described herein carry regulatory sequences that control the expression of the CTLA4 protein-encoding nucleic acids in a host cell. The term "regulatory sequence" is intended to include promoters, enhancers and other expression control elements (*e.g.,* polyadenylation signals) that control the transcription or translation of the nucleic acids encoding the CTLA4 proteins of the disclosure. Such regulatory sequences are described, for example, in Goeddel, Gene Expression Technology: Methods in Enzymology 185 (Academic Press, San Diego, CA, 1990). It will be appreciated by those skilled in the art that the design of the expression vector, including the selection of regulatory sequences may depend on such factors as the choice of the host cell to be transformed, the level of expression of protein desired, etc. Suitable regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (*e.g.,* the adenovirus major late promoter (AdMLP)) and polyoma. For further description of viral regulatory elements, and sequences thereof, see *e.g.,* U.S. Patent No. 5,168,062 by Stinski, U.S. Patent No. 4,510,245 by Bell et al., and U.S. Patent No. 4,968,615 by Schaffner et al.

In addition to the CTLA4 protein-encoding nucleic acids and regulatory sequences, the recombinant expression vectors described herein can carry additional sequences, such as sequences that regulate replication of the vector in host cells (*e.g.,* origins of replication) and selectable marker genes. The selectable marker gene facilitates selection of host cells into which the vector has been introduced (see *e.g.,* U.S. Patents Nos. 4,399,216, 4,634,665 and 5,179,017, all by Axel et al.). For example, typically the selectable marker gene confers resistance to drugs, such as G418, puromycin, blasticidin, hygromycin or methotrexate, on a host cell into which the vector has been introduced. Suitable selectable marker genes include the dihydrofolate reductase (DHFR) gene (for use in DHFR- host cells with methotrexate selection/amplification) and the neo gene (for G418 selection). For expression of the CTLA4 proteins, the expression vector(s) encoding the CTLA4 proteins is transfected into a host cell by standard techniques. The various forms of the term "transfection" are intended to encompass a wide variety of techniques commonly used for the introduction of exogenous DNA into a prokaryotic or eukaryotic host cell, *e.g.,* electroporation, lipofection, calcium-phosphate precipitation, DEAE- dextran transfection and the like.

It is possible to express the CTLA4 proteins of the disclosure in either prokaryotic or eukaryotic host cells. In certain instances, expression of CTLA4 proteins is performed in eukaryotic cells, *e.g.,* mammalian host cells or yeast host cells, for optimal secretion of a properly folded, post-translationally modified and immunologically active protein. Exemplary mammalian host cells for expressing the recombinant proteins of the disclosure include Chinese Hamster Ovary (CHO cells) (including DHFR- CHO cells, described in Urlaub and Chasin, 1980, Proc. Natl. Acad. Sci. USA 77:4216-4220, used with a DHFR selectable marker, *e.g.,* as described in Kaufman and Sharp, 1982, Mol. Biol. 159:601-621), NS0 myeloma cells, COS cells, 293 cells and SP2/0 cells. When recombinant expression vectors encoding CTLA4 proteins are introduced into mammalian host cells, the proteins are produced by culturing the host cells for a period of time sufficient to allow for expression of the protein in the host cells or secretion of the protein into the culture medium in which the host cells are grown. CTLA4 proteins can be recovered from the culture medium using standard protein purification methods.

Recombinant DNA technology can also be used to remove some or all of the DNA encoding the CTLA4 proteins that is not necessary for binding to a CTLA4 ligand. The molecules expressed from such truncated DNA molecules are also encompassed by the proteins of the disclosure.

Once a nucleic acid encoding one or more portions of wild-type abatacept or of a CTLA4 protein with CDR-like sequences related to the CDR-like sequences of abatacept is generated, further alterations or mutations can be introduced into the coding sequence, for example to generate nucleic acids encoding CTLA4 proteins with different CDR-like sequences, or proteins with reduced affinity to the Fc receptor.

The CTLA4 proteins of the disclosure can also be produced by chemical synthesis (*e.g.,* by the methods described in Solid Phase Peptide Synthesis, 2nd ed., 1984 The Pierce Chemical Co., Rockford, Ill.). Variant proteins can also be generated using a cell-free platform (see, *e.g.,* Chu et al., Biochemia No. 2, 2001 (Roche Molecular Biologicals)).

Once a CTLA4 protein of the disclosure has been produced by recombinant expression, it can be purified by any method known in the art for purification of proteins, for example, using antibodies reactive with the CTLA4 binding domain of a protein of the disclosure, or chromatography (*e.g.,* ion exchange, affinity, such as affinity for CD80 and/or CD86, and sizing column chromatography), centrifugation, differential solubility, or by any other standard technique. Further, the CTLA4 proteins of the present disclosure can be fused to heterologous polypeptide sequences described herein or otherwise known in the art to facilitate purification.

Once isolated, a CTLA4 protein can, if desired, be further purified, *e.g.,* by high performance liquid chromatography (See, *e.g*., Fisher, Laboratory Techniques In Biochemistry And Molecular Biology (Work and Burdon, eds., Elsevier, 1980)), or by gel filtration chromatography on a SuperdexTM 75 column (Pharmacia Biotech AB, Uppsala, Sweden).

### 6.6. Biological Activities of CTLA4 Proteins

In various embodiments, the CTLA4 proteins of the disclosure have certain biological activities, such as competing with abatacept for binding to CD80 and/or CD86, or blocking T-cell response.

Accordingly, in certain embodiments, CTLA4 proteins of the disclosure compete with abatacept for binding to CD80 or CD86. The ability to compete for binding to CD80 or CD86 can be tested using a competition assay. In one example of a competition assay, the AlphaLISA® (Amplified Luminescent Proximity Homogeneous) or AlphaScreen® assay can be used as a non-wash alternative to ELISA. In the AlphaLISA® assay, the ligand, CD80 or CD86 fused with a tag (*e.g.*, Cλ-HA) is bound to AlphaLISA® acceptor beads conjugated with an anti-tag antibody (*e.g*., goat anti-human Cλ) and biotinylated abatacept is bound to AlphaScreen® strepavidin-coated donor beads (PerkinElmer, Waltham, MA). In the absence of competing CTLA4 variants, abatacept interacts with CD80 or CD86 and produces a signal at 615 nm. Addition of unlabeled CTLA4 variant competes with the abatacept/CD80 or CD86 interaction, reducing fluorescence quantitatively to enable determination of relative binding affinities. Abatacept can be biotinylated with Sulfo-NHS-Biotin using standard methods and dialyzed in PBS. Conjugation of AlphaLISA® acceptor beads is performed by following the "AlphaLISA® Assay Development Guide" (PerkinElmer). The binding is performed in a 384-well AlphaPlate (PerkinElmer) in assay buffer (*e.g.,* 1% BSA, 0.01% Tween20 in PBS (pH 7.1)). Each well contains 0.5 nM biotinylated abatacept, 1:4 serially diluted CTLA4 competitor starting from 40nM, 0.078 µg/ml CD80, and 5 µg/ml goat anti-human Cλ-conjugated acceptor beads. The plate is incubated in the dark for 1 h at room temperature. Streptavidin donor beads are then added to each well at 5 µg /ml. The plate is incubated in the dark at room temperature for an additional 30 min, after which it is read on an EnVision reader (PerkinElmer). Data are fit using nonlinear regression with the software GRAPHPAD PRISM (GraphPad, San Diego).

For CD86 binding, 8nM biotinylated abatacept, 1:4 serially diluted competitor starting from 100nM, 0.1 µg/ml CD86 and 10 µg/ml goat anti-human Cλ-conjugated acceptor beads are first incubated and then 10 µg/ml streptavidin donor beads are added. Variations on this competition assay can also be used to test competition between CTLA4 proteins of the disclosure and abatacept. For example, in certain aspects, the CTLA4 protein is used as a reference and abatacept is used as a test molecule. Additionally, instead of soluble CD80 or CD86, membrane-bound ligand expressed on the surface of cells (*e.g.,* mammalian cells such as CHO) in culture can be used. Other formats for competition assay are known in the art and can be employed.

In various embodiments, a CTLA4 protein of the disclosure reduces the binding of labeled abatacept by at least 10%, by at least 20%, by at least 30%, by at least 40%, by at least 50%, by at least 60%, by at least 70%, by at least 80%, by at least 90%, by at least 95%, by at least 99%, or by a percentage ranging between any of the foregoing values (*e.g.*, a CTLA4 protein of the disclosure reduces the binding of labeled abatacept by 10% to 40%, 50% to 70%, by 40% to 60% or by 80% to 99%) when the CTLA4 protein is used at a concentration of 0.01 µg/ml, 0.025 µg/ml, 0.05 µg/ml, 0.075 µg/ml, 0.1 µg/ml, 0.25 µg/ml, 0.5 µg/ml, 0.75 µg/ml, 1 µg/ml, 2 µg/ml, 10 µg/ml, 50 µg/ml, 100 µg/ml or at a concentration ranging between any of the forgoing values (*e.g*., at a concentration ranging from 0.1 µg/ml to 2 µg/ml).

In certain aspects, the CTLA4 variants of the disclosure are tested for their ability to elicit a T-cell response. In particular, CTLA4 peptides are synthesized using a multi-pin format by Mimotopes (Adelaide, Australia). The CTLA4 polypeptide sequences can be synthesized, for example, as 15-mer peptides overlapping by 12 amino acids. Peptides are resuspended in DMSO (Sigma-Aldrich) at approximately 1-2 mg/ml. Stock peptides are kept frozen at -20°C. For isolation of peripheral blood mononuclear cells, community donor buffy coat products can be purchased from the Stanford Blood Center, Palo Alto, CA. Buffy coat material is diluted 1:1 v:v with DPBS containing no calcium or magnesium. Diluted buffy coat material (25-35 mls) is underlayed in 50 ml conical centrifuge tubes (Sarsted or Costar) with 12.5 mls of FicollPaque-PLUS (GE Healthcare). The samples are centrifuged at 900 g for 30 minutes at room temperature. Peripheral blood mononuclear cells (PBMC) are collected from the interface. DPBS is added to bring the final volume to 50 mls and the cells are centrifuged at 350 g for 5 minutes. Pelleted cells are resuspended in DPBS and counted. For isolation of dendritic cells, T75 culture flasks (Costar) are seeded with 108 freshly isolated PBMC in a total volume of 30 mls AIM V media (Invitrogen). Excess PBMC are frozen at -80°C in 90% fetal calf serum (FCS), 10% DMSO at 5 x 107 cells/ml. T75 flasks were incubated at 37°C in 5% CO2 for 2 hours. Nonadherent cells are removed, and the adherent monolayer is washed with DPBS. To differentiate dendritic cells from monocytes, 30 mls of AIM V media containing 800 units/ml of GM-CSF (R and D Systems) and 500 units/ml IL-4 (R and D Systems) are added. Flasks are incubated for 5 days. On day 5 IL-1α (Endogen) and TNFα (Endogen) are added to 50 pg/ml and 0.2 ng/ml, respectively. Flasks are incubated for two more days. On day 7, dendritic cells are collected by the addition of 3 mls of 100 mM EDTA containing 0.5 to 1.0 mg Mitomycin C (Sigma-Aldrich) for a final concentration of 10 mM EDTA and 16.5 to 33 µg/ml Mitomycin C. Flasks are incubated an additional hour at 37°C and 5% CO2. Dendritic cells are collected, and washed in AIM V media 2-3 times. For cell culture, on day 7, previously frozen autologous PBMC are thawed quickly in a 37°C water bath. Cells are immediately diluted into DPBS or AIM V media and centrifuged at 350g for 5 minutes. CD4⁺ cells are enriched by negative selection using magnetic beads (Easy-Sep CD4⁺ kit, Stem Cell Technologies). Autologous CD4+ T cells and dendritic cells are cocultured at 2 x 10⁵ CD4⁺ T-cells per 2 x 10⁴ dendritic cells per well in 96 well round bottomed plates (Costar 9077). Peptides are added at approximately 5 µg/ml. Control wells contain the DMSO (Sigma) vehicle alone at 0.25% v:v. Positive control wells contain DMSO at 0.25% and tetanus toxoid (List Biologicals or CalBioChem) at 1 µg/ml. Cultures are incubated for 5 days. On day 5, 0.25 µCi per well of tritiated thymidine (Amersham or GE Healthcare) is added. Cultures are harvested on day 6 to filtermats using a Packard Filtermate Cell harvester. Scintillation counting is performed using a Wallac MicroBeta 1450 scintillation counter (Perkin Elmer). Average background CPM values are calculated by averaging individual results from 6 to 12 replicates. The CPM values of the four positive control wells are averaged. Replicate or triplicate wells for each peptide are averaged.

Stimulation index values for the positive control and the peptide wells are calculated by dividing the average experimental CPM values by the average control values. In order to be included in the dataset, a stimulation index of greater than 3.0 in the tetanus toxoid positive control wells is required. A response is noted for any peptide resulting in a stimulation index of 2.95 or greater. Peptides are tested using peripheral blood samples from a group of 100 donors. Responses to all peptides are compiled. For each peptide tested, the percentage of the donor set that responds with a stimulation index of 2.95 or greater is calculated. In addition, the average stimulation index for all donors is calculated. CD4⁺ T-cell epitope peptides are identified by an analysis of the percent responses to the peptides within the set of 100 donors. The average percent response and standard deviation are calculated for all peptides tested describing the CTLA4 polypeptide. A response rate greater than or equal to the average background response plus three standard deviations is considered a potential CD4⁺ T-cell epitope.

In other aspects, a CTLA4 polypeptide of the disclosure inhibits activity of CD80 and/or CD86 in a range of *in vitro* assays, such as cell proliferation, cytokine (*e.g.,* IL-2) production and cell adhesion.

In some embodiments, the activity assayed is immunosuppressive activity of the CTLA4 polypeptides of the disclosure. In particular, human blood freshly collected from donors is diluted with an equal volume of PBS and fractionated to isolate PMBCs using a Histopaque (Sigma) Ficoll gradient as per the manufacturer's instructions. PMBCs are diluted in RPMI medium (Sigma) supplemented with 10% FBS (Hyclone) and 1x PSG (penicillin, streptomycin and glutamine) (Invitrogen) and added to 96-well culture plates (BD Biosciences) at a density of 1 x 10⁵ cells/well. CTLA4 proteins to be tested are serially diluted in the same medium and added to wells in quadruplicate. Cell proliferation is initiated by addition of PPD antigen (purified protein derivative from *mycobacterium tuberculosis*, Mycos) to a final concentration of 5µl/ml. After incubation at 37°C for 5 days, ³H thymidine (GE Healthcare) is added at 1 µCi/well and plates are incubated at 37 °C for an additional 18 hours. Cells are harvested with a cell harvester (FilterMate Omnifilter-96 Harvester, Perkin Elmer) using the manufacturer's recommended conditions and measured for ³H thymidine incorporation using a scintillation counter (Wallac Trilux). Cell proliferation data are analyzed with GraphPad Prism 5 software using a non-linear regression curve fit model (sigmoidal dose-response, variable slope) and the least-squares fit method. The IC₅₀ parameters for each variant and their associated 95% confidence intervals are determined.

In certain embodiments, the activity assayed is inhibition of proliferation of primary and secondary allo-stimulated T-cells. In particular, the activity can be measured by the proliferation of human peripheral blood CD4⁺ T-cells, for example as described by Larsen et al., 2005, Am. J. Transplant. 5:443-453. In primary allo-response assays, human CD4⁺ T-cells (3-10 x 10⁴/well) are isolated and incubated with irradiated human B lymphoblastoid cell lines expressing equivalent amounts of CD80 and CD86 (e.g., PM cells, 8.0 x 10³/well), along with varying concentrations of wild-type CTLA4-Ig or CTLA4 proteins of the disclosure. Primary allogenic stimulation assays proceed for 6 days before addition of 0.5µCi/well of [³H]-thymidine for the final 7h of culture. For secondary allogenic stimulation assays, viable cells from 7-day primary allo-stimulated T-cells are isolated by differential density centrifugation and rested for 24h. T-cells are then restimulated (secondary stimulation) by adding PM cells in the same ratio as above in the presence of titrating amounts of wild-type CTLA4-Ig or CTLA4 proteins of the disclosure. T-cells are stimulated for 3 days, pulsed with [³H]-thymidine for the final 7h of culture and harvested as above. Alternatively, the proliferation of primary and secondary allo-stimulated T-cells can be assayed as described in Example 2 below.

In various embodiments, the activity of CTLA4 variants of the disclosure can be measured by inhibition of cytokine production. For example, in specific embodiments, the cytokine is IL-2 produced by Jurkat T cells (Cox et al., 1999, Protein Expr. Purif. 17(1):26-32). Jurkat cells (1.0 x 10⁵/well) are stimulated with 10µg/mL PHA-L and cells (*e.g.,* CHO-K1, 2.5 x10⁴/well) expressing recombinant CD80 or CD86 in the presence of titrating amounts of wild-type CTLA4-Ig or CTLA4 proteins of the disclosure. After 24 hours of culture, IL-2 concentration in the culture supernatant is measured using, for example, an AlphaLISA® Human IL-2 kit (Perkin Elmer). Alternatively, IL-2 concentration can be measured by other methods known in the art (*e.g.,* ELISA).

Alternatively, in another embodiment, the inhibition of cytokine production can be measured in the culture media of secondary allo-stimulated T-cells (Larsen et al., 2005, Am. J. Transplant. 5:443-453). Aliquots of culture supernatants from T-cells allo-stimulated for 3 days as described in secondary allo-stimulation assay above are removed prior to addition of [³H]-thymidine. The level of cytokine production (*e.g*., IFNγ, IL-2, IL-4) is measured using bead-based assays such as Luminex® (Invitrogen) or other methods known in the art (*e.g.,* ELISA).

In certain embodiments, the activity assayed is cell adhesion. In particular, cells (*e.g.,* CHO cells) recombinantly expressing CD80 or CD86 on the cells surface are diluted in medium, *e.g.,* 10% FBS in RPMI, and seeded in 96-well plates and grown to confluence. When confluent, cells are incubated with a CTLA4 protein of the disclosure or abatacept or are used as a negative control. Unbound protein is removed from the cells by washing with warm medium. Purified T-cells that are either unstimulated or are prestimulated with 1 µg/ml anti-CD3 monoclonal antibody (30 min at 37°C). T-cells (1 x 10⁵ cells/well in 100µl medium) are seeded onto the CD80 or CD86 expressing cells and allowed to adhere for 30 min at 37°C. Nonadherent cells are removed by washing each well three times with warm medium and counted (*e.g.,* by trypan blue exclusion) in triplicate. Cell adherence to the CHO cells is calculated as (the number of nonadherent cells/total number of input cells) X 100%. Alternatively, T-cells can be incubated with a fluorescent dye such as calcein AM (Molecular Probes, Eugene OR) according to the manufacturer's instructions before being seeded into the wells. After incubation with T-cells, removal of medium containing non-adherent T-cells and washing, plates are read by a fluorescence multi-well plate reader. Percent of T-cell adhesion is derived from the formula: (specific adhesion in the presence of test fusion protein - background adhesion)/(specific adhesion in the presence of abatacept - background adhesion).

### 6.7. Kinetic Properties of CTLA4-Ig Fusion Proteins

In certain embodiments, the CTLA4 proteins of the disclosure have high affinity for CD80 and CD86. As used herein, variant proteins with "high affinity" for CD80 and/or CD86 have at least a 1.5-fold improvement in binding affinity as compared to abatacept. In other embodiments, the CTLA4 proteins of the disclosure have high affinity for CD80 and neutral affinity for CD86. In various embodiments, the CTLA4 proteins of the disclosure have high affinity for CD80 and low affinity for CD86. In still other embodiments, the CTLA4 proteins of the disclosure have high affinity for CD86 and neutral affinity for CD80. As used herein, variant proteins with "neutral affinity" for CD80 and/or CD86 have at least 0.5-fold but less than 1.5-fold improvement in binding affinity as compared to abatacept. In various embodiments, the CTLA4 proteins of the disclosure have low affinity for CD80 and high affinity for CD86. Also described are CTLA4 proteins which have neutral affinity for both CD80 and CD86. Also described are CTLA4 proteins which have neutral affinity for CD80 and low affinity for CD86. Also described are CTLA4 proteins which have low affinity for CD80 and neutral affinity for CD86. As used herein, a variant with "low affinity" for CD80 and/or CD86 has less than 0.5-fold improvement in binding affinity as compared to abatacept.

In specific embodiments, the CTLA4 proteins of the present disclosure have specific association rate constants (kₒₙ or kₐ values), dissociation rate constants (k_{off} or k_{d} values), affinity constants (K_{A} values), dissociation constants (K_{D} values) and/or IC₅₀ values. In various embodiments, binding constants for the interaction of the CTLA4 proteins with either CD80 or CD86 extracellular domain can be determined using surface plasmon resonance according to the method disclosed in Karlsson et al., 1991, J. Immunol. Methods 145:229-240. In certain aspects, such values are selected from the following embodiments.

In specific embodiments, the CTLA4 proteins of the disclosure bind to CD80 with a kₒₙ of at least 10² M⁻¹s⁻¹,at least 5 X 10² M⁻¹s⁻¹, at least 10³ M⁻¹s⁻¹, at least 5 X 10³M⁻¹s⁻¹, at least 10⁴ M⁻¹s⁻¹, at least 5 X 10⁴ M⁻¹s⁻¹, at least 10⁵ M⁻¹s⁻¹, at least 5 X 10⁵ M⁻¹s⁻¹, at least 10⁶ M⁻¹s⁻¹, at least 5 X 10⁶ M⁻¹s⁻¹, at least 10⁷ M⁻¹s⁻¹, at least 5 X 10⁷ M⁻¹s⁻¹, at least 10⁸ M⁻¹s⁻¹, at least 5 X 10⁸ M⁻¹s⁻¹, at least 10⁹M⁻¹s⁻¹, at least 5 X 10⁹ M⁻¹s⁻¹ or with a kₒₙ of any range between any pair of the foregoing values (*e.g.,* 5 X 10⁴ M⁻¹s⁻¹ to 10⁶ M⁻¹s⁻¹ or 10² M⁻¹s⁻¹ to 5 X 10⁵ M⁻¹s⁻¹).

In certain embodiments, the CTLA4 proteins of the disclosure bind to CD86 with a kₒₙ of at least 10² M⁻¹s⁻¹, at least 5 X 10² M⁻¹s⁻¹, at least 10³ M⁻¹s⁻¹, at least 5 X 10³ M¹s¹, at least 10⁴ M⁻¹s⁻¹, at least 5 X 10⁴ M⁻¹s⁻¹, at least 10⁵ M⁻¹s⁻¹, at least 5 X 10⁵ M⁻¹s⁻¹, at least 10⁶ M⁻¹s⁻¹, at least 5 X 10⁶ M⁻¹s⁻¹, at least 10⁷ M⁻¹s⁻¹, at least 5 X 10⁷ M⁻¹s⁻¹, at least 10⁸ M⁻¹s⁻¹, at least 5 X 10⁸ M⁻¹s⁻¹, at least 10⁹M⁻¹s⁻¹, at least 5 X 10⁹ M⁻¹s⁻¹ or with a kₒₙ of any range between any pair of the foregoing values (*e.g.,* 5 X 10⁴ M⁻¹s⁻¹ to 10⁶ M⁻¹s⁻¹ or 10³ M⁻¹s⁻¹ to 5 X 10⁵ M⁻¹s⁻¹).

In certain embodiments, the CTLA4 proteins of the disclosure bind to CD80 with a k_{off} of 2 s⁻¹ ore less, 1.5 s⁻¹ or less, 1 s⁻¹ or less, 0.5 s⁻¹ or less, 0.1 s⁻¹ or less, 5 X 10⁻² s⁻¹ or less, 10⁻² s⁻¹ or less, 5 X 10⁻³ s⁻¹ or less, 10⁻³ s⁻¹ or less, 5 X 10⁻⁴ s⁻¹ or less, 10⁻⁴ s⁻¹ or less, 5 X 10⁻⁵ s⁻¹ or less, 10⁻⁵ s⁻¹ or less, 5 X 10⁻⁶ s⁻¹ or less, 10⁻⁶ s⁻¹ or less, 5 X 10⁻⁷ s⁻¹ or less, 10⁻⁷ s⁻¹ or less, 5 X 10⁻⁸ s⁻¹ or less, 10⁻⁸ s⁻¹ or less, or with a k_{off} rate of any range between any pair of the foregoing values (*e.g.,* 1 s⁻¹ to 5 X 10⁻³ s⁻¹, or 5 X 10⁻⁵ s⁻¹ to 10⁻⁸ s⁻¹).

In some embodiments, the CTLA4 proteins of the disclosure bind to CD86 with a k_{off} of 10 s⁻¹ or less, 5 s⁻¹ or less, 1 s⁻¹ or less, 0.5 s⁻¹ or less, 0.1 s⁻¹ or less, 5 X 10⁻² s⁻¹ or less, 10⁻² s⁻¹ or less, 5 X 10⁻³ s⁻¹ or less, 10⁻³ s⁻¹ or less, 5 X 10⁻⁴ s⁻¹ or less, 10⁻⁴ s⁻¹ or less, 5 X 10⁻⁵ s⁻¹ or less, 10⁻⁵ s⁻¹ or less, 5 X 10⁻⁶ s⁻¹ or less, 10⁻⁶ s⁻¹ or less, 5 X 10⁻⁷ s⁻¹ or less, 10⁻⁷ s⁻¹ or less, 5 X 10⁻⁸ s⁻¹ or less, 10⁻⁸ s⁻¹ or less, or with a k_{off} rate of any range between any pair of the foregoing values (*e.g*., 1 s⁻¹ to 5 X 10⁻³ s⁻¹, or 5 X 10⁻⁵ s⁻¹ to 10⁻⁸ s⁻¹).

In some embodiments, a CTLA4 protein of the disclosure binds to CD80 with a K_{A} (kon/koff) of at least at least 5 X 10⁵ M⁻¹, at least 10⁵ M⁻¹, at least 5 X 10⁶ M⁻¹, at least 10⁶ M⁻¹, at least 5 X 10⁷ M⁻¹, at least 10⁷ M⁻¹, at least 10⁸ M⁻¹, at least 5 X 10⁸ M⁻¹, at least 10⁹ M⁻¹, at least 5 X 10⁹ M⁻¹, at least 5 X 10¹⁰ M⁻¹, at least 10¹⁰ M⁻¹, at least 5 X 10¹¹ M⁻¹, at least 10¹¹ M⁻¹, at least 5 X 10¹² M⁻¹, at least 10¹² M⁻¹, at least 5 X 10¹³ M⁻¹, at least 10¹³M⁻¹, at least 5 X 10¹⁴M⁻¹, at least 10¹⁴ M⁻¹, at least 5 X 10¹⁵ M⁻¹, at least 10¹⁵ M⁻¹, or with a K_{A} of any range between any pair of the foregoing values (*e.g.,* 5 X 10⁵ M-¹ to 5 X 10¹³ M⁻¹ or 5 X 10⁸ M⁻¹ to 5 X 10¹⁵ M⁻¹).

In some embodiments, a CTLA4 protein of the disclosure binds to CD86 with a K_{A} (kon/koff) of at least 5 X 10⁴ M⁻¹, at least 10⁴ M⁻¹, at least 5 X 10⁵ M⁻¹, at least 10⁵ M⁻¹, at least 5 X 10⁶ M⁻¹, at least 10⁶ M⁻¹, at least 10⁷ M⁻¹, at least 5 X 10⁷ M⁻¹, at least 10⁸ M⁻¹, at least 5 X 10⁸ M⁻¹, at least 5 X 10⁹ M⁻¹, at least 10⁹ M⁻¹, at least 5 X 10¹⁰ M⁻¹, at least 10¹⁰ M⁻¹, at least 5 X 10¹¹ M⁻¹, at least 10¹¹ M⁻¹, at least 5 X 10¹²M⁻¹, at least 10¹²M⁻¹, at least 5 X 10¹³M⁻¹, at least 10¹³ M⁻¹, at least 5 X 10¹⁴ M⁻¹, at least 10¹⁴ M⁻¹, or with a K_{A} of any range between any pair of the foregoing values (*e.g.,* 5 X 10⁴ M⁻¹ to 10¹³ M⁻¹ or 5 X 10⁷ M⁻¹ to 5 X 10¹³ M⁻¹).

In various embodiments, a CTLA4 of the disclosure binds to CD80 with a K_{D} (k_{off}/kₒₙ) of 5 X 10⁻⁶ M or less, 10⁻⁶ M or less, 5 X 10⁻⁷ M or less, 10⁻⁷ M or less, 5 X 10⁻⁸ M or less, 10⁻⁸ M or less, 5 X 10⁻⁹ M or less, 10⁻⁹ M or less, 5 X 10⁻¹⁰ M or less, 10⁻¹⁰ M or less, 5 X 10⁻¹¹ M or less, 10⁻¹¹ M or less, 5 X 10⁻¹² M or less, 10⁻¹² M or less, 5 X 10⁻¹³ M or less, 10⁻¹³ M or less, 5 X 10⁻¹⁴ M or less, 10⁻¹⁴ M or less, 5 X 10⁻¹⁵ M or less, 10⁻¹⁵ M or less, or with a K_{D} of any range between any pair of the foregoing values (*e.g*., 10⁻⁷ M to 5 X 10⁻¹³ M, or 5 X 10⁻⁹ M to 10⁻¹⁵ M).

In various embodiments, a CTLA4 protein of the disclosure binds to CD86 with a K_{D} (k_{off}/kₒₙ) of 10⁻⁵ M or less, 5 X 10⁻⁶ M or less, 10⁻⁶ M or less, 5 X 10⁻⁷ M or less, 10⁻⁷ M or less, 5 X 10⁻⁸ M or less, 10⁻⁸ M or less, 5 X 10⁻⁹ M or less, 10⁻⁹ M or less, 5 X 10⁻¹⁰ M or less, 10⁻¹⁰ M or less, 5 X 10⁻¹¹ M or less, 10⁻¹¹ M or less, 5 X 10⁻¹² M or less, 10⁻¹² M or less, 5 X 10⁻¹³ M or less, 10⁻¹³ M or less, 5 X 10⁻¹⁴M or less, 10⁻¹⁴ M or less, 5 X 10⁻¹⁵ M or less, 10⁻¹⁵ Mor less, or with a K_{D} of any range between any pair of the foregoing values (*e.g*., 10⁻⁶ M to 5 X 10⁻¹⁰M, or 5 X 10⁻⁹ M to 10⁻¹⁵ M).

In some embodiments, the CTLA4 protein of the disclosure binds to CD80 and inhibits the binding of CD80 to abatacept in a binding assay (*e.g*., an AlphaLISA® competition assay) at an IC₅₀ value of less than 1000 nM, less than 750 nM, less than 500 nM, less than 100 nM, less than 50 nM, less than 10 nM, less than 5 nM, less than 1 nM, less than 0.75 nM, less than 0.5 nM, less than 0.25 nM, less than 0.1 nM, less than 5 X 10⁻² nM, less than 10⁻² nM, less than 5 X 10⁻³ nM, less than 10⁻³ nM, less than 5 X 10⁻⁴ nM, or less than 10⁻⁴ nM, or with an IC₅₀ of any range between any pair of the foregoing values (*e*.*g*.,100 nM to 5 X 10⁻² nM or 0.5 nM and 5 X 10⁻⁴ nM).

In some embodiments, the CTLA4 protein of the disclosure binds to CD86 and inhibits the binding of CD86 to abatacept in a binding assay (*e.g*., an AlphaLISA® competition assay) at an IC₅₀ value of less than 1000 nM, less than 500 nM, less than 100 nM, less than 50 nM, less than 10 nM, less than 5 nM, less than 1 nM, less than 0.75 nM, less than 0.5 nM, less than 0.25 nM, less than 0.1 nM, less than 5 X 10⁻² nM, less than 10⁻² nM, less than 5 X 10⁻³ nM, less than 10⁻³ nM, less than 5 X 10⁻⁴ nM, or less than 10⁻⁴ nM, or with an IC₅₀ of any range between any pair of the foregoing values (*e.g.,100* nM to 5 X 10⁻² nM or 0.5 nM and 5 X 10⁻⁴ nM).

In certain embodiments, the kinetic properties of a fusion protein of the disclosure are comparable to, or improved relative to, abatacept in a comparable assay. For example, in certain embodiments, a CTLA4 protein of the disclosure binds to CD80 and/or CD86 with a kₒₙ rate ranging from approximately 0.05x to 1000x of the kₒₙ of abatacept, for example a kₒₙ of 0.06x of the kₒₙ of abatacept, a kₒₙ of 0.08x of the kₒₙ of abatacept, a kₒₙ of 0.1x of the kₒₙ of abatacept, a kₒₙ of 0.5x of the kₒₙ of abatacept, a kₒₙ of 1x of the kₒₙ of abatacept, a kₒₙ of 1.1x of the kₒₙ of abatacept, a kₒₙ of 1.2x of the kₒₙ of abatacept, a kₒₙ of 1.3x of the kₒₙ of abatacept, a kₒₙ of 1.4x of the kₒₙ of abatacept, a kₒₙ of 1.5x of the kₒₙ of abatacept, a kₒₙ of 1.6x of the kₒₙ of abatacept, a kₒₙ of 1.6x of the kₒₙ of abatacept, a kₒₙ of 1.7x of the kₒₙ of abatacept, a kₒₙ of 1.8x of the kₒₙ of abatacept, a kₒₙ of 1.9x of the kₒₙ of abatacept, a kₒₙ of 2x of the kₒₙ of abatacept, a kₒₙ of 2.25x of the kₒₙ of abatacept, a kₒₙ of 2.5x of the kₒₙ of abatacept, a kₒₙ of 2.75x of the kₒₙ of traztuzumab, a kₒₙ of 3x of the kₒₙ of abatacept, a kₒₙ of 4x of the kₒₙ of abatacept, a kₒₙ of 5x of the kₒₙ of abatacept, a kₒₙ of 6x of the kₒₙ of abatacept, a kₒₙ of 7x of the kₒₙ of abatacept, a kₒₙ of 8x of the kₒₙ of abatacept, a kₒₙ of 9x of the kₒₙ of abatacept, a kₒₙ of 10x of the kₒₙ of abatacept, a kₒₙ of 15x of the kₒₙ of abatacept, a kₒₙ of 20x of the kₒₙ of abatacept, a kₒₙ of 50x of the kₒₙ of abatacept, a kₒₙ of 75x of the kₒₙ of abatacept, a kₒₙ of 100x of the kₒₙ of abatacept, a kₒₙ of 150x of the kₒₙ of abatacept, a kₒₙ of 200x of the kₒₙ of abatacept, or a kₒₙ ranging between any pair of the foregoing values, e.g., a kₒₙ of 0.1x-75x of the kₒₙ of abatacept, a kon of 5x-100x of the kon of abatacept, a kₒₙ of 0.05x-1000x of the kₒₙ of abatacept, a kₒₙ of 0.75x-250x of the kₒₙ of abatacept, *etc.*

In various embodiments, a CTLA4 protein of the disclosure binds to CD80 and/or CD86 with a k_{off} rate ranging from about 0.001x to about 3x of the k_{off} of abatacept, for example a k_{off} of 0.002x of the k_{off} of abatacept, a k_{off} of 0.003x of the k_{off} of abatacept, a ko_{ff} o f0.004x of the k_{off} of abatacept, a k_{off} of 0.005x of the k_{off} of abatacept, a k_{off} of 0.006x of the k_{off} of abatacept, a k_{off} of 0.0075x of the k_{off} of abatacept, a k_{off} of 0.01x of the k_{off} of abatacept, a k_{off} of 0.025x of the k_{off} of abatacept, a k_{off} of 0.05x of the k_{off} of abatacept, a k_{off} of 0.075x of the k_{off} of abatacept, a k_{off} of 0.1x of the k_{off} of abatacept, a k_{off} of 0.25x of the k_{off} of abatacept, a k_{off} of 0.5x of the k_{off} of abatacept, a k_{off} of 0.75x of the k_{off} of abatacept, a k_{off} of 1x of the k_{off} of abatacept, a k_{off} of 1.25x of the k_{off} of abatacept, a k_{off} of 1.5x of the k_{off} of abatacept, a k_{off} of 1.75x of the k_{off} of abatacept, a k_{off} of 2x of the k_{off} of abatacept, a k_{off} of 2.25x of the k_{off} of abatacept, a k_{off} of 2.5x of the k_{off} of abatacept, a k_{off} of 3x of the k_{off} of abatacept, a k_{off} of 4x of the k_{off} of abatacept, a k_{off} of 5x of the k_{off} of abatacept, or a k_{off} ranging between any pair of the foregoing values, *e.g*., a k_{off} of 0.01x to 1.25x of the k_{off} of abatacept, a k_{off} of 0.05x to 2.5x of the k_{off} of abatacept, or a k_{off} of 0.006x to 0.1 x of the k_{off} of abatacept, *etc.*

In other embodiments, a CTLA4 protein of the disclosure binds to CD80 and/or CD86 with a K_{A} (kₒₙ/k_{off}) ranging from about 0.1x to about 1000x of the K_{A} of abatacept, for example a K_{A} of 0.2x of the K_{A} of abatacept, a K_{A} of 0.25x of the K_{A} of abatacept, a K_{A} of 0.5x of the K_{A} of abatacept, a K_{A} of 0.75 of the K_{A} of abatacept, a K_{A} of 1x of the K_{A} of abatacept, a K_{A} of 2x of the K_{A} of abatacept, a K_{A} of 3x of the K_{A} of abatacept, a K_{A} of 4x of the K_{A} of abatacept, a K_{A} of 5x of the K_{A} of abatacept, a K_{A} of 10x of the K_{A} of abatacept, a K_{A} of 15x ofthe K_{A} of abatacept, a K_{A} of 20x ofthe K_{A} of abatacept, a K_{A} of 30x of the K_{A} of abatacept, a K_{A} of 40x ofthe K_{A} of abatacept, a K_{A} of 50x of the K_{A} of abatacept, a K_{A} of 75x of the K_{A} of abatacept, a K_{A} of 100x of the K_{A} of abatacept, a K_{A} of 200x of the K_{A} of abatacept, a K_{A} of 250x of the K_{A} of abatacept, a K_{A} of 300x of the K_{A} of abatacept, a K_{A} of 350X of the K_{A} of abatacept, a K_{A} of 400x of the K_{A} of abatacept, a K_{A} of 500x of the K_{A} of abatacept, a K_{A} of 750x of the K_{A} of abatacept, a K_{A} of 1000x of the K_{A} of abatacept, or a K_{A} ranging between any pair of the foregoing values, *e.g*., a K_{A} of 0.1x to 100x of the K_{A} of abatacept, a K_{A} of 10x to 50x of the K_{A} of abatacept, or a K_{A} of 5x to 50x of the K_{A} of abatacept, *etc.*

In still other embodiments, a CTLA4 protein of the disclosure binds to CD80 and/or CD86 with a K_{D} (k_{off}/kₒₙ) ranging from about 0.001x to 10x of the K_{D} of abatacept, or example a K_{D} of 0.001x of the K_{D} of abatacept, a K_{D} of 0.002x of the K_{D} of abatacept, a K_{D} of 0.003x of the K_{D} of abatacept, a K_{D} of 0.004x ofthe K_{D} of abatacept, a K_{D} of 0.005x of the K_{D} of abatacept, a K_{D} of 0.0075x of the K_{D} of abatacept, a K_{D} of 0.01x of the K_{D} of abatacept, a K_{D} of 0.025x of the K_{D} of abatacept, a K_{D} of 0.05x of the K_{D} of abatacept, a K_{D} of 0.075x of the K_{D} of abatacept, a K_{D} of 0.1x ofthe K_{D} of abatacept, a K_{D} of 0.2x of the K_{D} of abatacept, a K_{D} of 0.3x of the K_{D} of abatacept, a K_{D} of 0.4x of the K_{D} of abatacept, a K_{D} of 0.5x of the K_{D} of abatacept, a K_{D} of 0.75x of the K_{D} of abatacept, a K_{D} of 1x of the K_{D} of abatacept, a K_{D} o f 1.5x of the K_{D} of abatacept, a K_{D} of 2x of the K_{D} of abatacept, a K_{D} of 3x of the K_{D} of abatacept, a K_{D} of 4x of the K_{D} of abatacept, a K_{D} of 5x of the K_{D} of abatacept, a K_{D} of 6x ofthe K_{D} of abatacept, a K_{D} of 7x of the K_{D} of abatacept, a K_{D} of 8x of the K_{D} of abatacept, a K_{D} of 9x of the K_{D} of abatacept, a K_{D} of 10x of the K_{D} of abatacept, or a K_{D} ranging between any pair of the foregoing values, *e.g*., a K_{D} of 0.001x o 0.5x of the K_{D} of abatacept, a K_{D} of 0.1x to 4x of the K_{D} of abatacept, a K_{D} of 0.05 to 1x of the K_{D} of abatacept, *etc.*

In certain embodiments, a CTLA4 protein ofthe disclosure binds to CD80 and/or CD86 and inhibits cell growth or IL2 production at a IC₅₀ value ranging from about 0.001x to 10x of the IC₅₀ of abatacept, for example at an IC₅₀ of 0.005 of the IC₅₀ of abatacept, at an IC₅₀ of 0.01x of the IC₅₀ of abatacept, at an IC₅₀ of 0.05x ofthe IC₅₀ of abatacept, at an IC₅₀ of 0.1x of the IC₅₀ of abatacept, at an IC₅₀ of 0.2x of the IC₅₀ of abatacept, at an IC₅₀ of 0.3x of the IC₅₀ of abatacept, at an IC₅₀ of 0.4x of the IC₅₀ of abatacept, at an IC₅₀ of 0.5x of the IC₅₀ of abatacept, at an IC₅₀ of 0.6x of the IC₅₀ of abatacept, at an IC₅₀ of 0.7x of the IC₅₀ of abatacept, at an IC₅₀ of 0.8x of the IC₅₀ of abatacept, at an IC₅₀ of 0.9x of the IC₅₀ of abatacept, at an IC₅₀ of 1x of the IC₅₀ of abatacept, at an IC₅₀ of 1.5x of the IC₅₀ of abatacept, at an IC₅₀ of 2x of the IC₅₀ of abatacept, at an IC₅₀ of 3x of the IC₅₀ of abatacept, at an IC₅₀ of 4x of the IC₅₀ of abatacept, at an IC₅₀ of 5x of the IC₅₀ of abatacept, at an IC₅₀ of 6x of the IC₅₀ of abatacept, at an IC₅₀ of 7x of the IC₅₀ of abatacept, at an IC₅₀ of 8x of the IC₅₀ of abatacept, at an IC₅₀ of 9x of the IC₅₀ of abatacept, at an IC₅₀ of 10x of the IC₅₀ of abatacept, at an IC₅₀ of 100x of the IC₅₀ of the IC₅₀ of abatacept, at an IC₅₀ of 500x of the IC₅₀ of abatacept, at an IC₅₀ of 1000x of the IC₅₀ of abatacept, or an IC₅₀ ranging between any pair of the foregoing values, *e.g*., an IC₅₀ of 0.005 to 0.2 of the IC₅₀ of abatacept, an IC₅0 of 0.1x to 1.5x of the IC₅₀ of abatacept, an IC₅₀ of 0.2x to 2x of the IC₅₀ of abatacept, *etc.* In certain embodiments, a single substitution in the CDR-like loops can result in the foregoing differences in IC₅₀ as compared to abatacept, wherein a CTLA4-Ig fusion protein of the disclosure can comprise such substitution and up to 5 additional substitutions, up to 10 additional substitutions, up to 15 additional substitutions or up to 20 additional substitutions in the CDR-like loops as compared to abatacept.

### 6.8. CTLA4 Protein Conjugates

The CTLA4 proteins of the disclosure include conjugates that are modified, *e.g*., by the covalent attachment of any type of molecule to the protein, such that covalent attachment does not interfere with binding to CD80 or CD86.

In certain aspects, a CTLA4 protein of the disclosure can be conjugated to an effector moiety or a label. The term "effector moiety" as used herein includes, for example, antineoplastic agents, drugs, toxins, biologically active proteins, for example enzymes, antibody or antibody fragments, synthetic or naturally occurring polymers, nucleic acids (*e.g*., DNA and RNA), radionuclides, particularly radioiodide, radioisotopes, chelated metals, nanoparticles and reporter groups such as fluorescent compounds or compounds which can be detected by NMR or ESR spectroscopy.

In one example, CTLA4 proteins can be conjugated to an effector moiety, such as a cytotoxic agent, a radionuclide or drug moiety to modify a given biological response. The effector moiety can be a protein or polypeptide, such as, for example and without limitation, a toxin (such as abrin, ricin A, Pseudomonas exotoxin, or Diphtheria toxin), a signaling molecule (such as α-interferon, β-interferon, nerve growth factor, platelet derived growth factor or tissue plasminogen activator), an immunosuppressant (such as cyclosporine A, tacrolimus, Campath-1H or a glucocorticoid) or anti-inflammatory agent (such as aspirin or naproxen) or a biological response modifier such as an antibody, anti-lymphocyte globulin, protein or cytokine (*e.g*., an anti-TNFα antibody or an antibody that affects cell migration, such as natalizumab).

In another example the effector moieties can be cytotoxins or cytotoxic agents. Examples of cytotoxins and cytotoxic agents include taxol, cytochalasin B, gramicidin D, ethidium bromide, emetine, mitomycin, etoposide, tenoposide, vincristine, vinblastine, colchicin, doxorubicin, daunorabicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof.

Effector moieties also include, but are not limited to, antimetabolites (*e.g*., methotrexate, 6-mercaptopurine, 6-thioguanine, cytarabine, 5-fluorouracil decarbazine), alkylating agents (*e.g*., mechlorethamine, thioepa chlorambucil, melphalan, carmustine (BSNU) and lomustine (CCNU), cyclothosphamide, busulfan, dibromomannitol, streptozotocin, mitomycin C5 and cis-dichlorodiamine platinum (II) (DDP) cisplatin), anthracyclines (*e.g*., daunorubicin (formerly daunomycin) and doxorubicin), antibiotics (*e.g*., dactinomycin (formerly actinomycin), bleomycin, mithramycin, anthramycin (AMC), calicheamicins or duocarmycins), and anti-mitotic agents (*e.g*., vincristine and vinblastine).

Other effector moieties can include radionuclides such as, but not limited to, ¹¹¹In and ⁹⁰Y, Lu¹⁷⁷, Bismuth²¹³, Californium²⁵², Iridium¹⁹² and Tungsten^{18s}/Rhenium¹⁸⁸ and drugs such as, but not limited to, alkylphosphocholines, topoisomerase I inhibitors, taxoids and suramin.

Techniques for conjugating such effector moieties to proteins are well known in the art (see, *e.g.,* Hellstrom et al., Controlled Drug Delivery, 2nd Ed., at pp. 623-53 (Robinson et al., eds., 1987)); Thorpe et al., 1982, Immunol. Rev. 62:119-58 and Dubowchik et al., 1999, Pharmacology and Therapeutics 83:67-123).

In certain aspects, a CTLA4 protein is conjugated to a small molecule toxin. In certain exemplary embodiments, a CTLA4 protein of the disclosure is conjugated to a dolastatin or dolostatin peptidic analog or derivative, *e.g*., an auristatin (U.S. Patent Nos. 5,635,483 and 5,780,588). The dolastatin or auristatin drug moiety may be attached to the antibody through its N (amino) terminus, C (carboxyl) terminus or internally (WO 02/088172). Exemplary auristatin embodiments include the N-terminus linked monomethylauristatin drug moieties DE and DF, as disclosed in U.S. Patent No. 7,498,298, which is hereby incorporated by reference in its entirety (disclosing, *e.g*., linkers and methods of preparing monomethylvaline compounds such as MMAE and MMAF conjugated to linkers).

In other exemplary embodiments, small molecule toxins include but are not limited to calicheamicin, maytansine (U.S. Patent No. 5,208,020), trichothene, and CC1065. In one embodiment of the disclosure, the CTLA4 protein is conjugated to one or more maytansine molecules (*e.g*., about 1 to about 10 maytansine molecules per protein molecule). Maytansine may, for example, be converted to May-SS-Me which may be reduced to May-SH3 and reacted with a protein (Chari et al., 1992, Cancer Research 52: 127-131) to generate a maytansinoid-protein or maytansinoid-Fc fusion conjugate. Structural analogues of calicheamicin that can also be used include but are not Research 53:3336-3342; Lode et al., 1998, Cancer Research 58:2925-2928; U.S. Patent No. 5,714,586; U.S. Patent No. 5,712,374; U.S. Patent No. 5,264,586; U.S. Patent No. 5,773,001).

In certain aspects, a CTLA4 protein of the disclosure can be attached to an immunosuppressant agent. Such agents include glucocorticoids, cytostatic agents, antibodies, drugs that act on immunophilins, statins and other agents such as interferons (*e.g*., INF-β and INF-γ), opioids, TNF binding agents (*e.g.,* infliximab, etanercept, adalimumab, curcumin and catechins), mycophenolate, IL-1 receptor antagonists, and other small molecule agents (*e.g*., fingolimod, myriocin). Exemplary glucocorticoids include, but are not limited to, hydrocortisone, prednisone, prednisolone, methylprednisone, dexamethasone, betamethasone, triamcinolone, beclometasone, fludrocortisone acetate, deoxycorticosterone acetate, and aldosterone. Exemplary cytostatic agents include but are not limited to cyclophosphamide, nitrosoureas, platinum compounds, methotrexate, azathioprine, mercaptopurine, pyrimidine analogs, protein synthesis inhibitors, and antibiotics such as dactinomycin, anthracyclines, mitomycin C, bleomycin and mithramycin. Exemplary immunosuppressant antibodies include, but are not limited to, anti-CD20 antibodies, anti-IL2 receptor antibodies (daclizumab, basiliximab), Campath-1H, anti-α₄β₁ integrin antibodies, anti-IL-15 antibodies, anti-IL-6 receptor antibodies, and anti-CD3 antibodies (muromonab). Exemplary agents that act on immunophilins include but are not limited to cyclosporin, tacrolimus, and sirolimus.

In certain embodiments, CTLA4 proteins of the present disclosure can be attached to an anti-inflammatory agent. Exemplary anti-inflammatory agents include, but are not limited to, non-steroidal anti-inflammatories such as ibuprofen, aspirin, naproxen, diflunisal, ketoprofen, nabumetone, piroxicam, diclofenac, indomethacin, sulindac, tolmetin, etodolac, ketorolac, oxaprozin, and celecoxib, and glucocorticoids, which also have immunosuppressive activity.

In one example CTLA4 proteins of the present disclosure can be attached to poly(ethyleneglycol) (PEG) moieties. In one particular example the PEG moieties can be attached through any available amino acid side-chain or terminal amino acid functional group located in the protein, for example any free amino, imino, thiol, hydroxyl or carboxyl group. Such amino acids can occur naturally in the protein or can be engineered into the protein using recombinant DNA methods. See for example U.S. Patent No. 5,219,996. Multiple sites can be used to attach two or more PEG molecules. PEG moieties can be covalently linked through a thiol group of at least one cysteine residue located in the CTLA4 protein. Where a thiol group is used as the point of attachment, appropriately activated effector moieties, for example thiol selective derivatives such as maleimides and cysteine derivatives, can be used. The CTLA4 proteins can be attached to PEG or other hydrophilic synthetic polymers, such as polypropylene glycol or polyoxyalkylene, using techniques well known in the art, such as those described in U.S. Patent Nos. 4,179,337, 4,301,144, 4,496,689, 4,640,835, 4,670,417, and 4,791,192, or to a polymer such as polyvinylalcohol or polyvinylpyrrolidone (PVP).

The word "label" when used herein refers to a detectable compound or composition which can be conjugated directly or indirectly to a CTLA4 protein of the disclosure. The label can itself be detectable (*e.g*., radioisotope labels or fluorescent labels) or, in the case of an enzymatic label, can catalyze chemical alteration of a substrate compound or composition which is detectable. Useful fluorescent moieties include, but are not limited to, fluorescein, fluorescein isothiocyanate, rhodamine, 5-dimethylamine-1-napthalenesulfonyl chloride, phycoerythrin and the like. Useful enzymatic labels include, but are not limited to, alkaline phosphatase, horseradish peroxidase, glucose oxidase and the like.

Additional CTLA4 protein conjugates that are useful for, *inter alia*, diagnostic purposes, are described in Section 5.7 below.

### 6.9. Diagnostic uses of CTLA4 Proteins

The CTLA4 proteins of the disclosure, including those proteins that have been modified, *e.g*., by biotinylation, horseradish peroxidase, or any other detectable moiety (including those described in Section 5.6), can be advantageously used for diagnostic purposes.

In particular, the CTLA4 proteins can be used, for example, but not limited to, to bind to CD80 and/or CD86 for leukocyte typing *in vitro* to define B-cell maturational stages, for detecting B-cell associated diseases, for isolation of CD80-and/or CD86-positive cells, and for *in vitro* and *in vivo* diagnostic methods. (See, *e.g.,* Yokochi et al., 1982, J. Immunol. 128(2):823-27.) For example, the proteins can be used in immunoassays for qualitatively and quantitatively measuring levels of CD80- and/or CD86-positive cells in biological samples. General guidance for performing such diagnostic techniques are found in, for example, Harlow et al., Antibodies: A Laboratory Manual, Second Edition (Cold Spring Harbor Laboratory Press, 1988) and Hampton R et al., Serological Methods A Laboratory Manual, (APS Press, 1990).

In certain embodiments, the CTLA4 proteins of the disclosure can be used in assays to identify other agents, *e.g*., anti-CTLA4 antibodies or fragments thereof, that are capable of regulating T-cell/B-cell interactions. In particular, the assays identify agents capable of inhibiting the binding of a protein of the disclosure to CD80 and/or CD86. Assays useful for screening agents that regulate T-cell/B-cell interactions are known in the art and include, but are not limited to, competition ELISA assays.

The present disclosure further encompasses CTLA4 proteins or fragments thereof conjugated to a diagnostic agent. The proteins can be used diagnostically, for example, to detect expression of a target of interest in specific cells, tissues, or serum; or to monitor the development or progression of an immunologic response as part of a clinical testing procedure to, *e.g*., determine the efficacy of a given treatment regimen. Detection can be facilitated by coupling the protein to a detectable substance. Examples of detectable substances include various enzymes, prosthetic groups, fluorescent materials, luminescent materials, bioluminescent materials, radioactive materials, positron emitting metals using various positron emission tomographies, and nonradioactive paramagnetic metal ions. The detectable substance can be coupled or conjugated either directly to the protein (or fragment thereof) or indirectly, through an intermediate (such as, for example, a linker known in the art) using techniques known in the art. Examples of enzymatic labels include luciferases (*e.g*., firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β- galactosidase, acetylcholinesterase, glucoamylase, lysozyme, saccharide oxidases (*e.g*., glucose oxidase, galactose oxidase, and glucose-6-phosphate dehydrogenase), heterocyclic oxidases (such as uricase and xanthine oxidase), lactoperoxidase, microperoxidase, and the like. Examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include ¹²⁵I, ¹³¹I, ¹¹¹In or ⁹⁹Tc.

The disclosure provides for the detection of expression of CD80 and/or CD86 comprising contacting a biological sample (*e.g*., cells, tissues or bodily fluids) using one or more CTLA4 proteins of the disclosure (optionally conjugated to a detectable moiety), and detecting whether or not the sample is positive for CD80- and/or CD86-expressing cells, or whether the sample has altered (*e.g*., reduced or increased) numbers of cells expressing CD80 and/or CD86 as compared to a control sample.

Diseases that can be diagnosed using the methods described herein include, but are not limited to, the diseases described herein. In certain embodiments, the tissue or body fluid is peripheral blood, peripheral blood leukocytes, biopsy tissues such as breast or lymph node biopsies, and tissue.

### 6.10. Therapeutic Methods Using CTLA4 Proteins

### 6.10.1. Clinical Benefits

The CTLA4 proteins of the disclosure interact with CD80 and/or CD86 on antigen presenting cells and regulate the interaction of T-cells with CD80/CD86-positive cells. Accordingly, the proteins of the disclosure are useful for treating pathological conditions involving dysregulated T-cell interactions with CD80/CD86-positive cells, including antigen presenting cells.

As described above in Section 5.1, in certain embodiments, the variant CTLA4 polypeptides of the disclosure have different binding affinities for CD80 and CD86. In other embodiments, the variant CTLA4 polypeptides of the disclosure have comparable binding affinities for both CD80 and CD86. CD80 and CD86 bind to T-cell surface proteins (e.g., CD28 and CTLA4) and elicit different biological effects. For example, the binding of CD86 to CD28 on naive T-cells initiates T-cell responses. Thus, inhibiting or reducing CD86 binding to CD28, for example, by administering a variant CTLA4 polypeptide of the disclosure with high or neutral binding affinity for CD86, can inhibit the initiation of the T-cell response and lead to T-cell anergy. Furthermore, binding of CD80 to CTLA4 terminates the T-cell response. Therefore, inhibiting or reducing CD80 binding to CTLA4 on T-cells, for example, by administering a variant CTLA4 polypeptide of the disclosure for high or neutral binding affinity for CD80, can block the termination of responses and prolong T-cell responses.

Accordingly, variant CTLA4 polypeptides with high binding affinity for CD80 and with low binding affinity for CD86, or variant CTLA4 polypeptides with neutral binding affinity for CD80 and with low binding affinity for CD86 may be useful for treating conditions requiring enhanced T-cell mediated killing of cells, such as cancer. Variant CTLA4 proteins with high affinity or neutral binding affinity for CD86 and low affinity for CD80, or variant CTLA4 proteins with high binding affinity for both CD80 and CD86 may be useful for treating conditions resulting from enhanced T-cell mediated killing of cells, such as autoimmune diseases, or tissue, organ or cell transplant rejection.

In certain embodiments, the proteins of the disclosure are useful for treating neoplasms. The proteins of the disclosure are useful in the treatment of tumors, including cancers and benign tumors. Examples of cancer to be treated herein include, but are not limited to, melanoma, breast cancer, prostate cancer, bladder cancer and colorectal cancer. More particular examples of such cancers include squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as multiple myeloma, B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome.

More particularly, cancers that are amenable to treatment with the CTLA4 proteins of the disclosure include those that are CD80 and/or CD86 positive. In some embodiments, the variant proteins of the disclosure useful for cancer treatment have high affinity for CD80 and low affinity for CD86. The variant proteins described herein are useful for cancer treatment and have neutral affinity for CD80 and low affinity for CD86.

The cancer treatment methods may include before the step of administering to the patient a CTLA4 protein of the disclosure, a step of detecting CD80 and/or CD86 on cancer cells. Said detecting step can be accomplished by any method known in the art, including, but not limited to, an immunohistochemistry assay to measure CD80 and/or CD86 protein levels on cancer cells. In certain embodiments, immunohistochemistry assays can be performed using a labeled anti-CD80 and/or anti-CD86 antibodies.

In various embodiments, the proteins of the disclosure are useful for treating autoimmune diseases. In some embodiments, the proteins of the disclosure are useful for treating patients with an autoimmune disease who have not been previously treated for the disease. In other embodiments, the proteins of the disclosure are useful for treating patients with an autoimmune disease who had an inadequate response to treatment with methotrexate, a disease-modifying antirheumatic drug, or an anti-TNFα antagonist. In various embodiments, the CTLA4 proteins of the disclosure are useful for treating an autoimmune disease selected from rheumatoid arthritis and juvenile idiopathic arthritis. In some embodiments, the proteins of the disclosure are useful for treating an autoimmune disease selected from ankylosing spondilitis, scleroderma, multiple sclerosis, and systemic lupus erythematosus.

More particularly, the CTLA4 proteins of the disclosure are useful for treating an autoimmune disease selected from acute disseminated encephalomyelitis (ADEM), acute necrotizing hemorrhagic leukoencephalitis, Addison's disease, agammaglobulinemia, allergic asthma, allergic rhinitis, alopecia areata, amyloidosis, ankylosing spondylitis, anti-GBM/anti-TBM nephritis, antiphospholipid syndrome (APS), autoimmune aplastic anemia, autoimmune dysautonomia, autoimmune hepatitis, autoimmune hyperlipidemia, autoimmune immunodeficiency, autoimmune inner ear disease (AIED), autoimmune myocarditis, autoimmune pancreatitis, autoimmune retinopathy, autoimmune thrombocytopenic purpura (ATP), autoimmune thyroid disease, axonal & neuronal neuropathies, Balo disease, Behcet's disease, bullous pemphigoid, cardiomyopathy, Castleman disease, celiac sprue (nontropical), Chagas disease, autoimmune conditions associated with chronic fatigue syndrome or fibromyalgia, chronic inflammatory demyelinating polyneuropathy (CIDP), chronic recurrent multifocal ostomyelitis (CRMO), Churg-Strauss syndrome, icatricial pemphigoid/benign mucosal pemphigoid, Crohn's disease, Cogans syndrome, cold agglutinin disease, congenital heart block, coxsackie myocarditis, CREST disease, essential mixed cryoglobulinemia, demyelinating neuropathies, dermatomyositis, Devic's disease (neuromyelitis optica), discoid lupus, Dressler's syndrome, endometriosis, eosinophilic fasciitis, erythema nodosum, experimental allergic encephalomyelitis, Evans syndrome, fibrosing alveolitis, giant cell arteritis (temporal arteritis), glomerulonephritis, Goodpasture's syndrome, Graves' disease, Guillain-Barre syndrome, Hashimoto's encephalitis, Hashimoto's thyroiditis, hemolytic anemia, Henoch-Schonlein purpura, herpes gestationis, hypogammaglobulinemia, idiopathic thrombocytopenic purpura (ITP), IgA nephropathy, immunoregulatory lipoproteins, inclusion body myositis, insulin-dependent diabetes (type1), interstitial cystitis, juvenile arthritis, juvenile diabetes, Kawasaki syndrome, Lambert-Eaton syndrome, leukocytoclastic vasculitis, lichen planus, lichen sclerosus, ligneous conjunctivitis, linear IgA disease (LAD), lupus (SLE), Lyme disease, Meniere's disease, microscopic polyangiitis, mixed connective tissue disease (MCTD), Mooren's ulcer, Mucha-Habermann disease, multiple sclerosis, myasthenia gravis, myositis, narcolepsy, neuromyelitis optica (see Devic's), neutropenia, ocular cicatricial pemphigoid, optic neuritis, palindromic rheumatism, PANDAS (Pediatric Autoimmune Neuropsychiatric Disorders Associated with Streptococcus), paraneoplastic cerebellar degeneration, paroxysmal nocturnal hemoglobinuria (PNH), Parry Romberg syndrome, Parsonnage-Turner syndrome, pars planitis (peripheral uveitis), pemphigus, peripheral neuropathy, perivenous encephalomyelitis, pernicious anemia, POEMS syndrome, polyarteritis nodosa, type I, II, & III autoimmune polyglandular syndromes, polymyalgia rheumatica, polymyositis, postmyocardial infarction syndrome, postpericardiotomy syndrome, progesterone dermatitis, primary biliary cirrhosis, primary sclerosing cholangitis, psoriasis, psoriatic arthritis, idiopathic pulmonary fibrosis, pyoderma gangrenosum, pure red cell aplasia, Raynauds phenomenon, reflex sympathetic dystrophy, Reiter's syndrome, relapsing polychondritis, restless legs syndrome, retroperitoneal fibrosis, rheumatic fever, rheumatoid arthritis, sarcoidosis, Schmidt syndrome, scleritis, scleroderma, Sjogren's syndrome, sperm & testicular autoimmunity, stiff person syndrome, subacute bacterial endocarditis (SBE), sympathetic ophthalmia, Takayasu's arteritis, temporal arteritis/Giant cell arteritis, thrombocytopenic purpura (TTP), Tolosa-Hunt syndrome, transverse myelitis, ulcerative colitis, undifferentiated connective tissue disease (UCTD), uveitis, vasculitis, vesiculobullous dermatosis, vitiligo, and Wegener's granulomatosis.

In certain embodiments, the variant proteins of the disclosure useful for treating an autoimmune disease have high affinity for CD86 and low affinity for CD80. Also described are variant proteins useful for treating an autoimmune disease that have neutral affinity for CD86 and low affinity for CD80. In other embodiments, the variant proteins of the disclosure have high affinity for both CD86 and CD80.

In various embodiments, the CLTA4 proteins of the disclosure are useful for treating rejection of cell, organ and tissue graft transplants, graft versus host disease and/or for inducing xenograft tolerance, *e.g*., islet xenograft tolerance in patients with type 1 diabetes mellitus. In some embodiments, the variant proteins of the disclosure have high affinity for CD86 and low affinity for CD80. Also described herein are variant proteins with neutral affinity for CD86 and low affinity for CD80. In still other embodiments, the variant proteins of the disclosure have high affinity for both CD86 and CD80.

In some embodiments, the proteins of the disclosure are useful for treating infections caused by the proliferation of viruses that depend on T-cell activation. Such viruses include, but are not limited to, HTLV-1, HIV-1 and HIV2 viruses.

Accordingly, described herein are methods of treating any of the foregoing diseases in a patient in need thereof, comprising: administering to the patient a CTLA4 protein of the disclosure. Optionally, said administration is repeated, *e.g*., after one day, two days, three days, five days, one week, two weeks, three weeks, one month, five weeks, six weeks, seven weeks, eight weeks, two months or three months. The repeated administration can be at the same dose or at a different dose. The administration can be repeated once, twice, three times, four times, five times, six times, seven times, eight times, nine times, ten times, or more. For example, according to certain dosage regimens a patient receives CTLA4 therapy for a prolonged period of time, *e.g.,* 6 months, 1 year or more. Periods of CTLA4 therapy may be alternated with periods of no CTLA4 therapy. The amount of protein administered to the patient may be a therapeutically effective amount. As used herein, a "therapeutically effective" amount of a CTLA4 protein can be administered as a single dose or over the course of a therapeutic regimen, *e.g*., over the course of a week, two weeks, three weeks, one month, three months, six months, one year, or longer. Exemplary therapeutic regimens are described in Section 5.8.4 below.

In certain instances, the CTLA4 therapy may be commenced at or near the onset of disease, such as from 1 day to 6 months after onset of the disease. In some instances, CTLA4 therapy may be commenced after the disease has progressed, such as from 6 months to 1 year, 2 years, or 3 years or more after the onset of disease. In other instances, CTLA4 therapy may be administered during periods of disease remission. In still other instances, CTLA4 therapy is administered during periods of active disease. The timing of administration of CTLA4 therapy will depend upon factors known to the skilled artisan, such as the nature of the disease and the role of the CD28-CD80/CD86 costimulatory pathway in the disease being treated.

According to the present disclosure, treatment of a disease encompasses the treatment of patients already diagnosed as having any form of the disease at any clinical stage or manifestation; the delay of the onset or evolution or aggravation or deterioration of the symptoms or signs of the disease; and/or preventing and/or reducing the severity of the disease.

A "subject" or "patient" to whom the CTLA4 proteins of the disclosure is administered is preferably a mammal such as a non-primate (e.g., cow, pig, horse, cat, dog, rat, *etc.)* or a primate *(e.g.,* monkey or human). The subject or patient may be a human. The human may be an adult patient or a pediatric patient.

### 6.10.2. Pharmaceutical Compositions and Routes of Administration

Compositions comprising a CTLA4 protein of the disclosure and, optionally one or more additional therapeutic agents, such as the combination therapeutic agents described in Section 5.8.3 below, are provided herein. The compositions will usually be supplied as part of a sterile, pharmaceutical composition that will normally include a pharmaceutically acceptable carrier. This composition can be in any suitable form (depending upon the desired method of administering it to a patient).

The CTLA4 proteins of the disclosure can be administered to a patient by a variety of routes such as orally, transdermally, subcutaneously, intranasally, intravenously, intramuscularly, intraocularly, topically, intrathecally and intracerebroventricularly. The most suitable route for administration in any given case will depend on the particular protein, the subject, and the nature and severity of the disease and the physical condition of the subject.

For treatment of indications described herein, the effective dose of CTLA4 protein of the disclosure can range from about 0.1 to about 75 mg/kg per single (*e.g*., bolus) administration, multiple administrations or continuous administration, or to achieve a serum concentration of 0.01-5000 µg/ml serum concentration per single (*e.g*., bolus) administration, multiple administrations or continuous administration, or any effective range or value therein depending on the condition being treated, the route of administration and the age, weight and condition of the subject. In certain embodiments, *e.g.,* for the treatment of rheumatoid arthritis, each dose can range from about 0.2 mg to about 50 mg per kilogram of body weight, for example from about 0.5 mg to about 20 mg per kilogram body weight. The CTLA4 protein can be formulated as an aqueous solution and administered by infusion.

Pharmaceutical compositions can be conveniently presented in unit dose forms containing a predetermined amount of a CTLA4 protein of the disclosure per dose. Such a unit can contain for example but without limitation 0.1 mg to 5 g, for example 1 mg to 1 g, 100 mg to 500 mg, or 10 mg to 50 mg. Pharmaceutically acceptable carriers for use in the disclosure can take a wide variety of forms depending, *e.g*., on the condition to be treated or route of administration.

Therapeutic formulations of the CTLA4 proteins of the disclosure can be prepared for storage as lyophilized formulations or aqueous solutions by mixing the protein having the desired degree of purity with optional pharmaceutically-acceptable carriers, excipients or stabilizers typically employed in the art (all of which are referred to herein as "carriers"), i.e., buffering agents, stabilizing agents, preservatives, isotonifiers, non-ionic detergents, antioxidants, and other miscellaneous additives. See, Remington's Pharmaceutical Sciences, 16th edition (Osol, ed. 1980). Such additives must be nontoxic to the recipients at the dosages and concentrations employed.

Buffering agents help to maintain the pH in the range which approximates physiological conditions. They can be present at concentration ranging from about 2 mM to about 50 mM. Suitable buffering agents for use with the present disclosure include both organic and inorganic acids and salts thereof such as citrate buffers (*e.g*., monosodium citrate-disodium citrate mixture, citric acid-trisodium citrate mixture, citric acid-monosodium citrate mixture, *etc.*), succinate buffers (*e.g.,* succinic acid monosodium succinate mixture, succinic acid-sodium hydroxide mixture, succinic acid disodium succinate mixture, *etc*.), tartrate buffers (*e.g.,* tartaric acid-sodium tartrate mixture, tartaric acid-potassium tartrate mixture, tartaric acid-sodium hydroxide mixture, *etc*.), fumarate buffers (*e.g.,* fumaric acid-monosodium fumarate mixture, fumaric acid disodium fumarate mixture, monosodium fumarate-disodium fumarate mixture, *etc*.), gluconate buffers (*e.g*., gluconic acid-sodium glyconate mixture, gluconic acid-sodium hydroxide mixture, gluconic acid-potassium glyuconate mixture, *etc*.), oxalate buffer (*e.g*., oxalic acid-sodium oxalate mixture, oxalic acid-sodium hydroxide mixture, oxalic acid-potassium oxalate mixture, *etc*.), lactate buffers (*e.g.,* lactic acid-sodium lactate mixture, lactic acid-sodium hydroxide mixture, lactic acid-potassium lactate mixture, *etc*.) and acetate buffers (*e.g.,* acetic acid-sodium acetate mixture, acetic acid-sodium hydroxide mixture, *etc*.). Additionally, phosphate buffers, histidine buffers and trimethylamine salts such as Tris can be used.

Preservatives can be added to retard microbial growth, and can be added in amounts ranging from 0.2%-1 % (w/v). Suitable preservatives for use with the present disclosure include phenol, benzyl alcohol, meta-cresol, methyl paraben, propyl paraben, octadecyldimethylbenzyl ammonium chloride, benzalconium halides (*e.g*., chloride, bromide, and iodide), hexamethonium chloride, and alkyl parabens such as methyl or propyl paraben, catechol, resorcinol, cyclohexanol, and 3-pentanol. Isotonicifiers sometimes known as "stabilizers" can be added to ensure isotonicity of liquid compositions of the present disclosure and include polhydric sugar alcohols, for example trihydric or higher sugar alcohols, such as glycerin, erythritol, arabitol, xylitol, sorbitol and mannitol. Stabilizers refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, *etc.,* organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, α-monothioglycerol and sodium thio sulfate; low molecular weight polypeptides (*e.g*., peptides of 10 residues or fewer); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccacharides such as raffinose; and polysaccharides such as dextran. Stabilizers can be present in the range from 0.1 to 10,000 weights per part of weight active protein.

Non-ionic surfactants or detergents (also known as "wetting agents") can be added to help solubilize the therapeutic agent as well as to protect the therapeutic protein against agitation-induced aggregation, which also permits the formulation to be exposed to shear surface stressed without causing denaturation of the protein. Suitable non-ionic surfactants include polysorbates (20, 80, *etc.*), polyoxamers (184, 188 *etc*.), Pluronic polyols, polyoxyethylene sorbitan monoethers (TWEEN®-20, TWEEN®-80, *etc*.). Nonionic surfactants can be present in a range of about 0.05 mg/ml to about 1.0 mg/ml, for example about 0.07 mg/ml to about 0.2 mg/ml.

Additional miscellaneous excipients include bulking agents (*e.g*., starch), chelating agents (*e.g.,* EDTA), antioxidants (*e.g.,* ascorbic acid, methionine, vitamin E), and cosolvents.

The formulation herein can also contain a combination therapeutic agent in addition to the CTLA4 protein of the disclosure. Examples of suitable combination therapeutic agents are provided in Section 5.8.3 below.

The dosing schedule for administration by infusion can vary from once every six months to daily depending on a number of clinical factors, including the type of disease, severity of disease, and the patient's sensitivity to the CTLA4 protein.

The dosage of a CTLA4 protein of the disclosure to be administered will vary according to the particular antibody, the type of disease, the subject, and the severity of the disease, the physical condition of the subject, the therapeutic regimen (e.g., whether a combination therapeutic agent is used), and the selected route of administration; the appropriate dosage can be readily determined by a person skilled in the art.

It will be recognized by one of skill in the art that the optimal quantity and spacing of individual dosages of a CTLA4 protein of the disclosure will be determined by the nature and extent of the condition being treated, the form, route and site of administration, and the age and condition of the particular subject being treated, and that a physician will ultimately determine appropriate dosages to be used. This dosage can be repeated as often as appropriate. If side effects develop, the amount and/or frequency of the dosage can be altered or reduced, in accordance with normal clinical practice.

### 6.10.3. Combination Therapy

Described below are combinatorial methods in which the CTLA4 proteins of the disclosure can be utilized. The combinatorial methods involve the administration of at least two agents to a patient, the first of which is a CTLA4 protein of the disclosure, and the second of which is a combination therapeutic agent. The CTLA4 protein and the combination therapeutic agent can be administered simultaneously, sequentially or separately.

The combinatorial therapy methods can result in a greater than additive effect, providing therapeutic benefits where neither the CTLA4 protein nor the combination therapeutic agent is administered in an amount that is alone therapeutically effective.

In the methods described herein, the CTLA4 protein of the disclosure and the combination therapeutic agent can be administered concurrently, either simultaneously or successively. As used herein, the CTLA4 protein of the disclosure and the combination therapeutic agent are said to be administered successively if they are administered to the patient on the same day, for example during the same patient visit. Successive administration can occur 1, 2, 3, 4, 5, 6, 7 or 8 hours apart. In contrast, the CTLA4 antibody of the disclosure and the combination therapeutic agent are said to be administered separately if they are administered to the patient on the different days, for example, the CTLA4 protein of the disclosure and the combination therapeutic agent can be administered at a 1-day, 2-day or 3-day, one-week, 2-week or monthly intervals. In the methods described herein, administration of the CTLA4 protein of the disclosure can precede or follow administration of the combination therapeutic agent.

As a non-limiting example, the CTLA4 protein of the disclosure and combination therapeutic agent can be administered concurrently for a period of time, followed by a second period of time in which the administration of the CTLA4 protein of the disclosure and the combination therapeutic agent is alternated.

Because of the potentially synergistic effects of administering a CTLA4 protein of the disclosure and a combination therapeutic agent, such agents can be administered in amounts that, if one or both of the agents is administered alone, is/are not therapeutically effective.

The combination therapeutic agent may be a chemotherapeutic agent, an anti-rheumatic, an anti-inflammatory agent, a radiotherapeutic, an immunosuppressive agent, or a cytotoxic drug.

Anti-rheumatic drugs for use in the combination therapies described herein include, but are not limited to, anakinra, auranofin, azathioprine, chloroquine, D-penicillamine, gold sodium thiomalate, hydroxychloroquine, Myocrisin, sulfasalazine methotrexate, cyclophosphamide, leflunomide, and minocycline.

In certain instances, the anti-rheumatic drug is a TNF-α antagonist including, but not limited to, agents such as infliximab (REMICADE®; Centocor) or a derivative, analog or antigen-binding fragment thereof, adalimumab (HUMIRA®; Abbott) or a derivative, analog or antigen-binding fragment thereof, golimumab (SIMPONI™; Centocor), or a derivative, analog or antigen-binding fragment thereof, certolizumab pegol (CIMZIA®, UCB) or a derivative, analog or antigen-binding fragment thereof, etanercept (ENBREL®, Amgen) or a fragment, derivative or analog thereof, IL-10, thalidomide, xanthine derivatives such as pentoxifylline, and Bupropion (WELLBUTRIN®, GlaxoSmithKline).

For treatment of cancer, a cancer vaccine can be used in combination with a CTLA4 protein of the disclosure. Suitable cancer vaccines include those that are targeted to cancer-causing viruses, such as the hepatitis B vaccine for liver cancer and the human papilloma virus vaccine for cervical cancer (Gardasil® and Cervarix®). Other cancer vaccines that can be used in the combination with the methods of the disclosure include, but are not limited to, Provenge® (Dendreon, Seattle WA) and GVAX® for prostate cancer, OncoVex^{GM-CSF} for melanoma(BioVex, Woburn MA), Lucanix™ for non-small cell lung cancer, Stimuvax® for breast cancer, and Oncophage® for kidney cancer (Antigenics, Lexington MA). In certain instances, a cancer vaccine is administered prior to administration of a CTLA4 polypeptide of the disclosure.

For treatment of cancer, autoimmune diseases, or transplant rejection an anti-inflammatory agent can be used in combination with the CTLA4 proteins of the disclosure. Suitable anti-inflammatory agents for use in the methods described herein include, but are not limited to, ibuprofen, aspirin, naproxen, diflunisal, ketoprofen, nabumetone, piroxicam, diclofenac, indomethacin, sulindac, tolmetin, etodolac, ketorolac, oxaprozin, celecoxib, acetaminophen, diphenhydramine, meperidine, dexamethasone, pentasa, mesalazine, asacol, codeine phosphate, benorylate, and fenbufen.

For treatment of autoimmune diseases or transplant rejection, an immunosuppressant can be used in combination with the CTLA4 proteins of the disclosure. Suitable immunosuppressants for use in the methods described herein include, but are not limited to, hydrocortisone, prednisone, prednisolone, methylprednisone, dexamethasone, betamethasone, triamcinolone, beclometasone, fludrocortisone acetate, deoxycorticosterone acetate, aldosterone, cyclophosphamide, nitrosoureas, platinum compounds, methotrexate, azathioprine, mercaptopurine, pyrimidine analogs, protein synthesis inhibitors, dactinomycin, anthracyclines, mitomycin C, bleomycin, mithramycin, rapamycin, cyclosporin, tacrolimus, sirolimus, mycophenolic acid, mizoribine, 15-deoxyspergualin, mycophenolate mofetil (MMF), anti-thymocyte globulin, an anti-CD20 antibody or derivative, analog or antigen binding fragment thereof, an anti-IL2 receptor antibody (daclizumab, basiliximab) or a derivative, analog or antigen binding fragment thereof, Campath-1H, an anti-α₄β₁ integrin antibody or a derivative, analog or antigen binding fragment thereof, an anti-IL-15 antibody or a derivative, analog or antigen binding fragment thereof, an anti-IL-6 receptor antibody or a derivative, analog or antigen binding fragment thereof, an anti-CD3 antibody(muromonab) or a derivative, analog or antigen binding fragment thereof, an anti-MHC antibody, or a derivative, analog or antigen binding fragment thereof, an anti-CD2 antibody, or a derivative, analog or antigen binding fragment thereof, an anti-CD4 antibody, or a derivative, analog or antigen binding fragment thereof, an anti-CD11a/CD18 antibody, or a derivative, analog or antigen binding fragment thereof, an anti-CD7 antibody, or a derivative, analog or antigen binding fragment thereof, an anti-CD27 antibody, or a derivative, analog or antigen binding fragment thereof, an anti-CD80 and/or anti-CD86 antibody (*e.g*., ATCC HB-253, ATCC CRL-2223, ATCC CRL-2226, ATCC HB-301, ATCC HB-11341, etc), or a derivative, analog or antigen binding fragment thereof, an anti-CD40 antibody (*e.g*., ATCC HB-9110), or a derivative, analog or antigen binding fragment thereof, or a derivative, analog or antigen binding fragment thereof, an anti-CD45 antibody, or a derivative, analog or antigen binding fragment thereof, an anti-CD58 antibody, or a derivative, analog or antigen binding fragment thereof, an anti-CD 137 antibody, or a derivative, analog or antigen binding fragment thereof, an anti-ICOS antibody, or a derivative, analog or antigen binding fragment thereof, an anti-CD 150 antibody, or a derivative, analog or antigen binding fragment thereof, an anti-OX40 antibody, or a derivative, analog or antigen binding fragment thereof, an anti-4-1BB antibody, or a derivative, analog or antigen binding fragment thereof, and low molecular weight adhesion antagonists such as LFA-1 antagonists, selectin antagonists and VLA-4 antagonists.

Other immunomodulatory compounds for use in the combination methods described herein include, but are not limited to, soluble gp39 (also known as CD40 ligand (CD40L), CD154, T-BAM, TRAP), soluble CD29, soluble CD40, soluble CD80 (*e.g.,* ATCC 68627), soluble CD86, soluble CD28 (*e.g*., 68628), soluble CD56, soluble Thy-1, soluble CD3, soluble TCR, soluble VLA-4, soluble VCAM-1, soluble LECAM-1, soluble ELAM-1, soluble CD44, antibodies reactive with gp39 (*e.g*., ATCC HB-10916, ATCC HB-12055 and ATCC HB-12056), antibodies reactive with CD28 (*e.g*., ATCC HB-11944 or mAb 9.3 as described by Martin et al. (J. Clin. Immun. 4(1):18-22, 1980), antibodies reactive with LFA-1 (*e.g*., ATCC HB-9579 and ATCC TIB-213), antibodies reactive with LFA-2, antibodies reactive with IL-12, antibodies reactive with IFN-γ, antibodies reactive with CD48, antibodies reactive with any ICAM (*e.g*., ICAM-1 (ATCC CRL-2252), ICAM-2 and ICAM-3), antibodies reactive with CTLA4 (*e.g*., ATCC HB-304), antibodies reactive with Thy-1, antibodies reactive with CD56, antibodies reactive with CD29, antibodies reactive with TCR, antibodies reactive with VLA-4, antibodies reactive with VCAM-1, antibodies reactive with LECAM-1, antibodies reactive with ELAM-1, antibodies reactive with CD44.

For treatment of cancers, chemotherapeutic agents can suitably be used in combination with the CTLA4 proteins of the disclosure. Chemotherapeutic agents include, but are not limited to, radioactive molecules, toxins, also referred to as cytotoxins or cytotoxic agents, which includes any agent that is detrimental to the viability of cells, agents, and liposomes or other vesicles containing chemotherapeutic compounds. Examples of suitable chemotherapeutic agents include but are not limited to 1- dehydrotestosterone, 5-fluorouracil decarbazine, 6-mercaptopurine, 6-thioguanine, actinomycin D, adriamycin, aldesleukin, an anti-α5β1 integrin antibody, alkylating agents, allopurinol sodium, altretamine, amifostine, anastrozole, anthramycin (AMC)), anti-mitotic agents, cisdichlorodiamine platinum (II) (DDP) cisplatin), diamino dichloro platinum, anthracyclines, antibiotics, antimetabolites, asparaginase, BCG live (intravesical), betamethasone sodium phosphate and betamethasone acetate, bicalutamide, bleomycin sulfate, busulfan, calcium leucouorin, calicheamicin, capecitabine, carboplatin, lomustine (CCNU), carmustine (BSNU), Chlorambucil, Cisplatin, Cladribine, Colchicin, conjugated estrogens, Cyclophosphamide, Cyclothosphamide, Cytarabine, Cytarabine, cytochalasin B, Cytoxan, Dacarbazine, Dactinomycin, dactinomycin (formerly actinomycin), daunirubicin HCL, daunorucbicin citrate, denileukin diftitox, Dexrazoxane, Dibromomannitol, dihydroxy anthracin dione, Docetaxel, dolasetron mesylate, doxorubicin HCL, dronabinol, *E. coli* L-asparaginase, eolociximab, emetine, epoetin-α, *Erwinia* L-asparaginase, esterified estrogens, estradiol, estramustine phosphate sodium, ethidium bromide, ethinyl estradiol, etidronate, etoposide citrororum factor, etoposide phosphate, filgrastim, floxuridine, fluconazole, fludarabine phosphate, fluorouracil, flutamide, folinic acid, gemcitabine HCL, glucocorticoids, goserelin acetate, gramicidin D, granisetron HCL, hydroxyurea, idarubicin HCL, ifosfamide, interferon α-2b, irinotecan HCL, letrozole, leucovorin calcium, leuprolide acetate, levamisole HCL, lidocaine, lomustine, maytansinoid, mechlorethamine HCL, medroxyprogesterone acetate, megestrol acetate, melphalan HCL, mercaptipurine, mesna, methotrexate, methyltestosterone, mithramycin, mitomycin C, mitotane, mitoxantrone, nilutamide, octreotide acetate, ondansetron HCL, paclitaxel, pamidronate disodium, pentostatin, pilocarpine HCL, plimycin, polifeprosan 20 with carmustine implant, porfimer sodium, procaine, procarbazine HCL, propranolol, rituximab, sargramostim, streptozotocin, tamoxifen, taxol, teniposide, tenoposide, testolactone, tetracaine, thioepa chlorambucil, thioguanine, thiotepa, topotecan HCL, toremifene citrate, tretinoin, valrubicin, vinblastine sulfate, vincristine sulfate, and vinorelbine tartrate.

Sometimes, for treatment of cancers it may be beneficial to also administer one or more cytokines to the patient. In a preferred instance, the CTLA4 protein is co-administered with a growth inhibitory agent. Suitable dosages for the growth inhibitory agent are those presently used and may be lowered due to the combined action (synergy) of the growth inhibitory agent and CTLA4 protein.

### 6.10.4. Therapeutic Regimens

Described herein are therapeutic regimens involving the administration of the CTLA4-Ig fusion proteins of the disclosure. The therapeutic regimen will vary depending on the patient's age, weight, and disease condition. The therapeutic regimen can continue for 2 weeks to indefinitely. In specific instances, the therapeutic regimen is continued for 2 weeks to 6 months, from 3 months to 5 years, from 6 months to 1 or 2 years, from 8 months to 18 months, or the like. The therapeutic regimen can be a non-variable dose regimen or a multiple-variable dose regimen.

For the dosage exemplary regimens described below, the CTLA4-Ig fusion proteins can be administered as a sterile, preservative-free solution for intravenous infusion.

For treatment of rheumatoid arthritis in adults weighing less than 60 kg, a CTLA4-Ig fusion protein of the disclosure is administered intravenously at an initial dose of 50 mg to 750 mg. In specific embodiments, the initial dose is 100-600 mg, 250-500 mg, 300-550 mg, 350-500 mg, 50-250 mg, or 400-600 mg. Following the initial dose, a CTLA4-Ig fusion protein of the disclosure is administered intravenously at 2 weeks and 4 weeks after the first infusion and every 4 weeks thereafter at a dose of 50-750 mg, such as 100-600 mg, such as 250-500 mg, such as 300-550 mg, such as 50-250 mg or at a dose of 400-600 mg.

For treatment of rheumatoid arthritis in adults weighing between 60 kg and 100 kg, a CTLA4-Ig fusion protein of the disclosure is administered intravenously at an initial dose of 75 mg to 1000 mg. In specific embodiments, the initial dose is 100-850 mg, 250-800 mg, 300-750 mg, 400-700 mg, 75-500 mg, 500-1000 mg or 600-800 mg. Following the initial dose, a CTLA4-Ig fusion protein of the disclosure is administered intravenously at 2 weeks and 4 weeks after the first infusion and every 4 weeks thereafter at a dose of 75-900 mg, such as 100-850 mg, such as 250-800 mg, such as 300-750 mg, such as 400-700 mg, such as 75-500 mg, such as 500-1000 mg or such as 600-800 mg.

For treatment of rheumatoid arthritis in adults weight more than 100 kg, a CTLA4-Ig fusion protein of the disclosure is administered intravenously at an initial dose of 100 mg to 1500 mg. In specific embodiments, the initial dose is 250-1250 mg, 500-1100 mg, 750-1000 mg, 100-800 mg, 250-900 mg or 700-1200 mg. Following the initial dose, a CTLA4-Ig fusion protein of the disclosure is administered intravenously at 2 weeks and 4 weeks after the first infusion and every 4 weeks thereafter at a dose of 100 mg to 1500 mg, such as 250-1250 mg, such as 500-1100 mg, such as 750-1000 mg, such as 100-800 mg, such as 250-900 mg or such as 700-1200 mg.

For treatment of juvenile idiopathic arthritis in patients between the ages of 6 and 17 who weigh less than 75 kg, a CTLA4-Ig fusion protein of the disclosure is administered intravenously at an initial dose of 0.1 to 15 mg/kg. In specific embodiments, the initial dose is 0.2-12.5 mg/kg, 0.5-12 mg/kg, 1-10.5 mg/kg, 2-10 mg/kg, 3-9 mg/kg, or 4-8.5 mg/kg. Following the initial dose, a CTLA4-Ig fusion protein of the disclosure is administered intravenously at 2 weeks and 4 weeks after the first infusion and every 4 weeks thereafter at a dose of 0.1 to 15 mg/kg, such as 0.2-12.5 mg/kg, 0.5-12 mg/kg, 1-10.5 mg/kg, 2-10 mg/kg, 3-9 mg/kg, or 4-8.5 mg/kg.

For treatment of juvenile idiopathic arthritis in patients aged 6 to 17 years who weigh more than 75 kg, a CTLA4-Ig fusion protein of the disclosure is administered intravenously at an initial dose of 75 mg to 1000 mg. In specific embodiments, the initial dose is 100-850 mg, 250-800 mg, 300-750 mg, 400-700 mg, 75-500 mg, 500-1000 or 600-800 mg. Following the initial dose, a CTLA4-Ig fusion protein of the disclosure is administered intravenously at 2 weeks and 4 weeks after the first infusion and every 4 weeks thereafter at a dose of 75-900 mg, such as 100-850 mg, such as 250-800 mg, such as 300-750 mg, such as 400-700 mg, such as 75-500 mg, such as 500-1000 mg or such as 600-800 mg. In these embodiments, the maximum does should not exceed 1000 mg.

In certain instances, the CTLA4-Ig fusion proteins of the disclosure are used to treat patients with rheumatoid arthritis who have had an inadequate response to methotrexate therapy or to therapy with an anti-TNFα agent (e.g., etanercept (Enbrel®), infliximab (Remicade®), adalimumab (Humira®), certolizumab pegol (Cimzia®), or sTNFR-IgG (lenercept)).

### 6.10.5. Diagnostic and Pharmaceutical Kits

Encompassed by the present disclosure are pharmaceutical kits containing the CTLA4 proteins (including conjugates of CTLA4 proteins) of the disclosure. The pharmaceutical kit is a package comprising the CTLA4 protein of the disclosure (e.g., either in lyophilized form or as an aqueous solution) and one or more of the following:
- A combination therapeutic agent, for example as described in Section 5.8.3 above;
- A device for administering the CTLA4 protein, for example a pen, needle and/or syringe; and
- Pharmaceutical grade water or buffer to resuspend the antibody if the protein is in lyophilized form.

In certain aspects, each unit dose of the CTLA4 protein is packaged separately, and a kit can contain one or more unit doses (e.g., two unit doses, three unit doses, four unit doses, five unit doses, eight unit doses, ten unit doses, or more). In a specific embodiment, the one or more unit doses are each housed in a syringe or pen.

Diagnostic kits containing the CTLA4 proteins (including protein conjugates) of the disclosure are also encompassed herein. The diagnostic kit is a package comprising the CTLA4 protein of the disclosure (e.g., either in lyophilized form or as an aqueous solution) and one or more reagents useful for performing a diagnostic assay. Where the CTLA4 protein is labeled with an enzyme, the kit can include substrates and cofactors required by the enzyme (e.g., a substrate precursor which provides the detectable chromophore or fluorophore). In addition, other additives can be included, such as stabilizers, buffers (e.g., a block buffer or lysis buffer), and the like. In certain embodiments, the CTLA4 protein included in a diagnostic kit is immobilized on a solid surface, or a solid surface (e.g., a slide) on which the protein can be immobilized is included in the kit. The relative amounts of the various reagents can be varied widely to provide for concentrations in solution of the reagents which substantially optimize the sensitivity of the assay. In a specific embodiment, the protein and one or more reagents can be provided (individually or combined) as dry powders, usually lyophilized, including excipients which on dissolution will provide a reagent solution having the appropriate concentration.

### 7. EXAMPLE 1: IDENTIFICATION OF VARIANTS OF CTLA4-IG WITH AFFINITY TO CD80 AND CD86

### 7.1. Materials and Methods

### 7.1.1. Production of CD80 and CD86 proteins

CD80 and CD86 protein were expressed as monomeric secreted fusion proteins. The extracellular domain of either CD80 or CD86 was fused with human lambda constant region containing a Cys to Ser mutation to disrupt the intermolecular disulfide bond (Akamatsu et al., 2007, J. Immunol. Methods 327:40). The CD80-Cλ or CD86-Cλ fusion protein was expressed transiently in the culture supernatant of 293T cells. The production levels of these fusion proteins were estimated by ELISA, using goat anti-human lambda light chain antibody (Biosource, Camarillo, CA) for capturing and horseradish peroxidase (HRP)-conjugated goat anti-human lambda chain antibody (Southern Biotech, Birmingham, AL) for detection.

### 7.1.2. Binding of CTLA4-Ig variants to CD80 and CD86

CD80 or CD86 fused with a Cλ-HA tag was bound to AlphaLISA® acceptor beads conjugated with goat anti-human Cλ antibody and biotinylated abatacept was bound to AlphaScreen® strepavidin-coated donor beads (Perkin Elmer, Waltham, MA). Abatacept was biotinylated with Sulfo-NHS-Biotin using standard methods and dialyzed in PBS. Conjugation of AlphaLISA® acceptor beads was performed by following the manufacturer's instructions ("AlphaLISA® Assay Development Guide", Perkin Elmer). The binding assay was performed in a 384-well AlphaPlate (Perkin Elmer) in assay buffer (1% BSA, 0.01% Tween20 in PBS (pH 7.1)). Each well contained 0.5 nM biotinylated abatacept, 1:4 serially diluted unlabeled CTLA4-Ig variant protein starting from 40nM, 0.078 µg/ml CD80, and 5 µg/ml goat anti-human Cλ-conjugated acceptor beads. The plate was incubated in the dark for 1 h at room temperature. Streptavidin donor beads were subsequently added to each well at 5 µg/ml. The plate was incubated in the dark at room temperature for an additional 30 min, after which it was read on an EnVision reader (Perkin Elmer). Data were fit using nonlinear regression with the software GRAPHPAD PRISM (GraphPad, San Diego). For the CD86 binding assays, 8nM biotinylated abatacept, 1:4 serially diluted unlabeled CTLA4-Ig variant protein starting from 100nM, 0.1 µg/ml CD86 and 10 µg/ml goat anti-human Cλ-conjugated acceptor beads were incubated in the AlphaPlate as described above, followed by addition of 10 µg/ml streptavidin donor beads.

### 7.1.3. Production Of Cell Lines Expressing Cell-Surface CD80 Or CD86

Genes encoding full-length human CD80 and CD86 were subcloned into appropriate mammalian expression vectors. Each plasmid containing cDNA of CD80 or CD86 was transfected into CHO-K1 (ATCC) cells by Lipofectamine 2000 (Invitrogen) and selected for stable transfection in F12K media (45% DMEM, 45% F12K, 10% FBS) containing 0.5 mg/ml G418. The 1% of single clones with the highest expression of CD80 or CD86 on the cell surface were sorted by FACS and cultured to establish stable cell lines. The expression levels between two cell lines were confirmed to be roughly equivalent to each other.

### 7.1.4. Potency Of CTLA4-Ig Variants In The Inhibition Of IL-2 Production Of Jurkat T Cells

Jurkat cells (3.0 x 10⁵/well) and CHO-K1 that stably expressed CD80 or CD86 (1.0 x10⁴/well) were incubated in the presence of 10µg/ml PHA-P with titrating amounts of abatacept or CTLA4 proteins of the disclosure. After 24 hours, supernatants were assayed using an AlphaLISA® Human IL-2 kit to measure the amount of secreted IL-2.

### 7.2. Results

A total of 27 high affinity variants and 4 neutral affinity variants for binding of either CD80 or CD86 were confirmed by AlphaLISA®. The CTLA4-Ig variant having the mutation K28H showed the highest affinity among 19 high affinity CTLA4-Ig variants for CD80, with a binding affinity that was 11.9-fold greater than the binding affinity of wild-type CTLA4-Ig for CD80 (Figure 2A). The CTLA4-Ig variant having the mutation A29H showed the highest affinity among 18 high affinity CTLA4-Ig variants for CD86, with a binding affinity that was 14.2-fold greater than the binding affinity of wild-type CTLA4-Ig for CD86 (Figure 2A).

The mutations A29Y and L104E are those incorporated into belatacept (LEA29, Bristol-Myers Squibb) and were re-identified as high affinity variants in the present analysis (Figure 3). Belatacept showed 22-fold and 32-fold improvement over wild-type CTLA4-Ig in binding to CD80 and CD86, respectively, in the AlphaLISA® assay (data not shown).

In a typical 24 hour assay without blocking agent, 2.1-3.5 ng/ml of IL-2 was produced in the culture supernatant of Jurkat T-cells stimulated by PMA and CHO-K1 cells stably expressing CD80 or CD86. In the presence of blocking agents such as CTLA4-Ig proteins or anti-CD80 or anti-CD86 antibodies, the production of IL-2 was inhibited through blocking of the costimulatory signal. No IL-2 was produced by Jurkat cells in the absence of PHA-P or CHO-K1 negative for CD80 or CD86 expression. Similarly, no IL-2 was detected in the culture supernatant of CHO-K1 cultured in the absence of Jurkat cells (Figure 4A).

Inhibition curves of wild-type CTLA4-Ig and abatacept were found to overlap substantially when Jurkat T-cells were stimulated by PMA and CHO-K1 cells expressing either CD80 or CD86 (Figure 4B). This result indicates that wild-type CTLA4-Ig and abatacept inhibit IL-2 production by Jurkat cells to the same extent (*i.e.,* have similar potency). The average IC₅₀ value of wild-type CTLA4-Ig was determined to be 1.2 nM and 17.2 nM for CD80 and CD86, respectively. Although the two amino acid mutations in belatacept as compared to wild-type CTLA4-Ig resulted in a 22-fold improvement in binding toward CD80, belatacept showed only a 2.0-fold improvement in potency for inhibition of CD80-mediated T-cell activation over wild-type CTLA4-Ig or abatacept. In contrast, the resulting 32-fold improvement in binding of CD86 in belatacept produced an 11.5-fold improvement in potency for inhibition of CD86-mediated T-cell activation.

The 7.2-fold to 11.9-fold improvement in affinity toward CD80 of the CTLA4 variants of disclosure resulted in from 1.1-fold to 2.2-fold improvement in potency for inhibition of CD80-mediated T-cell activation. In contrast, the 7.0-fold to 14.2-fold improvement in affinity toward CD86 of the CTLA4 variants resulted in from 2.4-fold to 6.2-fold improvement in potency for inhibition of CD86-mediated T-cell activation. The K28H and A29H CTLA4-Ig variants respectively showed the highest affinity for CD80 and CD86 as well as the highest potency for inhibition of T-cell activation. Accordingly, a mutation that confers higher affinity binding to a ligand typically results in improved potency in inhibition of T-cell activation. Likewise, a variant with neutral affinity resulted in no significant difference in biological activity (Y52F).

These results indicate that variants having higher binding affinity for CD86 as compared to wild-type protein show better potency as compared to variants having higher binding affinity for CD80, which is similar to the results obtained for belatacept. This can be explained by the avidity effect, which has a greater impact on biological activity when the base-line binding affinity is lower. The binding affinity of wild-type CTLA4-Ig to CD80 is likely to be too high to gain further improvement through avidity. In contrast, wild-type CTLA4-Ig has a 13-fold lower affinity for CD86 than for CD80 (Collins et al, 2002, Immunity 17: 201-210), which means that the affinity of CTLA4-Ig variants for CD86 can be improved. The avidity effect can be seen in the results obtained with the CTLA4-Ig variant having the mutation L96K (Figure 4C). Although this variant showed significant reduction in CD80 binding affinity (10-fold lower as compared to wild-type CTLA4-Ig), the potency of this variant was only 2.4-fold lower than that of wild-type CTLA4-Ig due to the increased impact of avidity on biological activity of this low affinity variant.

### 8. EXAMPLE 2: EVALUATION OF POTENCY OF CTLA4-IG VARIANTS TO INHIBIT T-CELL PROLIFERATION

### 8.1. Materials and Methods: Potency of CTLA4-Ig Variants for Inhibiting T-cell Proliferative Responses In Vitro

The human diffuse large B cell lymphoma cell line SU-DHL-6 (ATCC CRL-2959) expresses both CD80 and CD86 on the cell surface. SU-DHL-6 cells were maintained in RPMI 1640 containing 10% FBS. Peripheral blood mononuclear cells (PBMC) were isolated from human whole blood or buffy coat by Ficoll-Paque Plus (GE Healthcare, Piscataway, NJ) density gradient centrifugation as described in the instruction manual of Leucosep Greiner Bio-One, Germany). Human peripheral CD4⁺ T cells were isolated from PBMC using EasySep Negative Selection Human CD4⁺ T cell Enrichment Kit (STEMCELL Technologies, Canada). T-cells were cultured in RPMI 1640 medium containing 10% heat-inactivated FBS, β-mercaptoethanol and 100U/ml Penicillin-Streptomycin. The resulting culture contained CD4⁺ above 90% in purity.

To set up the assays, varying concentrations of CTLA4-Ig or its variants diluted in 50 µL of media were added to each well of round-bottom 96-well microtiter plates in triplicate. CD4⁺ T cells (1x10⁵) were then mixed with freshly irradiated (10,000 rad) B lymphoma cell line (2 x10⁴) in a 5:1 responder-stimulator ratio in 150 µL of culture media. The cells were added to CTLA4-Ig or variant-containing wells resulting in a final volume of 200 µL per well. The primary allogenic stimulation assays were incubated at 37°C in 5% CO₂ for 5 days. tritiated thymidine at 0.5 µCi/well were added for additional 18hr of culture. Cells were harvested with a cell harvester (Filtermate Omnifilter-96 Harvester, Perkin Elmer) using the manufacturer's recommended conditions and [³H] thymidine incorporation was measured using a scintillation counter (Wallac Trilux). For secondary allogenic stimulation assays, 6-day primary allo-stimulated T cells were harvested, isolated by Ficoll-Paque Plus density gradient centrifugation and rested in complete medium for 24 hr. T cells were then restimulated for 3 days with freshly irradiated B lymphoma cells at the same ratio as above in the presence of CTLA4-Ig and its mutants. The secondary stimulation assays were incubated at 37°C for 2 days before addition of 0.5 µCi/well of [³H]-thymidine for the final 18hr of culture. Cell harvesting was performed as indicated above. Cell proliferation data are analyzed with GraphPad Prism 5 software using a non-linear regression curve fit method.

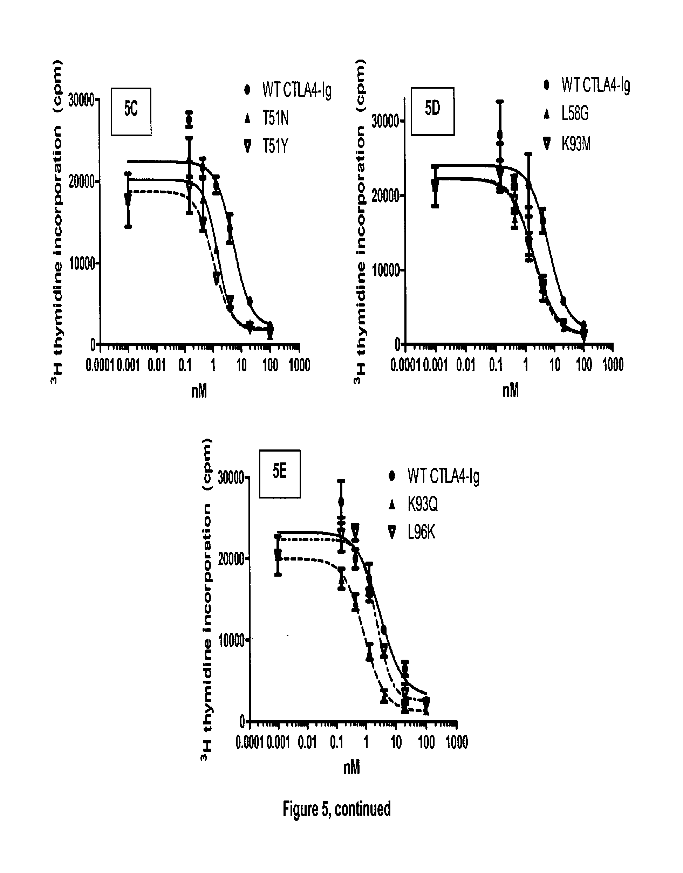

To evaluate the potency of CTLA4-Ig variants for inhibiting alloantigen-stimulated T- cell proliferation, purified CD4⁺ T-cells were stimulated with an allogenic B-cell line, SU-DHL-6, positive for both CD80 and CD86 (confirmed by FACS, data not shown). Inhibition curves of wild-type CTLA4-Ig and commercial abatacept closely overlap each other, indicating equivalent potency in inhibition of proliferative response in CD4⁺ T-cells. All variants with improved binding to CD80 and/or CD86 showed greater inhibition of proliferative responses of CD4⁺ T-cells in both primary and secondary responses. Improvement in IC₅₀ was observed in a range of 1.5 to 20-fold and 1.5 to 10-fold for primary and secondary responses, respectively (Figures 5 and 6). Overall, affinity improvement to CD80 and/or CD86 inhibits primary proliferative responses of CD4⁺ T-cells greater than secondary responses, reflecting the reduced dependency of memory T-cells on costimulatory molecules for secondary responses (Croft et al., 1997, Crit. Rev. Immunol. 17: 89).

Among the eight variants tested, the A29H mutation resulted in the maximum potency for inhibition of both primary and secondary proliferative responses, approximately 20-fold and 10-fold, respectively. The K28H mutation was the second best in potency with approximately 12-fold and 8-fold improvement in inhibiting primary and secondary responses, respectively (Figures 5 and 6 The A29H mutation showed a 14-fold improvement in binding activity toward CD86, but only a 3.0-fold improvement in binding to CD80. On the other hand, the K28H mutant showed more balanced improvement to both ligands, being 12-fold and 9.5-fold for CD80 and CD86, respectively (Figure 2A). Since the A29H mutation resulted in the best improvement in inhibition of both primary and secondary proliferative response, increased affinity for in CD86 may have more impact on potency, assuming that the binding kinetics and protein stability of the A29H and K28H mutations are equivalent.

The L96K mutation resulted in ~11-fold reduction in CD80 binding while improving CD86-binding for ~11-fold. Another example of higher CD86 binding with reduced binding toward CD80 is T51N (10-fold to CD86 and 0.25-fold to CD80). Because both variants did not show reduction in potency in inhibiting primary or secondary responses, loss of binding toward CD80 at least up to 11-fold seems to be well compensated by the improved binding to CD86. Another possible explanation is that, since CD80 is known to form dimers (Ikemuzu et al., 2000, Immunity 12:51), loss of affinity of at least up to 11-fold may have minimal impact on the avidity provided by CD80.

In conclusion, higher affinity to either ligand results in improved potency in inhibition of proliferative response in alloantigenic stimulation, consistent with the results from 24hr assay using Jurkat and CHO cell lines expressing CD80 or CD86 (Figure 4). Both CD80 and CD86 can contribute to primary and secondary allo-responses, however, it is possible to contribute at different levels under the condition where differential levels of ligand expression and other costimulatory/adhesion molecules are involved.

### 9. SPECIFIC EMBODIMENTS, CITATION OF REFERENCES

While various specific embodiments have been illustrated and described, it will be appreciated that various changes can be made without departing from the scope of the invention.

## Claims

1. A variant CTLA4 polypeptide that has greater affinity to CD80 and/or CD86 than the CTLA4 binding domain of amino acids 21-112 of SEQ ID NO:1; said variant CTLA4 polypeptide comprising an amino acid sequence having at least 90%, at least 95%, at least 97%, at least 98% or at least 99% sequence identity to said CTLA4 binding domain and the amino acid substitution K28H as compared to SEQ ID NO:1.

2. The variant CTLA4 polypeptide of claim 1, which comprises one or more of the amino acid substitutions A29H, L58G, K93Q, K93M and L96K as compared to a polypeptide of sequence SEQ ID NO:1.

3. The variant CTLA4 polypeptide of claim 1, which comprises the amino acid substitution T51N or T51Y as compared to a polypeptide of sequence SEQ ID NO:1.

4. The variant CTLA4 polypeptide of claim 1, which comprises the amino acid substitution L61H as compared to a polypeptide of sequence SEQ ID NO:1.

5. The variant CTLA4 polypeptide of claim 1, which comprises the amino acid substitution L104E as compared to a polypeptide of sequence SEQ ID NO:1.

6. The variant CTLA4 polypeptide of any one of claims 1 to 5, which comprises:
(i) up to 10, up to 9, up to 8, up to 7, up to 6, up to 5, up to 4 amino acid, or up to 3 or up to 2 substitutions as compared to amino acids 21 to 112 of SEQ ID NO:1; and/or
(ii) up to 10, up to 9, up to 8, up to 7, up to 6, up to 5, up to 4, up to 3 or up to 2 substitutions as compared to amino acids 1 to 124 of SEQ ID NO:1.

7. The variant CTLA4 polypeptide of any one of claims 1 to 6 which is a fusion protein.

8. The variant CTLA4 polypeptide of claim 7, wherein the fusion protein comprises a CTLA4 extracellular domain linked to an immunoglobulin constant region and optionally a signal sequence.

9. The variant CTLA4 polypeptide of claim 7 or claim 8, which comprises an amino acid sequence having at least 90%, at least 95%, at least 97%, at least 98% or at least 99% sequence identity to abatacept (SEQ ID NO:6).

10. The CTLA4 polypeptide of claim 8 , wherein the CTLA4 extracellular domain and the immunoglobulin constant region are linked by way of a hinge.

11. The CTLA4 polypeptide of any one of claims 8, 9 or 10, wherein the immunoglobulin constant region is selected from an IgG1, an IgG2, and IgG3 or an IgG4, preferably wherein the immunoglobulin constant region is an IgG1.

12. The CTLA4 polypeptide of claim 11, wherein at least one cysteine residue in the immunoglobulin constant region has been substituted with another amino acid to eliminate one or more disulfide bonds formed between two Ig chains, optionally wherein the at least one cysteine residue has been substituted with a serine.

13. A variant CTLA4 polypeptide-drug conjugate comprising a variant CTLA4 polypeptide of any one of claims 1 to 12.

14. A pharmaceutical composition comprising a CTLA4 polypeptide according to any one of claims 1 to 12 or a CTLA4 polypeptide-drug conjugate according to claim 13, and a pharmaceutically acceptable carrier.

15. A CTLA4 polypeptide according to any one of claims 1-12, a CTLA4 polypeptide-drug conjugate according to claim 13, or a pharmaceutical composition according to claim 14 for use in a therapeutically effective amount in treating cancer in a human.

16. A CTLA4 polypeptide according to any one of claims 1-12, a CTLA4 polypeptide-drug conjugate according to claim 13, or a pharmaceutical composition according to claim 14 for use in a therapeutically effective amount in treating an autoimmune disease.

17. The CTLA4 polypeptide, CTLA4 polypeptide-drug conjugate or pharmaceutical composition for use according to claim 16, wherein the autoimmune disease is selected from rheumatoid arthritis and juvenile idiopathic arthritis.

18. A CTLA4 polypeptide according to any one of claims 1-12, a CTLA4 polypeptide-drug conjugate according to claim 13, or a pharmaceutical composition according to claim 14 for use in a therapeutically effective amount for treating or preventing rejection of a cell, organ or tissue graft transplant in a human.

19. The CTLA4 polypeptide, CTLA4 polypeptide-drug conjugate or pharmaceutical composition for use according to any one of claims 15 to 18, wherein, prior to administration, the patient is to be tested for CTLA4 expression in a tissue affected by said cancer, autoimmune disease or rejection, respectively.

## Patentansprüche

1. Variantes CTLA4-Polypeptid, welches eine größere Affinität für CD80 und/oder CD86 besitzt als die CTLA4-Bindungsdomäne der Aminosäuren 21-112 von SEQ ID NO:1, wobei das variante CTLA4-Polypeptid eine Aminosäuresequenz mit mindestens 90%, mindestens 95%, mindestens 97%, mindestens 98% oder mindestens 99% Sequenzidentität zu der CTLA4-Bindungsdomäne, und die Aminosäuresubstitution K28H in Bezug auf SEQ ID NO:1 umfasst.

2. Variantes CTLA4-Polypeptid gemäß Anspruch 1, welches eine oder mehrere der Aminosäuresubstitutionen A29H, L58G, K93Q, K93M und L96K, in Bezug auf ein Polypeptid der Sequenz SEQ ID NO:1, umfasst.

3. Variantes CTLA4-Polypeptid gemäß Anspruch 1, welches die Aminosäuresubstitution T51N oder T51Y, in Bezug auf ein Polypeptid der Sequenz SEQ ID NO:1, umfasst.

4. Variantes CTLA4-Polypeptid gemäß Anspruch 1, welches die Aminosäuresubstitution L61H, in Bezug auf ein Polypeptid der Sequenz SEQ ID NO:1, umfasst.

5. Variantes CTLA4-Polypeptid gemäß Anspruch 1, welches die Aminosäuresubstitution L104E, in Bezug auf ein Polypeptid der Sequenz SEQ ID NO:1, umfasst.

6. Variantes CTLA4-Polypeptid gemäß einem der Ansprüche 1 bis 5, welches umfasst:
(i) bis zu 10, bis zu 9, bis zu 8, bis zu 7, bis zu 6, bis zu 5, bis zu 4 Aminosäure-, oder bis zu 3 oder bis zu 2 Substitutionen, in Bezug auf die Aminosäuren 21 bis 112 von SEQ ID NO:1 und/oder
(ii) bis zu 10, bis zu 9, bis zu 8, bis zu 7, bis zu 6, bis zu 5, bis zu 4, bis zu 3 oder bis zu 2 Substitutionen in Bezug auf die Aminosäuren 1 bis 124 von SEQ ID NO:1.

7. Variantes CTLA4-Polypeptid gemäß einem der Ansprüche 1 bis 6, welches ein Fusionsprotein ist.

8. Variantes CTLA4-Polypeptid gemäß Anspruch 7, wobei das Fusionsprotein eine extrazelluläre CTLA4-Domäne, verknüpft mit einer konstanten Immunglobulinregion und gegebenenfalls eine Signalsequenz umfasst.

9. Variantes CTLA4-Polypeptid gemäß Anspruch 7 oder 8, welches eine Aminosäuresequenz mit mindestens 90%, mindestens 95%, mindestens 97%, mindestens 98% oder mindestens 99% Sequenzidentität mit Abatacept (SEQ ID NO:6) umfasst.

10. CTLA4-Polypeptid gemäß Anspruch 8, wobei die extrazelluläre CTLA4-Domäne und die konstante Immunglobulinregion durch ein Gelenk verknüpft sind.

11. CTLA4-Polypeptid gemäß einem der Ansprüche 8, 9 oder 10, wobei die konstante Immunglobulinregion aus IgG1, IgG2 und IgG3 oder IgG4 ausgewählt ist, wobei die konstante Immunglobulinregion bevorzugt IgG1 ist.

12. CTLA4-Polypeptid gemäß Anspruch 11, wobei mindestens ein Cysteinrest in der konstanten Immunglobulinregion mit einer anderen Aminosäure substituiert worden ist, um ein oder mehrere Disulfidbindungen zu eliminieren, welche zwischen zwei Ig-Ketten ausgebildet sind, wobei der mindestens eine Cysteinrest gegebenenfalls durch ein Serin substituiert worden ist.

13. Variantes CTLA4-Polypeptid-Wirkstoffkonjugat, umfassend ein variantes CTLA4-Polypeptid gemäß einem der Ansprüche 1 bis 12.

14. Pharmazeutische Zusammensetzung, umfassend ein CTLA4-Polypeptid gemäß einem der Ansprüche 1 bis 12, oder ein CTLA4-Polypeptid-Wirkstoffkonjugat gemäß Anspruch 13, und einen pharmazeutisch annehmbaren Träger.

15. CTLA4-Polypeptid gemäß einem der Ansprüche 1 bis 12, CTLA4-Polypeptid-Wirkstoffkonjugat gemäß Anspruch 13 oder pharmazeutische Zusammensetzung gemäß Anspruch 14 zur Verwendung in einer therapeutisch wirksamen Menge bei der Behandlung von Krebs bei einem Menschen.

16. CTLA4-Polypeptid gemäß einem der Ansprüche 1 bis 12, CTLA4-Polypeptid-Wirkstoffkonjugat gemäß Anspruch 13 oder pharmazeutische Zusammensetzung gemäß Anspruch 14 zur Verwendung in einer therapeutisch wirksamen Menge bei der Behandlung einer Autoimmunerkrankung.

17. CTLA4-Polypeptid, CTLA4-Polypeptid-Wirkstoffkonjugat oder pharmazeutische Zusammensetzung zur Verwendung gemäß Anspruch 16, wobei die Autoimmunerkrankung aus rheumatoider Arthritis und juveniler idiopathischer Arthritis ausgewählt ist.

18. CTLA4-Polypeptid gemäß einem der Ansprüche 1 bis 12, CTLA4-Polypeptid-Wirkstoffkonjugat gemäß Anspruch 13 oder pharmazeutische Zusammensetzung gemäß Anspruch 14 zur Verwendung in einer therapeutisch wirksamen Menge zur Behandlung oder Prävention der Abstoßung eines Zell-, Organ- oder Gewebetransplantats bei einem Menschen.

19. CTLA4-Polypeptid, CTLA4-Polypeptid-Wirkstoffkonjugat oder pharmazeutische Zusammensetzung zur Verwendung gemäß einem der Ansprüche 15 bis 18, wobei der Patient vor der Verabreichung auf CTLA4-Expression in einem Gewebe zu testen ist, welches von dem Krebs, der Autoimmunerkrankung bzw. der Abstoßung betroffen ist.

## Revendications

1. Polypeptide, variante de CTLA4, qui possède une plus grande affinité pour CD80 et/ou CD86 que le domaine de liaison de CTLA4 des acides aminés n° 21 à 112 de la Séquence N° 1, lequel polypeptide variante de CTLA4 comprend une séquence d'acides aminés identique, à un degré d'au moins 90 %, d'au moins 95 %, d'au moins 97 %, d'au moins 98 % ou d'au moins 99 %, à celle dudit domaine de liaison de CTLA4, et comporte, par rapport à la Séquence N° 1, la substitution d'acide aminé K28H.

2. Polypeptide variante de CTLA4, conforme à la revendication 1, qui comporte, par rapport à un polypeptide de Séquence N° 1, l'une ou plusieurs des substitutions d'acide aminé A29H, L58G, K93Q, K93M et L96K.

3. Polypeptide variante de CTLA4, conforme à la revendication 1, qui comporte, par rapport à un polypeptide de Séquence N° 1, la substitution d'acide aminé T51N ou T51Y.

4. Polypeptide variante de CTLA4, conforme à la revendication 1, qui comporte, par rapport à un polypeptide de Séquence N° 1, la substitution d'acide aminé L61H.

5. Polypeptide variante de CTLA4, conforme à la revendication 1, qui comporte, par rapport à un polypeptide de Séquence N° 1, la substitution d'acide aminé L104E.

6. Polypeptide variante de CTLA4, conforme à l'une des revendications 1 à 5, qui comporte :
i) jusqu'à 10, jusqu'à 9, jusqu'à 8, jusqu'à 7, jusqu'à 6, jusqu'à 5, jusqu'à 4, jusqu'à 3 ou jusqu'à 2 substitutions, par rapport aux acides aminés n° 21 à 112 de la Séquence N° 1,
ii) et/ou jusqu'à 10, jusqu'à 9, jusqu'à 8, jusqu'à 7, jusqu'à 6, jusqu'à 5, jusqu'à 4, jusqu'à 3 ou jusqu'à 2 substitutions, par rapport aux acides aminés n° 1 à 124 de la Séquence N° 1.

7. Polypeptide variante de CTLA4, conforme à l'une des revendications 1 à 6, qui est une protéine de fusion.

8. Polypeptide variante de CTLA4, conforme à la revendication 7, dans lequel la protéine de fusion comprend un domaine extracellulaire de CTLA4 raccordé à une région constante d'immunoglobuline et, en option, une séquence signal.

9. Polypeptide variante de CTLA4, conforme à la revendication 7 ou 8, qui comprend une séquence d'acides aminés identique, à un degré d'au moins 90 %, d'au moins 95 %, d'au moins 97 %, d'au moins 98 % ou d'au moins 99 %, à celle de l'abatacept (Séquence N° 6).

10. Polypeptide CTLA4, conforme à la revendication 8, dans lequel le domaine extracellulaire de CTLA4 et la région constante d'immunoglobuline sont raccordés par une charnière.

11. Polypeptide CTLA4, conforme à l'une des revendications 8, 9 et 10, dans lequel la région constante d'immunoglobuline est choisie parmi celles provenant d'une IgG1, d'une IgG2, d'une IgG3 ou d'une IgG4, et dans lequel, de préférence, la région constante d'immunoglobuline provient d'une IgG1.

12. Polypeptide CTLA4, conforme à la revendication 11, dans lequel on a remplacé au moins un résidu de cystéine, dans la région constante d'immunoglobuline, par un autre acide aminé afin de détruire un ou plusieurs ponts disulfure entre deux chaînes Ig, étant entendu que, en option, le résidu de cystéine au nombre d'au moins un a été remplacé par un résidu de sérine.

13. Conjugué de polypeptide variante de CTLA4 et de médicament, comprenant un polypeptide variante de CTLA4 conforme à l'une des revendications 1 à 12.

14. Composition pharmaceutique comprenant un polypeptide CTLA4 conforme à l'une des revendications 1 à 12, ou un conjugué de polypeptide CTLA4 et de médicament, conforme à la revendication 13, et un véhicule pharmacologiquement admissible.

15. Polypeptide CTLA4 conforme à l'une des revendications 1 à 12, conjugué de polypeptide CTLA4 et de médicament, conforme à la revendication 13, ou composition pharmaceutique conforme à la revendication 14, pour utilisation, en une quantité à effet thérapeutique, dans le traitement d'un cancer chez un humain.

16. Polypeptide CTLA4 conforme à l'une des revendications 1 à 12, conjugué de polypeptide CTLA4 et de médicament, conforme à la revendication 13, ou composition pharmaceutique conforme à la revendication 14, pour utilisation, en une quantité à effet thérapeutique, dans le traitement d'une maladie auto-immune.

17. Polypeptide CTLA4, conjugué de polypeptide CTLA4 et de médicament, ou composition pharmaceutique pour utilisation conforme à la revendication 16, étant entendu que la maladie auto-immune est choisie parmi la polyarthrite rhumatoïde et l'arthrite juvénile idiopathique.

18. Polypeptide CTLA4 conforme à l'une des revendications 1 à 12, conjugué de polypeptide CTLA4 et de médicament, conforme à la revendication 13, ou composition pharmaceutique conforme à la revendication 14, pour utilisation, en une quantité à effet thérapeutique, dans le but de traiter ou de prévenir, chez un humain, le rejet d'un greffon, cellules, organe ou tissu transplantés.

19. Polypeptide CTLA4, conjugué de polypeptide CTLA4 et de médicament, ou composition pharmaceutique pour utilisation conforme à l'une des revendications 15 à 18, étant entendu que, avant l'administration, le patient doit subir un test ayant pour but de déterminer l'expression de CTLA4 dans un tissu affecté par ledit cancer, ladite maladie auto-immune ou ledit rejet, respectivement.
